(19) Europäisches Patentamt
European Patent Office
Office européen des brevets

(11) EP 3 326 642 B1

(12) EUROPEAN PATENT SPECIFICATION

(45) Date of publication and mention of the grant of the patent:
**25.11.2020 Bulletin 2020/48**

(21) Application number: **18150731.0**

(22) Date of filing: **05.02.2013**

(51) Int Cl.:
***A61K 38/36*** *(2006.01)* ***A61K 38/24*** *(2006.01)*
***C07K 14/475*** *(2006.01)* ***A61P 7/04*** *(2006.01)*
***C07K 14/755*** *(2006.01)*

# (54) LONG-ACTING COAGULATION FACTORS AND USES THEREOF

GERINNUNGSFAKTOREN MIT LANGZEITWIRKUNG UND VERWENDUNGEN DAVON

FACTEURS DE COAGULATION A ACTION PROLONGEE ET LEURS UTILIZATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.02.2012 US 201213372540**

(43) Date of publication of application:
**30.05.2018 Bulletin 2018/22**

(62) Document number(s) of the earlier application(s) in accordance with Art. 76 EPC:
**13749077.7 / 2 822 576**

(73) Proprietor: **OPKO Biologics Ltd.**
**Kiryat Gat, 8211804 (IL)**

(72) Inventors:
- **FIMA, Udi Eyal**
  **52330 Dvira (IL)**
- **HART, Gili**
  **6081809 Shoham (IL)**

(74) Representative: **Pearl Cohen Zedek Latzer Baratz UK LLP**
**The Gridiron Building**
**One Pancras Square**
**London N1C 4AG (GB)**

(56) References cited:
**WO-A2-2014/080401**
**US-A1- 2010 317 585**
**US-A1- 2012 208 759**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 326 642 B1

## Description

## FIELD OF INVENTION

The present invention relates to medical uses of polypeptides comprising three carboxy-terminal peptides (CTPs) of chorionic gonadotrophin attached to the carboxy terminus of a coagulation factor.

## BACKGROUND OF THE INVENTION

The development of coagulation factor replacement therapy has transformed the lives of many individuals with hemophilia. Hemophilia is a group of hereditary genetic disorders that impair the body's ability to control blood clotting or coagulation. Patients with hemophilia do not produce adequate amounts of Factor VIII or Factor IX proteins, which are necessary for effective blood clotting. In severe hemophiliacs even a minor injury can result in blood loss that continues for days or weeks, and complete healing may not occur, leading to the potential for debilitating permanent damage to joints and other organs, and premature death.

One type of hemophilia, Hemophilia B, is an X-linked bleeding disorder caused by a mutation in the Factor IX (FIX) gene, resulting in a deficiency of the procoagulant activity of FIX. Hemophilia B patients have spontaneous soft tissue hemorrhages and recurrent hemarthroses that often lead to a crippling arthopathy. Current treatment for these patients includes an intravenous administration of recombinant FIX. However issues of cost and relatively rapid clearance of FIX from the circulation make developing a long-acting FIX a challenging task.

Commercial availability of FVIII and FIX has lead to improved control of life-threatening bleedings episodes. Many patients receive prophylactic therapy, which reduces the risk of bleeding and its associated complications. However, a significant proportion of patients (10-30%) develop inhibitory antibodies to exogenously administered FVIII and FIX. Administration of FVIIa, which is a bypassing product, can induce homeostasis and provide an effective treatment for patients with inhibitory Abs.

Recombinant FVIIa (NovoSeven®) is commercially available and was approved in 1996 for treatment of bleeding episodes in hemophilia patients with inhibitors. However, rFVIIa is rapidly cleared with a terminal half-life of 2.5 hours. As a result, patients generally require multiple, frequent infusions (2-3 doses given in 2-3 hour intervals) to achieve adequate homeostasis following a mild to moderate bleed. Consequently, there is much interest in developing a long-acting form of FVIIa that would prolong the duration of haemostatic activity following a single dose and allow much less frequent dosing. A long-acting FVIIa would also increase the feasibility of long-term prophylactic therapy.

Various technologies are being developed for prolonging the half-life of FVIIa. However, the challenge is to achieve a prolonged half-life of this protein while preserving its biological activity and ensuring that the modifications do not induce significant immunogenicity.

## SUMMARY OF THE INVENTION

The invention provides a chorionic gonadotropin carboxy terminal peptide (CTP)-modified coagulation factor consisting of a coagulation factor and three CTPs attached to the carboxy terminus of said coagulation factor, wherein said CTP-modified coagulation factor is:

a CTP-modified Factor IX (FIX) polypeptide consisting of the amino acid sequence of SEQ ID NO: 31 or of amino acids 47-545 of SEQ ID NO: 31;
a CTP-modified Factor VII (FVII) polypeptide consisting of the amino acid sequence of SEQ ID NO: 25 or of amino acids 39-528 of SEQ ID NO: 25; or
a CTP-modified activated Factor VIIa (FVIIa) consisting of the amino acid sequence of amino acids 39-528 of SEQ ID NO: 25,

for use in reducing excessive bleeding or bruising in a subject.

The invention also provides a chorionic gonadotropin carboxy terminal peptide (CTP)-modified coagulation factor consisting of a coagulation factor and three CTPs attached to the carboxy terminus of said coagulation factor, wherein said CTP-modified coagulation factor is:

a CTP-modified Factor IX (FIX) polypeptide consisting of the amino acid sequence of SEQ ID NO: 31 or of amino acids 47-545 of SEQ ID NO: 31;
a CTP-modified Factor VII (FVII) polypeptide consisting of the amino acid sequence of SEQ ID NO: 25 or of amino acids 39-528 of SEQ ID NO: 25; or
a CTP-modified activated Factor VIIa (FVIIa) consisting of the amino acid sequence of amino acids 39-528 of SEQ ID NO: 25,

for use in maintaining hemostasis during surgery or during surgical interventions in a subject.

In one embodiment, at least one of the attached CTPs is glycosylated.

In an embodiment, the CTP-modified coagulation factor is a CTP-modified FIX polypeptide consisting of the amino acid sequence of SEQ ID NO: 31 or of amino acids 47-545 of SEQ ID NO: 31.

In another embodiment, the CTP-modified coagulation factor is a CTP-modified FVII polypeptide consisting of the amino acid sequence of SEQ ID NO: 25 or of amino acids 39-528 of SEQ ID NO: 25.

In another embodiment, the CTP-modified coagulation factor is a CTP-modified FVIIa polypeptide consisting of the amino acid sequence of amino acids 39-528 of SEQ ID NO: 25.

In an embodiment, the CTP-modified coagulation factor is administered to the subject in the form of a pharmaceutical composition comprising a pharmaceutically acceptable carrier.

In another embodiment, the CTP-modified coagulation factor is administered by subcutaneous administration.

In another embodiment, the CTP-modified coagulation factor is administered by intravenous administration.

In another embodiment, the subject is a hemophiliac subject, or is a subject suffering from a vitamin K deficiency or a liver disease.

In another embodiment of the invention, the subject is a child.

Other features and advantages of the present invention will become apparent from the following detailed description examples and figures. It should be understood, however, that the detailed description and the specific examples while indicating preferred embodiments of the invention are given by way of illustration only, since various changes and modifications within the scope of the invention will become apparent to those skilled in the art from this detailed description.

## BRIEF DESCRIPTION OF THE DRAWINGS

**Figure 1A.** Shows a bar graph showing harvests limited, diluted, transfected, and selected cells with FIX-CTP and FIX-CTP-CTP variants in the presence of 5μg/ml of Vitamin K3. The level of FIX was quantified using Human FIX ELISA kit (Affinity Biologicals; Cat. No. FIX-AG RUO), and the calculated protein concentration (μg/ml) is the average of two independent runs. Figure 1B shows SDS-PAGE gel micrographs of FIX Ab recognition Micrograph A depicts recognition of anti-FIX antibody in Western-blot; Micrograph B depicts recognition of anti-γ carboxylation antibody in Western-blot. Lane 1 in A-B was loaded with a sample containing recombinant FIX Lane 2 in A-B was loaded with a sample containing FIX-CTP harvests. Lane 3 in A-B was loaded with a sample containing FIX-$(CTP)_2$ harvest.

**Figure 2.** Shows a graph showing FIX-CTP and FIX-$(CTP)_2$ harvests comparative chromogenic activity (measured by a the $EC_{50}$. concentration) compared to rhFIX (American Diagnostics).

**Figure 3.** Shows a graph showing PK profile of rhFIX, harvest of FIX-CTP-CTP, and harvest of FIX-CTP.

**Figure 4.** Shows a bar graph showing harvests of FIX-CTP and FIX-CTP-CTP harvests and FIX-CTP-CTP purified protein FIX antigen level as determined using Human FIX ELISA kit (Affinity Biologicals; cat. No. FIX-AG RUO). The calculated protein concentration (μg/ml) is the average of two independent runs.

**Figure 5.** Shows SDS-PAGE gel micrographs of FIX Ab recognition. Micrograph A depicts a coomassie blue staining; Micrograph B depicts recognition of anti-FIX antibody in Western-blot; Micrograph C depicts recognition of anti-γ carboxylation antibody in Western-blot. Lane 1 in A-C was loaded with a sample containing FIX-$(CTP)_2$. Lane 2 in A-C was loaded with a sample containing unbound FIX-$(CTP)_2$. Lane 3 in A-C was loaded with a sample containing a concentrated elution of FIX-$(CTP)_2$.

**Figure 6.** Shows a graph showing FIX-$(CTP)_2$ chromogenic activity (sample concentration/O.D.) compared to human normal pool plasma and rhFIX (American Diagnostics).

**Figure 7.** Shows a graph showing the PK profile of purified FIX-CTP-CTP, rhFIX, harvest of FIX-CTP-CTP, and harvest of FIX-CTP.

**Figure 8.** Shows an anti-CTP and anti-gamma carboxylation antibodies Western blots of FIX fused to three, four or five CTPs. FIX-$CTP_3$, FIX-$CTP_4$, and FIX-$CTP_5$ harvests were loaded on 12% Tris-Glycine gel using Precision plus dual color protein marker (Bio-Rad). The SDS-PAGE analysis was performed by Western immuno-blot using anti-CTP polyclonal Ab (Adar Biotech Production) or anti-Gla Ab (American Diagnostica).

**Figure 9.** Shows a coomassie blue detection of FIX-$CTP_3$, FIX-$CTP_4$, and FIX-$CTP_5$. After a purification process utilizing Jacalin column (immunoaffinity purification of glycosylated proteins), FIX-$CTP_3$, FIX-$CTP_4$, and FIX-$CTP_5$ were loaded on 12% Tris-Glycine gel using Precision Plus Dual Color Protein Marker (Bio-Rad). The SDS-PAGE was stained by Coomassie blue dye for sample detection.

**Figure 10.** Shows FIX Chromogenic activity. A comparative assessment of the *in vitro* potency of fully purified (HA column) FIX-$CTP_3$ FIX-$CTP_4$ and FIX-$CTP_5$ versus human pool normal plasma was performed using a commercially available chromogenic activity test kit, BIOPHEN (Hyphen BioMed 221802). All samples were serially diluted and the potency was assessed by comparing a dose response curve to a reference preparation consisting of normal human plasma.

**Figure 11.** Shows the comparative pharacokinetic (PK) profile of FIX-$CTP_3$ FIX-$CTP_4$ and FIX-$CTP_5$. FIX concentration in plasma samples were quantified using human FIX Elisa kits (Affinity Biologicals). Pharmacokinetic profile was calculated and is the mean of 3 animals at each time point. Terminal half lives were calculated using PK Solutions 2.0 software.

**Figure 12.** Shows the FIX-$CTP_3$ SDS-PAGE analysis - Coomassie SDS-PAGE. FIX-$CTP_3$ γ-carboxylated enriched protein, rhFIX and rFIXa (activated FIX) were loaded on 12% Tris-Glycine gel using Precision Plus Dual Color Protein Marker (Bio-Rad). The SDS-PAGE Coomassie analysis was performed by staining the gel with Commasie blue reagent (800 ng of protein) (Figure 12A). A Western immunoblot was performed using 100 ng of protein with anti-human FIX polyclonal Ab (Figure 12B), anti-human gamma carboxylation monoclonal antibody (American Diagnostics Cat #499, 3570) (Figure 12C), anti-FIX propeptide polyclonal Ab (Figure 12D), and anti-CTP polyclonal Ab (Figure 12E).

**Figure 13:** Shows the FIX-$CTP_3$ chromogenic activity. A comparative assessment of the *in vitro* potency of FIX-$CTP_3$ harvest and FIX-$CTP_3$ γ-carboxylated enriched protein, versus human pool normal plasma was performed using a commercially available chromogenic activity test kit, BIOPHEN (Hyphen BioMed 221802). FIX-$CTP_3$ harvest and protein were serially diluted, and the potency was assessed by comparing a dose-response curve to a reference preparation consisting of normal human plasma.

**Figure 14:** Shows the comparative clotting time. An *in vitro* aPTT (activated Partial Thrombin Time Assay) was performed comparing the clotting activity of FIX-$CTP_3$ to BeneFIX. The proteins were serially diluted and spiked into human FIX-depleted plasma, and the clotting time was evaluated.

**Figure 15.** Shows FIX-$CTP_3$ comparative PK profile. FIX concentration was quantitated using human FIX ELISA kits (Affinity Biologicals; Cat. # FIX-AG RUO). The pharmacokinetic profile was calculated for each protein and is the mean of 3 animals at each time point.

**Figure 16.** Shows the activity profile parameters. In parallel to PK sampling, FIX-deficient animals administered with either BeneFIX® or FIX-$CTP_3$, citrated plasma samples, were evaluated for their clotting activity by aPTT assay, which was translated to % activity. The % activity at each collection point was calculated as the current clotting time/clotting time of normal pool mice plasma*100.

**Figure 17.** Shows a first challenge bleeding parameters. FIX-deficient mice were administered a single intravenous injection of 100 IU/Kg of BeneFIX® or rFIX-$CTP_3$. The tail vein was slightly clipped 48 hours post-dosing and tail vein bleeding time (TVBT) and bleeding intensity (hemoglobin OD) were evaluated. A second bleeding challenge was performed 15 minutes after reaching homeostasis, and the same parameters were measured.

**Figure 18.** Shows a second challenge bleeding parameters. Once the first bleeding described in the legend to Figure 19 was spontaneously or manually stopped, a second bleeding challenge was performed 15 minutes following the first one, and the time and bleeding intensity were re-measured.

**Figure 19.** Shows a diagram illustrating the rFVII-CTP construct (A), rFVII-CTP-CTP construct (B), rFIX-CTP construct (C), and rFIX-CTP-CTP construct (D).

**Figure 20A.** Shows a bar graph showing harvests limited diluted clone transfected and selected cells with FVII-CTP variants in the presence of 5μg/ml of Vitamin K3. The level of FVII was quantified using FVII ELISA (AssayPro).

**Figure 20B.** Shows a bar graph showing harvests of limited diluted transfected and selected cells with FVII-CTP variants in the presence of 5μg of Vitamin K3.activity. FVII activity was quantified using FVII chromogenic activity assay (AssayPro).

**Figure 20C.** Shows a bar graph showing harvests of limited diluted transfected and selected cells with FVII-CTP variants in the presence of 5μg of Vitamin K3. The specific activity of FVII was calculated for each version by dividing the activity value by the harvest FVII concentration.

**Figure 20D.** Shows a graph showing PK profile of FVII, FVII-CTP-CTP, and FVII-CTP harvests.

**Figure 21.** Shows western blots of FVII fused to three, four and five CTPs, detected using anti-FVII, anti-CTP, and anti-gamma carboxylation antibodies. FVII-$CTP_3$, FVII-$CTP_4$, and FVII-$CTP_5$ harvests were loaded on 12% Tris -Glycine gel (*expedeon*) using Precision plus dual color protein marker (Bio-Rad). The SDS-PAGE analysis was performed by Western immunoblot using anti-FVII Ab, anti-CTP polyclonal Ab (Adar Biotech Production) or anti-Gla Ab (American Diagnostica).

**Figure 22.** Shows the FVII Activity - Chromogenic activity. A comparative assessment of the *in vitro* potency of HA purified (highly gamma carboxylated fraction) FVII-$CTP_3$, FVII-$CTP_4$, and FVII-$CTP_5$ versus normal human pool plasma was performed using a commercially available chromogenic activity test kit, BIOPHEN (Hyphen BioMed 221304). All samples were serially diluted and the potency was assessed by comparing a dose response curve to a reference preparation consisting of normal human plasma.

**Figure 23.** Shows a first comparative pharmacokinetic (PK) profile-FVII 3, 4 and 5 CTPs. FVII-$CTP_3$, FVII-$CTP_4$,

and FVII-$CTP_5$ (Group A, B and C, respectively) were administered in a single intravenous injection to Sprague Dawley rats (six rats per treatment) in a dose of 250 μg/kg body weight. Blood samples were drawn retro-orbitally from 3 rats alternately at 0.083, 0.5 2, 5, 8, 24, 48, 72 and 96 hours post dosing. Citrated plasma (0.38%) was prepared immediately after sampling and stored at -20°C until analysis. FVII-$CTP_5$ demonstrated a superior profile as compared to the two other versions.

**Figure 24.** Shows a second comparative PK profile-FVII 3, 4 and 5 CTPs. FVII-$CTP_3$, FVII-$CTP_4$, and FVII-$CTP_5$ following FVII selection and the HA purification process (Group A, B and C, respectively) were administered in a single intravenous injection to Sprague Dawley rats (three rats per substance) in a dose of 29.45 μg/kg body weight. Blood samples were drawn retro-orbital at 0.083, 0.5 2, 8, 24, 48, and 72 hours post-dosing. Citrated plasma (0.38%) was prepared immediately after sampling and stored at -20°C until analysis.

**Figure 25.** Shows a schematic diagram of FVII-$CTP_3$ purification process. Batch 31 was produced for the PK/PD study. Batch 38 was produced for the survival study.

**Figure 26.** Shows an SDS -PAGE and Western blot of Final FVII and FVIIa. 10 μg (Batch 31) or 5 μg (Batch 38) were loaded in each lane of Coomassie stained SDS-PAGE. 1 μg protein was loaded in each lane of Western blot. 1. FVII-$CTP_3$ polypeptide; 2. Heavy chain, including 3x CTP; 3. Light Chain. All three antibodies detect FVII. FVIIa heavy chain was detected by α-CTP, and light chain is detected with both α-FVII and α-Gla.

**Figure 27.** Shows that FVII-$CTP_3$ chromogenic activity is enhanced as a result of purification on ceramic hydroxyapatite (HA) column. A comparative assessment of the *in vitro* potency of FVII-$CTP_3$ harvest, in-process fractions, and purified FVII-$CTP_3$ versus human pool normal plasma was performed using a commercially available chromogenic activity test kit, BIOPHEN (Hyphen BioMed 221304). FVII-$CTP_3$ harvest and protein were serially diluted and the potency was assessed by comparing a dose-response curve to a reference preparation of normal human plasma.

**Figure 28.** Shows the PK profile of FVIIa-$CTP_3$ vs. NovoSeven® in FVIII-deficient mice. FVIIa-$CTP_3$ was produced following **FVII** selection, HA purification process and activation. FVIIa-$CTP_3$ or NovoSeven® was administered in a single intravenous injection to FVIII-/- hemophilic mice. Blood samples were drawn retro-orbitally at 0.083, 0.5 2, 8, 24, 48, and 72 hours post-dosing. Citrated plasma (0.38%) was prepared immediately after sampling and stored at -20°C until analysis, and a PK profile was established based on FVIIa clotting activity using a STACLOT commercial kit.

**Figure 29.** Shows that FVIIa-$CTP_3$ was produced following FVII selection, HA purification process and activation. FVIIa-$CTP_3$ or NovoSeven® was administered in a single intravenous injection to FVIII-/- hemophilic mice. Blood samples were drawn retro-orbitally at 0.083, 0.5 2, 8, 24, 48, and 72 hours post-dosing. Citrated plasma (0.38%) was prepared immediately after sampling and stored at -20°C until analysis. Thrombin generation parameters were evaluated during the PK experiment, and parameters including maximal amount to peak, amount of thrombin to time point and rate of thrombin generation were evaluated.

**Figure 30.** Shows hemophilic mice survival curves post tail vain transection (TVT). TVT was performed **(A)** 15 min, **(B)** 24 hours or **(C)** 48 hours post administration. Mice Survival was observed for 24 hours after TVT and recorded every single hour for the first 12 hours, and after 24 hours. Figure 33D summarizes mouse survival as recorded 24 hours post TVT. Control group data (vehicle) is the sum of the 3 experiments with 5 mice/experiment.

**Figure 31.** Shows FVII - 3- CTP and FVII- 5 CTP immune-blots. A) blotted for GLA. B) blotted for FVIIa. C) blotted for CTP.

**Figure 32.** Shows a comparative PK profile-FVII 3 & 5 CTP- from select and HA column purification (FVIIS vs. FVII HA).

**Figure 33.** Shows a comparative PK profile-FVII 3 & 5 CTP-The second study (IV vs. SC).

**Figure 34.** Shows hemophilic mice survival curves post tail vain transection (TVT). TVT was performed 12 hours post SC administration. Mice Survival was observed for 24 hours after TVT and recorded every single hour for the first 12 hours, and after 24 hours.

**Figure 35.** Shows the PK profile of MOD-5014 vs. NovoSeven® following IV or SC administration. A) shows IV administration; B) shows SC administration.

**Figure 36.** Shows the PK profile of MOD-5014 (Clone 61 #75, #81) vs. NovoSeven® following single SC administration.

## DETAILED DESCRIPTION OF THE INVENTION

The present invention relates to medical uses of long-acting coagulation factors. The long-acting coagulation factors comprise three carboxy terminal peptides (CTPs, also referred to as CTP units) of human Chorionic Gonadotropin (hCG). It is disclosed that the CTP acts as a protectant against the degradation of the coagulation factor, extends the $C_{max}$ of the coagulation factor, \extends the $T_{max}$ of the coagulation factor, extends the circulatory half-life of the coagulation factor, and/or enhances the potency of the coagulation factor.

The present invention thus provides a chorionic gonadotropin carboxy terminal peptide (CTP)-modified coagulation factor consisting of a coagulation factor and three CTPs attached to the carboxy terminus of said coagulation factor, wherein said CTP-modified coagulation factor is:

a CTP-modified Factor IX (FIX) polypeptide consisting of the amino acid sequence of SEQ ID NO: 31 or of amino acids 47-545 of SEQ ID NO: 31;
a CTP-modified Factor VII (FVII) polypeptide consisting of the amino acid sequence of SEQ ID NO: 25 or of amino acids 39-528 of SEQ ID NO: 25; or
a CTP-modified activated Factor VIIa (FVIIa) consisting of the amino acid sequence of amino acids 39-528 of SEQ ID NO: 25,

for use in reducing excessive bleeding or bruising in a subject, or for use in maintaining hemostasis during surgery or during surgical interventions in a subject.

Coagulation Factor VII (FVII) is a 444 amino acid glycoprotein (50KDa) secreted by hepatocytes into the bloodstream as an inactive pro-enzyme. Upon tissue injury and exposure to circulating blood, FVII forms a complex with Tissue Factor (TF) which is a true receptor protein to FVII and is expressed by various cells localized in the deeper layers of the vessel wall. The formation of this FVII-TF complex leads to activation of FVII. Activated FVII (FVIIa) initiates the extrinsic coagulation pathway by activating Factor IX and Factor X.

FVII belong to a group of Vitamin K-dependent glycoproteins associated with the coagulation system. Besides FVII, this group consists of Factor IX, Factor X, Protein C and prothrombin. These proteins have similar domain organizations and are synthesized as precursors with an N-terminal propeptide followed by a mature amino acid sequence. The propeptide contains a docking site for gammacarboxylase which converts glutamic acids (Glu) into gamma carboxy glutamic acids (Gla). This domain is followed by two epidermal growth factor-like (EGF) domains, a connecting region (CR) and a C-terminal serine protease domain. Prior to secretion, FVII propeptide is cleaved forming a 406 amino acid single chain zymogen FVII glycoprotein. After secretion, the protein can be activated into a disulfide-linked two chain heterodimer, FVIIa, by cleavage in the CR. The plasma concentration of FVII is 10 nM and approximately 1% circulates in the active form in healthy individuals.

Factor IX (FIX) is a 415 Amino acid (55KDa) glycoprotein; it belongs to a group of vitamin K dependent glycoproteins associated with the coagulation system. FIX has a similar domain organization as factor FVII, Factor X, Protein C and prothrombin that are synthesized as precursors with an N-terminal propeptide followed by a mature amino acid sequence.

FIX is secreted as a single chain molecule that undergoes complex post-transcriptional modifications, many of which are critical to its biochemical and pharmacokinetic properties. Among all the post-transcriptional modifications, 12 glutamic acid residues near the amino terminus of FIX that are gamma carboxylated by the vitamin K-dependent gamma carboxylase are the most crucial ones. Carboxylation is required for the interaction of FIX with the phospholipid surfaces and for optimal FIX activity. The amino terminus propeptide serves as a recognition site for the gamma carboxylase and thus, following gamma carboxylation, it is cleaved off by the Golgi apparatus serine protease known as Paired basic Amino acid Cleave Enzyme (PACE/Furin). Four additional post-transcriptional modifications might occur at the Golgi apparatus: sulfation of tyrosine 155, phosphorylation of serine 158, O- glycosylation on Ser 63 and on 61 and finally, N-glycosylation on Asn 157 and 16, but were shown not to be necessary for proper activity of FIX.

FIX circulates in the plasma (average concentration of 5 $\mu$g/ml) as a single chain inactive zymogen. Upon proteolytic cleavage at two peptide bonds: Arg 145 and Arg 180 by either one or two physiological activators, FVIIa-TF complex or FIXa, the activation peptide is removed, converting FIX to a fully active enzyme consisting of a light and heavy chain held together by a single disulfide bond. The N-terminal light chain contains the non-catalytic gamma carboxyglutamic acid (Gla) and two epidermal growth factor-like domains, while the C-terminal heavy chain contains the trypsin-like catalytic domain of the molecule. FIXa alone is characterized by poor catalytic activity. However when complexed with FVIII, its proteolytic activity increase by 4-5 orders of magnitude towards its natural substrate FX.

Accordingly, disclosed herein is a method of extending the biological half-life of or improving the area under the curve (AUC) of a coagulation factor, comprising the step of attaching three CTPs to the carboxy terminus of the coagulation factor, thereby extending the biological half-life or improving the AUC of the coagulation factor. Disclosed herein is a method of extending the biological half-life or a method of improving the area under the curve (AUC) of FVII or FVIIa, comprising the step of attaching three CTPs to the carboxy terminus of FVII or FVIIa, thereby extending the biological half-life or improving the AUC of FVII or FVIIa.

The present application also discloses a method of extending the biological half-life of a Factor IX (FIX) polypeptide, comprising the step of attaching three chorionic gonadotrophin carboxy terminal peptides (CTPs) to the carboxy terminus of said FIX polypeptide, thereby extending the biological half-life of said FIX polypeptide.

The present application also discloses a method of improving the area under the curve (AUC) of a Factor IX (FIX) polypeptide, comprising the step of attaching three chorionic gonadotrophin carboxy terminal peptides (CTPs) to the carboxy terminus of said FIX polypeptide, thereby improving the AUC of said FIX polypeptide. The present application also discloses a method of improving the area under the curve (AUC) of a Factor VIIa (FVIIa) polypeptide, comprising the step of attaching three chorionic gonadotrophin carboxy terminal peptides (CTPs) to the carboxy terminus of said FVIIa polypeptide, thereby improving the AUC of said FVIIa polypeptide.

In another embodiment, a coagulation factor of the invention is a protein. In another embodiment, a coagulation factor of the invention is a peptide. In another embodiment, a coagulation factor of the invention is a polypeptide. In another embodiment, the coagulation factor is an enzyme. It is disclosed that the coagulation factor is a serine protease. It is disclosed that the coagulation factor is a glycoprotein. It is disclosed that the coagulation factor is a transglutaminase. It is disclosed that the coagulation factor is an inactive zymogen.

In an embodiment, the coagulation factor is Factor VIIa (FVIIa). In another embodiment, the coagulation factor is Factor VII (FVII). In another embodiment, the coagulation factor is Factor IX (FIX). In another embodiment, the coagulation factor is Factor IXa (FIXa).

In one embodiment of the invention, the coagulation factor is a glycoprotein.It is disclosed that the coagulation factor is a vitamin K-dependent glycoprotein.

In another embodiment of the invention, the coagulation factor is a recombinant protein. In another embodiment of the invention, the coagulation factor is a recombinant glycoprotein. In another embodiment, the coagulation factor comprises a signal peptide.

In another embodiment of the invention, the coagulation factor comprises three CTP repeats attached to the C-terminus and no CTPs attached to the N-terminus.

In one embodiment, the present invention provides a CTP-modified Factor IX (FIX) polypeptide consisting of a FIX polypeptide and three gonadotropin carboxy terminal peptides (CTPs) attached to the carboxy terminus of said CTP-modified FIX polypeptide.

In another embodiment, the present invention further provides a CTP-modified Factor VIIa (FVIIa) polypeptide consisting of a FVIIa polypeptide and three gonadotropin carboxy terminal peptides (CTPs) attached to the carboxy terminus of said FVIIa.

In another embodiment, the coagulation factor is synthesized as a precursor with an N-terminal propeptide. In another embodiment, the coagulation factor as used herein is in an inactive pro-enzyme form. In another embodiment, the coagulation factor is produced in hepatocytes. In another embodiment, the coagulation factor comprises a docking site for gammacarboxylase which converts glutamic acids (Glu) into gamma carboxy glutamic acids (Gla). In another embodiment, the coagulation factor as used herein is a commercially available coagulation factor.

In one embodiment, the nucleic acid sequence encoding Factor VII comprises the following nucleic acid sequence:

ctcgaggacatggtctcccaggccctcaggctcctctgccttctgcttgggcttcagggctgcctggctgcagtcttcgtaacccaggag gaagcccacggcgtcctgcaccggcgccggcgcgccaacgcgttcctggaggagctgcggccgggctccctggagagggagtgc aaggaggagcagtgctccttcgaggaggcccgggagatcttcaaggacgcggagaggacgaagctgttctggatttcttacagtgat ggggaccagtgtgcctcaagtccatgccagaatgggggctcctgcaaggaccagctccagtcctatatctgcttctgcctccctgccttc gagggccggaactgtgagacgcacaaggatgaccagctgatctgtgtgaacgagaacggcggctgtgagcagtactgcagtgacca cacgggcaccaagcgctcctgtcggtgccacgaggggtactctctgctggcagacggggtgtcctgcacacccacagttgaatatcc atgtggaaaaatacctattctagaaaaaagaaatgccagcaaaccccaaggccgaattgtgggggggcaaggtgtgccccaaaggggg agtgtccatggcaggtcctgttgttggtgaatggagctcagttgtgtgggggggaccctgatcaacaccatctgggtggtctccgcggcc cactgtttcgacaaaatcaagaactggaggaacctgatcgcggtgctgggcgagcacgacctcagcgagcacgacggggatgagca gagccggcgggtggcgcaggtcatcatccccagcacgtacgtcccgggcaccaccaaccacgacatcgcgctgctccgcctgcac cagcccgtggtcctcactgaccatgtggtgcccctctgcctgcccgaacggacgttctctgagaggacgctggccttcgtgcgcttctc attggtcagcggctggggccagctgctggaccgtggcgccacggccctggagctcatggtcctcaacgtgccccggctgatgaccc aggactgcctgcagcagtcacggaaggtgggagactccccaaatatcacggagtacatgttctgtgccggctactcggatggcagca aggactcctgcaagggggacagtggaggcccacatgccacccactaccggggcacgtggtacctgacgggcatcgtcagctgggg ccagggctgcgcaaccgtgggccactttggggtgtacaccagggtctcccagtacatcgagtggctgcaaaagctcatgcgctcaga gccacgcccaggagtcctcctgcgagccccatttccctgaggatgcggccgc (SEQ ID NO: 11).

In another embodiment, the amino acid sequence of Factor VII comprises the following amino acid sequence:

MVSQALRLLCLLLGLQGCLAAVFVTQEEAHGVLHRRRRANAFLEELRPGSLERECKE EQCSFEEAREIFKDAERTKLFWISYSDGDQCASSPCQNGGSCKDQLQSYICFCLPAFE GRNCETHKDDQLICVNENGGCEQYCSDHTGTKRSCRCHEGYSLLADGVSCTPTVEY PCGKIPILEKRNASKPQGRIVGGKVCPKGECPWQVLLLVNGAQLCGGTLINTIWVVS AAHCFDKIKNWRNLIAVLGEHDLSEHDGDEQSRRVAQVIIPSTYVPGTTNHDIALLRL HQPVVLTDHVVPLCLPERTFSERTLAFVRFSLVSGWGQLLDRGATALELMVLNVPRL MTQDCLQQSRKVGDSPNITEYMFCAGYSDGSKDSCKGDSGGPHATHYRGTWYLTGI VSWGQGCATVGHFGVYTRVSQYIEWLQKLMRSEPRPGVLLRAPFP (SEQ ID NO: 9).

In another embodiment, the amino acid sequence of Factor VII comprises the following amino acid sequence:

MVSQALRLLCLLLGLQGCLAAVFVTQEEAHGVLHRRRRANAFLEELRPGSLERECKE
EQCSFEEAREIFKDAERTKLFWISYSDGDQCASSPCQNGGSCKDQLQSYICFCLPAFE
GRNCETHKDDQLICVNENGGCEQYCSDHTGTKRSCRCHEGYSLLADGVSCTPTVEY
PCGKIPILEKRNASKPQGRIVGGKVCPKGECPWQVLLLVNGAQLCGGTLINTIWVVS
AAHCFDKIKNWRNLIAVLGEHDLSEHDGDEQSRRVAQVIIPSTYVPGTTNHDIALLRL
HQPVVLTDHVVPLCLPERTFSERTLAFVRFSLVSGWGQLLDRGATALELMVLNVPRL
MTQDCLQQSRKVGDSPNITEYMFCAGYSDGSKDSCKGDSGGPHATHYRGTWYLTGI
VSWGQGCATVGHFGVYTRVSQYIEWLQKLMRSEPRPGVLLRAPFP*GCGR (SEQ ID NO: 10).

It is disclosed that the nucleic acid sequence encoding Factor VII-CTP (attached to the carboxy terminus) comprises the following nucleic acid sequence:

ctcgaggacatggtctcccaggccctcaggctcctctgccttctgcttgggcttcagggctgcctggctgcagtcttcgtaacccaggag
gaagcccacggcgtcctgcaccggcgccggcgcgccaacgcgttcctggaggagctgcggccgggctccctggagagggagtgc
aaggaggagcagtgctccttcgaggaggcccgggagatcttcaaggacgcggagaggacgaagctgttctggatttcttacagtgat
ggggaccagtgtgcctcaagtccatgccagaatgggggctcctgcaaggaccagctccagtcctatatctgcttctgcctccctgccttc
gagggccggaactgtgagacgcacaaggatgaccagctgatctgtgtgaacgagaacggcggctgtgagcagtactgcagtgacca
cacgggcaccaagcgctcctgtcggtgccacgaggggtactctctgctggcagacggggtgtcctgcacacccacagttgaatatcc
atgtggaaaaatacctattctagaaaaaagaaatgccagcaaaccccaaggccgaattgtgggggggcaaggtgtgccccaaagggg
agtgtccatggcaggtcctgttgttggtgaatggagctcagttgtgtgggggggaccctgatcaacaccatctgggtggtctccgcggcc
cactgtttcgacaaaatcaagaactggaggaacctgatcgcggtgctgggcgagcacgacctcagcgagcacgacggggatgagca
gagccggcgggtggcgcaggtcatcatccccagcacgtacgtcccgggcaccaccaaccacgacatcgcgctgctccgcctgcac
cagcccgtggtcctcactgaccatgtggtgcccctctgcctgcccgaacggacgttctctgagaggacgctggccttcgtgcgcttctc
attggtcagcggctggggccagctgctggaccgtggcgccacggccctggagctcatggtcctcaacgtgccccggctgatgaccc
aggactgcctgcagcagtcacggaaggtgggagactccccaaatatcacggagtacatgttctgtgccggctactcggatggcagca
aggactcctgcaaggggggacagtggaggcccacatgccacccactaccggggcacgtggtacc
tgaccggcatcgtgagctggggccagggctgcgccaccgtgggccacttcggcgtgtacaccagggtgtcccagtacatcgagtgg
ctgcagaaactgatgagaagcgagcccagacccggcgtgctgctgagagccccccttccccagcagcagctccaaggcccctccccc
tagcctgcccagccctagcagactgcctgggcccagcgacacccccatcctgccccagtgaggatccgcggccgc (SEQ ID NO: 12).

It is disclosed that the amino acid sequence of Factor VII-CTP (attached to the carboxy terminus) comprises the following amino acid sequence:

MVSQALRLLCLLLGLQGCLAAVFVTQEEAHGVLHRRRRANAFLEELRPGSLERECKE
EQCSFEEAREIFKDAERTKLFWISYSDGDQCASSPCQNGGSCKDQLQSYICFCLPAFE
GRNCETHKDDQLICVNENGGCEQYCSDHTGTKRSCRCHEGYSLLADGVSCTPTVEY
PCGKIPILEKRNASKPQGRIVGGKVCPKGECPWQVLLLVNGAQLCGGTLINTIWVVS
AAHCFDKIKNWRNLIAVLGEHDLSEHDGDEQSRRVAQVIIPSTYVPGTTNHDIALLRL
HQPVVLTDHVVPLCLPERTFSERTLAFVRFSLVSGWGQLLDRGATALELMVLNVPRL
MTQDCLQQSRKVGDSPNITEYMFCAGYSDGSKDSCKGDSGGPHATHYRGTWYLTGI
VSWGQGCATVGHFGVYTRVSQYIEWLQKLMRSEPRPGVLLRAPFPSSSSKAPPPSLPS
PSRLPGPSDTPILPQ* (SEQ ID NO: 13).

It is disclosed that the nucleic acid sequence encoding Factor VII-CTP-CTP (attached to the carboxy terminus) comprises the following nucleic acid sequence:

ctcgaggacatggtctcccaggccctcaggctcctctgccttctgcttgggcttcagggctgcctggctgcagtcttcgtaacccaggag
gaagcccacggcgtcctgcaccggcgccggcgcgccaacgcgttcctggaggagctgcggccgggctccctggagagggagtgc
aaggaggagcagtgctccttcgaggaggcccgggagatcttcaaggacgcggagaggacgaagctgttctggatttcttacagtgat
ggggaccagtgtgcctcaagtccatgccagaatgggggctcctgcaaggaccagctccagtcctatatctgcttctgcctccctgccttc
gagggccggaactgtgagacgcacaaggatgaccagctgatctgtgtgaacgagaacggcggctgtgagcagtactgcagtgacca
cacgggcaccaagcgctcctgtcggtgccacgaggggtactctctgctggcagacggggtgtcctgcacacccacagttgaatatcc
atgtggaaaaatacctattctagaaaaaagaaatgccagcaaaccccaaggccgaattgtgggggggcaaggtgtgccccaaagggg
agtgtccatggcaggtcctgttgttggtgaatggagctcagttgtgtgggggggaccctgatcaacaccatctgggtggtctccgcggcc
cactgtttcgacaaaatcaagaactggaggaacctgatcgcggtgctgggcgagcacgacctcagcgagcacgacggggatgagca
gagccggcgggtggcgcaggtcatcatccccagcacgtacgtcccgggcaccaccaaccacgacatcgcgctgctccgcctgcac
cagcccgtggtcctcactgaccatgtggtgcccctctgcctgcccgaacggacgttctctgagaggacgctggccttcgtgcgcttctc
attggtcagcggctggggccagctgctggaccgtggcgccacggccctggagctcatggtcctcaacgtgccccggctgatgaccc
aggactgcctgcagcagtcacggaaggtgggagactccccaaatatcacggagtacatgttctgtgccggctactcggatggcagca
aggactcctgcaagggggacagtggaggcccacatgccacccactaccggggcacgtggtacctgaccggcatcgtgagctgggg
ccagggctgcgccaccgtgggccacttcggcgtgtacaccagggtgtcccagtacatcgagtggctgcagaaactgatgagaagcg
agcccagacccggcgtgctgctgagagccccttccccagcagcagctccaaggcccctcccccctagcctgcccagccctagcaga
ctgcctgggccctccgacacaccaatcctgccacagagcagctcctctaaggcccctcctccatccctgccatcccccctcccggctgc
caggcccctctgacacccctatcctgcctcagtgatgaaggtctggatccgcggccgc (SEQ ID NO: 14).

It is disclosed that the amino acid sequence of Factor VII-CTP-CTP (attached to the carboxy terminus) comprises the following amino acid sequence:

MVSQALRLLCLLLGLQGCLAAVFVTQEEAHGVLHRRRRANAFLEELRPGSLERECKE

EQCSFEEAREIFKDAERTKLFWISYSDGDQCASSPCQNGGSCKDQLQSYICFCLPAFE
GRNCETHKDDQLICVNENGGCEQYCSDHTGTKRSCRCHEGYSLLADGVSCTPTVEY
PCGKIPILEKRNASKPQGRIVGGKVCPKGECPWQVLLLVNGAQLCGGTLINTIWVVS
AAHCFDKIKNWRNLIAVLGEHDLSEHDGDEQSRRVAQVIIPSTYVPGTTNHDIALLRL
HQPVVLTDHVVPLCLPERTFSERTLAFVRFSLVSGWGQLLDRGATALELMVLNVPRL
MTQDCLQQSRKVGDSPNITEYMFCAGYSDGSKDSCKGDSGGPHATHYRGTWYLTGI
VSWGQGCATVGHFGVYTRVSQYIEWLQKLMRSEPRPGVLLRAPFPSSSSKAPPPSLPS
PSRLPGPSDTPILPQSSSSKAPPPSLPSPSRLPGPSDTPILPQ** (SEQ ID NO: 15).

In another embodiment, the nucleic acid sequence encoding Factor VII-CTP-CTP-CTP (attached to the carboxy terminus) comprises the following nucleic acid sequence:

ctcgaggacatggtctcccaggccctcaggctcctctgccttctgcttgggcttcagggctgcctggctgcagtcttcgtaacccaggag
gaagcccacggcgtcctgcaccggcgccggcgcgccaacgcgttcctggaggagctgcggccgggctccctggagagggagtgc
aaggaggagcagtgctccttcgaggaggcccgggagatcttcaaggacgcggagaggacgaagctgttctggatttcttacagtgat
ggggaccagtgtgcctcaagtccatgccagaatgggggctcctgcaaggaccagctccagtcctatatctgcttctgcctccctgccttc
gagggccggaactgtgagacgcacaaggatgaccagctgatctgtgtgaacgagaacggcggctgtgagcagtactgcagtgacca
cacgggcaccaagcgctcctgtcggtgccacgaggggtactctctgctggcagacggggtgtcctgcacacccacagttgaatatcc
atgtggaaaaatacctattctagaaaaaagaaatgccagcaaaccccaaggccgaattgtgggggcaaggtgtgccccaaagggg
agtgtccatggcaggtcctgttgttggtgaatggagctcagttgtgtgggggaccctgatcaacaccatctgggtggtctccgcggcc
cactgtttcgacaaaatcaagaactggaggaacctgatcgcggtgctgggcgagcacgacctcagcgagcacgacggggatgagca
gagccggcgggtggcgcaggtcatcatccccagcacgtacgtcccgggcaccaccaaccacgacatcgcgctgctccgcctgcac
cagcccgtggtcctcactgaccatgtggtgcccctctgcctgcccgaacggacgttctctgagaggacgctggccttcgtgcgcttctc
attggtcagcggctggggccagctgctggaccgtggcgccacggccctggagctcatggtcctcaacgtgccccggctgatgaccc
aggactgcctgcagcagtcacggaaggtgggagactccccaaatatcacggagtacatgttctgtgccggctactcggatggcagca
aggactcctgcaagggggacagtggaggcccacatgccacccactaccggggcacgtggtacctgaccggcatcgtgagctgggg
ccagggctgcgccaccgtgggccacttcggcgtgtacaccagggtgtcccagtacatcgagtggctgcagaaactgatgagaagcg
agcccagacccggcgtgctgctgagagccccccttccccagcagcagctccaaggcccctcccccctagcctgcccagccctagcaga
ctgcctgggcccagtgacacccctatcctgcctcagtccagctccagcaaggccccacccccctagcctgccttctccttctcggctgcct
ggccccagcgatactccaattctgccccagtcctccagcagtaaggctccccctccatctctgccatcccccagcagactgccaggcc
cttctgatacacccatcctcccacagtgatgaggatccgcggccgcttaattaa (SEQ ID NO: 24).

In another embodiment, the amino acid sequence of Factor VII-CTP-CTP-CTP (attached to the carboxy terminus) comprises the following amino acid sequence:

MVSQALRLLCLLLGLQGCLAAVFVTQEEAHGVLHRRRRANAFLEELRPGSLERECKE
EQCSFEEAREIFKDAERTKLFWISYSDGDQCASSPCQNGGSCKDQLQSYICFCLPAFE
GRNCETHKDDQLICVNENGGCEQYCSDHTGTKRSCRCHEGYSLLADGVSCTPTVEY

PCGKIPILEKRNASKPQGRIVGGKVCPKGECPWQVLLLVNGAQLCGGTLINTIWVVS
AAHCFDKIKNWRNLIAVLGEHDLSEHDGDEQSRRVAQVIIPSTYVPGTTNHDIALLRL
HQPVVLTDHVVPLCLPERTFSERTLAFVRFSLVSGWGQLLDRGATALELMVLNVPRL
MTQDCLQQSRKVGDSPNITEYMFCAGYSDGSKDSCKGDSGGPHATHYRGTWYLTGI
VSWGQGCATVGHFGVYTRVSQYIEWLQKLMRSEPRPGVLLRAPFPSSSSKAPPPSLPS
PSRLPGPSDTPILPQSSSSKAPPPSLPSPSRLPGPSDTPILPQSSSSKAPPPSLPSPSRLPGP
SDTPILPQ** (SEQ ID NO: 25).

It is disclosed that the nucleic acid sequence encoding Factor VII-$(CTP)_4$ (attached to the carboxy terminus) comprises the following nucleic acid sequence:

ctcgaggacatggtctcccaggccctcaggctcctctgccttctgcttgggcttcagggctgcctggctgcagtcttcgtaacccaggag
gaagcccacggcgtcctgcaccggcgccggcgcgccaacgcgttcctggaggagctgcggccgggctccctggagagggagtgc
aaggaggagcagtgctccttcgaggaggcccgggagatcttcaaggacgcggagaggacgaagctgttctggatttcttacagtgat
ggggaccagtgtgcctcaagtccatgccagaatgggggctcctgcaaggaccagctccagtcctatatctgcttctgcctccctgccttc
gagggccggaactgtgagacgcacaaggatgaccagctgatctgtgtgaacgagaacggcggctgtgagcagtactgcagtgacca
cacgggcaccaagcgctcctgtcggtgccacgaggggtactctctgctggcagacggggtgtcctgcacacccacagttgaatatcc
atgtggaaaaatacctattctagaaaaaagaaatgccagcaaaccccaaggccgaattgtgggggggcaaggtgtgccccaaagggg
agtgtccatggcaggtcctgttgttggtgaatggagctcagttgtgtggggggaccctgatcaacaccatctgggtggtctccgcggcc
cactgtttcgacaaaatcaagaactggaggaacctgatcgcggtgctgggcgagcacgacctcagcgagcacgacggggatgagca
gagccggcgggtggcgcaggtcatcatccccagcacgtacgtcccgggcaccaccaaccacgacatcgcgctgctccgcctgcac
cagcccgtggtcctcactgaccatgtggtgcccctctgcctgcccgaacggacgttctctgagaggacgctggccttcgtgcgcttctc
attggtcagcggctggggccagctgctggaccgtggcgccacggccctggagctcatggtcctcaacgtgccccggctgatgaccc
aggactgcctgcagcagtcacggaaggtgggagactccccaaatatcacggagtacatgttctgtgccggctactcggatggcagca
aggactcctgcaagggggacagtggaggcccacatgccacccactaccggggcacgtggtacctgaccggcatcgtgagctgggg
ccagggctgcgccaccgtgggccacttcggcgtgtacaccagggtgtcccagtacatcgagtggctgcagaaactgatgagaagcg
agcccagacccggcgtgctgctgagagccccctteccagcagcagctccaaggcccctcccccctagcctgcccagccctagcaga
ctgcctgggcccagtgacacccctatcctgcctcagtccagctccagcaaggccccacccccctagcctgccttctccttctcggctgcct
ggccccagcgatactccaattctgccccagtcctccagcagtaaggctccccctccatctctgccatccccagcagactgccaggcc
cttctgatacacccatcctcccacagtgatgaggatccgc (SEQ ID NO: 26).

It is disclosed that the amino acid sequence of Factor VII-$(CTP)_4$ (attached to the carboxy terminus) comprises the following amino acid sequence:

LEDMVSQALRLLCLLLGLQGCLAAVFVTQEEAHGVLHRRRRANAFLEELRPGSLERE CKEEQCSFEEAREIFKDAERTKLFWISYSDGDQCASSPCQNGGSCKDQLQSYICFCLP AFEGRNCETHKDDQLICVNENGGCEQYCSDHTGTKRSCRCHEGYSLLADGVSCTPT VEYPCGKIPILEKRNASKPQGRIVGGKVCPKGECPWQVLLLVNGAQLCGGTLINTIW

VVSAAHCFDKIKNWRNLIAVLGEHDLSEHDGDEQSRRVAQVIIPSTYVPGTTNHDIAL LRLHQPVVLTDHVVPLCLPERTFSERTLAFVRFSLVSGWGQLLDRGATALELMVLNV PRLMTQDCLQQSRKVGDSPNITEYMFCAGYSDGSKDSCKGDSGGPHATHYRGTWYL TGIVSWGQGCATVGHFGVYTRVSQYIEWLQKLMRSEPRPGVLLRAPFPSSSSKAPPPS LPSPSRLPGPSDTPILPQSSSSKAPPPSLPSPSRLPGPSDTPILPQSSSSKAPPPSLPSPSRLP GPSDTPILPQ**G (SEQ ID NO: 27).

It is disclosed that the nucleic acid sequence encoding Factor VII-$(CTP)_5$ (attached to the carboxy terminus) comprises the following nucleic acid sequence:

ctcgaggacatggtctcccaggccctcaggctcctctgccttctgcttgggcttcagggctgcctggctgcagtcttcgtaacccaggag
gaagcccacggcgtcctgcaccggcgccggcgcgccaacgcgttcctggaggagctgcggccgggctccctggagagggagtgc
aaggaggagcagtgctccttcgaggaggcccgggagatcttcaaggacgcggagaggacgaagctgttctggatttcttacagtgat
ggggaccagtgtgcctcaagtccatgccagaatgggggctcctgcaaggaccagctccagtcctatatctgcttctgcctccctgccttc
gagggccggaactgtgagacgcacaaggatgaccagctgatctgtgtgaacgagaacggcggctgtgagcagtactgcagtgacca
cacgggcaccaagcgctcctgtcggtgccacgaggggtactctctgctggcagacggggtgtcctgcacacccacagttgaatatcc
atgtggaaaaatacctattctagaaaaaagaaatgccagcaaaccccaaggccgaattgtgggggggcaaggtgtgccccaaagggg
agtgtccatggcaggtcctgttgttggtgaatggagctcagttgtgtgggggggaccctgatcaacaccatctgggtggtctccgcggcc
cactgtttcgacaaaatcaagaactggaggaacctgatcgcggtgctgggcgagcacgacctcagcgagcacgacggggatgagca
gagccggcgggtggcgcaggtcatcatccccagcacgtacgtcccgggcaccaccaaccacgacatcgcgctgctccgcctgcac
cagcccgtggtcctcactgaccatgtggtgcccctctgcctgcccgaacggacgttctctgagaggacgctggccttcgtgcgcttctc
attggtcagcggctggggccagctgctggaccgtggcgccacggccctggagctcatggtcctcaacgtgccccggctgatgaccc
aggactgcctgcagcagtcacggaaggtgggagactccccaaatatcacggagtacatgttctgtgccggctactcggatggcagca
aggactcctgcaagggggacagtggaggcccacatgccacccactaccggggcacgtggtacctgaccggcatcgtgagctgggg
ccagggctgcgccaccgtgggccacttcggcgtgtacaccagggtgtcccagtacatcgagtggctgcagaaactgatgagaagcg
agcccagacccggcgtgctgctgagagccccccttccccagcagcagctccaaggcccctcccccctagcctgcccagccctagcaga
ctgcctgggccctctgacacccctatcctgcctcagtccagctcctctaaggctccaccaccttccctgcctagcccttcaagactgcca
ggccctagcgatacaccaattctgccccagtcctccagcagcaaggctcccccacctagcctgccttctccatcaaggctgcctggcc
catccgataccccaattttgcctcagagcagctctagcaaggcacctcccccccagtctgccctctccaagcagactccctggcccttca
gacactccaatcctcccacagtcctctagctctaaagctccacctcccagcctgcccagccctagtagactccccggaccttctgatacc
cccatcttgccccagtgatgaggatccgc (SEQ ID NO: 28).

It is disclosed that the amino acid sequence of Factor VII-$(CTP)_5$ (attached to the carboxy terminus) comprises the following amino acid sequence:

LEDMVSQALRLLCLLLGLQGCLAAVFVTQEEAHGVLHRRRRANAFLEELRPGSLERE
CKEEQCSFEEAREIFKDAERTKLFWISYSDGDQCASSPCQNGGSCKDQLQSYICFCLP
AFEGRNCETHKDDQLICVNENGGCEQYCSDHTGTKRSCRCHEGYSLLADGVSCTPT

VEYPCGKIPILEKRNASKPQGRIVGGKVCPKGECPWQVLLLVNGAQLCGGTLINTIWVVSAAHCFDKIKNWRNLIAVLGEHDLSEHDGDEQSRRVAQVIIPSTYVPGTTNHDIALLRLHQPVVLTDHVVPLCLPERTFSERTLAFVRFSLVSGWGQLLDRGATALELMVLNVPRLMTQDCLQQSRKVGDSPNITEYMFCAGYSDGSKDSCKGDSGGPHATHYRGTWYLTGIVSWGQGCATVGHFGVYTRVSQYIEWLQKLMRSEPRPGVLLRAPFPSSSSKAPPPSLPSPSRLPGPSDTPILPQSSSSKAPPPSLPSPSRLPGPSDTPILPQSSSSKAPPPSLPSPSRLPGPSDTPILPQSSSSKAPPPSLPSPSRLPGPSDTPILPQSSSSKAPPPSLPSPSRLPGPSDTPILPQ**GS (SEQ ID NO: 29).

In another embodiment, the nucleic acid sequence encoding Factor IX comprises the following nucleic acid sequence:

gcgatcgccatgcagcgcgtgaacatgatcatggcagaatcaccaggcctcatcaccattgccttttaggatatctactcagtgctgaatgtacagtttttcttgatcatgaaaacgccaacaaaattctgaatcggccaaagaggtataattcaggtaaattggaagagtttgttcaagggaaccttgagagagaatgtatggaagaaaagtgtagttttgaagaagcacgagaagtttttgaaaacactgaaagaacaactgaattttggaagcagtatgttgatggagatcagtgtgagtccaatccatgtttaaatggcggcagttgcaaggatgacattaattcctatgaatgttggtgtccctttggatttgaaggaaagaactgtgaattagatgtaacatgtaacattaagaatggcagatgcgagcagttttgtaaaaatagtgctgataacaaggtggtttgctcctgtactgagggatatcgacttgcagaaaaccagaagtcctgtgaaccagcagtgccatttccatgtggaagagtttctgtttcacaaacttctaagctcacccgtgctgagactgtttttcctgatgtggactatgtaaattctactgaagctgaaaccattttggataacatcactcaaagcacccaatcatttaatgacttcactcgagttgttggtggagaagatgccaaaccaggtcaattcccttggcaggttgttttgaatggtaaagttgatgcattctgtggaggctctatcgttaatgaaaaatggattgtaactgctgcccactgtgttgaaactggtgttaaaattacagttgtcgcaggtgaacataatattgaggagacagaacatacagagcaaaagcgaaatgtgattcgaattattcctcaccacaactacaatgcagctattaataagtacaaccatgacattgcccttctggaactggacgaacccttagtgctaaacagctacgttacacctatttgcattgctgacaaggaatacacgaacatcttcctcaaatttggatctggctatgtaagtggctggggaagagtcttccacaaagggagatcagctttagttctccagtaccttagagttccacttgttgaccgagccacatgtcttcgatctacaaagttcaccatctataacaacatgttctgtgctggcttccatgaaggaggtagagattcatgtcaaggagatagtgggggaccccatgttactgaagtggaagggaccagtttcttaactggaattattagctggggtgaagagtgtgcaatgaaaggcaaatatggaatatataccaaggtatcccggtatgtcaactggattaaggaaaaaacaaagctcacttgaacgcggccgc (SEQ ID NO: 16).

In another embodiment, the amino acid sequence of Factor IX comprises the following amino acid sequence:

MQRVNMIMAESPGLITICLLGYLLSAECTVFLDHENANKILNRPKRYNSGKLEEFVQG NLERECMEEKCSFEEAREVFENTERTTEFWKQYVDGDQCESNPCLNGGSCKDDINSY ECWCPFGFEGKNCELDVTCNIKNGRCEQFCKNSADNKVVCSCTEGYRLAENQKSCE PAVPFPCGRVSVSQTSKLTRAETVFPDVDYVNSTEAETILDNITQSTQSFNDFTRVVG GEDAKPGQFPWQVVLNGKVDAFCGGSIVNEKWIVTAAHCVETGVKITVVAGEHNIE ETEHTEQKRNVIRIIPHHNYNAAINKYNHDIALLELDEPLVLNSYVTPICIADKEYTNIF

LKFGSGYVSGWGRVFHKGRSALVLQYLRVPLVDRATCLRSTKFTIYNNMFCAGFHE GGRDSCQGDSGGPHVTEVEGTSFLTGIISWGEECAMKGKYGIYTKVSRYVNWIKEKT KLT* (SEQ ID NO: 17).

It is disclosed that the nucleic acid sequence encoding Factor IX-CTP (attached to the carboxy terminus) comprises the following nucleic acid sequence:

gcgatcgccatgcagcgcgtgaacatgatcatggcagaatcaccaggcctcatcaccatctgccttttaggatatctactcagtgctgaa tgtacagtttttcttgatcatgaaaacgccaacaaaattctgaatcggccaaagaggtataattcaggtaaattggaagagtttgttcaagg gaaccttgagagagaatgtatggaagaaaagtgtagttttgaagaagcacgagaagtttttgaaaacactgaaagaacaactgaattttg gaagcagtatgttgatggagatcagtgtgagtccaatccatgtttaaatggcggcagttgcaaggatgacattaattcctatgaatgttggt gtccctttggatttgaaggaaagaactgtgaattagatgtaacatgtaacattaagaatggcagatgcgagcagttttgtaaaaatagtgct gataacaaggtggtttgctcctgtactgagggatatcgacttgcagaaaaccagaagtcctgtgaaccagcagtgccatttccatgtgga agagtttctgtttcacaaacttctaagctcacccgtgctgagactgtttttcctgatgtggactatgtaaattctactgaagctgaaaccatttt ggataacatcactcaaagcacccaatcatttaatgacttcactcgagttgttggtggagaagatgccaaaccaggtcaattcccttggca ggttgttttgaatggtaaagttgatgcattctgtggaggctctatcgttaatgaaaaatggattgtaactgctgcccactgtgttgaaactggt gttaaaattacagttgtcgcaggtgaacataatattgaggagacagaacatacagagcaaaagcgaaatgtgattcgaattattcctcac cacaactacaatgcagctattaataagtacaaccatgacattgcccttctggaactggacgaacccttagtgctaaacagctacgttacac ctatttgcattgctgacaaggaatacacgaacatcttcctcaaatttggatctggctatgtaagtggctggggaagagtcttccacaaagg gagatcagctttagttcttcagtaccttagagttccacttgttgaccgagccacatgtcttcgatctacaaagttcaccatctataacaacatg ttctgtgctggcttccatgaaggaggtagagattcatgtcaaggagatagtgggggaccccatgttactgaagtggaagggaccagttt cttaactggaattattagctggggtgaagagtgtgcaatgaaaggcaaatatggaatatataccaaggtatcccggtatgtcaactggatt aaggaaaaaacaaagctcactagctccagcagcaaggcccctcccccgagcctgccctccccaagcaggctgcctgggccctccga cacaccaatcctgccacagtgatgaaggtctggatccgcggccgc (SEQ ID NO: 18).

It is disclosed that the amino acid sequence of Factor IX-CTP (attached to the carboxy terminus) comprises the following amino acid sequence:

MQRVNMIMAESPGLITICLLGYLLSAECTVFLDHENANKILNRPKRYNSGKLEEFVQG NLERECMEEKCSFEEAREVFENTERTTEFWKQYVDGDQCESNPCLNGGSCKDDINSY ECWCPFGFEGKNCELDVTCNIKNGRCEQFCKNSADNKVVCSCTEGYRLAENQKSCE PAVPFPCGRVSVSQTSKLTRAETVFPDVDYVNSTEAETILDNITQSTQSFNDFTRVVG GEDAKPGQFPWQVVLNGKVDAFCGGSIVNEKWIVTAAHCVETGVKITVVAGEHNIE ETEHTEQKRNVIRIIPHHNYNAAINKYNHDIALLELDEPLVLNSYVTPICIADKEYTNIF LKFGSGYVSGWGRVFHKGRSALVLQYLRVPLVDRATCLRSTKFTIYNNMFCAGFHE GGRDSCQGDSGGPHVTEVEGTSFLTGIISWGEECAMKGKYGIYTKVSRYVNWIKEKT KLTSSSSKAPPPSLPSPSRLPGPSDTPILPQ** (SEQ ID NO: 19).

It is disclosed that the nucleic acid sequence encoding Factor IX-CTP-CTP (attached to the carboxy terminus) comprises the following nucleic acid sequence:

gcgatcgccatgcagcgcgtgaacatgatcatggcagaatcaccaggcctcatcaccatctgccttttaggatatctactcagtgctgaa tgtacagtttttcttgatcatgaaaacgccaacaaaattctgaatcggccaaagaggtataattcaggtaaattggaagagtttgttcaagg gaaccttgagagagaatgtatggaagaaaagtgtagttttgaagaagcacgagaagtttttgaaaacactgaaagaacaactgaattttg gaagcagtatgttgatggagatcagtgtgagtccaatccatgtttaaatggcggcagttgcaaggatgacattaattcctatgaatgttggt gtccctttggatttgaaggaaagaactgtgaattagatgtaacatgtaacattaagaatggcagatgcgagcagttttgtaaaaatagtgct gataacaaggtggtttgctcctgtactgagggatatcgacttgcagaaaaccagaagtcctgtgaaccagcagtgccatttccatgtgga agagtttctgtttcacaaacttctaagctcacccgtgctgagactgtttttcctgatgtggactatgtaaattctactgaagctgaaaccatttt ggataacatcactcaaagcacccaatcatttaatgacttcactcgagttgttggtggagaagatgccaaaccaggtcaattcccttggca ggttgttttgaatggtaaagttgatgcattctgtggaggctctatcgttaatgaaaaatggattgtaactgctgcccactgtgttgaaactggt gttaaaattacagttgtcgcaggtgaacataatattgaggagacagaacatacagagcaaaagcgaaatgtgattcgaattattcctcac cacaactacaatgcagctattaataagtacaaccatgacattgcccttctggaactggacgaacccttagtgctaaacagctacgttacac ctatttgcattgctacaaggaatacacgaacatcttcctcaaatttggatctggctatgtaagtggctggggaagagtcttccacaaaggg agatcagctttagttcttcagtaccttagagttccacttgttgaccgagccacatgtcttcgatctacaaagttcaccatctataacaacatgt tctgtgctggcttccatgaaggaggtagagattcatgtcaaggagatagtgggggaccccatgttactgaagtggaagggaccagtttc ttaactggaattattagctggggtgaagagtgtgcaatgaaaggcaaatatggaatatataccaaggtatcccggtatgtcaactggatta aggaaaaaacaaagctcactagctccagcagcaaggcccctcccccgagcctgccctccccaagcaggctgcctgggccctccgac acaccaatcctgccacagagcagctcctctaaggcccctcctccatccctgccatccccctcccggctgcctggcccctctgacacccc tatcctgcctcagtgatgaaggtctggatccgcggccgc (SEQ ID NO: 20).

It is disclosed that the amino acid sequence of Factor IX-CTP-CTP (attached to the carboxy terminus) comprises the following amino acid sequence:

MQRVNMIMAESPGLITICLLGYLLSAECTVFLDHENANKILNRPKRYNSGKLEEFVQG
NLERECMEEKCSFEEAREVFENTERTTEFWKQYVDGDQCESNPCLNGGSCKDDINSY
ECWCPFGFEGKNCELDVTCNIKNGRCEQFCKNSADNKVVCSCTEGYRLAENQKSCE
PAVPFPCGRVSVSQTSKLTRAETVFPDVDYVNSTEAETILDNITQSTQSFNDFTRVVG
GEDAKPGQFPWQVVLNGKVDAFCGGSIVNEKWIVTAAHCVETGVKITVVAGEHNIE
ETEHTEQKRNVIRIIPHHNYNAAINKYNHDIALLELDEPLVLNSYVTPICIADKEYTNIF
LKFGSGYVSGWGRVFHKGRSALVLQYLRVPLVDRATCLRSTKFTIYNNMFCAGFHE
GGRDSCQGDSGGPHVTEVEGTSFLTGIISWGEECAMKGKYGIYTKVSRYVNWIKEKT
KLTSSSSKAPPPSLPSPSRLPGPSDTPILPQSSSSKAPPPSLPSPSRLPGPSDTPILPQ**
(SEQ ID NO: 21).

In another embodiment, the nucleic acid sequence encoding Factor IX-$(CTP)_3$ (attached to the carboxy terminus) comprises the following nucleic acid sequence:

tctagagtcgaccccgccatgcagcgcgtgaacatgatcatggcagaatcaccaggcctcatcaccatctgccttttaggatatctactc
agtgctgaatgtacagtttttcttgatcatgaaaacgccaacaaaattctgaatcggccaaagaggtataattcaggtaaattggaagagtt
tgttcaagggaaccttgagagagaatgtatggaagaaaagtgtagttttgaagaagcacgagaagtttttgaaaacactgaaagaacaa
ctgaattttggaagcagtatgttgatggagatcagtgtgagtccaatccatgtttaaatggcggcagttgcaaggatgacattaattcctat
gaatgttggtgtccctttggatttgaaggaaagaactgtgaattagatgtaacatgtaacattaagaatggcagatgcgagcagttttgtaa
aaatagtgctgataacaaggtggtttgctcctgtactgagggatatcgacttgcagaaaaccagaagtcctgtgaaccagcagtgccatt
tccatgtggaagagtttctgtttcacaaacttctaagctcacccgtgctgaggcagtttttcctgatgtggactatgtaaattctactgaagct
gaaaccattttggataacatcactcaaagcacccaatcatttaatgacttcactcgagttgttggtggagaagatgccaaaccaggtcaat
tcccttggcaggttgttttgaatggtaaagttgatgcattctgtggaggctctatcgttaatgaaaaatggattgtaactgctgcccactgtgt
tgaaactggtgttaaaattacagttgtcgcaggtgaacataatattgaggagacagaacatacagagcaaaagcgaaatgtgattcgaat
tattcctcaccacaactacaatgcagctattaataagtacaaccatgacattgcccttctggaactggacgaacccttagtgctaaacagct
acgttacacctatttgcattgctgacaaggaatacacgaacatcttcctcaaatttggatctggctatgtaagtggctggggaagagtcttc
cacaaagggagatcagctttagttcttcagtaccttagagttccacttgttgaccgagccacatgtcttcgatctacaaagttcaccatctat
aacaacatgttctgtgctggcttccatgaaggaggtagagattcatgtcaaggagatagtgggggaccccatgttactgaagtggaagg
gaccagtttcttaactggaattattagctggggtgaagagtgtgcaatgaaaggcaaatatggaatatataccaaggtatcccggtatgtc
aactggattaaggaaaaaacaaagctcactagctccagcagcaaggcccctcccccgagcctgccctccccaagcaggctgcctgg
gcccagtgacacccctatcctgcctcagtccagctccagcaaggccccacccccctagcctgccttctccttctcggctgcctggcccca
gcgatactccaattctgccccagtcctccagcagtaaggctcccccctccatctctgccatcccccagcagactgccaggcccttctgata
cacccatcctcccacagtgatgaggatccgcggccgc (SEQ ID NO: 30).

In another embodiment, the amino acid sequence of Factor IX-$(CTP)_3$ (attached to the carboxy terminus) comprises the following amino acid sequence:

MQRVNMIMAESPGLITICLLGYLLSAECTVFLDHENANKILNRPKRYNSGKLEEFVQG
NLERECMEEKCSFEEAREVFENTERTTEFWKQYVDGDQCESNPCLNGGSCKDDINSY
ECWCPFGFEGKNCELDVTCNIKNGRCEQFCKNSADNKVVCSCTEGYRLAENQKSCE
PAVPFPCGRVSVSQTSKLTRAEAVFPDVDYVNSTEAETILDNITQSTQSFNDFTRVVG
GEDAKPGQFPWQVVLNGKVDAFCGGSIVNEKWIVTAAHCVETGVKITVVAGEHNIE
ETEHTEQKRNVIRIIPHHNYNAAINKYNHDIALLELDEPLVLNSYVTPICIADKEYTNIF
LKFGSGYVSGWGRVFHKGRSALVLQYLRVPLVDRATCLRSTKFTIYNNMFCAGFHE
GGRDSCQGDSGGPHVTEVEGTSFLTGIISWGEECAMKGKYGIYTKVSRYVNWIKEKT
KLTSSSSKAPPPSLPSPSRLPGPSDTPILPQSSSSKAPPPSLPSPSRLPGPSDTPILPQSSSS
KAPPPSLPSPSRLPGPSDTPILPQ** (SEQ ID NO: 31).

It is disclosed that the nucleic acid sequence encoding Factor IX-$(CTP)_4$ (attached to the carboxy terminus) comprises the following nucleic acid sequence:

tctagagtcgaccccgccatgcagcgcgtgaacatgatcatggcagaatcaccaggcctcatcaccatctgccttttaggatatctactc

agtgctgaatgtacagtttttcttgatcatgaaaacgccaacaaaattctgaatcggccaaagaggtataattcaggtaaattggaagagtt
tgttcaagggaaccttgagagagaatgtatggaagaaaagtgtagttttgaagaagcacgagaagtttttgaaaacactgaaagaacaa
ctgaattttggaagcagtatgttgatggagatcagtgtgagtccaatccatgtttaaatggcggcagttgcaaggatgacattaattcctat
gaatgttggtgtccctttggatttgaaggaaagaactgtgaattagatgtaacatgtaacattaagaatggcagatgcgagcagttttgtaa
aaatagtgctgataacaaggtggtttgctcctgtactgagggatatcgacttgcagaaaaccagaagtcctgtgaaccagcagtgccatt
tccatgtggaagagtttctgtttcacaaacttctaagctcacccgtgctgaggcagtttttcctgatgtggactatgtaaattctactgaagct
gaaaccattttggataacatcactcaaagcacccaatcatttaatgacttcactcgagttgttggtggagaagatgccaaaccaggtcaat
tcccttggcaggttgttttgaatggtaaagttgatgcattctgtggaggctctatcgttaatgaaaaatggattgtaactgctgcccactgtgt
tgaaactggtgttaaaattacagttgtcgcaggtgaacataatattgaggagacagaacatacagagcaaaagcgaaatgtgattcgaat
tattcctcaccacaactacaatgcagctattaataagtacaaccatgacattgcccttctggaactggacgaacccttagtgctaaacagct
acgttacacctatttgcattgctgacaaggaatacacgaacatcttcctcaaatttggatctggctatgtaagtggctggggaagagtcttc
cacaaagggagatcagctttagttcttcagtaccttagagttccacttgttgaccgagccacatgtcttcgatctacaaagttcaccatctat
aacaacatgttctgtgctggcttccatgaaggaggtagagattcatgtcaaggagatagtgggggaccccatgttactgaagtggaagg
gaccagtttcttaactggaattattagctggggtgaagagtgtgcaatgaaaggcaaatatggaatatataccaaggtatcccggtatgtc
aactggattaaggaaaaaacaaagctcactagctccagcagcaaggcccctcccccgagcctgccctccccaagcaggctgcctgg
gccctctgacacccctatcctgcctcagtccagctcctctaaggccccaccaccttccctgcctagcccttcaagactgccaggccctag
cgatacaccaattctgccccagtcctccagcagcaaggctcccccacctagcctgccttctccatcaaggctgcctggcccatccgata
ccccaattttgcctcagagcagctctagcaaggcacctcccccagtctgccctctccaagcagactccctggcccttcagacactccc
attctgccacagtgatgaggatccgcggccgc (SEQ ID NO: 32).

It is disclosed that the amino acid sequence of Factor IX-$(CTP)_4$ (attached to the carboxy terminus) comprises the following amino acid sequence:

SRVDPAMQRVNMIMAESPGLITICLLGYLLSAECTVFLDHENANKILNRPKRYNSGKL
EEFVQGNLERECMEEKCSFEEAREVFENTERTTEFWKQYVDGDQCESNPCLNGGSC
KDDINSYECWCPFGFEGKNCELDVTCNIKNGRCEQFCKNSADNKVVCSCTEGYRLA
ENQKSCEPAVPFPCGRVSVSQTSKLTRAEAVFPDVDYVNSTEAETILDNITQSTQSFN
DFTRVVGGEDAKPGQFPWQVVLNGKVDAFCGGSIVNEKWIVTAAHCVETGVKITVV
AGEHNIEETEHTEQKRNVIRIIPHHNYNAAINKYNHDIALLELDEPLVLNSYVTPICIAD
KEYTNIFLKFGSGYVSGWGRVFHKGRSALVLQYLRVPLVDRATCLRSTKFTIYNNMF
CAGFHEGGRDSCQGDSGGPHVTEVEGTSFLTGIISWGEECAMKGKYGIYTKVSRYVN
WIKEKTKLTSSSSKAPPPSLPSPSRLPGPSDTPILPQSSSSKAPPPSLPSPSRLPGPSDTPIL
PQSSSSKAPPPSLPSPSRLPGPSDTPILPQSSSSKAPPPSLPSPSRLPGPSDTPILPQ**GSA
A (SEQ ID NO: 33).

It is disclosed that the nucleic acid sequence encoding Factor IX-$(CTP)_5$ (attached to the carboxy terminus) comprises the following nucleic acid sequence:

ctagagtcgaccccgccatgcagcgcgtgaacatgatcatggcagaatcaccaggcctcatcaccatctgccttttaggatatctactcagtgctgaatgtacagtttttcttgatcatgaaaacgccaacaaaattctgaatcggccaaagaggtataattcaggtaaattggaagagtttgttcaagggaaccttgagagagaatgtatggaagaaaagtgtagttttgaagaagcacgagaagtttttgaaaacactgaaagaacaactgaattttggaagcagtatgttgatggagatcagtgtgagtccaatccatgtttaaatggcggcagttgcaaggatgacattaattcctatgaatgttggtgtccctttggatttgaaggaaagaactgtgaattagatgtaacatgtaacattaagaatggcagatgcgagcagttttgtaaaaatagtgctgataacaaggtggtttgctcctgtactgagggatatcgacttgcagaaaaccagaagtcctgtgaaccagcagtgccatttccatgtggaagagtttctgtttcacaaacttctaagctcacccgtgctgaggcagtttttcctgatgtggactatgtaaattctactgaagctgaaaccattttggataacatcactcaaagcacccaatcatttaatgacttcactcgagttgttggtggagaagatgccaaaccaggtcaattcccttggcaggttgttttgaatggtaaagttgatgcattctgtggaggctctatcgttaatgaaaaatggattgtaactgctgcccactgtgttgaaactggtgttaaaattacagttgtcgcaggtgaacataatattgaggagacagaacatacagagcaaaagcgaaatgtgattcgaattattcctcaccacaactacaatgcagctattaataagtacaaccatgacattgcccttctggaactggacgaacccttagtgctaaacagctacgttacacctatttgcattgctgacaaggaatacacgaacatcttcctcaaatttggatctggctatgtaagtggctggggaagagtcttccacaaagggagatcagctttagttcttcagtaccttagagttccacttgttgaccgagccacatgtcttcgatctacaaagttcaccatctataacaacatgttctgtgctggcttccatgaaggaggtagagattcatgtcaaggagatagtgggggaccccatgttactgaagtggaagggaccagtttcttaactggaattattagctggggtgaagagtgtgcaatgaaaggcaaatatggaatatataccaaggtatcccggtatgtcaactggattaaggaaaaaacaaagctcactagctccagcagcaaggcccctcccccgagcctgccctccccaagcaggctgcctggggccctctgacacccctatcctgcctcagtccagctcctctaaggctccaccaccttccctgcctagcccttcaagactgccaggccctagcgatacaccaattctgccccagtcctccagcagcaaggctcccccacctagcctgccttctccatcaaggctgcctggcccatccgataccccaattttgcctcagagcagctctagcaaggcacctcccccagtctgccctctccaagcagactccctggcccttcagacactccaatcctcccacagtcctctagctctaaagctccacctcccagcctgcccagccctagtagactccccggaccttctgataccccatcttgccccagtgatgaggatccgcggccgc (SEQ ID NO: 34).

It is disclosed that the amino acid sequence of Factor IX-$(CTP)_5$ (attached to the carboxy terminus) comprises the following amino acid sequence:

RVDPAMQRVNMIMAESPGLITICLLGYLLSAECTVFLDHENANKILNRPKRYNSGKL EEFVQGNLERECMEEKCSFEEAREVFENTERTTEFWKQYVDGDQCESNPCLNGGSC KDDINSYECWCPFGFEGKNCELDVTCNIKNGRCEQFCKNSADNKVVCSCTEGYRLA ENQKSCEPAVPFPCGRVSVSQTSKLTRAEAVFPDVDYVNSTEAETILDNITQSTQSFN DFTRVVGGEDAKPGQFPWQVVLNGKVDAFCGGSIVNEKWIVTAAHCVETGVKITVV AGEHNIEETEHTEQKRNVIRIIPHHNYNAAINKYNHDIALLELDEPLVLNSYVTPICIAD KEYTNIFLKFGSGYVSGWGRVFHKGRSALVLQYLRVPLVDRATCLRSTKFTIYNNMF CAGFHEGGRDSCQGDSGGPHVTEVEGTSFLTGIISWGEECAMKGKYGIYTKVSRYVN WIKEKTKLTSSSSKAPPPSLPSPSRLPGPSDTPILPQSSSSKAPPPSLPSPSRLPGPSDTPIL PQSSSSKAPPPSLPSPSRLPGPSDTPILPQSSSSKAPPPSLPSPSRLPGPSDTPILPQSSSSK APPPSLPSPSRLPGPSDTPILPQ**GSAA (SEQ ID NO: 35).

In another embodiment, furin may be added to a cell expressing the coagulation factor-CTP of the invention. It is disclosed that furin increases the production efficiency of a coagulation factor-CTP of the invention in a cell. In another embodiment, furin is co-transfected with the vector comprising the coding sequence of the coagulation factor-CTP of the invention. In another embodiment, furin is encoded by a separate vector. In another embodiment, furin and a coagulation factor-CTP are encoded by one vector. In another embodiment, the coding sequence of furin is inserted into pCI-DHFR. In another embodiment, the coding sequence of furin is engineered in pCI-dhfr/smaI+NotI, Furin/AsisI F.I.+NotI.

The nucleic acid sequence encoding furin comprises the following nucleic acid sequence:

tctagagtcgaccccgccatggagctgaggccctggttgctatgggtggtagcagcaacaggaaccttggtcctgctagcagctgatgctcagggccagaaggtcttcaccaacacgtgggctgtgcgcatccctggaggcccagcggtggccaacagtgtggcacggaagcatgggttcctcaacctgggccagatcttcggggactattaccacttctggcatcgaggagtgacgaagcggtccctgtcgcctcaccgcccgcggcacagccggctgcagagggagcctcaagtacagtggctggaacagcaggtggcaaagcgacggactaaacgggacgtgtaccaggagcccacagaccccaagtttcctcagcagtggtacctgtctggtgtcactcagcgggacctgaatgtgaaggcggcctgggcgcagggctacacagggcacggcattgtggtctccattctggacgatggcatcgagaagaaccacccggacttggcaggcaattatgatcctggggccagttttgatgtcaatgaccaggaccctgacccccagcctcggtacacacagatgaatgacaacaggcacggcacacggtgtgcgggggaagtggctgcggtggccaacaacggtgtctgtggtgtaggtgtggcctacaacgcccgcattggaggggtgcgcatgctggatggcgaggtgacagatgcagtggaggcacgctcgctgggcctgaaccccaaccacatccacatctacagtgccagctggggccccgaggatgacggcaagacagtggatgggccagcccgcctcgccgaggaggccttcttccgtggggttagccagggccgaggggggctgggctccatctttgtctgggcctcggggaacgggggccgggaacatgacagctgcaactgcgacggctacaccaacagtatctacacgctgtccatcagcagcgccacgcagtttggcaacgtgccgtggtacagcgaggcctgctcgtccacactggccacgacctacagcagtggcaaccagaatgagaagcagatcgtgacgactgacttgcggcagaagtgcacggagtctcacacgggcacctcagcctctgccccccttagcagccggcatcattgctctcaccctggaggccaataagaacctcacatggcgggacatgcaacacctggtggtacagacctcgaagccagcccacctcaatgccaacgactgggccaccaatggtgtgggccggaaagtgagccactcatatggctacgggcttttggacgcaggcgccatggtggccctggcccagaattggaccacagtggccccccagcggaagtgcatcatcgacatcctcaccgagcccaaagacatcgggaaacggctcgaggtgcggaagaccgtgaccgcgtgcctgggcgagcccaaccacatcactcggctggagcacgctcaggcgcggctcaccctgtcctataatcgccgtggcgacctggccatccacctggtcagccccatgggcacccgctccaccctgctggcagccaggccacatgactactccgcagatgggtttaatgactgggccttcatgacaactcattcctgggatgaggatccctctggcgagtgggtcctagagattgaaaacaccagcgaagccaacaactatgggacgctgaccaagttcaccctcgtactctatggcaccgcccctgaggggctgcccgtacctccagaaagcagtggctgcaagaccctcacgtccagtcaggcctgtgtggtgtgcgaggaaggcttctccctgcaccagaagagctgtgtccagcactgccctccaggcttcgcccccccaagtcctcgatacgcactatagcaccgagaatgacgtggagaccatccgggccagcgtctgcgcccccctgccacgcctcatgtgccacatgccaggggccggccctgacagactgcctcagctgccccagccacgcctccttggaccctgtggagcagacttgctcccggcaaagccagagcagccgagagtccccgccacagcagcagccacctcggctgcccccggaggtggaggcggggcaacggctgcgggcagggctgctgccctcacacctgcctgaggtggtggccggcctcagctgcgccttcatcgtgctggtcttcgtcactgtcttcctggtcctgcagctgcgctctggctttagttttcgggggtgaaggtgtacaccatggaccgtggcctcatctcctacaaggggctgcccccctgaagcctggcaggaggagtgcccgtctgactcagaagaggacgagggccggggcgagaggaccgcctttatcaaagaccagagcgccctctgaacgcggccgc (SEQ ID NO: 22).

The amino acid sequence of furin comprises the following amino acid sequence:

MELRPWLLWVVAATGTLVLLAADAQGQKVFTNTWAVRIPGGPAVANSVARKHGFL
NLGQIFGDYYHFWHRGVTKRSLSPHRPRHSRLQREPQVQWLEQQVAKRRTKRDVY
QEPTDPKFPQQWYLSGVTQRDLNVKAAWAQGYTGHGIVVSILDDGIEKNHPDLAGN
YDPGASFDVNDQDPDPQPRYTQMNDNRHGTRCAGEVAAVANNGVCGVGVAYNAR
IGGVRMLDGEVTDAVEARSLGLNPNHIHIYSASWGPEDDGKTVDGPARLAEEAFFRG
VSQGRGGLGSIFVWASGNGGREHDSCNCDGYTNSIYTLSISSATQFGNVPWYSEACS
STLATTYSSGNQNEKQIVTTDLRQKCTESHTGTSASAPLAAGIIALTLEANKNLTWRD
MQHLVVQTSKPAHLNANDWATNGVGRKVSHSYGYGLLDAGAMVALAQNWTTVA
PQRKCIIDILTEPKDIGKRLEVRKTVTACLGEPNHITRLEHAQARLTLSYNRRGDLAIH
LVSPMGTRSTLLAARPHDYSADGFNDWAFMTTHSWDEDPSGEWVLEIENTSEANNY
GTLTKFTLVLYGTAPEGLPVPPESSGCKTLTSSQACVVCEEGFSLHQKSCVQHCPPGF
APQVLDTHYSTENDVETIRASVCAPCHASCATCQGPALTDCLSCPSHASLDPVEQTCS
RQSQSSRESPPQQQPPRLPPEVEAGQRLRAGLLPSHLPEVVAGLSCAFIVLVFVTVFLV
LQLRSGFSFRGVKVYTMDRGLISYKGLPPEAWQEECPSDSEEDEGRGERTAFIKDQSA
L* (SEQ ID NO: 23).

In one embodiment of the disclosure, the term coagulation factor further includes a homologue of a known coagulation factor which has a coagulating activity. In some embodiments, homology according to the present disclosure also encompasses deletion, insertion, or substitution variants, including an amino acid substitution, thereof and biologically active polypeptide fragments thereof. In one embodiment, the variant comprises conservative substitutions, or deletions, insertions, or substitutions that do not significantly alter the three dimensional structure of the coagulation factor.

In another embodiment, homologues e.g., are polypeptides which are at least 50%, at least 55%, at least 60%, at least 65%, at least 70%, at least 75%, at least 80%, at least 85%, at least 87%, at least 89%, at least 91%, at least 93%, at least 95%, at least 96%, at least 97%, at least 98%, or at least 99% homologous as determined using BlastP software of the National Center of Biotechnology Information (NCBI) using default parameters.

In another embodiment, disclosed herein is a polypeptide consisting essentially of a coagulation factor and three CTPs attached to the carboxy terminus of the coagulation factor.

It is to be understood that the compositions for use according to the present invention comprising the elements or steps as described herein may consist of those elements or steps, or consist essentially of those elements or steps. In some embodiments, the term "comprise" refers to the inclusion of the indicated active agent, such as the CTP-modified coagulation factor, as well as inclusion of other active agents, and pharmaceutically acceptable carriers, excipients, emollients, stabilizers, etc., as are known in the pharmaceutical industry. In some embodiments, the term "consisting essentially of' refers to a composition, whose only active ingredient is the indicated active ingredient, however, other compounds may be included which are for stabilizing, preserving, etc. the formulation, but are not involved directly in the therapeutic effect of the indicated active ingredient. In some embodiments, the term "consisting essentially of' may refer to components which facilitate the release of the active ingredient. In some embodiments, the term "consisting" refers to a composition, which contains the active ingredient and a pharmaceutically acceptable carrier or excipient.

In another embodiment, the invention utilizes a polypeptide consisting of a coagulation factor having three CTPs on its carboxy terminus as described herein and no CTPs on its amino terminus.

In one embodiment, the present invention utilizes a recombinant coagulation factor as described hereinabove. In one embodiment, the present invention utilizes an engineered coagulation factor as described hereinabove. In one embodiment, the engineered coagulation factor as described hereinabove is referred to as a CTP-modified coagulation factor.

In one embodiment, the CTPs that are attached to the carboxy terminus of the coagulation factor are attached in tandem to the carboxy terminus.

In one embodiment, an engineered coagulation factor as described herein has equivalent or improved biological activity compared to the non-CTP-modified coagulation factor. In another embodiment, an engineered coagulation factor as described herein has equivalent or improved pharmacological measurements compared to the non-CTP-modified coagulation factor. In another embodiment, an engineered coagulation factor as described herein has equivalent or improved pharmacokinetics compared to the non-CTP-modified coagulation factor. In another embodiment, an engineered coagulation factor as described herein has equivalent or improved pharmacodynamics compared to the non-CTP-modified coagulation factor.

In an embodiment, the subject to be treated is a hemophilic subject. In one embodiment, the hemophilia is hemophilia B. In one embodiment, hemophilia B is known as factor IX deficiency or Christmas disease. In one embodiment, the hemophilia is severe hemophilia, which in one embodiment, describes hemophilia in which the coagulation factor levels are 0-1%. In another embodiment, the hemophilia is moderate hemophilia, which in one embodiment, describes hemophilia in which the coagulation factor levels are 1-5%. In another embodiment, the hemophilia is mild hemophilia, which in one embodiment, describes hemophilia in which the coagulation factor levels are 5-50%.

In other embodiments, the engineered coagulation factor can reduce the number of infusions required for a patient, reduce the required doses for a patient, or a combination thereof.

In one embodiment, coagulation Factor IX comprising 3 CTPs in tandem at its carboxy terminus exhibits an improved PK profile while maintaining its coagulation activity vs. FIX-CTP-CTP harvest, FIX-CTP harvest or rhFIX. In one embodiment, the elimination half-life of rFIX-CTP3 is 2.5- to 4-fold longer than rFIX in rats and in FIX-deficient mice. It is disclosed herein that the administration of rFIX-CTP3 significantly prolonged the procoagulatory effect in FIX-deficient mice for at least 76 hr after dosing. It is also disclosed herein that the administration of rFIX-CTP3 produced a higher activity peak than rFIX in FIX-deficient mice. It is also disclosed that coagulation Factor IX comprising 2 CTPs in tandem in its carboxy terminus exhibits an improved PK profile while maintaining its coagulation activity vs. FIX-CTP harvest or rhFIX and that coagulation Factor IX comprising 2 CTPs in tandem in its carboxy terminus exhibits 3-fold increase in half-life and 4.5-fold higher AUC compared to rhFIX.

In another embodiment of the invention, subcutaneous (SC) administration results in higher bioavailability of CTP-modified FVII as compared to recombinant FVII. It is disclosed that half life is longer and bioavailability (AUC SC/AUC IV) is higher following FVIIa-CTP3 and 5 SC administration when compared to SC administration of NovoSeven®. It is disclosed that subcutaneously injected MOD-5014 and MOD-5019 shows improved mice survival in comparison to recombinant FVII (NovoSeven®) (see Example 8 below).

In another embodiment, the terms "CTP peptide," "carboxy terminal peptide" and "CTP sequence" are used interchangeably herein. In another embodiment, the carboxy terminal peptide is a full-length CTP.

In another embodiment, a signal peptide may be attached to the amino terminus of the CTP, as described in US 7,553,940.

In other embodiments, the term engineered coagulation factor refers to the amino acid sequence of a matured coagulation factor. In other embodiments, the term engineered coagulation factor refers to the amino acid sequence of the coagulation factor including its signal sequence or signal peptide.

In another embodiment, "signal sequence" and "signal peptide" are used interchangeably herein. In another embodiment, "sequence" when in reference to a polynucleotide molecule can refer to a coding portion.

It is disclosed herein that an engineered coagulation factor comprising at least one CTP as described herein has enhanced in vivo biological activity compared the same coagulation factor without at least one CTP. In one embodiment, the enhanced biological activity stems from the longer half-life of the engineered coagulation factor while maintaining at least some biological activity. In another embodiment, the enhanced biological activity stems from enhanced biological activity resulting from the CTP modification. In another embodiment, the enhanced biological activity stems from both a longer half life and from enhanced functionality of the CTP-modified coagulation factor.

As disclosed herein, at least one CTP sequence at the carboxy terminal end of the coagulation factor provides enhanced protection against degradation of a coagulation factor, provides enhanced protection against clearance, provides prolonged clearance time, enhances its Cmax, enhances its Tmax, and/or prolongs its T½.

In another embodiment, a conjugated coagulation factor of this invention is used in the same manner as an unmodified conjugated coagulation factor. In another embodiment, a conjugated coagulation factor of this invention has an increased circulating half-life and plasma residence time, decreased clearance, and increased clinical activity in vivo. In another embodiment, due to the improved properties of the conjugated coagulation factor as described herein, this conjugate is administered less frequently than the unmodified form of the same coagulation factor.

In another embodiment, decreased frequency of administration will result in improved treatment strategy, which in one embodiment, will lead to improved patient compliance leading to improved treatment outcomes, as well as improved patient quality of life. In another embodiment, compared to conventional conjugates of coagulation factors, it has been found that conjugates having the molecular weight and linker structure of the conjugates of this invention have an improved potency, improved stability, elevated AUC levels, and enhanced circulating half-life.

In an embodiment, the CTP-modified coagulation factor for use according to the inventin is in the form of a pharmaceutical composition comprising a pharmaceutically acceptable carrier. In another embodiment, the pharmaceutical composition comprises a therapeutically effective amount of the conjugated coagulation factor. In one embodiment, a therapeutically effective amount of a conjugated coagulation factor is determined according to factors such as the specific condition being treated, the condition of the patient being treated, as well as the other ingredients in the composition.

In one embodiment, a CTP-modified coagulation factor of the present invention has therapeutic uses. In another embodiment, a CTP-modified coagulation factor of the present invention has prophylactic uses.

In another embodiment, a conjugated coagulation factor for use according to the invention is useful in the treatment of subjects experiencing excessive bleeding or bruising or having a prolonged Prothrombin Time (PT) or Partial Thromboplastin Time (PTT). In another embodiment, a conjugated coagulation factor for use according to the invention is useful in the treatment of subjects having an acquired condition that is causing bleeding, such as vitamin K deficiency or liver disease. In another embodiment, a conjugated coagulation factor for use according to the invention is useful in the treatment of subjects having deficiencies in coagulation factors that are acquired (due to other diseases) or inherited, mild or severe, permanent or temporary. In another embodiment, a conjugated coagulation factor for use according to the invention is useful in the treatment of subjects afflicted with hemophilia A. In another embodiment, a conjugated coagulation factor for use according to the invention is useful in the treatment of subjects afflicted with hemophilia B. In another embodiment, a conjugated coagulation factor for use according to the invention is useful in the treatment of subjects having acquired deficiencies due to chronic diseases, such as liver disease or cancer; to an acute condition such as disseminated intravascular coagulation (DIC), which uses up clotting factors at a rapid rate; or to a deficiency in vitamin K or treatment with a vitamin K antagonist like warfarin (the production of factors II, VII, IX, and X require vitamin K). In another embodiment, a conjugated coagulation factor for use according to the invention is useful in the treatment of subjects afflicted with a disease in which causes clotting imbalances such as but not limited to: a liver disease, uremia, a cancer, a bone marrow disorder, an exposure to snake venom, a vitamin K deficiency, an anticoagulation therapy, an accidental ingestion of the anticoagulant warfarin, multiple blood transfusions (stored units of blood lose some of their clotting factors), or a combination thereof.

In one embodiment, the compositions for use according to the present invention provide therapeutic or prophylactic treatment of hemorrhage, which in one embodiment, is uncontrollable hemorrhage, and, in another embodiment, intracerebral hemorrhage. In another embodiment, the compositions for use according to the present invention provide therapeutic or prophylactic treatment of neonatal coagulopathies; severe hepatic disease; high-risk surgical procedures; traumatic blood loss; bone marrow transplantation; thrombocytopenias and platelet function disorders; urgent reversal of oral anticoagulation; congenital deficiencies of factors V, VII, X, and XI; or von Willebrand disease, in one embodiment, von Willebrand disease with inhibitors to von Willebrand factor.

In one embodiment, the subject is human. In another embodiment, the subject is a domesticated animal. In another embodiment, the subject is a mammal. In another embodiment, the subject is a farm animal. In another embodiment, the subject is a monkey. In another embodiment, the subject is a horse. In another embodiment, the subject is a cow. In another embodiment, the subject is a mouse. In another embodiment, the subject is a rat. In another embodiment, the subject is canine. In another embodiment, the subject is feline. In another embodiment, the subject is bovine, ovine, porcine, equine, murine, or cervine. In one embodiment, the subject is male. In another embodiment, the subject is female. In one embodiment, the subject is a child, in another embodiment, an adolescent, in another embodiment, an adult or, in another embodiment, an elderly subject. In another embodiment, the subject is a pediatric subject, in another embodiment, a geriatric subject.

A [(CTP)n>1-coagulation factor] as described herein may comprise a full length coagulation factor or an active fragment thereof connected via a peptide bond on its carboxy terminus to at least one CTP unit with no CTPs on its amino terminus. A [(CTP)n>1-coagulation factor] as described herein may also comprise a coagulation factor or an active fragment thereof connected via a peptide bond to at least one CTP unit which is connected to an additional CTP unit via a peptide bond with no CTPs on its amino terminus. In another embodiment, one nucleic acid molecule encodes an engineered coagulation factor comprising at least one CTP attached to its C-terminus and no CTPs on its amino terminus.

In another embodiment, the CTP is attached to the coagulation factor via a linker. In another embodiment, the linker which connects the CTP sequence to the coagulation factor is a covalent bond. In another embodiment, the linker which connects the CTP sequence to the coagulation factor is a peptide bond. In another embodiment, the linker which connects the CTP sequence to the coagulation factor is a substituted peptide bond. In another embodiment, the CTP sequence comprises: DPRFQDSSSSKAPPPSLPSPSRLPGPSDTPIL (SEQ ID NO: 1). In another embodiment, the CTP sequence comprises: SSSSKAPPPSLPSPSRLPGPSDTPILPQ (SEQ ID NO: 2). In another embodiment, the CTP sequence comprises an amino acid sequence selected from the sequences set forth in SEQ ID NO: 1 and SEQ ID NO: 2.

In another embodiment, the carboxy terminal peptide (CTP) peptide of the present invention comprises the amino acid sequence from amino acid 112 to position 145 of human chorionic gonadotrophin, as set forth in SEQ ID NO: 1. In another embodiment, the CTP sequence of the present invention comprises the amino acid sequence from amino acid 118 to position 145 of human chorionic gonadotropin, as set forth in SEQ ID NO: 2. In another embodiment, the CTP sequence also commences from any position between positions 112-118 and terminates at position 145 of human chorionic gonadotrophin. In some embodiments, the CTP sequence peptide is 28, 29, 30, 31, 32, 33 or 34 amino acids long and commences at position 112, 113, 114, 115, 116, 117 or 118 of the CTP amino acid sequence.

In another embodiment, the CTP peptide is a variant of chorionic gonadotrophin CTP which differs from the native CTP by 1-5 conservative amino acid substitutions as described in U.S. Pat. No. 5,712,122. In another embodiment, the CTP peptide is a variant of chorionic gonadotrophin CTP which differs from the native CTP by 1 conservative amino acid substitution. In another embodiment, the CTP peptide is a variant of chorionic gonadotrophin CTP which differs from the native CTP by 2 conservative amino acid substitutions. In another embodiment, the CTP peptide is a variant of chorionic gonadotrophin CTP which differs from the native CTP by 3 conservative amino acid substitutions. In another embodiment, the CTP peptide is a variant of chorionic gonadotrophin CTP which differs from the native CTP by 4 conservative amino acid substitutions. In another embodiment, the CTP peptide is a variant of chorionic gonadotrophin CTP which differs from the native CTP by 5 conservative amino acid substitutions.

In another embodiment, the CTP peptide amino acid sequence of the present invention is at least 70%, or at least 80%, or at least 90%, or at least 95%, or at least 98% homologous to the native CTP amino acid sequence or a peptide thereof.

In another embodiment, the polynucleotide encoding the CTP peptide of the present invention is at least 70%, or at least 80%, or at least 90%, or at least 95%, or at least 98% homologous to the native CTP DNA sequence or a peptide-coding fragment thereof.

In one embodiment, at least one of the chorionic gonadotrophin CTP amino acid sequences may be truncated. In another embodiment, 2 of the chorionic gonadotrophin CTP amino acid sequences are truncated. In another embodiment, all 3 of the chorionic gonadotrophin CTP amino acid sequences are truncated. In one embodiment, the truncated CTP comprises the first 10 amino acids of SEQ ID NO: 3. SEQ ID NO: 3 comprises the following amino acid (AA) sequence: SSSSKAPPPSLP.

In one embodiment, the truncated CTP comprises the first 10 amino acids of SEQ ID NO: 4. SEQ ID NO: 4 comprises the following amino acid (AA) sequence: SSSSKAPPPSLPSPSRLPGPSDTPILPQ.

In one embodiment, the truncated CTP comprises the first 11 amino acids of SEQ ID NO: 4 or the first 12 amino acids of SEQ ID NO: 4. In one embodiment, the truncated CTP comprises the first 8 amino acids of SEQ ID NO: 4 or SEQ ID NO: 3. In one embodiment, the truncated CTP comprises the first 13 amino acids of SEQ ID NO: 4 or the first 14 amino acids of SEQ ID NO: 4. In one embodiment, the truncated CTP comprises the first 6 amino acids of SEQ ID NO: 4 or SEQ ID NO: 3. In one embodiment, the truncated CTP comprises the first 5 amino acids of SEQ ID NO: 4 or SEQ ID NO: 3.

In one embodiment, at least one of the chorionic gonadotrophin CTP amino acid sequences is glycosylated. In another embodiment, 2 of the chorionic gonadotrophin CTP amino acid sequences are glycosylated. In another embodiment, all 3 of the chorionic gonadotrophin CTP amino acid sequences are glycosylated.

In one embodiment, the CTP sequence of the present invention comprises at least one glycosylation site. In one embodiment, the CTP sequence comprises 2 glycosylation sites. In one embodiment, the CTP sequence comprises 3 glycosylation sites. In one embodiment, the CTP sequence comprises 4 glycosylation sites. In one embodiment, one or more of the chorionic gonadotrophin CTP amino acid sequences is fully glycosylated. In another embodiment, one or more of the chorionic gonadotrophin CTP amino acid sequences is partially glycosylated. In one embodiment, partially glycosylated indicates that one of the CTP glycosylation sites is glycosylated. In another embodiment, two of the CTP glycosylation sites are glycosylated. In another embodiment, three of the CTP glycosylation sites are glycosylated.

In some embodiments, the CTP sequence modification is advantageous in permitting the usage of lower dosages. In some embodiments, the CTP sequences modification is advantageous in permitting fewer dosages. In some embodiments, the CTP sequences modification is advantageous in permitting a safe, long-acting effect.

In some embodiments, "polypeptide", "engineered coagulation factor", or "protein" as used herein encompasses native polypeptides (either degradation products, synthetically synthesized polypeptides or recombinant polypeptides) and peptidomimetics (typically, synthetically synthesized polypeptides), as well as peptoids and semipeptoids which are polypeptide analogs, which have, in some embodiments, modifications rendering the polypeptides comprising a coagulation factor even more stable while in a body or more capable of penetrating into cells.

As used herein, the term "amino acid" or "amino acid sequence" is understood to include the 20 naturally occurring amino acid; those amino acid often modified post-translationally in vivo, including, for example, hydroxyproline, phosphoserine and phosphothreonine; and other unusual amino acid including, but not limited to, 2-aminoadipic acid, hydroxylysine, isodesmosine, nor-valine, nor-leucine and ornithine. In one embodiment, "amino acid" includes both D- and L-amino acids.

In some embodiments, the polypeptides of the present invention are utilized in therapeutics which requires the polypeptides comprising a coagulation factor to be in a soluble form. In some embodiments, the polypeptides of the present invention include one or more non-natural or natural polar amino acid, including but not limited to serine and threonine which are capable of increasing polypeptide solubility due to their hydroxyl-containing side chain.

In some embodiments, the engineered coagulation factors of the present invention are biochemically synthesized such as by using standard solid phase techniques. In some embodiments, these biochemical methods include exclusive solid phase synthesis, partial solid phase synthesis, fragment condensation, or classical solution synthesis.

In some embodiments, recombinant protein techniques are used to generate the engineered coagulation factors of the present invention. In some embodiments, recombinant protein techniques are used for the generation of relatively long polypeptides (e.g., longer than 18-25 amino acids). In some embodiments, recombinant protein techniques are used for the generation of large amounts of the engineered coagulation factors of the present invention. In some embodiments, recombinant techniques are described by Bitter et al., (1987) Methods in Enzymol. 153:516-544, Studier et al. (1990) Methods in Enzymol. 185:60-89, Brisson et al. (1984) Nature 310:511-514, Takamatsu et al. (1987) EMBO J. 6:307-311, Coruzzi et al. (1984) EMBO J. 3:1671-1680 and Brogli et al., (1984) Science 224:838-843, Gurley et al. (1986) Mol. Cell. Biol. 6:559-565 and Weissbach & Weissbach, 1988, Methods for Plant Molecular Biology, Academic Press, NY, Section VIII, pp 421-463.

The present application discloses a method of producing a CTP-modified Factor IX (FIX) polypeptide, comprising the step of attaching three chorionic gonadotrophin carboxy terminal peptides (CTPs) to the carboxy terminus of said FIX polypeptide, thereby producing a CTP-modified FIX polypeptide. The application also discloses a method of producing a CTP-modified Factor VIIa (FVIIa) polypeptide, comprising the step of attaching three chorionic gonadotrophin carboxy terminal peptides (CTPs) to the carboxy terminus of said FVIIa polypeptide, thereby producing a CTP-modified FVIIa polypeptide.

In another embodiment, the engineered coagulation factors of the present invention may be synthesized using a polynucleotide molecule encoding the polypeptide. In some embodiments, the polynucleotide molecule encoding the engineered coagulation factors is ligated into an expression vector, comprising a transcriptional control of a cis-regulatory sequence (e.g., promoter sequence). In some embodiments, the cis-regulatory sequence is suitable for directing constitutive expression of an engineered coagulation factor or for directing inducible expression of the engineered coagulation factors.

In one embodiment, following expression and secretion, the signal peptides are cleaved from the precursor engineered coagulation factors resulting in the mature engineered coagulation factors.

In some embodiments, the polynucleotides may be prepared using PCR techniques, or any other method or procedure known to one skilled in the art. In some embodiments, the procedure involves the ligation of two different DNA sequences (See, for example, "Current Protocols in Molecular Biology", eds. Ausubel et al., John Wiley & Sons, 1992).

In one embodiment, polynucleotides which encode the engineered coagulation factors are inserted into expression vectors (i.e., a nucleic acid construct) to enable expression of the recombinant polypeptide. In one embodiment, the expression vector includes additional sequences which render this vector suitable for replication and integration in prokaryotes. In another embodiment, the expression vector includes additional sequences which render this vector suitable for replication and integration in eukaryotes. In one embodiment, the expression vector includes a shuttle vector which renders this vector suitable for replication and integration in both prokaryotes and eukaryotes. In some embodiments, cloning vectors comprise transcription and translation initiation sequences (e.g., promoters, enhances) and transcription and translation terminators (e.g., polyadenylation signals).

A variety of prokaryotic or eukaryotic cells can be used as host-expression systems to express the coagulation factors. These include, but are not limited to, microorganisms, such as bacteria transformed with a recombinant bacteriophage DNA, plasmid DNA or cosmid DNA expression vector containing the polypeptide coding sequence; yeast transformed with recombinant yeast expression vectors containing the polypeptide coding sequence; plant cell systems infected with recombinant virus expression vectors (e.g., cauliflower mosaic virus, CaMV; tobacco mosaic virus, TMV) or transformed with recombinant plasmid expression vectors, such as Ti plasmid, containing the polypeptide coding sequence.

In some embodiments, non-bacterial expression systems are used (e.g. mammalian expression systems such as CHO cells) to express the coagulation factors of the present invention. In one embodiment, the expression vector used to express polynucleotides in mammalian cells is pCI-DHFR vector comprising a CMV promoter and a neomycin resistance gene. Construction of the pCI-dhfr vector is described, according to one embodiment, in Example 1.

In bacterial systems a number of expression vectors can be advantageously selected depending upon the use intended for the polypeptide expressed. In one embodiment, large quantities of polypeptide are desired. Vectors that direct the expression of high levels of the protein product, possibly as a fusion with a hydrophobic signal sequence, which directs the expressed product into the periplasm of the bacteria or the culture medium where the protein product is readily purified may be desired. Certain fusion proteins are engineered with a specific cleavage site to aid in recovery of the polypeptide. Vectors adaptable to such manipulation include, but are not limited to, the pET series of E. coli expression vectors [Studier et al., Methods in Enzymol. 185:60-89 (1990)].

In one embodiment, yeast expression systems are used. A number of vectors containing constitutive or inducible promoters can be used in yeast as disclosed in U.S. Pat. Application. No: 5,932,447. Vectors which promote integration of foreign DNA sequences into the yeast chromosome may also be used.

The expression vector can further include additional polynucleotide sequences that allow, for example, the translation of several proteins from a single mRNA such as an internal ribosome entry site (IRES) and sequences for genomic integration of the promoter-chimeric polypeptide.

Suitable mammalian expression vectors include, but are not limited to, pcDNA3, pcDNA3.1(+/-), pGL3, pZeoSV2(+/-), pSecTag2, pDisplay, pEF/myc/cyto, pCMV/myc/cyto, pCR3.1, pSinRep5, DH26S, DHBB, pNMT1, pNMT41, pNMT81, which are available from Invitrogen, pCI which is available from Promega, pMbac, pPbac, pBK-RSV and pBK-CMV which are available from Strategene, pTRES which is available from Clontech, and their derivatives.

Expression vectors containing regulatory elements from eukaryotic viruses such as retroviruses are used in the present invention. SV40 vectors include pSVT7 and pMT2. It is also disclosed that vectors derived from bovine papilloma virus include pBV-1MTHA, and vectors derived from Epstein Bar virus include pHEBO, and p2O5. Other exemplary vectors include pMSG, pAV009/A+, pMTO10/A+, pMAMneo-5, baculovirus pDSVE, and any other vector allowing expression of proteins under the direction of the SV-40 early promoter, SV-40 later promoter, metallothionein promoter, murine mammary tumor virus promoter, Rous sarcoma virus promoter, polyhedrin promoter, or other promoters shown effective for expression in eukaryotic cells.

In some embodiments, recombinant viral vectors are useful for *in vivo* expression of the coagulation factors since they offer advantages such as lateral infection and targeting specificityIn one embodiment, viral vectors are produced that are unable to spread laterally; this characteristic can be useful if the desired purpose is to introduce a specified gene into only a localized number of targeted cells.

Various methods can be used to introduce the expression vector into cells. Such methods are generally described in Sambrook et al., Molecular Cloning: A Laboratory Manual, Cold Springs Harbor Laboratory, New York (1989, 1992), in Ausubel et al., Current Protocols in Molecular Biology, John Wiley and Sons, Baltimore, Md. (1989), Chang et al., Somatic Gene Therapy, CRC Press, Ann Arbor, Mich. (1995), Vega et al., Gene Targeting, CRC Press, Ann Arbor Mich. (1995), Vectors: A Survey of Molecular Cloning Vectors and Their Uses, Butterworths, Boston Mass. (1988) and Gilboa et at. [Biotechniques 4 (6): 504-512, 1986] and include, for example, stable or transient transfection, lipofection, electroporation and infection with recombinant viral vectors. In addition, see U.S. Pat. Nos. 5,464,764 and 5,487,992 for positive-negative selection methods.

It will be appreciated that other than containing the necessary elements for the transcription and translation of the inserted coding sequence (encoding the polypeptide), the expression construct of the present invention can also include sequences engineered to optimize stability, production, purification, yield or activity of the expressed polypeptide.

In some embodiments, transformed cells are cultured under effective conditions, which allow for the expression of high amounts of recombinant engineered coagulation factors. In some embodiments, effective culture conditions include, but are not limited to, effective media, bioreactor, temperature, pH and oxygen conditions that permit protein production. In one embodiment, an effective medium refers to any medium in which a cell is cultured to produce the recombinant polypeptide of the present invention. In some embodiments, a medium typically includes an aqueous solution having assimilable carbon, nitrogen and phosphate sources, and appropriate salts, minerals, metals and other nutrients, such as vitamins. In some embodiments, the cells can be cultured in conventional fermentation bioreactors, shake flasks, test tubes, microtiter dishes and petri plates. In some embodiments, culturing is carried out at a temperature, pH and oxygen content appropriate for a recombinant cell. The determination of culturing conditions are within the expertise of one of ordinary skill in the art.

Depending on the vector and host system used for production, resultant engineered coagulation factors of the present invention either remain within the recombinant cell, are secreted into the fermentation medium, are secreted into a space between two cellular membranes, such as the periplasmic space in E. coli; or are retained on the outer surface of a cell or viral membrane.

In one embodiment, following a predetermined time in culture, recovery of the recombinant engineered coagulation factor is effected. The phrase "recovering the recombinant engineered coagulation factor" used herein may refer to collecting the whole fermentation medium containing the polypeptide and need not imply additional steps of separation or purification.

In one embodiment, engineered coagulation factors of the present invention are purified using a variety of standard protein purification techniques, such as, but not limited to, affinity chromatography, ion exchange chromatography, filtration, electrophoresis, hydrophobic interaction chromatography, gel filtration chromatography, reverse phase chromatography, concanavalin A chromatography, chromatofocusing and differential solubilization.

In one embodiment, to facilitate recovery, the expressed coding sequence can be engineered to encode the engineered coagulation factor of the present invention and fused cleavable moiety. In one embodiment, a fusion protein can be designed so that the polypeptide can be readily isolated by affinity chromatography; e.g., by immobilization on a column specific for the cleavable moiety. In one embodiment, a cleavage site is engineered between the engineered coagulation factor and the cleavable moiety and the polypeptide can be released from the chromatographic column by treatment with an appropriate enzyme or agent that specifically cleaves the fusion protein at this site [e.g., see Booth et al., Immunol. Lett. 19:65-70 (1988); and Gardella et al., J. Biol. Chem. 265:15854-15859 (1990)].

In one embodiment, the engineered coagulation factor of the present invention is retrieved in "substantially pure" form. In one embodiment, the phrase "substantially pure" refers to a purity that allows for the effective use of the protein in the applications described herein.

In one embodiment, the engineered coagulation factor of the present invention can also be synthesized using in vitro expression systems. In one embodiment, in vitro synthesis methods are well known in the art and the components of the system are commercially available.

In some embodiments, the recombinant engineered coagulation factors are synthesized and purified; their therapeutic efficacy can be assayed either in vivo or in vitro. In one embodiment, the binding activities of the recombinant engineered coagulation factors can be ascertained using various assays as known to one of skill in the art.

It is disclosed herein that the engineered coagulation factor can be provided to the individual per se. In one embodiment, the engineered coagulation factor of the present invention can be provided to the individual as part of a pharmaceutical composition where it is mixed with a pharmaceutically acceptable carrier.

In another embodiment, a "pharmaceutical composition" refers to a preparation of one or more of the active ingredients described herein with other chemical components such as physiologically suitable carriers and excipients. The purpose of a pharmaceutical composition is to facilitate administration of a compound to an organism.

In another embodiment, "active ingredient" refers to the polypeptide sequence of interest, which is accountable for the biological effect.

The disclosure provides combined preparations where "a combined preparation" defines especially a "kit of parts" in the sense that the combination partners as defined above can be dosed independently or by use of different fixed combinations with distinguished amounts of the combination partners i.e., simultaneously, concurrently, separately or sequentially. The parts of the kit of parts can then, e.g., be administered simultaneously or chronologically staggered, that is at different time points and with equal or different time intervals for any part of the kit of parts. The ratio of the total amounts of the combination partners can be administered in the combined preparation.The combined preparation can be varied, e.g., in order to cope with the needs of a patient subpopulation to be treated or the needs of the single patient which different needs can be due to a particular disease, severity of a disease, age, sex, or body weight as can be readily made by a person skilled in the art.

In another embodiment, the phrases "physiologically acceptable carrier" and "pharmaceutically acceptable carrier" which are interchangeably used refer to a carrier or a diluent that does not cause significant irritation to an organism and does not abrogate the biological activity and properties of the administered compound. An adjuvant is included under these phrases. In one embodiment, one of the ingredients included in the pharmaceutically acceptable carrier can be for example polyethylene glycol (PEG), a biocompatible polymer with a wide range of solubility in both organic and aqueous media (Mutter et al. (1979)).

In another embodiment, "excipient" refers to an inert substance added to a pharmaceutical composition to further facilitate administration of an active ingredient. In one embodiment, excipients include calcium carbonate, calcium phosphate, various sugars and types of starch, cellulose derivatives, gelatin, vegetable oils and polyethylene glycols.

Techniques for formulation and administration of drugs are found in "Remington's Pharmaceutical Sciences," Mack Publishing Co., Easton, PA, latest edition.

Various embodiments of dosage ranges are contemplated by this invention. The dosage of the engineered coagulation factor of the present invention, in one embodiment, is in the range of 0.005-100 mg/day. In another embodiment, the dosage is in the range of 0.005-5 mg/day. In another embodiment, the dosage is in the range of 0.01-50 mg/day. In another embodiment, the dosage is in the range of 0.1-20 mg/day. In another embodiment, the dosage is in the range of 0.1-10 mg/day. In another embodiment, the dosage is in the range of 0.01-5 mg/day. In another embodiment, the dosage is in the range of 0.001-0.01 mg/day. In another embodiment, the dosage is in the range of 0.001-0.1 mg/day. In another embodiment, the dosage is in the range of 0.1-5 mg/day. In another embodiment, the dosage is in the range of 0.5-50 mg/day. In another embodiment, the dosage is in the range of 0.2-15mg/day. In another embodiment, the dosage is in the range of 0.8-65 mg/day. In another embodiment, the dosage is in the range of 1-50 mg/day. In another embodiment, the dosage is in the range of 5-10 mg/day. In another embodiment, the dosage is in the range of 8-15 mg/day. In another embodiment, the dosage is in a range of 10-20mg/day. In another embodiment, the dosage is in the range of 20-40 mg/day. In another embodiment, the dosage is in a range of 60-120 mg/day. In another embodiment, the dosage is in the range of 12-40 mg/day. In another embodiment, the dosage is in the range of 40-60 mg/day. In another embodiment, the dosage is in a range of 50-100mg/day. In another embodiment, the dosage is in a range of 1-60 mg/day. In another embodiment, the dosage is in the range of 15-25 mg/day. In another embodiment, the dosage is in the range of 5-10 mg/day. In another embodiment, the dosage is in the range of 55-65 mg/day.

In another embodiment, the dosage is in a range of 50-500 mg/day. In another embodiment, the dosage is in a range of 50-150 mg/day. In another embodiment, the dosage is in a range of 100-200 mg/day. In another embodiment, the dosage is in a range of 150-250 mg/day. In another embodiment, the dosage is in a range of 200-300 mg/day. In another embodiment, the dosage is in a range of 250-400 mg/day. In another embodiment, the dosage is in a range of 300-500 mg/day. In another embodiment, the dosage is in a range of 350-500 mg/day.

In one embodiment, the dosage is 20 mg/day. In one embodiment, the dosage is 30 mg/day. In one embodiment, the dosage is 40 mg/day. In one embodiment, the dosage is 50 mg/day. In one embodiment, the dosage is 0.01 mg/day. In another embodiment, the dosage is 0.1 mg/day. In another embodiment, the dosage is 1 mg/day. In another embodiment, the dosage is 0.530 mg/day. In another embodiment, the dosage is 0.05 mg/day. In another embodiment, the dosage is 50 mg/day. In another embodiment, the dosage is 10 mg/day. In another embodiment, the dosage is 20-70 mg/day. In another embodiment, the dosage is 5 mg/day.

In one embodiment, the dosage of the CTP-modified coagulation factor is 1-5 mg/day. In one embodiment, the dosage of the CTP-modified coagulation factor is 1-3 mg/day. In another embodiment, the dosage of the CTP-modified coagulation factor is 2 mg/day.

In another embodiment, the dosage is 1-90 mg/day. In another embodiment, the dosage is 1-90 mg/2 days. In another embodiment, the dosage is 1-90 mg/3 days. In another embodiment, the dosage is 1-90 mg/4 days. In another embodiment, the dosage is 1-90 mg/5 days. In another embodiment, the dosage is 1-90 mg/6 days. In another embodiment, the dosage is 1-90 mg/week. In another embodiment, the dosage is 1-90 mg/9 days. In another embodiment, the dosage is 1-90 mg/11 days. In another embodiment, the dosage is 1-90 mg/14 days.

In another embodiment, the coagulation factor dosage is 10-50 mg/day. In another embodiment, the dosage is 10-50 mg/2 days. In another embodiment, the dosage is 10-50 mg/3 days. In another embodiment, the dosage is 10-50 mg/4 days. In another embodiment, the dosage is 10-50 micrograms mg/5 days. In another embodiment, the dosage is 10-50 mg/6 days. In another embodiment, the dosage is 10-50 mg/week. In another embodiment, the dosage is 10-50 mg/9 days. In another embodiment, the dosage is 10-50 mg/11 days. In another embodiment, the dosage is 10-50 mg/14 days.

In another embodiment, a polypeptide comprising the CTP-modified coagulation factor is formulated in an intranasal dosage form. In another embodiment, a polypeptide comprising the CTP-modified coagulation factor is formulated in an injectable dosage form. In another embodiment, a polypeptide comprising the CTP-modified coagulation factor is administered to a subject in a dose ranging from 0.0001 mg to 0.6 mg. In another embodiment, a polypeptide comprising the CTP-modified coagulation factor is administered to a subject in a dose ranging from 0.001 mg to 0.005 mg. In another embodiment, a polypeptide comprising the CTP-modified coagulation factor is administered to a subject in a dose ranging from 0.005 mg to 0.01 mg. In another embodiment, a polypeptide comprising the CTP-modified coagulation factor is administered to a subject in a dose ranging from 0.01 mg to 0.3 mg. In another embodiment, a polypeptide comprising the CTP-modified coagulation factor is administered to a subject in a dose ranging from 0.2 mg to 0.6 mg. In another embodiment, the coagulation factor is free of CTPs on its amino terminus.

It is disclosed that a polypeptide comprising the CTP-modified coagulation factor is administered to a subject in a dose ranging from 1-100 micrograms, or in a dose ranging from 10-80 micrograms, or in a dose ranging from 20-60 micrograms, or in a dose ranging from 10-50 micrograms, or in a dose ranging from 40-80 micrograms, or in a dose ranging from 10-30 micrograms, or in a dose ranging from 30-60 micrograms.

In another embodiment, a polypeptide comprising the CTP-modified coagulation factor is administered to a subject in a dose ranging from 0.2 mg to 2 mg, or in a dose ranging from 2 mg to 6 mg, or in a dose ranging from 4 mg to 10 mg, or in a dose ranging from 5 mg and 15 mg.

In one embodiment, the dosage of the CTP-modified FIX comprises 50% of the amount of FIX administered in the recommended dosage of recombinant FIX (e.g., Benefix®, Wyeth or Mononine®, CSL Behring) to patients over the same period of time. In another embodiment, the dosage of the CTP-modified FVIIa comprises 50% of the amount of FVIIa administered in the recommended dosage of recombinant FVIIa (e.g., NovoSeven®) to patients over the same period of time. In one embodiment, the dosage of the CTP-modified FVII comprises 50% of the amount of FVII administered in the recommended dosage of recombinant FVII to patients over the same period of time. For example, if NovoSeven® is given at a dose of 90 mcg/kg every two hours to a patient pre- or post-operatively (i.e., 7.65 mg every two hours or 45.9 mg in six doses over a 12 hour period, for an 85 kg patient), a CTP-modified coagulation factor of the present invention may be given at a dose that is 50% of the patient's 12-hour dose of recombinant FVIIa (i.e., at a dose of 23 mg given once over a 12-hour period).

In another embodiment, the dosage of CTP-modified coagulation factor is such that it contains 45% of the amount of the coagulation factor than that administered using the non-CTP-modified coagulation factor. In another embodiment, the dosage of CTP-modified coagulation factor is such that it contains 10% of the amount of the coagulation factor than that administered using the non-CTP-modified coagulation factor. In another embodiment, the dosage of CTP-modified coagulation factor is such that it contains 25% of the amount of the coagulation factor than that administered using the non-CTP-modified coagulation factor. In another embodiment, the dosage of CTP-modified coagulation factor is such that it contains 35% of the amount of the coagulation factor than that administered using the non-CTP-modified coagulation factor. In another embodiment, the dosage of CTP-modified coagulation factor is such that it contains 75% of the amount of the coagulation factor than that administered using the non-CTP-modified coagulation factor. In another embodiment, the dosage of CTP-modified coagulation factor is such that it contains 100% of the amount of the coagulation factor than that administered using the non-CTP-modified coagulation factor. However, even if the dosage contains the same amount of coagulation factor (e.g. FIX) as non-CTP-modified coagulation factor, it is still advantageous to subjects in that it will be administered less frequently because of its increased half-life compared to recombinant coagulation factors.

In another embodiment, a therapeutically effective amount of a conjugated coagulation factor is between 50-500 IU per kg body weight administered once a day to once a week for FIX or 10μg/Kg-500μg/Kg for FVIIa. In another embodiment, a therapeutically effective amount of a conjugated coagulation factor is 150-250 IU per kg body weight, administered once a day. In another embodiment, a pharmaceutical composition comprising a conjugated coagulation factor is formulated at a strength effective for administration by various means to a human patient.

In one embodiment, FIX is administered in an amount effective to bring circulating Factor IX activity to 20-30 IU/dL in a subject. In another embodiment, FIX is administered in an amount effective to bring circulating Factor IX activity to 25-50 IU/dL in a subject. In another embodiment, FIX is administered in an amount effective to bring circulating Factor IX activity to 50-100 IU/dL in a subject. In another embodiment, FIX is administered in an amount effective to bring circulating Factor IX activity to 100-200 IU/dL in a subject. In another embodiment, FIX is administered in an amount effective to bring circulating Factor IX activity to 10-50 IU/dL in a subject. In another embodiment, FIX is administered in an amount effective to bring circulating Factor IX activity to 20-100 IU/dL in a subject.

In one embodiment, the CTP-modified coagulation factor is administered to a subject on a weekly basis. In another embodiment, the CTP-modified coagulation factor is administered to a subject twice a week. In another embodiment, the CTP-modified coagulation factor is administered to a subject on a fortnightly (once every two weeks) basis. In another embodiment, the CTP-modified coagulation factor is administered to a subject twice a month. In another embodiment, the CTP-modified coagulation factor is administered to a subject once a month. In another embodiment, the CTP-modified coagulation factor is administered to a subject on a daily basis. In another embodiment, the CTP-modified coagulation factor is administered to a subject every two days.

In another embodiment, the CTP-modified coagulation factor is administered to a subject once every three days, once every four days, once every five days, once every six days, once every 7-14 days, once every 10-20 days, once every 5-15 days, or once every 15-30 days.

It is disclosed that the present invention provides a way to reduce the required dosing frequency of a Factor IX (FIX) polypeptideor of a Factor VIIa (FVIIa) polypeptide.

It is disclosed that the invention results in increasing the compliance in the use of coagulation factor therapy. The term compliance comprises adherence. It is also disclosed that the invention increases the compliance of patients in need of a coagulation factor therapy by reducing the frequency of administration of the coagulation factor. It is also disclosed that reduction in the frequency of administration of the coagulation factor is achieved due to the CTP modifications which render the CTP-modified coagulation factor more stable. It is also disclosed that reduction in the frequency of administration of the coagulation factor is achieved as a result of increasing T½ of the coagulation factor. It is also disclosed that reduction in the frequency of administration of the coagulation factor is achieved as a result of increasing the clearance time or reducing the clearance rate of the coagulation factor.

It is disclosed that the reduction in the required frequency of administration of the coagulation factor is achieved as a result of increasing the AUC measure of the coagulation factor.

In another embodiment, the present invention shows that the compositions provided herein are surprisingly more effectively absorbed into the bloodstream after SC administration (see Examples 7-9 herein). To be able to administer FVIIa subcutaneously serves as an advantage as it can be used for prophylactic applications. Subcutaneous injections are also much easier for patients to self-inject, and are advantage when the patients are very young and their veins are small and difficult to find.

Oral administration, in one embodiment, comprises a unit dosage form comprising tablets, capsules, lozenges, chewable tablets, suspensions, emulsions and the like. Such unit dosage forms comprise a safe and effective amount of the desired coagulation factor of the invention, each of which is in one embodiment, from about 0.7 or 3.5 mg to about 280 mg/70 kg, or in another embodiment, about 0.5 or 10 mg to about 210 mg/70 kg. The pharmaceutically-acceptable carriers suitable for the preparation of unit dosage forms for peroral administration are well-known in the art. In some embodiments, tablets typically comprise conventional pharmaceutically-compatible adjuvants as inert diluents, such as calcium carbonate, sodium carbonate, mannitol, lactose and cellulose; binders such as starch, gelatin and sucrose; disintegrants such as starch, alginic acid and croscarmelose; lubricants such as magnesium stearate, stearic acid and talc. In one embodiment, glidants such as silicon dioxide can be used to improve flow characteristics of the powder-mixture. In one embodiment, coloring agents, such as the FD&C dyes, can be added for appearance. Sweeteners and flavoring agents, such as aspartame, saccharin, menthol, peppermint, and fruit flavors, are useful adjuvants for chewable tablets. Capsules typically comprise one or more solid diluents disclosed above. In some embodiments, the selection of carrier components depends on secondary considerations like taste, cost, and shelf stability, which are not critical for the purposes of this invention, and can be readily made by a person skilled in the art.

In one embodiment, the oral dosage form comprises a predefined release profile. In one embodiment, the oral dosage form comprises extended release tablets, capsules, lozenges or chewable tablets. In one embodiment, the oral dosage form of the present invention comprises a slow release tablets, capsules, lozenges or chewable tablets. In one embodiment, the oral dosage form comprises an immediate release tablets, capsules, lozenges or chewable tablets. In one embodiment, the oral dosage form is formulated according to the desired release profile of the pharmaceutical active ingredient as known to one skilled in the art.

Peroral compositions, in some embodiments, comprise liquid solutions, emulsions, suspensions, and the like. In some embodiments, pharmaceutically-acceptable carriers suitable for preparation of such compositions are well known in the art. In some embodiments, liquid oral compositions comprise from about 0.001% to about 0.933% of the desired compound or compounds, or in another embodiment, from about 0.01% to about 10%.

In some embodiments, compositions for medical use of this invention comprise solutions or emulsions, which can be aqueous solutions or emulsions comprising a safe and effective amount of the compounds of the present invention and optionally, other compounds, intended for topical intranasal administration. In some embodiments, h compositions comprise from about 0.001% to about 10.0% w/v of a subject compound, more preferably from about 00.1% to about 2.0, which is used for systemic delivery of the compounds by the intranasal route.

In another embodiment, the CTP-modified coagulation factor may be injected into the muscle (intramuscular injection), or may be injected below the skin (subcutaneous injection), or injected into the skin, or administered via systemic administration, or administered by intravenous injection. In another embodiment, administration can be parenteral, pulmonary, oral, topical, intradermal, intramuscular, intraperitoneal, intravenous, subcutaneous, intranasal, transnasal, intraocular, ophthalmic, epidural, buccal, rectal, transmucosal, intestinal or parenteral delivery, including intramedullary injections as well as intrathecal or direct intraventricular administration.

In another embodiment, the preparation is administered in a local rather than systemic manner, for example, via injection of the preparation directly into a specific region of a patient's body.

In one embodiment, the route of administration may be enteral. In another embodiment, the route may be conjunctival, transdermal, intradermal, intra-arterial, vaginal, rectal, intratumoral, parcanceral, transmucosal, intramuscular, intravascular, intraventricular, intracranial, intra-nasal, sublingual, or a combination thereof.

In another embodiment, the pharmaceutical compositions are administered by intravenous, intra-arterial, or intramuscular injection of a liquid preparation. In some embodiments, liquid formulations include solutions, suspensions, dispersions, emulsions, oils and the like. In one embodiment, the pharmaceutical compositions are administered intravenously, and are thus formulated in a form suitable for intravenous administration. In another embodiment, the pharmaceutical compositions are administered intra-arterially, and are thus formulated in a form suitable for intra-arterial administration. In another embodiment, the pharmaceutical compositions are administered intramuscularly, and are thus formulated in a form suitable for intramuscular administration.

Further, in another embodiment, the pharmaceutical compositions are administered topically to body surfaces, and are thus formulated in a form suitable for topical administration. Suitable topical formulations include gels, ointments, creams, lotions, drops and the like. For topical administration, the compounds of the present invention are combined with an additional appropriate therapeutic agent or agents, prepared and applied as solutions, suspensions, or emulsions in a physiologically acceptable diluent with or without a pharmaceutical carrier.

In one embodiment, pharmaceutical compositions of the present invention are manufactured by processes well known in the art, e.g., by means of conventional mixing, dissolving, granulating, dragee-making, levigating, emulsifying, encapsulating, entrapping or lyophilizing processes.

In one embodiment, pharmaceutical compositions for use in accordance with the present invention is formulated in a conventional manner using one or more physiologically acceptable carriers comprising excipients and auxiliaries, which facilitate processing of the active ingredients into preparations which, can be used pharmaceutically. In one embodiment, formulation is dependent upon the route of administration chosen.

In one embodiment, injectables of the invention are formulated in aqueous solutions. In one embodiment, injectables of the invention are formulated in physiologically compatible buffers such as Hank's solution, Ringer's solution, or physiological salt buffer. In some embodiments, for transmucosal administration, penetrants appropriate to the barrier to be permeated are used in the formulation. Such penetrants are generally known in the art.

In one embodiment, the preparations described herein are formulated for parenteral administration, e.g., by bolus injection or continuous infusion. In some embodiments, formulations for injection are presented in unit dosage form, e.g., in ampoules or in multidose containers with optionally, an added preservative. In some embodiments, compositions are suspensions, solutions or emulsions in oily or aqueous vehicles, and contain formulatory agents such as suspending, stabilizing and/or dispersing agents.

The compositions also comprise, in some embodiments, preservatives, such as benzalkonium chloride and thimerosal and the like; chelating agents, such as edetate sodium and others; buffers such as phosphate, citrate and acetate; tonicity agents such as sodium chloride, potassium chloride, glycerin, mannitol and others; antioxidants such as ascorbic acid, acetylcystine, sodium metabisulfote and others; aromatic agents; viscosity adjustors, such as polymers, including cellulose and derivatives thereof; and polyvinyl alcohol and acid and bases to adjust the pH of these aqueous compositions as needed. The compositions also comprise, in some embodiments, local anesthetics or other actives.

The compositions can be used as sprays, mists, drops, and the like.

In some embodiments, pharmaceutical compositions for parenteral administration include aqueous solutions of the active preparation in water-soluble form. Additionally, suspensions of the active ingredients, in some embodiments, are prepared as appropriate oil or water based injection suspensions. Suitable lipophilic solvents or vehicles include, in some embodiments, fatty oils such as sesame oil, or synthetic fatty acid esters such as ethyl oleate, triglycerides or liposomes. Aqueous injection suspensions contain, in some embodiments, substances, which increase the viscosity of the suspension, such as sodium carboxymethyl cellulose, sorbitol or dextran. In another embodiment, the suspension also contains suitable stabilizers or agents which increase the solubility of the active ingredients to allow for the preparation of highly concentrated solutions.

In another embodiment, the active compound can be delivered in a vesicle, in particular a liposome (see Langer, Science 249:1527-1533 (1990); Treat et al., in Liposomes in the Therapy of Infectious Disease and Cancer, Lopez-Berestein and Fidler (eds.), Liss, New York, pp. 353-365 (1989); Lopez-Berestein, ibid., pp. 317-327; J. E. Diederichs and al., Pharm./nd. 56 (1994) 267- 275).

In another embodiment, the pharmaceutical composition delivered in a controlled release system is formulated for intravenous infusion, implantable osmotic pump, transdermal patch, liposomes, or other modes of administration. In one embodiment, a pump is used (see Langer, supra; Sefton, CRC Crit. Ref. Biomed. Eng. 14:201 (1987); Buchwald et al., Surgery 88:507 (1980); Saudek et al., N. Engl. J. Med. 321:574 (1989). In another embodiment, polymeric materials can be used. In yet another embodiment, a controlled release system can be placed in proximity to the therapeutic target, i.e., the brain, thus requiring only a fraction of the systemic dose (see, e.g., Goodson, in Medical Applications of Controlled Release, supra, vol. 2, pp. 115-138 (1984). Other controlled release systems are discussed in the review by Langer (Science 249:1527-1533 (1990).

In some embodiments, the active ingredient is in powder form for constitution with a suitable vehicle, e.g., sterile, pyrogen-free water based solution, before use. Compositions are formulated, in some embodiments, for atomization and inhalation administration. In another embodiment, compositions are contained in a container with attached atomizing means.

In one embodiment, the preparation of the present invention is formulated in rectal compositions such as suppositories or retention enemas, using, e.g., conventional suppository bases such as cocoa butter or other glycerides.

In some embodiments, pharmaceutical compositions suitable for use in context of the present invention include compositions wherein the active ingredients are contained in an amount effective to achieve the intended purpose. In some embodiments, a therapeutically effective amount means an amount of active ingredients effective for use in preventing, alleviating or ameliorating symptoms of disease or prolonging the survival of the subject being treated.

In one embodiment, determination of a therapeutically effective amount is well within the capability of those skilled in the art.

Some examples of substances which can serve as pharmaceutically-acceptable carriers or components thereof are sugars, such as lactose, glucose and sucrose; starches, such as corn starch and potato starch; cellulose and its derivatives, such as sodium carboxymethyl cellulose, ethyl cellulose, and methyl cellulose; powdered tragacanth; malt; gelatin; talc; solid lubricants, such as stearic acid and magnesium stearate; calcium sulfate; vegetable oils, such as peanut oil, cottonseed oil, sesame oil, olive oil, corn oil and oil of theobroma; polyols such as propylene glycol, glycerine, sorbitol, mannitol, and polyethylene glycol; alginic acid; emulsifiers, such as the Tween™ brand emulsifiers; wetting agents, such sodium lauryl sulfate; coloring agents; flavoring agents; tableting agents, stabilizers; antioxidants; preservatives; pyrogen-free water; isotonic saline; and phosphate buffer solutions. The choice of a pharmaceutically-acceptable carrier to be used in conjunction with the compound is basically determined by the way the compound is to be administered. If the subject compound is to be injected, in one embodiment, the pharmaceutically-acceptable carrier is sterile, physiological saline, with a blood-compatible suspending agent, the pH of which has been adjusted to about 7.4.

In addition, the compositions further comprise binders (e.g. acacia, cornstarch, gelatin, carbomer, ethyl cellulose, guar gum, hydroxypropyl cellulose, hydroxypropyl methyl cellulose, povidone), disintegrating agents (e.g. cornstarch, potato starch, alginic acid, silicon dioxide, croscarmelose sodium, crospovidone, guar gum, sodium starch glycolate), buffers (e.g., Tris-HCI., acetate, phosphate) of various pH and ionic strength, additives such as albumin or gelatin to prevent absorption to surfaces, detergents (e.g., Tween 20, Tween 80, Pluronic F68, bile acid salts), protease inhibitors, surfactants (e.g. sodium lauryl sulfate), permeation enhancers, solubilizing agents (e.g., glycerol, polyethylene glycerol), antioxidants (e.g., ascorbic acid, sodium metabisulfite, butylated hydroxyanisole), stabilizers (e.g. hydroxypropyl cellulose, hyroxypropylmethyl cellulose), viscosity increasing agents(e.g. carbomer, colloidal silicon dioxide, ethyl cellulose, guar gum), sweeteners (e.g. aspartame, citric acid), preservatives (e.g., Thimerosal, benzyl alcohol, parabens), lubricants (e.g. stearic acid, magnesium stearate, polyethylene glycol, sodium lauryl sulfate), flow-aids (e.g. colloidal silicon dioxide), plasticizers (e.g. diethyl phthalate, triethyl citrate), emulsifiers (e.g. carbomer, hydroxypropyl cellulose, sodium lauryl sulfate), polymer coatings (e.g., poloxamers or poloxamines), coating and film forming agents (e.g. ethyl cellulose, acrylates, polymethacrylates) and/or adjuvants.

Typical components of carriers for syrups, elixirs, emulsions and suspensions include ethanol, glycerol, propylene glycol, polyethylene glycol, liquid sucrose, sorbitol and water. For a suspension, typical suspending agents include methyl cellulose, sodium carboxymethyl cellulose, cellulose (e.g. Avicel™, RC-591), tragacanth and sodium alginate; typical wetting agents include lecithin and polyethylene oxide sorbitan (e.g. polysorbate 80). Typical preservatives include methyl paraben and sodium benzoate. In another embodiment, peroral liquid compositions also contain one or more components such as sweeteners, flavoring agents and colorants disclosed above.

In some embodiments, preparation of effective amount or dose can be estimated initially from in vitro assays. In one embodiment, a dose can be formulated in animal models and such information can be used to more accurately determine useful doses in humans.

In one embodiment, toxicity and therapeutic efficacy of the active ingredients described herein can be determined by standard pharmaceutical procedures in vitro, in cell cultures or experimental animals. In one embodiment, the data obtained from these in vitro and cell culture assays and animal studies can be used in formulating a range of dosage for use in human. In one embodiment, the dosages vary depending upon the dosage form employed and the route of administration utilized. The exact formulation, route of administration and dosage can be chosen by the individual physician in view of the patient's condition. [See e.g., Fingl, et al., (1975) "The Pharmacological Basis of Therapeutics", Ch. 1 p.1].

Depending on the severity and responsiveness of the condition to be treated, dosing can be of a single or a plurality of administrations, with course of treatment lasting from several days to several weeks or until cure is effected or diminution of the disease state is achieved.

The amount of a composition to be administered will, of course, be dependent on the subject being treated, the severity of the affliction, the manner of administration, the judgment of the prescribing physician, etc.

In one embodiment, compositions including the preparation of the present invention formulated in a compatible pharmaceutical carrier are also prepared, placed in an appropriate container, and labeled for treatment of an indicated condition.

In another embodiment, a coagulation factor as described herein is lyophilized (i.e., freeze-dried) preparation in combination with complex organic excipients and stabilizers such as nonionic surface active agents (i.e., surfactants), various sugars, organic polyols and/or human serum albumin. In another embodiment, a pharmaceutical composition comprises a lyophilized coagulation factor as described in sterile water for injection. In another embodiment, a pharmaceutical composition comprises a lyophilized coagulation factor as described in sterile PBS for injection. In another embodiment, a pharmaceutical composition comprises a lyophilized coagulation factor as described in sterile 0.9% NaCl for injection.

In another embodiment, the pharmaceutical composition comprises the CTP-modified coagulation factor as described herein and complex carriers such as human serum albumin, polyols, sugars, and anionic surface active stabilizing agents. In another embodiment, the pharmaceutical composition comprises the CTP-modified coagulation factor as described herein and lactobionic acid and an acetate/glycine buffer. In another embodiment, the pharmaceutical composition comprises the CTP-modified coagulation factor as described herein and amino acids, such as arginine or glutamate that increase the solubility of interferon compositions in water. In another embodiment, the pharmaceutical composition comprises a lyophilized CTP-modified coagulation factor as described herein and glycine or human serum albumin (HSA), a buffer (e g. acetate) and an isotonic agent (e.g NaCl). In another embodiment, the pharmaceutical composition comprises a lyophilized CTP-modified coagulation factor as described herein and phosphate buffer, glycine and HSA.

In another embodiment, the pharmaceutical composition comprising a coagulation factor as described herein is stabilized when placed in buffered solutions having a pH between about 4 and 7.2. In another embodiment, the pharmaceutical composition comprising a coagulation factor is in a buffered solution having a pH between about 4 and 8.5. In another embodiment, the pharmaceutical composition comprising a coagulation factor is in a buffered solution having a pH between about 6 and 7. In another embodiment, the pharmaceutical composition comprising a coagulation factor is in a buffered solution having a pH of about 6.5. In another embodiment, the pharmaceutical composition comprising a coagulation factor as described herein is stabilized with an amino acid as a stabilizing agent and in some cases a salt (if the amino acid does not contain a charged side chain).

In another embodiment, the pharmaceutical composition comprising a coagulation factor as described herein is a liquid composition comprising a stabilizing agent at between about 0.3% and 5% by weight which is an amino acid.

In another embodiment, the pharmaceutical composition comprising a coagulation factor as described herein provides dosing accuracy and product safety. In another embodiment, the pharmaceutical composition comprising a coagulation factor as described herein provides a biologically active, stable liquid formulation for use in injectable applications. In another embodiment, the pharmaceutical composition comprises a non-lyophilized coagulation factor as described herein.

In another embodiment, the pharmaceutical composition comprising a coagulation factor as described herein provides a liquid formulation permitting storage for a long period of time in a liquid state facilitating storage and shipping prior to administration.

In another embodiment, the pharmaceutical composition comprising a coagulation factor as described herein comprises solid lipids as matrix material. In another embodiment, the injectable pharmaceutical composition comprising a coagulation factor as described herein comprises solid lipids as matrix material. In another embodiment, the production of lipid microparticles by spray congealing was described by Speiser (Speiser and al., Pharm. Res. 8 (1991) 47-54) followed by lipid nanopellets for peroral administration (Speiser EP 0167825 (1990)). In another embodiment, lipids, which are used, are well tolerated by the body (e. g. glycerides composed of fatty acids which are present in the emulsions for parenteral nutrition).

The compositions for use according to the present invention may be presented in a pack or dispenser device, such as an FDA approved kit, which contain one or more unit dosage forms containing the active ingredient. In one embodiment, the pack, for example, comprise metal or plastic foil, such as a blister pack. In one embodiment, the pack or dispenser device is accompanied by instructions for administration. In one embodiment, the pack or dispenser is accommodated by a notice associated with the container in a form prescribed by a governmental agency regulating the manufacture, use or sale of pharmaceuticals, which notice is reflective of approval by the agency of the form of the compositions or human or veterinary administration. Such notice, in one embodiment, is labeling approved by the U.S. Food and Drug Administration for prescription drugs or of an approved product insert.

It will be appreciated that the coagulation factors for use according to the present invention can be provided to the individual with additional active agents to achieve an improved therapeutic effect as compared to treatment with each agent by itself. In an embodiment, measures (e.g., dosing and selection of the complementary agent) are taken to avoid adverse side effects which are associated with combination therapies.

As is generally known in the art, the modified peptides and proteins of the invention may be coupled to labels, drugs, targeting agents, carriers, solid supports, and the like, depending on the desired application. The labeled forms of the modified biologicals may be used to track their metabolic fate; suitable labels for this purpose include, especially, radioisotope labels such as iodine 131, technetium 99, indium 111, and the like. The labels may also be used to mediate detection of the modified proteins or peptides in assay systems; in this instance, radioisotopes may also be used as well as enzyme labels, fluorescent labels, chromogenic labels, and the like. The use of such labels is particularly helpful if the peptide or protein is itself a targeting agent such as an antibody or a receptor ligand.

Similar linking techniques, along with others, may be employed to couple the modified peptides and proteins of the invention to solid supports. When coupled, these modified peptides and proteins can then be used as affinity reagents for the separation of desired components with which specific reaction is exhibited.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

## EXAMPLES

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Culture of Animal Cells - A Manual of Basic Technique" by Freshney, Wiley-Liss, N. Y. (1994), Third Edition; "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996). Other general references are provided throughout this document.

***EXAMPLE 1***

***Generation and Utilization of Coagulation Factor IX***

**Cloning and expression of recombinant FIX molecule:**

Factor IX clones were constructed in our eukaryotic expression vector pCI-neo (Promega, catalog no. E1841). ORF Clone of Homo sapiens coagulation factor IX was ordered from "OriGene" (RC219065). Primers were ordered from **Sigma-Genosys.**

**Construction of 301-1-pCI-neo-p200-11** (**Factor IX-ctp x2**):

Primer 101: 5' GTTTAGTGAACCGTCAGAAT 3' (SEQ ID NO: 36)
Primer $103^{R:}$ 5' TTGAGGAAGATGTTCGTGTA 3' (contains the SspI site of factor IX) (SEQ ID NO: 37)

A PCR reaction was conducted with primer 101 and primer $103^{R}$ and plasmid DNA, cDNA clone of Factor IX (OriGene" RC219065) as a template; as a result of the PCR amplification , a ~ 1085 bp (pcr 10) product was formed and purified from the gel (the fragment containing the amino terminus of Factor IX sequence).

Primer 98: 5' ATTACAGTTGTCGCAGGTGA 3' (SEQ ID NO: 38)
Primer $99^{R}$ : 5' GCTGGAGCTAGTGAGCTTTGTTTTTTCCTT 3' (SEQ ID NO: 39)
Primer 100: 5' GCTCACTAGCTCCAGCAGCAAGGCC 3' (SEQ ID NO: 40)
Primer $27^{R}$: 5' TTTTCACTGCATTCTAGTTGTGG 3' (SEQ ID NO: 41)

Three PCR reactions were performed. The first reaction was conducted with primer 98 and primer $99^{R}$ and plasmid DNA, cDNA clone of Factor IX (OriGene" ,RC219065) as a template; as a result of the PCR amplification, a ~ 540 bp product was formed.

The second reaction was conducted with primer 100 and primer $27^{R}$ and plasmid DNA of 402-2-p72-3 (hGH-CTP-CTP) as a template; as a result of the PCR amplification, a ~ 258 bp product was formed.

The last reaction (pcr 3) was conducted with primers 98 and $27^{R}$ and a mixture of the products of the previous two reactions as a template; as a result of the PCR amplification, a ~ 790 bp product was formed and ligated into TA cloning vector (Invitrogen, catalog K2000-01). SspI -EcoRI fragment was isolated (TA 3-3).

Another PCR reaction was conducted (pcr 12) with primer 101 and primer $27^{R}$ and a mixture of the products of pcr 10 and SspI-EcoRI fragment from pcr 3 as a template; as a result of the PCR amplification, a ~ 1700 bp product was formed (Factor IX-ctp-ctp) and ligated into TA cloning vector (Invitrogen, catalog K2000-01) (lig 180).

A mistake was found in the Factor IX sequence so fragments were replaced in order to form an insert of Factor IX-ctp-ctp with the correct DNA sequence.

TA- pcr 3-3 was digested with SspI and XbaI and the large fragment was isolated (vector). TA 180-4 was digested with SspI and XbaI and the small fragment (insert) was isolated and ligated to the isolated large fragment of TA-pcr-3-3digested with SspI and XbaI. The new plasmid TA-183-2 was digated with Sal I and NotI, and the Factor IX-CTP-CTP insert was isolated (-1575 bp). This fragment was inserted into eukaryotic expression vector pCI-neo (digested with Sal I and Not I) to yield the 301-2-p200-11 clone.

**pCI-dhfr -Factor 9- ctpx2 (p223-4) construction:** Vector pCI-dhfr (p6-1) was digested with SmaI and NotI. Factor IX-CTP-CTP (p200-11) was digested with ASisI F.I. and NotI. The two fragments were ligated.

**pCI-dhfr Factor 9-ctp x3 (p225-7) construction:** Vector pCI-dhfr OXM-CTP×3 (p216-4) was digested with XbaI and ApaI. Factor IX-CTP-CTP (223-4) was digested with XbaI and ApaI. The two fragments were ligated.

**pCI-dhfr Factor 9-ctp x3 T148A (p243-2) construction:** Plasmid p225-7 contained Threonine at position 148, since the more common version of FIX contains Alanine at this position, Thr was replaced to Ala using site directed mutagenesis method.

Primer 75: ctcccagttcaattacagct (SEQ ID NO: 42)
Primer 122r: ggaaaaactgcctcagcacgggtgagc (SEQ ID NO: 43)
Primer 123: gtgctgaggcagtttttcctgatgtggactat (SEQ ID NO: 44)
Primer 124r: caacacagtgggcagcag (SEQ ID NO: 45)

Three PCR reactions were performed. The first reaction was conducted with primer 75 and primer 122r and plasmid DNA p225-7 as a template; as a result of the PCR amplification, a ~ 692 bp product was formed and purified from the gel. A second PCR reaction was conducted with primer 123 and primer 124r and plasmid DNA p225-7 as a template; as a result of the PCR amplification, a -237 bp product was formed and purified from the gel. The third - overlap PCR reaction reaction was conducted with primers 75 and 124r, and a mixture of the products of the previous two reactions as a template; as a result of the PCR amplification, a ~ 910 bp product was formed. This overlap PCR product was digested with XbaI and NsiI and re ligated into p225-7 plasmid (digested with XbaI and NsiI) to yield Factor IX-ctpx3 T148A designated p243-2.

**FIX-4CTP (p259-4) construction:** 3.5CTP fragment was isolated from oxym-4CTP (p254-3) by restriction enzymes Apa1 and XbaI. FIX+0.5CTP fragment was isolated from FIX-3CTP (p243-2) with restriction enzymes Apa1 and XbaI. The two fragments were ligated.

**FIX-5CTP (p260-18) construction:** 4.5CTP fragment was isolated from oxym-5CTP (255-1) by restriction enzymes Apa1 and XbaI. FIX+0.5CTP fragment was isolated from FIX-3CTP (p243-2) using enzymes Apa1 and XbaI. The two fragments were ligated.

Dg44 cells were plated in 100mm tissue culture dishes and grown to 50-60% confluence. A total of 2 μg (microgram) of FIX cDNA was used for the transfection of one 100mm plate using the FuGene reagent (Roche) in protein-free medium (Invitrogene CD Dg44). The media was removed 48 hours after transfection and replaced with a protein-free medium (Invitrogene CD Dg44) without nucleosides and in the presence of 800 μg/ml of G418 (Neomycin). After 14 days, the transfected cell population was transferred into T25 tissue culture flasks, and selection continued for an additional 10-14 days until the cells began to grow as stable clones. High expressing clones were selected. Approximately $2x10^7$ cells were used to inoculate 300 ml of growth medium in a 1700 $cm^2$ roller bottle (Corning, Corning NY) supplemented with 5 ng/ml of Vitamin K3 (menadione sodium bisulfate; Sigma). The production medium (harvest) was collected after a rapid decrease in cell viability to about 70%. The production medium was first clarified and then concentrated approximately 20-fold and dialyzed with PBS using flow filtration cassette (10KDa MWCO; Millipore Corp.).

**Determination of FIX antigen level:** FIX-CTP harvest antigen levels were determined using AssayMax Human FIX ELISA kit (AssayPro-EF1009-1). The calculated protein concentration is the average of three different dilutions in two independent runs (Figure 1A, Table 1).

**Table 1**: Calculated protein concentration

| | FIX-CTP | FIX-CTP-CTP |
|---|---|---|
| FIX Ag level (μg/ml) | 41.9 | 19.2 |
| SD | 8.76 | 3.67 |
| %CV | 20.92 | 19.15 |

**FIX SDS-PAGE - immune blot:** FIX-CTP harvests or purified rhFIX (American Diagnostics), 100 ng of protein, were loaded on 12% Tris-Glycine gel using Precision Plus Dual Color Protein Marker (Bio-Rad). The SDS-PAGE analysis was performed by Western immunoblot using anti-human FIX polyclonal antibody and anti-human gamma carboxylation monoclonal antibody (American Diagnostics). As previously reported, rhFIX migrated at 55KDa, while FIX fused to two CTPs migrated at 75KDa. Both variants of FIX-CTP proteins were shown to be gamma carboxylated, an essential post-tranaslation modification for FIX activity and function (Figure 1B).

**Determination of FIX chromogenic activity:** A comparative assessment of the *in vitro* potency of FIX-CTP harvests versus rhFIX protein (American Diagnostics) was performed using the commercially available chromogenic activity test kit, BIOPHEN (Hyphen BioMed 221802). In the presence of thrombin, phospholipids, calcium, excess amounts of FXIa activates sampled FIX into FIXa. FIXa forms an enzymatic complex with thrombin, activated FVIII:C (supplied in an excess amounts), phospholipids, and calcium and activates Factor X, present in the assay system, into FXa. The activity directly correlates with the amount of FIX, which is the limiting factor. The generated FXa is then measured by its specific activity on FXa chromogenic substrate (pNA). The amount of pNA generated is directly proportional to FIXa activity. rhFIX and FIX-CTP harvests were serially diluted, and the potency was assessed by comparing a dose-response curve of the FIX harvests to a reference preparation consisting of rhFIX or human plasma. The average EC50 of FIX was 21 ng/ml, while the FIX-$(CTP)_2$ harvest calculated EC50 was 382 ng/ml, and the FIX-CTP harvest calculated EC50 was 1644 ng/ml. An approximately 15-fold decrease in the enzymatic activity of the FIX-$(CTP)_2$ harvest was observed (Figure 2).

**FIX Clotting activity (aPTT):** The activated partial thromboplastin time (aPTT) is a measure of the integrity of the intrinsic and common pathways of the coagulation cascade. The aPTT is the time, in seconds, for plasma to clot following the addition of an intrinsic pathway activator, phospholipid and calcium. The aPTT reagent is called a partial thromboplastin because tissue factor is not included with the phospholipid as it is with the protime (PT) reagent. The activator initiates the system and then the remaining steps of the intrinsic pathway take place in the presence of phospholipid. Reference aPTT range varies from laboratory to laboratory, but is usually in the range of 27-34 seconds.

The principal of the assay was to quantitate the ability of FIX-CTP harvests to restore the clotting activity of FIX-depleted human plasma by the addition of rhFIX. 300 μl of FIX-deficient human plasma was mixed with 100μl of rhFIX or FIX-CTP harvests and serially diluted. Following a 60 second incubation at 37°C, thromboplastin, $CaCl_2$, and phospholipids were added to the mixture, and clotting time in seconds was determined (performed by American Medical Laboratories). The potency was assessed by comparing a dose-response curve of the FIX harvests to a reference preparation consisting of rhFIX or human plasma. One unit of FIX activity corresponds to the FIX concentration that equals the activity of one ml normal human plasma. The presented aPTT results indicate that FIX-$(CTP)_2$ exhibit a 5.7-fold reduction in its specific coagulation activity compared to rhFIX (Table 2). Moreover, the aPTT results together with the chromogenic activity *in vitro* assay suggest that FIX-$(CTP)_2$ harvest has an improved enzymatic activity vs. FIX-CTP harvest (Table 2). An improved activity of FIX-CTP proteins can be obtained following optimization of the expression system (i.e. co-transfection with Furin and optimization of Vitamin K3 medium concentration), which was strengthened following super-transfection with Furin (data not shown).

**Table 2**: FIX clotting activity

| rhFIX(AD) (μg/ml) | PTT(Sec) | FIX-CTP (μg/ml) | PTT (Sec) | FIX-CTP-CTP (μg/ml) | PTT (Sec) |
|---|---|---|---|---|---|
| 5 | 31.3 | 9 | 45.2 | 4 | 47.5 |
| 1.25 | 35.7 | 2.25 | 53.3 | 1 | 55.9 |
| 0.3125 | 43 | 0.5625 | 64.1 | 0.25 | 67 |
| 0.078125 | 52.1 | 0.140625 | 76.3 | 0.0625 | 77.4 |

**Pharmacokinetic study:** rhFIX (American Diagnostic) and FIX-CTP harvests were administered in a single intravenous injection to Sprague-Dawley rats (six rats per substance) at a dose of 75 μg/kg body weight (Table 3).

**Table 3:** PK study plan of operation

| Treated Groups | Test Article | No. of animals/ group | Dose Route | Gender | Dose Level (μg/kg ) | Dose Level (μg per animal ) | Injected Vol. (μl) | Con. (μg/ ml ) | *Time -Points (hours post-dose) |
|---|---|---|---|---|---|---|---|---|---|
| *1* | rFIX | *6* | IV | M | 75 | 15 | 500 | 30 | 0 (Pre-dose) 0.083, 0.5, 1.5,4, 8,24, 48, 72. |
| *2* | rFIX-CTP | *6* | IV | M | 75 | 15 | 500 | 30 | 0 (Pre-dose) 0.083, 0.5, 1.5,4, 8,24, 48, 72. |
| *3* | rFIX-CTP-CTP | *6* | IV | M | 75 | 15 | 1000 | 15 | 0 (Pre-dose) 0.083, 0.5, 1.5,4, 8,24, 48, 72. |

Blood samples were drawn retro-orbitally from 3 rats alternately at 0.083, 0.5 1.5, 4, 8, 24, 48, and 72 hours post-dosing. Plasma was prepared immediately after sampling and stored at -20°C until analysis. FIX concentration was quantitated by FIX ELISA-specific assay (AssayPro). A pharmacokinetic profile was calculated for each protein and represents the mean of 3 animals at each time point (Figure 3). The terminal half-lives were calculated using PK solutions 2.0 software. Table 4 summarizes the observed FIX concentrations at the different sampling time points.

**Table 4**: Observed FIX concentrations

| Time (Hr ) | FIX-AD (ng/ml) | FIX-CTP (ng/ml) | FIX-CTP-CTP (ng/ml) |
|---|---|---|---|
| 0.083 | 1506.7 | 1477.5 | 1914.8 |
| 0.5 | 1949.8 | 1150.1 | 1830.1 |
| 1.5 | 2189.4 | 1009.0 | 1264.3 |
| 4 | 733.90 | 709.33 | 1000.00 |
| 8 | 319.80 | 167.20 | 1234.67 |
| 24 | BLQ | 54.625 | 230 |
| 48 | BLQ | BLQ | 120.9 |

The PK profile and summary of the terminal half-lives are summarized in Table 5. FIX-CTP harvests exhibit an improved $T½_{\beta}$ values compared to rhFIX (2- and 5-fold increases, respectively). Since in FIX dosing collection, animal serum concentrations of FIX at 24hr were below limit of quantitation (BLQ), additional PK parameters were not calculated.

**Table 5**: Summary of PK parameters

| Product | Terminal half-life- (hr) | Ratio (FIX-(CTP)$_X$/rhFIX) |
|---|---|---|
| rhFIX (American Diagnostics) | 2.62 | – |
| FIX-CTP | 5.55 | 2.11 |
| FIX-CTP (FIX-CTP-CTP) | 12.9 | 4.92 |

In this study, a novel approach was described for prolonging FIX half-life while retaining the therapeutic potency. Adding a CTP peptide to an active protein has a harmful potential in interfering with the protein's activity. Therefore, the generation of an active recombinant FIX-CTP by adding a CTP sequence at the C-terminus of the FIX is unexpected.

***Characterization of an immunoaffinity purified FIX-CTP-CTP***

**FIX-CTP-CTP purification**

In order to evaluate a protein at high grade content with increased activity whose PK profile mimics and can be extrapolated to a clinical setting, FIX-CTP-CTP is a FIX modified with 2 CTP units in tandem in its carboxy-terminal. FIX-CTP-CTP was purified using matrix-bound monoclonal antibody against γ carboxyglutamyl (Gla) residues present in the N-terminal region of FIX (American Diagnostics Cat. # 3570MX). The monoclonal antibody was bound to Sepharose CL-4B. The FIX-CTP-CTP harvest at a concentration of 88 μg/ml was dialyzed against 20mM Tris, 150Mm NaCl and 10mM EDTA at PH =7.4. The loading rate was 0.5 ml/min, elution was performed using 20Mm Tris-HCl, 350 mM NaCl and 50 mM CaCl, and the unbound fraction was recycled five times. Finally, the elution fraction was dialyzed with PBS, pulled and concentrated.

**Determination of FIX antigen level:** FIX-CTP harvests, FIX-$(CTP)_2$ harvests, and FIX-$(CTP)_2$ purified protein levels were determined using the Human FIX ELISA kit (Affinity Biologicals; Cat. #FIX-AG RUO). The calculated protein concentration (μg/ml) is the average of two independent runs (Figure 4, Table 6).

**Table 6**: Calculated protein concentration

| | FIX-CTP | FIX-CTP-CTP | FIX-CTP-CTP (purified) |
|---|---|---|---|
| FIX Ag level (μg/ml) | 125.78 | 88.53 | 172.9 |
| SD | 17.28 | 21.31 | 2.63 |
| %CV | 13.74 | 24.08 | 1.52 |

Additionally, FIX-CTP-CTP was quantitated by Bradford assay. The calculated concentration was 202 μg/ml, which is similar to the concentration obtained by human FIX ELISA.

**SDS-PAGE blots:** FIX-CTP-CTP harvest, unbound fraction and purified protein, were loaded on a 12% Tris-Glycine gel using Precision Plus Dual Color Protein Marker (Bio-Rad). The SDS-PAGE Coomassie analysis was performed by staining the gel with Coommasie blue reagent (800ng of protein). A Western immunoblot was performed with 100 ng of protein, anti-human FIX polyclonal antibody (Ab), and anti-human gamma carboxylation monoclonal Ab (American Diagnostics Cat #499 and #3570). The immunoaffinity purification procedure significantly enriched the FIX-CTP-CTP portion while reduced impurity (Figure 5).

**N-terminal sequencing:** FIX-CTP-CTP purified protein was separated by 12% Tris-Glycine SDS-PAGE and subsequently electro-blotted to PVDF membrane. The band of interest was cut out and put on a purified Biobrene treated glass fiber filter. The N-terminal sequence analysis was carried out by Edmann degradation using a pulsed liquid protein sequencer equipped with a 140 C HPLC micro-gradient system. N-terminal sequencing revealed that FIX-CTP-CTP is a mixture of incomplete and complete pro-peptide cleaved proteins. Inadequate pro-peptide cleavage was shown to reduce FIX coagulation activity. By co-transfection with Furin, the pro-peptide cleavage process can be an improved.

**Determination of FIX chromogenic activity:** A comparative assessment of the *in vitro* potency of FIX-CTP-CTP purified protein versus rhFIX (American Diagnostics) and a pool of human normal plasma was performed using the commercially available chromogenic activity test kit, BIOPHEN (Hyphen BioMed 221802). In the presence of thrombin, phospholipids and calcium, excess amounts of FXIa activates FIX into FIXa. FIXa forms an enzymatic complex with thrombin (supplied in excess amounts), phospholipids and calcium activates Factor X, present in the assay system, into FXa. The activity directly correlates with the amount of FIX, which is the limiting factor. The generated FXa was measured by its specific activity on FXa chromogenic substrate (pNA). The amount of pNA generated was directly proportional to FIXa activity. rhFIX, human plasma and FIX-CTP-CTP were serially diluted, and potency was assessed by comparing a dose-response curve (Figure 6). The average $EC_{50}$ of rhFIX was 68.74 ng/ml while FIX-CTP-CTP calculated $EC_{50}$ was 505 ng/ml. An approximately 7-fold decrease in the enzymatic activity of FIX-CTP-CTP was observed vs. recombinant FIX and a 16.5-fold decrease versus normal human pulled plasma. This reduced activity could be explained by inadequate cleavage of N-terminal pro-peptide, which was identified by N-terminal analysis.

**FIX Clotting activity (aPTT):** The activated partial thromboplastin time (aPTT) is a measure of the integrity of the intrinsic and common pathways of the coagulation cascade. The aPTT is the time (measured in seconds) it takes plasma to clot following the addition of an intrinsic pathway activator, phospholipid and calcium.

The assay quantitated the ability of the FIX-CTP-CTP protein to restore the clotting activity of FIX depleted human plasma by the addition of rhFIX. 300 μl of FIX-deficient human plasma was mixed with 100 μl of rhFIX, FIX-CTP-CTP (FIX-CTP-CTP (the CTP are in tandem at the C-terminal)), or normal pool human plasma which was further diluted. Following a 60 second incubation at 37°C, Tissue Factor (TF), $CaCl_2$, and phospholipids were added to the mixture. Clotting time in seconds was determined. Potency was assessed by comparing a dose-response curve of FIX-CTP-CTP to a reference preparation of rhFIX or human plasma. One unit of FIX was defined as the amount of FIX which equals to the activity of 1 ml human normal plasma.

The aPTT results indicate that FIX-CTP-CTP coagulation activity is only 1.4 less then normal pool human plasma and similar to the rhFIX. The aPTT results together with the chromogenic activity *in vitro* assay suggest that FIX-CTP-CTP purification did not damage its activity.

**Pharmacokinetic activity of FIX-CTP-CTP:** Purified FIX-CTP-CTP, rhFIX (American Diagnostic) and harvests containing FIX-CTP-CTP and FIX-CTP were administered in a single intravenous injection to Sprague-Dawley rats (eight rats per substance) in a dose of 100μg/kg body weight (Table 7).

**Table 7:** PK study outline

| Treated Groups | Test Article | No.of animals/ group/ time point | Dose Level (μg/kg ) | Dose Level (μg per animal) | Injected Vol. (μl) | Con. (μg/ ml ) | Time-Points (hours post-dose) |
|---|---|---|---|---|---|---|---|
| A | rFIX | 8 | 100 | 20 | 500 | 40 | 0 (Pre-dose) 0.083, 0.5, 1, 2, 4, 7, 10, 24, 48, 72. |
| B | rFIX-CTP (harvest) | 8 | 100 | 20 | 500 | 40 | 0 (Pre-dose) 0.083, 0.5, 1, 2, 4, 7, 10, 24, 48, 72. |
| c | rFIX-CTP-CTP (harvest ) | 6 | 100 | 20 | 500 | 40 | 0 (Pre-dose) 0.083, 0.5, 1, 2, 4, 7, 10, 24, 48,72. |
| D | rFIX-CTP-CTP (purified) | 4 | 100 | 20 | 500 | 40 | 0.083, 0.5 1, 2, 4, 7, 10, 24, 4, 8,72. |

Blood samples were drawn retro-orbitally from 4 rats alternately at 0.083, 0.5, 2, 4, 7 10, 24, 48, and 72 hours post-dosing. Citrated plasma (0.32%) was prepared immediately after sampling and stored at -20°C until analysis. FIX concentration was quantitated using a human FIX ELISA kit (Affinity Biologicals). The pharmacokinetic profile was calculated for each protein as the mean of 4 animals at each time point (Figure 7). The terminal half-life was calculated using PK Solutions 2.0 Software. Table 8 summarizes the observed FIX concentrations at different sampling time points.

**Table 8**: Observed FIX concentrations

| Time (hr) | FIX-CTP harvest ng/ml | FIX-$(CTP)_2$ harvest ng/ml | rhFIX ng/ml | Purified FIX-CTP-CTP ng/ml |
|---|---|---|---|---|
| **0.085** | 1038.97 | 1123.62 | 325.05 | 886.48 |
| **0.5** | 939.12 | 956.80 | 274.58 | 670.92 |
| **1** | 791.97 | 843.85 | 222.90 | 674.17 |
| **2** | 304.98 | 673.31 | 186.00 | 503.91 |
| **4** | 315.37 | 525.50 | 109.69 | 357.36 |
| **7** | 171.45 | 384.36 | 67.62 | 257.02 |
| **10** | 50.34 | 250.73 | 40.20 | 158.66 |
| **24** | 10.07 | 78.50 | BLQ | 52.13 |
| **48** | BLQ | 23.40 | BLQ | 18.07 |

A summary of the PK parameters are presented in Table 9.

**Table 9**: Summary of PK parameters

| | T½ (hr) | AUC ng-hr/ml | MRT (hr) | Vd ml/Kg | CL Ml/hr/Kg |
|---|---|---|---|---|---|
| **FIX-CTP harvest** | 4.17 | 3622 | 4.5 | 155.1 | 27.6 |
| **FIX-(CTP)$_2$ harvest** | 10.44 | 9105.7 | 12 | 165.4 | 10.9 |
| **rhFIX** | 3.72 | 1416.8 | 5.1 | 373.8 | 70.183 |
| **Purified FIX-CTP-CTP** | 11.14 | 6314.2 | 12.3 | 254.5 | 15.83 |

The FIX-CTP-CTP harvest demonstrated an improved PK profile compared to FIX-CTP harvest. Furthermore, purified FIX-CTP-CTP exhibited a 3-fold increase in $T½_β$ value and a 4.5-fold increase in AUC compared to rhFIX.

The reduced amount of secreted FIX fused to tandem CTP molecules versus fusion of a single CTP appears to be due to the addition of an extra CTP and not to reduced detection by ELISA, because the Bradford-purified FIX-CTP-CTP calculated concentration was similar to the ELISA-calculated concentration.

FIX-CTP-CTP clotting activity was similar to pooled human plasma; however, its *in vitro* chromogenic activity was significantly lower when compared to rhFIX or pooled human plasma. The chromogenic activity assay was reported as a very sensitive assay compared to the coagulation assay. The reason for reduced activity of FIX-CTP-CTP may vary. Addition of CTP may decrease the affinity of FIX to FXIa or reduce post-transcriptional modifications (e.g. 12-10 GLA residues and pro-peptide cleavage). N-terminal analysis revealed that the proteolytic cleavage of the FIX-CTP-CTP pro-peptide was not fully completed prior to secretion. Since this post-transcriptional modification is crucial for the normal enzymatic activity of the protein, co-transfection with Furine-PACE plasmid is favorable and may improve FIX-CTP-CTP activity.

Finally, FIX-CTP-CTP comparative PK study in rats demonstrated that fusion of two tandem CTPs to the C-terminal of FIX generated a FIX with an extended half-life.

**FIX depleted mouse model:** In order to assess the *in vivo* activity, FIX knockout mice are obtained, and a breeding colony is established. 10μg of either commercial recombinant hFIX (BeneFIX®) or rFIX-(CTP)$_2$ (FIX-CTP-CTP) are injected into the tail vein of an anaesthetized FIX knockout mouse (22-28g). The amount of injected protein equals to the required concentration of FIX in normal plasma (5μg/ml). Blood samples are taken from the clipped tail into heparinized capillary tubes at specific time points. Plasma samples are assessed for FIX levels by ELISA and efficacy is measured by aPTT coagulation assay.

**Increasing FIX Propeptide cleavage efficacy:** CTP peptide cDNA was fused to the 3' end of human FIX cDNA. The corresponding rFIX and Furin expressing constructs were co-transfected into Dg44 cells; a human rFIX cDNA was also co-transfected with the Furin plasmid as a control. Secretion of high level of FIX leads to secretion of a mixture of pro-factor and a mature factor FIX, due to limited amount of the Furin protease in the cell. Co-transfection of a Furin expressing vector with a pro-factor expressing vector increases the recovery and result in the secretion of fully processed FIX in to the medium.

Following FIX-(CTP)$_2$ and Furin co-transfection, stable clones are generated and harvest is collected for pro-peptide cleavage evaluation. 100 ng of protein, are loaded on 12% Tris-Glycine gel using Precision Plus Dual Color Protein Marker (Bio-Rad). The SDS-PAGE analysis is performed by Western immunoblot using anti-human FIX polyclonal Ab (American Diagnostics) and anti-pro-peptide polyclonal antibody. As previously reported, rhFIX migrated at 55KDa, while FIX fused to two CTPs migrated at 75 kDa. Both variants of FIX proteins are shown to undergo a proper, full pro-peptide cleavage.

To determine whether proper pro-peptide cleavage improves FIX-(CTP)$_2$ enzymatic activity, a comparative assessment of chromogenic and coagulation activity of FIX-(CTP)$_2$ harvest co transfecated with Furin is performed. A significant improvement in FIX-(CTP)$_2$ specific activity is observed, which is similar to rhFIX.

In conclusion, the results described herein suggest that FIX-CTP-CTP can be used efficiently for treating Hemophilia B patients. FIX fused to CTP constructs benefit from improved *in vivo* pharmacologic performance that overcomes the drawback in certain *in vitro* measures. This proposed treatment is advantageous over previous treatments as the rate of infusions and the amount of required doses are reduced.

It is important to notice that when an albumin-fused molecule strategy was used to improve the FIX half-life, the recombinant FIX became inactive. The present novel approach lead to the design and purification of a novel recombinant FIX-fused protein that presents an improved long-lasting activity. Since mere size modifications did not improve the pharmacokinetics of injected FIX, the finding that CTP fused to FIX facilitates pharmacokinetic parameters was unexpected. The presence of highly glycosylated peptide-sialic acid residues stabilized the protein and protected it from interactions with vascular receptors without abrogating key determinants of FIX function.

FIX-CTP has a similar therapeutic efficacy to rFIX in hemophilia B patients and required less frequent dosing. A single injection of FIX-CTP is sufficient to control bleeding episodes and reduce the number of injections that are needed during surgical intervention in hemophilia B patients.

The CTP technology was utilized for the development of a long-acting FIX. Specifically, extending the half-life of recombinant rFIX molecule was performed by fusion of at least one human CTP to FIX. The recombinant FIX-CTP was expressed in mammalian cells and characterized *in vitro* and *in vivo.* It was demonstrated that the *in vitro* activity of rFIX-CTP was comparable to rFIX. Pharmacokinetics and efficacy studies in rats demonstrated improved properties of the rFIX-CTP. The results of this study demonstrate that it is feasible to develop a half-life extended rFIX molecule having similar haemostatic properties to the wild type enzyme.

### *EXAMPLE 2*

### *Comparative Assessment of Purified FIX-$CTP_3$ vs. FIX-$CTP_4$ and FIX-$CTP_5$*

#### 2.1 Study objective

A comparative assessment of the pharmacokinetic parameters of FIX-$CTP_4$ and FIX-$CTP_5$ versus FIX-$CTP_3$ following a partial purification process.

#### 2.2 Production of FIX-$CTP_4$ and FIX-$CTP_5$ harvests

FIX cDNA (OriGene RC219065) fused at the C-terminal to four or five tandem CTP sequences was expressed in Dg44 cells using Excellgene expression system in the presence of 10 ng/L of vitamin K3 (Sigma, Mennadion). The harvests were collected (300ml), filtered and frozen.

#### 2.3 Production of FIX-$CTP_3$ harvest

FIX-$CTP_3$ was expressed in-house in CHO cells using pCI-DHFR vector, clone 196, BR-9 in the presence of 25 ng/L of vitamin K3 (Sigma). The harvests were collected and filtered.

All FIX-CTP samples (3, 4 and 5 CTP) were purified only by Jacalin column because of a lack of material.

#### 2.4 Determination of FIX antigen level

FIX antigen level was determined using Human FIX ELISA kit (Affinity Biologicals; Cat. # FIX-AG RUO). The calculated protein concentration is the average of four independent runs. FIX-$CTP_3$ concentration was slightly higher as compared to the two additional versions (Table 10).

**Table 10**: FIX antigen level

| | 3 CTP Final Jacalin40 | 4 CTP Final Jacalin40 | 5 CTP Final Jacalin40 |
|---|---|---|---|
| Av. (ng/ml) | 1016.69 | 4644.11 | 1686.82 |
| SD | 225.41 | 925.63 | 160.07 |
| %CV | 22.17 | 19.93 | 9.49 |

**2.5 FIX-CTP Coomassie stain and immune-blot**

FIX-$CTP_3$, FIX-$CTP_4$, and FIX-$CTP_5$ harvests were loaded on 12% Tris-Glycine gel using Precision Plus Dual Color Protein Marker (Bio-Rad). The SDS-PAGE analysis was performed by Western immuno-blot using anti-CTP polyclonal Ab (Adar Biotech Production) or anti-Gla Ab (American Diagnostica).

As previously reported, FIX fused to three CTPs migrated at 80 kDa while FIX fused to four or five CTPs migrated at 85 KDa or 90 KDa, respectively. As expected, FIX-$CTP_4$ and FIX-$CTP_5$ harvests from Excellgene showed very low levels of gamma carboxylation compared to FIX-$CTP_3$ harvest, which was produced at Prolor (Figure 8).

After a purification process utilizing Jacalin column (immunoaffinity purification of glycosylated proteins), FIX-$CTP_3$, FIX-$CTP_4$, and FIX-$CTP_5$ were loaded on 12% Tris-Glycine gel using Precision Plus Dual Color Protein Marker (Bio-Rad). The SDS-PAGE was stained by Coomassie blue Dye for samples detection. All variants showed much cleaner band profiles (Figure 9), suggesting an improved purity.

**2.6 Determination of FIX chromogenic activity**

A comparative assessment of the *in vitro* potency of fully purified (HA column) FIX-$CTP_3$, FIX-$CTP_4$, and FIX-$CTP_5$ versus human pool normal plasma was performed using a commercially available chromogenic activity test kit, BIOPHEN (Hyphen BioMed 221802). All samples were serially diluted, and the potency was assessed by comparing a dose-response curve to a reference preparation of normal human plasma. The reduced chromogenic activity of FIX-$CTP_4$ and FIX-$CTP_5$ (Figure 10) as compared to plasma can be a consequence of improper post-transcriptional modifications of FIX proteins, e.g. inappropriate gamma carboxylation and pro-peptide cleavage or, alternatively, due to the addition of CTP cassettes. The fluctuation in the FIX-$CTP_4$ and FIX-$CTP_5$ activity (Table 11) might be caused by inappropriate quantitation capabilities of the FIX ELISA due to CTP masking of the antigen site.

**Table 11**: Sample/plasma EC50 ratio

| Sample | Sample/plasma EC50 ratio |
|---|---|
| Plasma | 1 |
| 3 CTP Final HA | 2 |
| 4 CTP Final HA | 5.35 |
| 5 CTP Final HA | 2.73 |

**2.7 Pharmacokinetic study**

Jacalin-purified FIX-$CTP_3$, FIX-$CTP_4$, and FIX-$CTP_5$ (Group A, B and C, respectively) were administered in a single intravenous injection to Sprague-Dawley rats (six rats per treatment group) at a dose of 250 μg/kg body weight. Blood samples were drawn retro-orbitally from 3 rats alternately at 0.083, 0.5 2, 5, 8, 24, 48, 72 and 96 hours post-dosing (Table 12). Citrated plasma (0.38%) was prepared immediately after sampling and stored at -20°C until analysis.

**Table 12:** PK study plan of operation

| Treatm ent Group | Treatmen t | No. of animals/ group | Dose Route | Dose Level (μg per animal) | Injecte d Vol. (μl) | Conc. (μg/ml) | Time-Points (hr post-dose) |
|---|---|---|---|---|---|---|---|
| A | FIX-CTP*3 Jacalin 40 | 6 | IV | 50 | 200 | 250 | 0.083, 0.5, 2, 5, 8, 24, 48, 72, 96 |
| B | FIX-CTP*4 | 6 | IV | 50 | 200 | 250 | 0.083, 0.5, 2, 5, 8, 24, 48, 72, 96 |
| | Jacalin 40 | | | | | | |
| c | FIX-CTP*5 Jacalin 40 | 6 | IV | 50 | 200 | 250 | 0.083, 0.5, 2, 5, 8, 24, 48, 72, 96 |

FIX concentration in plasma samples were quantified using human FIX ELISA kits (Affinity Biologicals). The pharmacokinetic profile was calculated and is the mean of 3 animals at each time point. Terminal half-lives were calculated using PK Solutions 2.0 Software. Table 13 below summarizes the calculated FIX concentrations at the different sampling time points.

**Table 13**: Calculated FIX concentrations

| Time (hr) | Av. 3 CTP ng/ml | SD 3 CTP | Av. 4 CTP ng/ml | SD 4 CTP | Av. 5 CTP ng/ml | SD 5 CTP |
|---|---|---|---|---|---|---|
| **0.083** | 1087.82 | 72.39 | 904.54 | 21.06 | 1097.23 | 82.24 |
| **0.5** | 774.18 | 86.31 | 736.82 | 66.93 | 998.79 | 70.43 |
| **2** | 562.23 | 3.70 | 627.09 | 32.47 | 747.85 | 14.02 |
| **5** | 357.44 | 8.63 | 431.23 | 29.41 | 576.49 | 27.36 |
| **8** | 239.20 | 7.82 | 327.46 | 30.26 | 394.96 | 36.48 |
| **24** | 77.08 | 4.26 | 107.38 | 5.18 | 142.42 | 16.13 |
| **48** | 27.73 | 2.02 | 39.83 | 1.85 | 53.66 | 3.33 |
| **72** | 12.55 | 1.48 | 21.53 | 1.55 | 23.54 | 3.32 |
| **96** | 6.66 | 1.23 | 10.63 | 0.13 | 18.54 | 3.39 |

The PK profile and a summary of the PK parameters are presented in Table 14 below and in Figure 11. A full PK analysis profile at all time points suggested that addition of 4 or 5 CTP cassettes to FIX did not increase its half-life as compared to FIX-$CTP_3$. The AUC following FIX-$CTP_5$ administration increased by 1.4- to 1.6-fold versus FIX-$CTP_3$, which was not statistically significant.

**Table 14:PK profile and a summary of the PK parameters**

| 24-96 (hr) | 3 CTP | 4 CTP | 5 CTP |
|---|---|---|---|
| **Half-life (hr)** | 20.43 | 22.02 | 23.96 |
| **AUC (ng-hr/ml)** | 8218.38 | 10504.49 | 13329.41 |
| **Vd (ml/kg)** | 700.76 | 586.02 | 494.89 |

(continued)

| 24-96 (hr) | 3 CTP | 4 CTP | 5 CTP |
|---|---|---|---|
| CL (ml/hr/kg) | 23.77 | 18.45 | 14.32 |

Since 96 hr post-dosing samples were shown to have very low FIX concentrations, which were at the lower limit of quantification of the assay, the terminal half-life was recalculated providing a more precise and scientifically appropriate calculation (Table 15). According to this calculation, even smaller differences were obtained between the half-life of FIX-$CTP_3$, FIX-$CTP_4$, and FIX-$CTP_5$.

**Table 15:** Recalculated terminal half-life

| 8-72 (hr) | 3 CTP | 4 CTP | 5 CTP |
|---|---|---|---|
| Half-life (hr) | 15.38 | 16.63 | 16.04 |

**2.8 Conclusions:**

In this study, the pharmacokinetic parameters and potential clotting activity of FIX-$CTP_3$, FIX-$CTP_4$, and FIX-$CTP_5$ were assessed. Fusion of 4 and 5 CTPs to FIX did not provide a superior or improved half-life extension, as compared to FIX-$CTP_3$, and reduced chromogenic activity was observed. Table 16 below summarizes the percent improvement of half-life for the different FIX-CTP fused variants (1 to 5 CTPs). Fusion of CTP to FIX improved its pharmacokinetic behavior, but, unpredictably, this improvement was limited. Surprisingly, following fusion of 3, 4 or 5 CTPs in tandem to FIX, a similar half-life value was calculated.

**Table 16**: Summary of the percent improvement of half-life

| FIX Version | T½ (8-72hr) % increase |
|---|---|
| rhFIX vs. 1CTP | 112 |
| 1CTP vs. 2CTP | 141 |
| 2CTP vs. 3CTP | 37 |
| 3CTP vs. 4CTP | 6 |
| 4CTP vs. 5CTP | 0 |

These data suggest that fusion of 3 CTPs to FIX produces a maximal improvement in protein half-life, confirming that FIX-$CTP_3$ is the optimal variant in terms of half-life, structure and potential clotting activity for further clinical development.

***EXAMPLE 3***

***FIX-$CTP_3$ TREATMENT OF FIX-/- HEMOPHILIC MOUSE MODEL***

As described above, a study testing FIX-CTP, FIX-$CTP_2$ and FIX-$CTP_3$ harvest PK profile and coagulation activity vs. rhFIX was conducted. FIX-$CTP_3$ exhibited an improved PK profile while maintaining its coagulation activity vs. FIX-$CTP_1$ and FIX-$CTP_2$ harvests or rhFIX. To further evaluate this result, FIX-$CTP_3$ $\gamma$-Carboxyglutamate protein was purified. FIX-$CTP_3$ exhibits a 3-fold increase in half-life and 4.5-fold higher AUC compared to rhFIX in normal rats following a single IV administration. FIX-$CTP_3$ demonstrated a reduced *in vitro* chromogenic and clotting activity, most likely due to insufficient cleavage of N-terminal pro-peptide and in appropriate post-transcriptional modifications (PTMs), such as appropriate gamma carboxylation.

In the current study, the pharmacokinetic and pharmacodynamic properties of human recombinant FIX fused to three tandem CTPs were tested in FIX-deficient mice.

**Study purpose:**

To determine the pharmacokinetic and pharmacodynamic parameters of rFIX-$(CTP)_3$ vs. commercial rhFIX (BeneFIX®) in FIX-deficient mice following a single IV administration of FIX-$(CTP)_3$ at a similar specific activity and dose (similar specific activity to PD and similar FIX constant for PK).

**Production of FIX-$CTP_3$ harvest:**

FIX cDNA (OriGene RC219065-Thr 148) fused at the C-terminal to three tandem CTP sequences was expressed in Dg44 cells using Excellgene expressing system in the presence of 25 ng/ml of Vitamin K3 (Sigma, Mennadion). Five separate batches containing 5 liters of cell suspension was cultured (total of twenty-five liters) and harvested following viability decline to 60-70%. The harvest was filtered and frozen at -70°C.

**Determination of harvest FIX antigen level:**

Harvest FIX antigen level was determined using a human FIX ELISA kit (Affinity Biologicals; Cat. # FIX-AG RUO). The antigen level was calculated per each batch. The FIX concentration was maintained through the different batches (Table 17).

**Table 17**: FIX antigen level

| | FIX antigen level | | |
|---|---|---|---|
| **Batch** | **#1** | **Bat #2** | **Bat #3** |
| **Av (μg/ml)** | 28.81 | 32.74 | 42.9 |
| **STD** | 2.5 | 2.69 | 4.0 |
| **%CV** | 8.84 | 8.38.2 | 9.4 |

**FIX-$CTP_3$** purification process:

Following a short purification study, a purification process using the following 3 columns was performed: DEAE Sepharose, Heparin Sepharose and HA Bio Rad Ceramic Hydroxyapatite type 1 (40 μm), FIX-$CTP_3$. γ-carboxylated enriched protein was purified. In brief: Five liters of clarified harvest was thawed at 4°C over a 4 day period. For each purification batch, the clarified harvest (2 liters) was concentrated 4-fold and dialyzed against 20 mM Tris-HCl pH 8.2 using a disposable hollow fiber cartridge with a nominal molecular weight cutoff size of 10 KDa. This process (UFDF1) was performed twice, and one liter of UFDF1 was loaded on DEAE Sepharose column, and Factor IX was eluted with 20 mM Tris-HCl, 200 mM NaCl, 10 mM CaCl2 pH 8.2. The product was diluted 1:1 with 20 mM Tris-HCl, 10 mM CaCl2 pH 7.5, and the pH was adjusted to 7.5 before loading on Heparin Sepharose column. The elution was performed with 20 mM Tris-HCl, 300 mM NaCl, and 10 mM CaCl2 pH 7.5. The eluted product was concentrated and dialyzed against 10 mM phosphate pH 6.8 using a Pellicon XL cassette 10 KDa cutoff membrane (UFDF2). The product was loaded on an HA column, and the activated fraction of Factor IX was eluted with 150 mM phosphate pH 6.8. The purification product was concentrated to a target concentration of 2 mg/ml and dialyzed against TBS pH 7.45, divided in aliquots and stored at -70°C.

The purification process was repeated five times, on a weekly basis in order to purify the total volume (25 liters). The purification processes were named HA# 6-10. Each purification product was separately evaluated (App # 1-5). At the end of the purification process, the different batches were pooled and further concentrated to a target concentration of 4 mg/ml.

**FIX-$CTP_3$ analytical properties:**

Determination of FIX antigen level

FIX-$CTP_3$ γ-carboxylated enriched protein antigen level was determined using a human FIX ELISA kit (Affinity Biologicals; Cat. # FIX-AG RUO). The calculated protein concentration is the average of two independent runs (Table 18).

**Table 18**: FIX-$CTP_3$ antigen level

| FIX-CTP$_3$ HA purified pool ELISA #1 | | | | FIX-CTP$_3$ HA purified pool- ELISA #2 | | | | Final Av. |
|---|---|---|---|---|---|---|---|---|
| Dil. | 1 | 2 | Av. | Dil. | 1 | 2 | Av. | |
| 130000 | 3412240 | 3781830 | 3597035 | 130000 | 3692260 | 3568240 | 3630250 | 3613643 |
| 260000 | 3915600 | 4158440 | 4037020 | 260000 | 3706820 | 3595540 | 3651180 | 3844100 |
| 520000 | 4158544 | 4334096 | 4246320 | 520000 | 3831464 | 3530748 | 3681106 | 3963713 |
| 1040000 | 4096352 | 4004104 | 4050228 | 1040000 | 3863392 | 3684304 | 3773848 | 3912038 |
| Av. (ng/ml) | 3895684 | 4069618 | 3982651 | Av. (ng/ml) | 3773484 | 3594708 | 3684096 | 3833373 |
| STD | 338367.5 | 234486.7 | 274313.5 | STD | 86576.66 | 65369.65 | 63369.86 | 154459.6 |
| %CV | 8.685703 | 5.761884 | 6.887712 | %CV | 2.294343 | 1.818497 | 1.720092 | 4.029338 |
| Av. (mg/ml) | 3.895684 | 4.069618 | 3.982651 | Av. (mg/ml) | 3.773484 | 3.594708 | 3.684096 | 3.833373 |


| FIX-CTP$_3$ HA purified pool ELISA #1 | | | | FIX-CTP$_3$ HA purified pool- ELISA #2 | | | | Final Av. |
|---|---|---|---|---|---|---|---|---|
| Dil. | 1 | 2 | Av. | Dil. | 1 | 2 | Av. | |
| 130000 | 3412240 | 3781830 | 3597035 | 130000 | 3692260 | 3568240 | 3630250 | 3613643 |
| 260000 | 3915600 | 4158440 | 4037020 | 260000 | 3706820 | 3595540 | 3651180 | 3844100 |
| 520000 | 4158544 | 4334096 | 4246320 | 520000 | 3831464 | 3530748 | 3681106 | 3963713 |
| 1040000 | 4096352 | 4004104 | 4050228 | 1040000 | 3863392 | 3684304 | 3773848 | 3912038 |
| Av. (ng/ml) | 3895684 | 4069618 | 3982651 | Av. (ng/ml) | 3773484 | 3594708 | 3684096 | 3833373 |
| STD | 338367.5 | 234486.7 | 274313.5 | STD | 86576.66 | 65369.65 | 63369.86 | 154459.6 |
| %CV | 8.685703 | 5.761884 | 6.887712 | %CV | 2.294343 | 1.818497 | 1.720092 | 4.029338 |
| Av. (mg/ml) | 3.895684 | 4.069618 | 3.982651 | Av. (mg/ml) | 3.773484 | 3.594708 | 3.684096 | 3.833373 |


SDS-PAGE blots:

FIX-CTP$_3$ γ-carboxylated enriched protein, rhFIX and rFIXa (activated FIX) were loaded on 12% Tris-Glycine gel using Precision Plus Dual Color Protein Marker (Bio-Rad). The SDS-PAGE Coomassie analysis was performed by staining the gel with Coomassie blue reagent (800 ng of protein) (Figure 12). A Western immunoblot was performed using 100 ng of protein with anti-human FIX polyclonal Ab (Figure 12B), anti-human gamma carboxylation monoclonal antibody (American Diagnostics Cat #499, 3570) (Figure 12C), anti-FIX pro-peptide polyclonal Ab (Figure 12D), and anti-CTP polyclonal Ab (Figure 12E). As previously reported, FIX-CTP$_3$ migrated at 75KDa.

The purification procedure significantly enriched FIX-CTP$_3$ portion while reducing impurities. The purification process yield was very low ranging around 2-3% (data not shown) due to the requirement to collect only the γ-carboxylated FIX-CTP$_3$ fractions, as demonstrated in the anti-Gla immunoblot (Figure 12B). Based on the Coomassie and FIX immunoblot, the FIX-CTP$_3$ portion is only around 60-70%, and additional lower molecular weight bands, presumably with lower glycosylation forms, were also detected.

**FIX-CTP$_3$ clotting activity:**

FIX-CTP$_3$ chromogenic activity:

A comparative assessment of the *in vitro* potency of FIX-CTP$_3$ harvest and FIX-CTP$_3$ γ-carboxylated enriched protein, versus human pool normal plasma was performed using a commercially available chromogenic activity test kit, BIOPHEN (Hyphen BioMed 221802). FIX-CTP$_3$ harvest and protein were serially diluted, and the potency was assessed by comparing a dose-response curve to a reference preparation consisting of normal human plasma. As previously demonstrated, FIX-CTP$_3$ harvest was 50 times less active then human pool plasma (Table 19, Figure 13). Following FIX-CTP$_3$ purification, the chromogenic activity was significantly improved and was only 4.72 times less active then human pool plasma (Table 19, Figure 13). Harvest reduced chromogenic activity can be a consequence of improper post-transcriptional modifications of FIX protein variants, e.g. inappropriate gamma carboxylation and pro-peptide cleavage. Following purification and enrichment of the FIX-CTP$_3$ γ-carboxylated fraction, the activity was improved, demonstrating the important contribution of γ-carboxylation to FIX activity.

**Table 19**: FIX-$CTP_3$ chromogenic activity

| Sample | $EC_{50}$ (ng/ml) | Sample /plasma $EC_{50}$ ratio |
|---|---|---|
| FIX-$CTP_3$ Harvest | 741.3 | 54.4 |
| Pur. FIX-$CTP_3$ | 64.6 | 4.72 |
| Plasma | 13.63 | 1 |

One stage clotting assay (aPTT):

The activated partial thromboplastin time (aPTT) is a measure of the integrity of the intrinsic and common pathways of the coagulation cascade. The aPTT is the time, in seconds, for plasma to clot following the addition of an intrinsic pathway activator, phospholipid and calcium. The principal of the assay was to quantitate the ability of FIX-$CTP_3$ to restore the clotting activity of FIX-depleted human plasma by the addition of rhFIX. 200 μl of FIX-deficient human plasma was mixed with 25 μg/ml of FIX-$CTP_3$ and further diluted in TBS. Following a 60 second incubation at 37°C, 50 μl of PTT activator (Actin FS) and 50 μl of calcium 25 mM were added to the mixture, and the clotting time in seconds was determined using a Sysmex® CA 1500 Coagulator (performed by Sheba hospital, National Coagulation Center using validated aPTT assay). The potency was assessed by comparison of FIX-$CTP_3$ to the dose-response curve of a reference preparation of normal human pool plasma. The results are expressed in percent of activity interpolated from the standard curve covering FIX levels of <1-110%. FIX-$CTP_3$ exhibited a 15-20-fold reduction in its coagulation activity versus normal human pool plasma since the activity at 5 μg/ml, which is the normal value of FIX in the body, was shown to be 6.5% (Table 20).

**Table 20:** FIX-$CTP_3$ clotting activity

| | **FIX Concentration by provider (mg/ml)** | **Concentration in tested sample (μg/ml)** | **FIX % of activity** (normalized to human normal pool plasma) |
|---|---|---|---|
| **FIX-$CTP_3$** | 3.83 | 25 | 34.7 |
| | | 5 | 6.5 |

FIX-$CTP_3$ also exhibited increased clotting time compared to BeneFIX® (Table 21 and Figure 14).

**Table 21**: Comparative clotting time (aPTT)

| Clotting time | | |
|---|---|---|
| | FIX-$CTP_3$ | BeneFIX® |
| **38ug/ml** | 77.6 | |
| **19ug/ml** | 83.4 | |
| **7.6ug/ml** | 93.2 | 50.6 |
| **3.8ug/ml** | 104.8 | 57.6 |
| **1.9ug/ml** | 112.2 | 63.7 |
| **0.95ug/ml** | 122.6 | 71.5 |
| **0.475ug/ml** | | 83.7 |
| **0.238ug/ml** | | 94.3 |

An additional clotting assay was performed independently in FIX-deficient mice by Dr. Paul Monahan at University of North Carolina prior to the initiation of the PK-PD study. The aPTT results suggested that FIX-$CTP_3$ coagulation activity is 40 times less then normal pooled human plasma as demonstrated by the longer period (as measured in seconds) and higher concentration that are required for proper clotting activity (Table 22).

**Table 22**: Comparative clotting activity

| FIX activity (Units) | | |
|---|---|---|
| | FIX-$CTP_3$ | BeneFIX® |
| **38ug/ml** | 13.9 | |
| **19ug/ml** | 8.8 | |
| **7.6ug/ml** | 4 | 116.8 |
| **3.8ug/ml** | 1.6 | 67.4 |
| **1.9ug/ml** | 0.9 | 41.7 |
| **0.95ug/ml** | 0.4 | 22.4 |
| **0.475ug/ml** | | 8.5 |
| **0.238ug/ml** | | 3.7 |

The specific activity (u/ml), which was based on FIX antigen level as calculated by ELISA for FIX-$CTP_3$ and BeneFIX®, was 4.46 and 198.9 respectively.

The inconsistency in the calculated FIX-$CTP_3$ activity as demonstrated in the chromogenic vs. aPTT assays can be explained by the superior sensitivity of the aPTT assay and *in vivo* relevance. In the chromogenic activity assay, an excess amount of reagents and enzymes are present which can activate less potent FIX versions. The difference in the FIX-CTP specific activity values can be explained by the use of different reagents and automated machines. The activity value as calculated at University of North Carolina was used for the PK-PD study design.

**FIXa Protein detection:**

In order to confirm that following the purification process, FIX activation (FIXa) did not occur, a FIXa detection assay was performed using FIXa Biophen Chromogenic Assay (Cat. # Ref. 221812). The assay measures the amount of FIXa present in a specific sample using the chromogenic activity cascade, as previously described. FIX-$CTP_3$ and rhFIX were diluted and FIXa levels were evaluated. FIX-$CTP_3$ wasn't activated through purification or storage (Table 23).

**Table 23**: FIXa detection

| **Sample** | **FIX-$CTP_3$** | **rhFIX** |
|---|---|---|
| **Initial** | **1000** | **5.7** |
| **Con.(mg/ml)** | | |
| **rFIXa (mg/ml)** | **BLQ** | **0.00487** |
| **%FIXa in sample** | **BLQ** | **0.085** |

**FIX-CTP$_3$ PK-PD study:** FIX-CTP$_3$ and rhFIX (BeneFIX®) were administered in a single intravenous injection to C57Bl FIX-deficient mice in a dose of 625 μg/kg body weight containing 100 IU FIX/kg body weight. Blood samples were drawn retro-orbitally from 3 mice alternately at 0.25, 4, 24, 48, 72, and 96 hours post-dosing. Citrated plasma (0.32%) was prepared immediately after sampling and stored at -20'IC until analysis. hFIX antigen level was evaluated, and a detailed PK analysis was performed. In order to evaluate the ability of FIX-CTP$_3$ to elongate the clotting activity of FIX-deficient animals compared to BeneFIX®, FIX activity in citrated plasma samples, collected from the FIX-/- treated mice, was calculated using an automated FIX activity assay (Table 24).

**Table 24:** Study outline

| | Product | Administ ration | Dose | # mic e | Collection Points (hr post-dosing) | Required amount |
|---|---|---|---|---|---|---|
| ****Coh ort 1** | FIX-CTP$_3$ | Single dose: IV | 100IU/Kg 2.5IU/mous e (553μg/mou se) | 12 mic e, | 0.25, 1, 4,8, 16, 24, 48 | **6636μg** |
| | | | | | | |
| **Cohort 2** | FIX-CTP$_3$ | Single dose: IV | **472μg/K g 12.57μg/m ouse | 18 mice | *0.25,1*, 4*,8 *,16*, 24*, 48*, 72*,96* | **200μg 12.57μg/mou se** |
| | | | | | | |
| ****Coh ort 3** | BeneFIX ® | Single dose: IV | 100IU/Kg 2.5IU/mou se | 18 mice, | 0.25, 1,4,8,16, 24, 48, *72,*96 | **226.3 μg 12.57μg/mou** se |
| ** PK collection points only*<br>*** Tail vein bleeding at T=48 post-dosing; cohorts 1 & 3* | | | | | | |

FIX-CTP$_3$ Pharmacokinetic profile in FIX$^{-/-}$ mice

FIX concentration was quantitated using human FIX ELISA kits (Affinity Biologicals; Cat. # FIX-AG RUO). The pharmacokinetic profile was calculated for each protein and is the mean of three animals at each time point. Table 25 below and Figure 15 summarize the calculated FIX concentrations at the different sampling time points for Cohorts 1 & 3. The PK profile and a summary of the PK parameters are presented below (Tables 26 & 27). A PK analysis was also performed for Cohort #2 in order to verify exposure (data not shown).

**Table 25**: FIX concentrations

| **Time point(hr)** | **FIX-CTP$_3$ ng/ml** | **BeneFIX® ng/ml** |
|---|---|---|
| **0.25** | 3645.397 | 2823.023 |
| **1** | 2411.09 | 2416.248 |
| **4** | 1703.205 | 1506.228 |
| **8** | 1139.736 | 864.764 |
| **16** | 415.32 | 347.465 |
| **24** | 238.37 | 158.7973 |
| **36** | 141.0105 | 94.40067 |
| **48** | 95.461 | 42.28833 |
| **72** | 76.90953 | 11.87567 |
| **96** | 24.955 | BLQ |

A two-compartmental module was used (WinLin software) to determine AUC0-inf, $T_{terminal}$ and clearance (CL). The PK parameters are described below in Table 26.

**Table 26**: PK properties

| FIX Version | T½α (1/hr) | T½ β (1/hr) | AUC ng/ml*hr | CL ml/Kg/hr | MRT (hr) | Vss (ml/Kg) |
|---|---|---|---|---|---|---|
| **BeneFIX®** | 3.4 | 12.7 | 22428 | 29 | 11.5 | 320.8 |
| **FIX-CTP3** | 4 | 28.7 | 31770 | 19 | 22 | 425.2 |

The addition of the three CTP "cassettes" to rhFIX elongated FIX half-life *in vivo* by at least 2.5-fold. AUC following *in vivo* FIX-CTP$_3$ administration increased 2-fold versus rhFIX. FIX-CTP$_3$-injected mice demonstrated an improved PK profile compared to BeneFIX®-injected mice.

FIX-CTP$_3$ Pharmacodynamic profile in FIX-deficient mice:

In parallel to PK sampling, FIX-deficient animals administered with either BeneFIX® or FIX-CTP$_3$, citrated plasma samples, were evaluated for their clotting activity by aPTT assay, which was translated to % activity. The % activity at each collection point was calculated as the current clotting time/clotting time of normal pool mice plasma*100. Table 27 summarizes the activity values following administration of either BeneFIX® or FIX-CTP$_3$.

Following FIX-CTP$_3$ administration, significant clotting activity was detected one hour after administration reaching 96% activity at four hours post-dosing, while BeneFIX® highest activity value was 40% (Table 27, Figure 16). FIX-CTP$_3$ clotting activity was maintained for a longer period of time, demonstrating elongated activity. Clotting activity for the BeneFIX®-treated mice was undetectable at time points later then 36 hours, while FIX-CTP$_3$-treated mice continued to retain measurable activity at 72 hours post-dosing (Table 27, Figure 16). Analysis of the % clotting pharmacokinetic profile suggest that FIX-CTP$_3$ clotting activity is maintained for a significantly longer period and its half life is almost 2-fold higher than Benefix® (Table 28).

**Table 27**: FIX % of activity

| Hr post-dosing | BeneFIX® % of activity | FIX-CTP3 % of activity |
|---|---|---|
| **0.25** | 39.9 | 1.0 |
| **1** | 33.4 | 15.5 |
| **4** | 24.9 | 93.6 |
| **8** | 18.8 | 65.2 |
| **16** | 10.3 | 39.9 |
| **24** | 1.7 | 11.9 |
| **36** | 1.4 | 11.0 |
| **48** | <1 | 4.6 |
| **72** | <1 | 1.4 |

**Table 28**: Clotting Activity

| FIX Version | T½α (1/hr) | T½ β (1/hr) |
|---|---|---|
| BeneFIX® | 5.7 | ----- |
| $FIX-CTP_3$ | 7.3 | 16 |

9.3 FIX-deficient mice bleeding challenge

FIX-deficient mice were administered a single intravenous injection of 100 IU/kg of BeneFIX® or $rFIX-CTP_3$. The tail vein was slightly clipped 48 hours post-dosing, and tail vein bleeding time (TVBT) and bleeding intensity (hemoglobin OD) were evaluated. A second bleeding challenge was performed 15 minutes after reaching homeostasis, and the same parameters were measured. Following the first bleeding challenge, $FIX-CTP_3$-administered animals' bleeding was significantly less intense then BeneFIX® bleeding as demonstrated by the Hemoglobin OD values (Figure 17).

Since it was previously reported that during the first bleeding challenge in hemophilic mice, the bleeding time does not necessarily correlate with treatment efficacy, it is recommended to evaluate the homeostasis following additional bleeding. Once the first bleeding was spontaneously or manually stopped, a second bleeding challenge was performed 15 minutes following the first one, and the time and bleeding intensity were re-measured. During the second bleeding episode $FIX-CTP_3$-administered animals had reduced bleeding time and intensity, demonstrating that $FIX-CTP_3$ was potent at a later time points (Figure 18).

Finally, the animals were further observed for the 12 hours following the second bleeding challenge, and all recurring bleeding events were documented. $FIX-CTP_3$-administered animals were able to maintain blood homeostasis for the next 12 hours with no re-occurring bleeding events. In contrast, 50% of BeneFIX®-treated mice had spontaneous bleeding episodes from the tail (Table 29).

**Table 29:** Outcome 12 hours after tail transection

| Mouse group | Delayed rebleeding | Death or Distress Requiring Euthanasia |
|---|---|---|
| $FIX-CTP_3$ (100IU/kg) | 0/5 (0%) | 0/5 |
| BeneFIX® (100IU/kg) | 3/6 (50%) | 0/6 |
| FIX-/- (untreated) | 5/6 (100%) | 1/6 |

Recombinant $FIX-CTP_3$, a fusion protein comprised of a single molecule of FIX fused to three CTP "cassettes" in tandem was developed to address the short half-life of currently available FIX products used to treat patients with hemophilia B. We have demonstrated that the elimination half-life of $rFIX-CTP_3$ was consistently 2.5- to 4-fold longer than rFIX in rats (as previously reported) and in FIX-deficient mice.

Without being bound by theory, the fusion protein reduces clearance of FIX and protects FIX from protease activity, degradation by masking and reduces the affinity of FIX for hepatic receptors. Taken together these characteristics of the CTP domain extend the half-life of FIX.

In addition to pharmacokinetic analysis of $rFIX-CTP_3$, we examined the pharmacodynamic properties of $FIX-CTP_3$ in FIX-deficient mice. $rFIX-CTP_3$ and rFIX, were administered at comparable doses (in units) to compensate for the clotting deficiency levels in FIX-deficient mice. However, the effect of $rFIX-CTP_3$ in FIX-deficient mice was significantly prolonged to at least 76 hr after dosing, reaching a higher activity peak. $FIX-CTP_3$ clotting activity began after a 1-hour delay compared to BeneFIX®. FIX activation may be required since the addition of three tandem CTPs might theoretically mask the activation site and delay cascade onset. Following $FIX-CTP_3$ administration, a 100% peak activity was observed, while BeneFIX® activity was only 40%. The superior initial activity is a very important parameter and demonstrates that addition of 3 CTPs has the potential to improve recovery.

Prophylactic FIX replacement therapy for patients with hemophilia B aims to maintain plasma levels of 1-2% normal clotting activity. The tail vein bleeding assay is a sensitive *in vivo* test that measures the ability to maintain bleeding homeostasis at low activity values mimicking human bleeding homeostasis model. In response to tail vein bleeding challenge 48 hours post-dosing, $rFIX-CTP_3$-administered animals maintained blood homeostasis with shorter and less severe bleeding episodes, demonstrating sustained clotting activity.

FIX is a complex protein that contains a number of functional domains which undergo extensive post-translational modifications. One of the essential post-translational modifications for FIX activity is gamma-carboxylation of the first 12 glutamic acids in the Gla domain by vitamin K-dependent $\gamma$-glutamyl carboxylase. This modification facilitates the binding of FIX to phospholipid membranes and, thus, is critical to its function. FIX that is not gamma-carboxylated is not functional, and hence gamma-carboxylation is a rate-limiting step.

This PK-PD study was conducted using transiently transfected cells. An extensive analytical evaluation of post-translational modifications is performed on the stable FIX-$CTP_3$ protein produced and secreted from stable optimized clone.

Based on the presented data, FIX-$CTP_3$ coagulation factor has the potential to reduce the frequency of injections in patients receiving routine prophylactic doses of FIX replacement therapy. It is anticipated that rFIX-$CTP_3$ can confer prolonged protection from bleeding following each dose of factor, decrease the overall units of factor needed to treat bleeding episodes, and/or maintain adequate hemostasis during surgical procedures with fewer injections.

### *EXAMPLE 4*

### *Generation and utilization of Coagulation Factor FVII*

A long-acting version of activated Factor VII (FVIIa) coagulation factor will be useful for the treatment of patients with hemophilia A and B. FVIIa-$CTP_3$ recombinant protein has the clinical potential to improve the treatment of hemophilia patients by reducing the frequency of infusions and even by reducing the drug load, enabling a prophylactic treatment approach which can significantly improves a patient's quality of life, avoid spontaneous bleeding episodes and accumulated damage to the joint and other organs.

The generation of a recombinant FVIIa-CTP molecule with an extended half-life based on fusion of FVII to a human CTP is described herein. The recombinant FVIIa-CTP was expressed in mammalian cells and characterized *in vitro* and *in vivo.* It was demonstrated that rFVII-CTP activity was comparable to rFVII. Pharmacokinetic and efficacy studies in rats demonstrated improved properties of rFVII-CTP. The results of this study demonstrated that it is feasible to develop a half-life extended rFVIIa molecule with very similar haemostatic properties to the wild-type enzyme.

**Cloning and expression of recombinant FVII molecule:** Several Factor VII clones were constructed in our eukaryotic expression vector (pCI-dhfrr) (Figure 19). Human MGC verified FL cDNA clone (IRCM) containing the sequence of *homo sapiens* coagulation Factor VII was ordered from "Open Biosystems" (OB-MHS4426). The following primers were synthesized by Sigma-Genosys in the following sequence: Primer 67: 5'CTCGAGGACATGGTCTCCCAGGCCC3' (contains the 5' end of Factor VII DNA and the restriction site of XhoI) (SEQ ID NO: 5); Primer $68^R$: 5' TCTAGAATAGGTATTTTTCCACATG3' (contains the restriction site of XbaI) (SEQ ID NO: 6); Primer 69: 5' TCTAGAAAAAAGAAATGCCAGC3' (contains the restriction site of XbaI) (SEQ ID NO: 7); and Primer $70^R$: 5'GCGGCCGCATCCTCAGGGAAATGGGGCTCGCA3' (contains the 3' end of Factor VII DNA and the restriction site of NotI) (SEQ ID NO: 8).

Cloning was performed in two sets of PCR reactions. The first reaction was conducted with primer 67 and primer $68^R$ using a cDNA plasmid with the Factor VII sequence (OB-MHS4426) as a template; as a result of the PCR amplification, a -534 bp product was formed, isolated and ligated into a TA cloning vector (Invitrogen, Catalog No: K2000-01). A XhoI -XbaI fragment containing the amino terminus of the Factor VII sequence was isolated. The second reaction was conducted with primer 69 and primer $70^R$ and again, a cDNA plasmid with the Factor VII sequence (OB-MHS4426) was used as a template. As a result of the PCR amplification, a -813 bp product was formed and ligated into TA cloning vector (Invitrogen, Catalog No: K2000-01). A XbaI-NotI fragment containing the carboxy terminus of Factor VII sequence was isolated. The two fragments were inserted into our eukaryotic expression vector pCI-dhfr (triple ligation) to yield the 501-0-p136-1 clone.

Plasmid 501-p136-1 (Factor VII in pCI-dhfr vector) was digested with restriction enzymes XhoI and KpnI. A fragment of -1186 bp was isolated. A partial Factor VII clone (1180 bp-1322 bp) followed by a CTP sequence, termination sequence and NotI sequence that was synthesized by GeneArt (0721543) was digested with restriction enzymes KpnI and NotI. A fragment of -253 bp was isolated. The two fragments were inserted into our eukaryotic expression vector pCI-dhfr (triple ligation) to yield the 501-1-p137-2 clone. pCI-dhfr-701-2-p24-2 was digested with restriction enzymes XhoI and ApaI, and the large fragment (vector) was isolated.

pCI-dhfr-501-2-p137-2 (Factor VII-ctp x1) was digested with restriction enzymes XhoI and ApaI, and a -1200 bp insert was isolated. The vector and insert were ligated to yield 501-2-p139-2. Dg44 cells were plated in 100 mm tissue culture dishes and grown to confluence of 50-60%. A total of 2 $\mu$g of DNA was used for transfection of one 100 mm plate using the FuGene reagent (Roche) in protein-free medium (Invitrogen CD Dg44). The medium was removed 48 hours post-transfection and replaced with a protein-free medium (Invitrogen CD Dg44) without nucleosides. After 14 days, the transfected cell population was transferred into T25 tissue culture flasks, and the selection was continued for 10-14 days until the cells began to grow well as a stable clone. High-expressing clones were selected and approximately

$2x10^7$ cells were used to inoculate 300 ml of growth medium in a $1700cm^2$ roller bottle (Corning, Corning NY) supplemented with 5 ng/ml of Vitamin K3 (menadione sodium bisulfate; Sigma). The production medium (harvest) was collected after a rapid decrease in the cell viability to around 70%. The production medium was first clarified and then concentrated approximately 20-fold and dialyzed to PBS using flow filtration cassette (10KDaMWCO; Millipore Corp, Billerica, MA).

**Determination of FVII antigen level**

The cDNA coding the CTP peptide was fused to the 3' end of the cDNA coding human FVII. The corresponding rFVII construct was transfected into Dg44 cells. As a control, a human rFVII cDNA was utilized. The production medium (harvest) was collected, concentrated and the secreted recombinant FVII was further evaluated. rFVII, rFVII-CTP and rFVII-CTP-CTP antigen levels were determined by AssayMax Human FVII ELISA kit (AssayPro) (Figure 20A). There was no significant difference in the secretion level of rFVII-CTP and rFVII-$(CTP)_2$ compared to native rFVII.

**SDS-PAGE blots**

SDS-PAGE analysis was done by loading 50 ng of either harvest, purified or activated rFVII protein. Samples were loaded on 12% Tris-Glycine gel using Precision Plus Dual Color Protein Marker (Bio-Rad). The SDS-PAGE analysis was done by performing a Western immunoblot using an anti-human FVII monoclonal antibody (Ab) (R&D systems) or anti-CTP polyclonal antibody generated in Rabbit.

The level of rFVII antigen correlated with the detected protein level in a SDS-PAGE immunoblotted with anti-FVII Ab. rFVII-CTP migrated as a single band, while the corresponding molecular weight of the FVII control was approximately 52 KDa (data not shown). Both proteins reacted with antibodies specific for FVII on immunoblots. The rFVII-CTP also reacted with antibodies specific for CTP. rFVII was secreted in its zymogene form with no trace of activated protein.

**FVII Chromogenic activity:**

rFVII, rFVII-CTP and rFVII-$(CTP)_2$ harvest activities were determined using a commercially available chromogenic test kit (AssaySense Human FVII Chromogenic Activity Assay Kit (AssayPro). For functional characterization of the rFVII-CTP and its ability to be further activated (FVIIa), concentrated rFVII-CTP (harvests) were placed in a commercially available chromogenic test kit that measure the ability of TF/FVIIa to activate Factor X to Factor Xa that in the presence of FXa specific substrate releases a quantitated signal (AssayPro). The addition of the CTP peptide at the C-terminal of the rFVII protein did not impair the FVII serine protease activity (Figure 20B, 20C).

**FVII clotting activity:**

Prothrombin time (PT) measures the extrinsic pathway of coagulation. The PT is the time (measured in seconds) it takes plasma to clot following the addition of an extrinsic pathway activator, phospholipid and calcium. It is used to determine the clotting tendency of blood, specifically in the measure of warfarin dosage, liver damage, and vitamin K status. The reference range for prothrombin time is usually around 12-15 seconds. Specifically, the assay quantitated the ability of FVII-CTP and FVII-$(CTP)_2$ harvest to restore the clotting activity of FVII-depleted human plasma by the addition of rhFVII. 300 $\mu$l of FVII-deficient human plasma was mixed with 100 $\mu$l of FVII, FVII-CTP and FVII-$(CTP)_2$ harvests at specific concentrations, or normal pool human plasma and were further diluted. Following a 60 second incubation at 37°C, Tissue Factor (TF), $CaCl_2$, and phospholipids were added to the mixture. The clotting time in seconds was determined. Potency was assessed by comparing a dose-response curve of FVII-CTP and FVII-$(CTP)_2$ harvests to a reference preparation consisting of rhFVII or human pool plasma. One unit of active FVII was defined as the amount of FVII which equals to the activity of one ml human normal plasma. The PT Clotting activity of rFVII and rFVII-CTP was measured on a coagulometer (Instrumentation Laboratory).

As previously shown, the addition of a CTP peptide at the C-terminal of the rFVII protein did not damage its serine protease activity and lead to the initiation and activation of a native Factor X and Factor IX in human plasma. Following the insertion of an additional CTP at the C terminal, there was a three-fold reduction in the serine protease activity (data not shown).

**Pharmacokinetics study:**

rFVII, rFVII-CTP, and rFVII-$(CTP)_2$ harvests were administered intravenously to Sprague-Dawley rats (six rats per substance) with a dose of 100$\mu$g/kg body weight. For all of the *in vivo* experiments, the amount of the respective protein was determined on the basis of FVII ELISA kit. For each FVII test substance, the injected amount was calculated by taking into account the differences in the molecular weight of rFVII versus rFVII-CTP, which leads to a different molar concentration.

Blood samples were drawn retro-orbitally using an altering sampling scheme to minimize interference of the sampling procedure levels to be quantified: from 3 rats at 30 and 90 min and at 2, 6, and 48 hrs, and from the remaining three rats at 15 and 60 min and at 1.5, 4, and 24 hrs alternately. Plasma was prepared immediately after sampling and stored at -20°C until analysis. FVII concentration was quantified by FVII ELISA specific assay. Half-life and area under the curve (AUC) were calculated using a linear trapezoidal rule. Comparison of these clearance parameters revealed that the *in vivo* half-life and rFVII-$(CTP)_2$ AUC are significantly higher than those of rFVII (Table 30).

**Table 30**: PK study parameters

| Group | Route | Dose | T½ | $AUC_{0-t}$ | CL/F | MRT |
|---|---|---|---|---|---|---|
| | | µg/kg | min | ng/min/mL | mL/min/kg | min |
| **FVII** | IV | 60 | 4.07 | 3314.7 | 6.195 | 6.2 |
| **FVII-CTP** | IV | 60 | ß=51.06 | 31353.9 | 0.287 | 73.7 |
| **FVII-CTP-CTP** | IV | 60 | ß=13.66 | 7626.8 | 1.18 | 15.4 |

**Characterization of recombinant FVIIa-CTP:**

During activation, FVII is cleaved at R152 resulting in heavy and light chain domains that are held together by a single disulfide bridge. rFVIIa-$(CTP)_2$ is purified and activated by an ion exchange column purification process. In order to fully evaluate rFVIIa-$(CTP)_2$, the protein is loaded on SDS-PAGE under reducing conditions to commercial FVIIa (NovoSeven®). The heavy and the light chain domains are separated and migrate as separated bands of molecular weights 55 and 25 KDa. Both proteins react with antibodies specific for FVII, but the heavy chain of the rFVIIa-CTP specifically reacts with anti-CTP-specific antibodies, indicating that this band represents the FVII heavy chain fused to CTP. The light chain reacts specifically with anti-gamma carboxylase Ab. The FVIIa protein concentration is determined by FVIIa-specific ELISA kit.

**FVIIa N-terminal sequencing:**

rFVII-CTP-CTP in activated or zymogene purified proteins is separated by SDS-PAGE (on 12% Tris-Glycine) and subsequently electroblotted to a PVDF membrane. The bands of interest are cut out and put on a purified Biobrene-treated glass fiber filter. The N-terminal sequence analysis is carried out by Edmann degradation using a pulsed liquid protein sequencer equipped with a 140C HPLC microgradient system. The identity of the recombinant protein and proper pro-peptide cleavage is further verified by N-terminal sequencing.

**FVIIa clotting activity:**

In order to evaluate FVII-$(CTP)_2$ coagulation activity, activated partial thromboplastin time assay (aPTT) is performed. FVII-deficient plasma sample is substituted with rFVIIa (NovoSeven®) or rFVIIa-$(CTP)_2$. 300 µl of FVII deficient human plasma is mixed with 100 µl of FVIIa or rFVIIa-$(CTP)_2$ at specific concentrations, or normal pooled human plasma which is further diluted. Following 60 seconds incubation at 37°C. Tissue Factor (TF), $CaCl_2$, and phospholipids are added to the mixture. Clotting time in seconds is determined. Potency is assessed by comparing a dose-response curve of rFVIIa-$(CTP)_2$ to a reference preparation consisting of rhFVIIa or human pool normal plasma. One unit of FVIIa is defined as the amount of FVIIa which equals to the activity of 1 ml human normal plasma. The aPTT clotting activity of rFVII and rFVIIa-$(CTP)_2$ is measured on a coagulometer (Instrumentation Laboratory). The aPTT clotting activity of rFVIIa and rFVIIa-$(CTP)_2$ is similar.

**Pharmacokinetics studies in rats:**

In order to characterize the influence of the CTP addition to the rFVIIa on its longevity potential, a comparative pharmacokinetic study in rats is performed. NovoSeven® (rFVIIa) and rFVIIa-$(CTP)_2$ in TBS are injected IV to 6 SD rats. The levels of FVIIa over time are detected using a FVIIa ELISA kit. The half-life and AUC are calculated for each protein. Comparison of these clearance parameters reveals that the *in vivo* measures of half-life, recovery, and AUC of the rFVIIa-$(CTP)_2$ are superior to those of NovoSeven®.

**FVIIa-CTP *in vivo* efficacy model (FVIII-deficient mouse model of hemophilia):**

In order to assess the *in vivo* activity model, FVIII knockout mice are obtained, and a breeding colony is established. 10 μg of either commercial recombinant hFVIIa (NovoSeven®) or rFVIIa-$(CTP)_2$ are injected into the tail vein of an anaesthetized FVIII knockout mouse (22-28g). The amount of injected protein equals to the required concentration of FVIII in normal plasma (5μg/ml). Blood samples are taken from the clipped tail into heparinized capillary tubes at specific time points. Plasma samples are assessed for FVIIa levels by ELISA, and efficacy is measured by a PTT coagulation assay.

In this study, a fusion construct of FVII with CTP is generated. This recombinant protein is the basis for a treatment that provides a prolonged half-life and retention of therapeutic potency.

These results suggest that rFVIIa-$(CTP)_2$ has a similar therapeutic efficacy to rFVIIa in hemophilia patients. Moreover, this technology requires less frequent dosing. It appears that a single injection of rFVIIa-$(CTP)_2$ is sufficient to control bleeding episodes and reduce the number of injections that are needed during surgical intervention. This recombinant protein may be used as a long term prophylactic treatment.

***EXAMPLE 5***

***Comparative assessment of Purified FVII-$CTP_3$, FVII-$CTP_4$, and FVII-$CTP_5$***

**5.1 Study objective**

Comparative assessment of pharmacokinetic parameters and clotting activity of FVII-$CTP_4$ and FVII-$CTP_5$ versus FVII-$CTP_3$.

**5.2 Production of FVII-$CTP_4$ and FVII-$CTP_5$ harvests**

FVII cDNA fused at the C-terminal to four or five tandem CTP sequences was expressed in Dg44 cells using the Excellgene expressing system in the presence of 20 μg/L of vitamin K3 (Sigma, Mennadion). The harvest was collected (300 ml), filtered and frozen.

**5.3 Production of FVII-$CTP_3$ harvest**

FVII-$CTP_3$ was expressed in-house in mammalian expressing system, CHO cells, using pCI-DHFR vector. Stable transfected pool #71 was grown in shake flasks, in the presence of 25 ng/L of vitamin K3 (Sigma). The harvests were collected and filtered.

All FVII-CTP harvests (3, 4 and 5 CTPs) were concentrated and dialyzed against TBS (50 mM Tris, 150mM NaCl, pH 7.4) using Pellicon XL MWCO 10kDa.

**5.4 Determination of FVII antigen level**

FVII antigen level was determined using Human FVII ELISA kit (Zymotest HyPhen) (Table 31). The calculated protein concentration is the average of two independent runs.

**Table 31**: FVII antigen level

| | FVII-$CTP_3$ | FVII-$CTP_4$ | FVII-$CTP_5$ |
|---|---|---|---|
| Av. (ng/ml) | 224357.3 | 87884.1 | 589423 |
| **SD** | 44789.5 | 3248.7 | 5309 |
| %CV | 20.0 | 3.7 | 9 |

**5.5 FVII-CTP immune-blot**

FVII-$CTP_3$, FVII-$CTP_4$, and FVII-$CTP_5$ harvests were loaded on 12% Tris-Glycine gel (*expedeon*) using Precision plus dual color protein marker (Bio-Rad). The SDS-PAGE analysis was performed by Western immune-blot using anti-CTP polyclonal Ab (Adar Biotech Production) or anti-Gla Ab (American Diagnostica).

FVII fused to three, four and five CTP migrated at 80, 90 and 100kDa, respectively. As expected, FVII-$CTP_4$ and FVII-$CTP_5$ harvests from Excellgene contain low gamma carboxylation content as compared to FVII-$CTP_3$ harvest which was produced at Prolor since the production process wasn't optimized (Figure 21).

**5.6 Comparative assessment of FVII *in vitro* potency**

A comparative assessment of the *in vitro* potency of HA purified (highly gamma carboxylated fraction) FVII-$CTP_3$, FVII-$CTP_4$, and FVII-$CTP_5$ versus normal human pool plasma was performed using a commercially available chromogenic activity test kit, BIOPHEN (Hyphen BioMed 221304). All samples were serially diluted, and the potency was assessed by comparing a dose-response curve to a reference preparation consisting of normal human plasma. FVII-$CTP_3$ and FVII-$CTP_5$ demonstrated chromogenic activity lower than pooled normal plasma (Figure 22). FVII-$CTP_4$ demonstrated higher activity as reflected by EC50 ratios, compared to FVII-$CTP_3$ and FVII-$CTP_5$ (Table 32).

**Table 32:** FVII *In Vitro* Clotting Activity

| Sample | EC50 (ng/ml) | Sample /plasma EC50 ratio |
|---|---|---|
| **Plasma** | 0.05 | |
| **FVII 3CTP** | 0.12 | 2.72 |
| **FVII 4CTP** | 0.03 | 0.71 |
| **FVII 5CTP** | 0.06 | 1.35 |

**5.7 FVII *In Vitro* Clotting Activity:**

Factor VII (FVII) activity assay, which was performed in Sheba Medical Center, the Israel National Coagulation Center, is a prothrombin (PT)-based assay using immunoadsorbed plasma deficient in Factor VII (Siemens). The PT reagent is innovin, and the assay is performed in the Sysmex® CA 1500 instrument. FVII normal range is within 55-145%.

**Table 33:** FVII *In Vitro* Chromogenic Activity

| Sample | FVII % of activity | Concentration in tested sample (μg/ml) | Concentration (μg/ml) |
|---|---|---|---|
| **FVII 3CTP** | 36 | 0.5 | 224.2 |
| | 18 | 0.25 | |
| | 6 | 0.125 | |
| **FVII 4 CTP** | 334 | 0.5 | 87.9 |
| | 176 | 0.25 | |
| | 93 | 6.25 | |
| **FVII 5 CTP** | 38 | 0.5 | 58.9 |
| | 19 | 0.25 | |
| | 10 | 0.125 | |

Since the normal level of circulating FVII in the body is around 0.5 μg/ml, FVII-$CTP_3$ and FVII-$CTP_5$ harvests exhibit 3-fold reductions in their coagulation activity versus normal human pool plasma; this result correlates with the obtained chromogenic activity (Table 33).

The FVII-$CTP_4$ harvest exhibits a 3-fold increase in its potential coagulation activity versus normal human pool plasma as observed in the chromogenic activity assay (Table 33). The activity percentage of FVII-$CTP_4$ is much higher compared to activity percentage of FVII-$CTP_3$ and FVII-$CTP_5$. Methodological limitations of the ELISA method may limit the accuracy of Ag level calculations of FVII-$CTP_4$.

**5.8 Pharmacokinetic study**

Two pharmacokinetic studies were performed in order to determine the FVII-$CTP_3$, FVII-$CTP_4$, and FVII-$CTP_5$ pharmacokinetics (PK) parameters. During the first study, FVII-$CTP_3$, FVII-$CTP_4$, and FVII-$CTP_5$ (Group A, B and C, respectively) were administered in a single intravenous injection to Sprague Dawley rats (six rats per treatment) in a dose of 250 μg/kg body weight. Blood samples were drawn retro-orbitally from 3 rats alternately at 0.083, 0.5 2, 5, 8, 24, 48, 72 and 96 hours post-dosing (Table 34). Citrated plasma (0.38%) was prepared immediately after sampling and stored at -20°C until analysis.

**Table 34**: Pharmacokinetic Study Design – Concentrated Harvest

| Treatment Group | Test Article | No. of animals/ group/ time point | Dose Route | Dose Level (μg per animal) | Injected Vol. (μl) | Conc. (μg/ml) | Time-Points (hours post-dose) |
|---|---|---|---|---|---|---|---|
| A | FVII-CTP*3 | 6 | IV | 50 | 200 | 250 | 0 (Pre-dose) 0.083, 0.5, 2, 5, 8, 24,48,72,96 |
| B | FVII-CTP*4 | 6 | IV | 50 | 200 | 250 | 0 (Pre-dose) 0.083, 0.5, 2, 5, 8, 24,48,72,96 |
| C | FVII-CTP*5 | 6 | IV | 50 | 200 | 250 | 0 (Pre-dose) 0.083, 0.5, 2, 5, 8, 24,48,72,96 |

FVII concentration in plasma samples were quantified using human FVII Elisa kits (Zymutest FVII-Biophen).

The pharmacokinetic profile was calculated and is the mean of 3 animals at each time point. Terminal half-life values were calculated using PK Solutions 2.0 Software. Table 35 below summarizes the calculated FVII concentrations at the different sampling time points. The PK profile (Figures 23-24) and a summary of the PK parameters (Table 36) are also presented below. FVII-$CTP_5$ demonstrated a superior profile as compared to FVII-$CTP_3$ and FVII-$CTP_4$ (Table 36).

**Table 35:** First Pharmacokinetic Study - FVII Concentrations

| Time (hr) | AVE-FVII-3-CTP (ng/ml) | SD | AVE-FVII-4-CTP (ng/ml) | SD | AVE-FVII-5-CTP (ng/ml) | SD |
|---|---|---|---|---|---|---|
| **0.083** | **4214** | 583 | **3600** | 427 | **4888** | 504 |
| **0.5** | **3386** | 892 | **5213** | 1682 | **5384** | 2549 |
| **2** | **1138** | 219 | **3603** | 1338 | **3082** | 289 |
| **5** | **1390** | 374 | **2726** | 1127 | **2480** | 561 |
| **8** | **333** | 167 | **1349** | 44 | **2316** | 633 |
| **24** | **133** | 12 | **476** | 98 | **788** | 34 |
| **48** | **38** | 3 | **165** | 24 | **384** | 61 |
| **72** | **12** | 2 | **91** | 62 | **167** | 31 |
| **96** | **26** | 1 | **42** | 8 | **93** | 49 |

**Table 36:** Pharmacokinetic Analysis

| | FVII 3-CTP | FVII-4-CTP | FVII-5CTP |
|---|---|---|---|
| half-life **(0.083-8 hr) (hr)** | 2.5 | 4.9 | 6.6 |
| half-life **(8-72hr) (hr)** | 13.3 | 16.6 | 17.7 |
| **AUC** (ng-hr/ml)**(8-72hr)** | 18374.6 | 51224.4 | 72954.2 |
| Vd **(ml/kg)(8-72hr)** | 203.7 | 91.9 | 67.7 |
| CL**(ml/hr/kg) (8-72hr)** | 10.6 | 3.8 | 2.7 |

The addition of four or five CTPs significantly elongated FVII half-life as compared to 3 CTPs by 2- and 3-fold, respectively (Table 36). This superiority was more significant in the initial part of the study (0.083-8 hr), suggesting a potential improved protein recovery and reduced extra vascular clearance. AUC following FVII-$CTP_4$ and FVII-$CTP_5$ administration increased by 3- and 4-fold, respectively, versus FVII-$CTP_3$. Clearance was also reduced while adding 4 and 5 CTPs to FVII (Table 36).

As observed in the study, the addition of four and five CTPs significantly elongated FVII half-life as compared to 3 CTPs, both in the initial and terminal half-life. The half-life values in the first and second study are different due to a different analysis approach which was effected by the dose and study duration, nevertheless the overall trend was maintained. The AUC following FVII-$CTP_4$ and FVII-$CTP_5$ administration increased by 2.5- and 7-fold, respectively, versus FVII-$CTP_3$.

**5.9 Conclusions:**

In this study, the PK parameters and potential clotting activity of FVII-$CTP_3$, FVII-$CTP_4$, and FVII-$CTP_5$ were

assessed. Fusion of 4 and 5 CTPs to FVII provided a superior and improved half-life, exposure and reduced clearance as compared to FVII-$CTP_3$ while maintaining a similar chromogenic and *in vitro* clotting activity. These results were observed at different concentrations of protein and were consistent for both harvest and purified protein. While evaluating the overall effect of fusion of CTP at the C terminus to FVII, fusion of 1-5 CTPs considerably increased the half-life and AUC of FVII in a CTP proportional manner, suggesting that as the CTP portion of the molecule increases, FVII longevity and stability is significantly improved while maintaining its initial *in vitro* clotting activity, as summarized in Table 37 hereinbelow.

**Table 37:**

| Comparative assessment | $T_{1/2}$ percent increase | AUC percent increase |
|---|---|---|
| FVII vs. FVII-$CTP_2$ | 268 | 200 |
| FVII-$CTP_2$ vs. FVII-$CTP_3$ | 67 | 57.8 |
| FVII-$CTP_3$ vs. FVII-$CTP_4$ | 24 | 178 |
| FVII-$CTP_4$ vs. FVII-$CTP_5$ | 6 | 42 |

As previously reported, FVII half-life correlates with the half-life of the activated form of FVII (FVIIa) both in humans and animals. Therefore, it is anticipated that a similar improvement in half-life will be obtained for the activated versions following CTP fusion.

***EXAMPLE 6***

***FVII-$CTP_3$ feasibility studies in FVIII-deficient hemophilic mice***

Studies described hereinabove testing FVII-CTP, FVII-$CTP_2$ and FVII-$CTP_3$ harvest PK profile and coagulation activity vs. a commercial FVII were conducted. FVII-$CTP_3$ exhibited an improved PK profile while maintaining its coagulation activity vs. FVII-CTP and FVII-$CTP_2$ harvests or rhFVII. In order to further characterize FVII-$CTP_3$ *in vitro* and *in vivo* properties, a mini stable pool expressing and secreting the protein was generated, and purification and activation processes were developed.

In the current study, the pharmacokinetic and pharmacodynamic properties of FVIIa-$CTP_3$ were tested in FVIII-deficient mice. The PK profile of the protein was evaluated. A FVIIa specific activity-based PK profile was established and compared to commercial product NovoSeven®. In addition, the long-lasting *in vivo* hemostatic capabilities of FVIIa-$CTP_3$ to induce coagulation in FVIII-deficient mice after a tail vain transection (survival study) were tested.

**Study Objectives:**

To evaluate the pharmacokinetic and pharmacodynamic parameters of FVIIa-$CTP_3$ vs. commercial rhFVIIa (NovoSeven®) in FVIII-deficient mice following a single IV administration at a similar activity dose.

To determine the *in vivo* ability of FVIIa-$CTP_3$ to maintain homoeostasis in FVIII-deficient mice by a single IV administration of FVIIa-$CTP_3$ and NovoSeven® at a similar activity dose followed by a challenge of tail vein transection (survival study).

**Production of FVII-$CTP_3$ harvest:**

FVII-$CTP_3$ was expressed in-house in Dg44 cells using a pCI-DHFR vector. Stable transfected pool #71 was grown in shake flasks, in the presence of 25 ng/L of Vitamin K3 (Sigma). Cell suspension was cultured and harvested following viability decline to 60-80%. The harvest was filtered and frozen at -70°C.

**Determination of harvest FVII antigen level:**

FVII antigen level was determined using human FVII ELISA kit (Zymotest HyPhen) (Table 38). The antigen level was calculated per each pooled harvest batch.

**Table 38**: FVII-$CTP_3$ antigen level

| FVII antigen level | | | |
|---|---|---|---|
| | PK-PD study | | Survival study |
| | harvest 31A | harvest 31B | harvest 38 |
| Av (µg/ml) | 16.0 | 15.9 | 16.6 |
| STD | 1.5 | 0.0 | 0.8 |
| %CV | 9.1 | 0.1 | 4.9 |

**FVII-$CTP_3$ purification process (Figure 25)**

**Process outline**

Following a short purification study, the following purification process using 2 columns was performed. VII-Select affinity column (GE) and Ceramic Hydroxyapatite type 1 (HA), 40 µm (Bio Rad), FVII-$CTP_3$ γ-carboxylated enriched protein was purified. Auto-activation was induced by incubation of purified FVII-$CTP_3$ in the presence of $CaCl_2$ overnight at 2-8°C. The purification process is in its final developmental stage and is being optimized, thus part of the purification steps are not identical in the two batches.

**Ultra-filtration/diafiltration (UFDF) using 10kDa hollow fiber or Pellicon cassette**

Clarified harvest was thawed at 4°C over the weekend (2-3 days).

In Batch 31, clarified harvest (12 liters) was concentrated 4-fold (in two successive runs) using a hollow fiber cartridge (GE Healthcare Catalog # UFP-10-C-4X2MA) with a 10 KDa molecular weight cut-off. Concentrated harvest was dia-filtrated against 1-2 volumes of TBS (50mM Tris 150mM NaCl pH 7.4).

In Batch 38, clarified harvest (8.5 liters) was concentrated 4-fold using a Pellicon 2 (Millipore) cassette with a 10 KDa molecular weight cut-off. Concentrated harvest was directly loaded on VII-Select column.

Both ultra-filtrations were performed on ice with ice cold buffers. UFDF samples were filtered 0.22 µm before loading.

**Capture on FVII-Select column**

The UFDF or concentrated harvest was loaded on VII-Select column (XK16/20, CV 18ml), pre-equilibrated with TBS pH 7.4. The column was washed with 50 mM Tris-HCl, 0.5M NaCl pH 7.5, and FVII-$CTP_3$ was eluted with 50 mM Tris-HCl, 1M NaCl 50% (v/v), Propylene Glycol pH 7.5. The process was performed in two successive cycles utilizing the same column.

**Gamma carboxylation-based separation on a ceramic hydroxyapatite column**

The eluted product was diluted 1:10 with 10 mM sodium phosphate pH 6.8 and loaded on ceramic hydroxyapatite columns (XK16/20, CV 24ml). The column was washed with 59 mM sodium phosphate pH 6.8 and the γ-carboxylated rich fraction of Factor VII was eluted with 500mM sodium phosphate pH 6.8. This process was performed in two successive cycles on the same column. At each batch, the eluates of the two cycles were pooled and concentrated to 1.7-2 mg/ml and dia-filtered with 20 mM Tris-HCl, 100 mM NaCl pH 8.2 to reduce volume and prepare the material for the activation step.

**FVII activation**

Purified FVII-$CTP_3$ was diluted to 1 mg/ml and incubated in 20 mM Tris-HCl, 100 mM NaCl and 1mM $CaCl_2$ pH 8.2 at 2-8°C for 24 hours. Activation was terminated by buffer exchange (UFDF) to preliminary formulation buffer (20 mM Citrate, 240 mM NaCl, 13.3 mM Glycine, pH 6.9).

**$FVII-CTP_3$ and $FVIIa-CTP_3$ analytical properties:**

**SDS-PAGE and Western blots**

Purified $FVII-CTP_3$, and $FVIIa-CTP_3$ were loaded on 12% Tris-Glycine gel using Precision Plus Dual Color Protein Marker (Bio-Rad). The SDS-PAGE Coomassie analysis was performed by staining the gel with Coomassie brilliant blue reagent (5 or 10 $\mu$g of protein/lane). Western blot analysis was performed (1 $\mu$g of protein/ lane) using anti-human FVII polyclonal Ab (R&D systems; AF2338), anti-human gamma carboxylation monoclonal antibody (American Diagnostics Catalog #499, 3570), and anti-CTP polyclonal Ab. Under reduced conditions, $FVII-CTP_3$ migrated at 75KDa, and $FVIIa-CTP_3$ migrated as two main bands: a heavy chain at 50 kDa, and a light chain at 25 kDa, represented in Figure 26 as Bands 2 and 3, respectively.

The purification procedure significantly enriched the $FVII-CTP_3$ portion while reducing impurities. The purification process yield was 25-30% FVII (according to ELISA). Most of the protein lost during purification had low FVII chromogenic activity or no activity. Based on Coomassie-stained SDS-PAGE, the reduced $FVIIa-CTP_3$ contains more than the predicted bands. A band migrating to around -75 kDa represents non-activated FVII (Figure 26, Band 1). This band consists of two bands with minor MW differences, which might reflect different $\gamma$-carboxylation content. Additional bands with MW lower than 20 kDa were observed. This was previously reported to be degradation products of the heavy chain.

**$FVII-CTP_3$ chromogenic activity:**

A comparative assessment of the *in vitro* potency of $FVII-CTP_3$ harvest, in-process fractions, and purified $FVII-CTP_3$ versus human pool normal plasma was performed using a commercially available chromogenic activity test kit, BIOPHEN (Hyphen BioMed 221304). $FVII-CTP_3$ harvest and protein were serially diluted and the potency was assessed by comparing a dose-response curve to a reference preparation of normal human plasma. Following $FVII-CTP_3$ purification, the chromogenic activity was significantly improved, and non-active fractions were separated mainly by HA column (Figure 27). A strong correlation between FVII chromogenic activity and detection of FVII with monoclonal anti-Gla antibodies in Western blot was observed. The potency of FVII chromogenic activity as reflected by EC50 value in harvest is affected from both carboxylated and non-carboxylated FVII fractions. Following purification and enrichment of $FVII-CTP_3$ $\gamma$-carboxylated fraction, the activity was improved, demonstrating the important contribution of $\gamma$-carboxylation to FVII activity (Figure 27). This parameter is crucial for proper FVII *in vivo* activity and will be further addressed in a clone development program.

**Protein determination by A280**

The theoretical extinction coefficient of $FVIIa-CTP_3$ and NovoSeven® was calculated using the ProtParam algorithm (http://web.expasy.org/protparam). The calculation is based on amino acid sequence. The calculated extinction coefficients for $FVII-CTP_3$ and NovoSeven® is 1.186 and 1.406, respectively. These values represent the absorbance of 1 g/L at 280 nm.

The extinction coefficient difference between the two proteins derives solely from the increase in molecular weight of $FVIIa-CTP_3$ compared to NovoSeven®, since CTP lacks aromatic and cysteine residues, thus does not contribute to the absorbance.

Protein determination by A280 is used for final FVII, and for purified in-process samples, starting from the elution of VII-Select column.

**Determination of FVIIa antigen level**

FVIIa antigen level was determined using Human FVIIa ELISA kit (IMUBIND, American Diagnostica). The antigen level was calculated per each batch. However, this tool was not useful for the determination of the dose for injection, since it did not represent the amount of active product.

**Clotting assay of FVIIa- Staclot® VIIa-rTF**

FVIIa is derived from an intra-chain cleavage of the single-chain FVII. Native tissue factor (TF) is a cofactor of FVIIa. Upon binding to TF, FVII mediates the activation of Factor X to Xa, while itself is transformed to FVIIa. The soluble tissue factor is the extracellular part of native tissue factor. It can no longer activate FVII by auto-activation, but the FVIIa bound to tissue factor can activate FX to FXa.

The recombinant soluble tissue factor (rsTF) used in this assay utilizes the FVIIa specificity to construct a FVIIa clotting test. rsTF, in the presence of FVIIa, calcium and phospholipids leads to coagulation of plasma, without activating FVII to FVIIa.

The observed clotting time in this system has an inverse relationship with the FVIIa content in the tested sample, with no interference of FVII presence in the sample.

The assay was performed by Omri Laboratories (Nes-Ziona, Israel). FVIIa activity was evaluated for both NovoSeven® following reconstitution and FVIIa-$CTP_3$ prior to each study. NovoSeven® activity did not correlate with the anticipated activity as reported on the vial, but the discrepancy might be due to a different approach for activity evaluation. Table 39 summarizes the FVIIa clotting activity per volume without considering the protein concentration.

**Table 39:** FVIIa clotting activity of batch products

| | PK study | | Survival Study | |
|---|---|---|---|---|
| | **FVIIa-3*CTP (FVIIa 31)** | **NovoSeven®** | **FVIIa-3*CTP (FVIIa 38)** | **NovoSeven®** |
| **Activity** (U/ml) | 1.3E+06 | 2.5E+05 | 1.3E+06 | 7.4E+05 |

**Specific activity of FVIIa-$CTP_3$**

FVIIa specific activity (which is calculated as the activity/ml divided by protein concentration) was calculated based on A280 and is presented in Table 40. When comparing the specific activity of the two molecules, which differ in MW, compensation must be made in order to normalize the activity (i.e. because of the molecular weight difference, the number of active sites in 1 mg of NovoSeven® is 1.185-fold higher than in FVIIa-$CTP_3$). Calculation of the conversion factor is presented in the following equation:

$$\mathrm{Normalized_SA} = \frac{\mathrm{SA(FVIa - CTP_3)}}{\mathrm{MW.(FVII\quad CTP_3)}} \times \mathrm{MW(Native_FVII)} =$$

$$= \frac{\mathrm{SA(FVIIa\quad CTP_3)}}{\mathrm{53419.5Da}} \times \mathrm{45079.1Da} = \mathrm{SA(FVIIa - CTP_3)} * 1.185$$

**Table 40:** FVIIa-$CTP_3$ specific activity compared to NovoSeven®

| Sample | Average A280 | STDV (n=9) | % CV | Extinction coefficient | Prot conc. (mg/ml) | U/ml | Specific Activity | | Fold decrease from NovoSeven® |
|---|---|---|---|---|---|---|---|---|---|
| | | | | | | | U/mg protein | U/mg FVIIa | |
| **NovoSeven®** | 1.274 | 0.031 | 2.398 | 1.406 | 0.906 | 8.36E+05 | 9.23E+05 | 9.23E+05 | 1.0 |
| **FVIIa-$CTP_3$** | 4.396 | 0.092 | 2.094 | 1.186 | 3.706 | 7.23E+05 | 1.95E+05 | 2.31E+05 | 4.0 |

**FVIIa-CTP$_3$ PK-PD study:**

**Study outline**

FVIIa-CTP$_3$ and rhFVIIa (NovoSeven®, NS) were administered in a single intravenous injection to C57B FVIII-deficient mice at a dose of 6.4E6 U/kg body weight (160,000 U/animal). Blood samples were drawn retro-orbitally from 4 mice alternately at 0.166, 0.5, 2, 4, 8, 12, 24, 34, 48, 58, and 72 hours post-dosing (Table 41). Citrated plasma (0.32%) was prepared immediately after sampling and stored at -20°C until analysis. FVIIa clotting activity level was evaluated, and a detailed PK analysis was performed. The study was performed by Omri Laboratories (Nes-Ziona, Israel).

**Table 41:** Study outline

| Treated Groups | Test Article | No. of animals/ group/ timepoint | Dose Route | Amount of Units/ animal | Injected Vol. (μl) | Time-Points (hours post-dose) |
|---|---|---|---|---|---|---|
| A | rhFVIIa | 4 | IV | 1.6e5 | 200 | 0 (Pre-dose) 0.166, 0.5, 2, 4, 8, 12, 24, 34, 48, 58, 72 |
| B | FVIIa - CTP$_3$ | 4 | IV | 1.6e5 | 200 | 0 (Pre-dose) 0.166, 0.5, 2, 4, 8, 12, 24, 34, 48, 58, 72 |

**FVIIa-CTP$_3$ PK profile in FVIII-deficient mice**

FVIIa activity in blood samples was quantitated using a Staclot® VIIa-rTF kit (Stago, Parsippany, NJ). The pharmacokinetic profile was calculated for each protein and represents the mean of 4 animals at each time point. Figure 28 presents the PK profile of FVIIa throughout the experiment. FVIIa recovery is presented in Table 42. A summary of the PK parameters is presented in Table 43.

Table 41 summarizes the clotting activity values following administration of either NovoSeven® or FVIIa-CTP$_3$. FVIIa-CTP$_3$ and NovoSeven® reached maximal activity half an hour post-dosing. NovoSeven®'s highest activity value reached only 43% of FVIIa-CTP$_3$'s maximal activity value. FVIIa-CTP$_3$ clotting activity was maintained for a longer period of time, demonstrating elongated activity. Clotting activity for the NovoSeven®-treated mice was undetectable at time points later than 12 hours, while FVII-CTP$_3$ treated mice continued to retain measurable activity at 48 hours post dosing (Table 41 and Figure 28).

The addition of three tandem CTP copies to FVIIa elevated recovery by 100% (Table 42), as measured by the highest activity post-dosing and compared to the anticipated activity based on *in vitro* analysis, and increased the half-life and mean resident time (MRT) 5-fold. The exposure time (AUC) was increased 3-fold (Table 43).

**Table 41**: FVIIa clotting activity following single IV injection

| Time after administration (hours) | Average FVIIa Clotting Activity (U/ml) | |
|---|---|---|
| | FVIIa-CTP$_3$ | NovoSeven® |
| 0.16 | 6.8E+07 | 3.2E+07 |
| 0.5 | 9.7E+07 | 4.3E+07 |
| 2 | 2.1E+07 | 3.9E+06 |

| | | |
|---|---|---|
| 4 | 7.7E+06 | 7.3E+05 |
| 8 | 2.7E+06 | 4.2E+04 |
| 12 | 3.7E+05 | 6.2E+03 |
| 24 | 2.4E+04 | BLQ |
| 34 | 4.6E+03 | BLQ |
| 48 | 1.5E+03 | BLQ |

**Table 42:** FVIIa-$CTP_3$ recovery

| Treated. Groups | Test Article | Amount of Units/ animal | Practical administered dose (U/ml) | *Anticipated Cmax (U/ml blood) | Cmax (U/ml) | %Recovery |
|---|---|---|---|---|---|---|
| A | rFVIIa | 1.60E+05 | 1.20E+06 | 1.40E+05 | 4.25E+04 | **30** |
| B | FVIIa-$CTP_3$ | 1.60E+05 | 1.29E+06 | 1.50E+05 | 9.74E+04 | **64.6** |
| *anticipated Cmax is derived from administered dose divided in blood volume | | | | | | |

**Table 43:** PK parameters of FVIIa-$CTP_3$ vs. NovoSeven®

| PK Parameters | NovoSeven® | FVIIa-$CTP_3$ |
|---|---|---|
| Half-life-$_{\alpha}$ (0.5-12hr) | **0.94** | 1.57 |
| Half-life-$_{\beta}$ (12-48hr) | NA | **4.62** |
| AUC (mU*hr/ml) | 5.80E+07 | 1.80E+08 |
| Vd/Kg (ml/Kg) | 1408 | 2375 |
| CL/Kg (ml/hr/Kg) | 1034 | 356 |
| MRT (hr) | 1.3 | 6.7 |

**Thrombin generation assay (TGA)**

The generation of thrombin is a fundamental part of the clotting cascade and as such an estimate of how well a particular individual can generate thrombin may correlate with either a risk of bleeding or thrombosis. Commonly measured variables when analyzing thrombin generation include: the lag time, the time to peak thrombin generation, the peak, the endogenous thrombin potential [ETP] (i.e., the area under the curve and the tail), the time course of the thrombogram ("TG"). After a lag time, a burst of thrombin is observed. However, clotting occurs at the end of the lag time, when more than 95% of all thrombin has not yet formed. The thrombin generation assay was performed at Omri Laboratories, using Thrombinoscope reagents supplemented with human hemophilic plasma. TGA reflects of the clotting ability in mice plasma, derived from injection of NovoSeven® and FVIIa-$CTP_3$. Figure 29 presents TGA parameter values for mice plasma following administration of either FVIIa-$CTP_3$ or NovoSeven®. Following FVIIa-$CTP_3$ administration, all three parameters (rate of thrombin generation, maximal amount of generated thrombin and KIIa) demonstrate an advantage of FVII-$CTP_3$ over NovoSeven® treatment. This further strengthens the notion of potential long-acting superiority of FVII-$CTP_3$ as compared to NovoSeven®.

**FVIIa-$CTP_3$ Tail Vain Transection (TVT) study:**

**Study outline**

The data obtained from the PK/PD test for FVIIa-$CTP_3$ provided insight into the functionality of FVIIa-$CTP_3$, and demonstrated that FVIIa-$CTP_3$ had a pharmacokinetic advantage when compared with NovoSeven®. However, the ability of the protein to induce a clot *in vivo,* after a traumatic event has not yet been demonstrated. In order to evaluate the ability of FVIIa-$CTP_3$ to stop bleeding, the same FVIII-deficient mice model was employed for a bleeding challenge.

FVIII-deficient mice were administered a single intravenous injection of FVIIa-$CTP_3$ or NovoSeven®. The mice were dosed with drug in amounts that provided equivalent FVIIa activity (1.6E05 units, 200 μl), calculated according to the potency of each drug evaluated in the FVIIa clot activity assay (Table 44). The administered doses were 9 mg/kg of NovoSeven®, and 40 mg/kg of FVII-$CTP_3$ due to the reduced activity of FVIIa-$CTP_3$. A control group was injected with 200 μl vehicle.

The tail vein was transected 2.7 cm from the tail tip 15 min (injection 1), 24 hours (injection 2) or 48 hours (injection 3) post-administration, and mice survival was recorded for 24 hours.

**Table 44:** Evaluation of injected samples

| | NovoSeven® | | | FVIIa-$CTP_3$ | | | |
|---|---|---|---|---|---|---|---|
| **Injection No.** | **protein cone. (mg/ml)** | **Activity (U/ml)** | **Specific Activity (U/mg)** | **protein conc. (mg/ml)** | **Activity (U/ml)** | **Specific Activity (U/mg)** | **Specific Activity (normalized)** |
| **1** | 0.91 | 8.0E+05 | 8.8E+05 | 3.63 | 6.6E+05 | 1.8E+05 | 2.2E+05 |
| **2** | 0.92 | 8.3E+05 | 9.0E+05 | 3.81 | 7.8E+05 | 2.0E+05 | 2.4E+05 |
| **3** | 0.89 | 8.8E+05 | 9.9E+05 | 3.68 | 7.3E+05 | 2.0E+05 | 2.3E+05 |

Protein concentration was determined by A280.

**Results**

Data from the vehicle-injected control groups for the three injections (5 animals x 3 injections), were summarized and are presented in Figure 30. 30% survival was observed 24 hours after tail vein transection.

NovoSeven® and FVIIa-$CTP_3$-treated mice demonstrated proper hemostatic activity after tail vein transection performed 15 min after FVIIa administration. A 100% survival rate was observed in FVIIa-$CTP_3$ and NovoSeven® treated animals (Figure 30).

The reduced clearance rate of FVII-$CTP_3$ which was demonstrated in the PK/PD study is most clearly appreciated after a tail vein transection performed 24 hours post-administration. A decline in the survival rate of NovoSeven® is observed. Similar to the control group, 50% death is observed within 10 hours. Meanwhile, 90% of FVIIa-$CTP_3$ treated mice survived (Figure 30). This result emphasizes the long-lasting efficacy of the FVIIa-$CTP_3$ treatment.

48 hours after administration, a decline in survival rate is demonstrated in groups treated with either FVIIa-$CTP_3$ or NovoSeven® (Figure 30C). A slight improvement in FVIIa-CTP mice was observed, but the difference did not reach statistical significance.

**Discussion:**

CTP fusion to recombinant proteins extends the circulatory half-life of proteins while maintaining comparable activity. While the mechanism behind the reduced clearance of protein above a threshold size of 70 KDa is well understood with respect to renal clearance, additional protection is achieved following CTP fusion. CTP fusion is believed to sweep around the protein shield and protect it from proteolytic cleavage, to increase its radial molecular weight due to the highly negative charge and to reduce its affinity to hepatic clearance receptors.

The present study was aimed to provide specific insight on the impact of CTP fusion to FVII on protein half-life and clearance and also address the paradigm of its specific activity following this modification. FVIII-deficient mice were administered with a single IV injection of FVIIa-$CTP_3$ or recombinant commercial FVIIa (NovoSeven®) at similar dose (unit based) and a PK activity-based analysis was performed. FVIIa-$CTP_3$ demonstrated a superior longevity as reflected by 5- and 3.5-fold increase in its half-life and AUC, respectively. The specific activity (U/mg) of FVIIa-CTP as calculated by the Staclot® activity kit divided by the protein concentration measured by A280 was shown to be 4-5 times lower than the specific activity of NovoSeven®.

To build on the understanding of how CTP affects the haemostatic effects of FVIIa *in vivo,* the ability of FVIIa-$CTP_3$ to reduce bleeding was investigated. In the tail vein transection bleeding model in hemophilic mice model, rFVIIa administration can improve the survival rate of challenged animals and avoid their bleeding to death. In the study described herein, animals were administered with FVIIa-$CTP_3$ or NovoSeven®. Both molecules were able to maintain homeostasis when the transection was performed 0.25 hours post-dosing. A significantly prolonged duration of activity was demonstrated for the FVIIa-$CTP_3$-treated group when the tail transection was performed 24 hr post dosing. The vehicle-treated group's survival rate was higher than anticipated and higher than that obtained in previous studies (50% vs. 20% in previous studies, data not shown). The percent survival of treated animals at is further evaluated at earlier time points, including at 36 hr post dosing.

In conclusion, it was demonstrated that FVIIa-$CTP_3$ has an increased duration of activity in hemophilic mice which translates into a longer duration of haemostatic effect when compared to NovoSeven®. The data gathered suggest that fusion of CTP to FVII is a technology with the potential to significantly improve prophylactic treatment in patients with hemophilia.

***EXAMPLE 7: COMPARATIVE ASSESSMENT OF PURIFIED FVII-$CTP_3$ vs. FVII-$CTP_5$ PROFILE FOLLOWING SINGLE IV or SC INJECTION TO SD RATS***

**Study objective**

Two studies were carried out:

The first study objective was to determine the pharmacokinetic parameters of rFVII-CTP3 versus rFVII- CTP5 following FVII select- and HA-column purification in male Sprague Dawley rats, after a single intravenous administration of 50μg/animal.

In the second study, rFVII-CTP3-HA versus rFVII-CTP5-HA pharmacokinetic parameters, were examined in male Sprague Dawley rats following a single intravenous or subcutaneous administration of 100 μg/animal.

**RESULTS**

**Determination of FVII-CTP 3 and FVII-CTP 5 antigen level**

FVII antigen level was determined using Human FVII ELISA kit (Zymotest HyPhen) (See table 45). T

**Table 45.** Summarizes the calculated protein concentration which is the average of three independent runs.

| | FVII 3 CTP | | FVII 5 CTP | |
|---|---|---|---|---|
| | FVIIS 46 el. Conc. Dial | FVII HA 46 el. Conc. Dial | FVIIS el. Conc. Dial | FVII HA 5 100%B Conc. Dial |
| AVE (ng\ml) | 3.78E+06 | 1.59E+06 | 1.88E+06 | 7.92E+05 |
| SD | 1.30E+06 | 6.03E+05 | 7.15E+05 | 3.57E+05 |
| CV (%) | 3.43E+01 | 3.80E+01 | 3.80E+01 | 4.51E+01 |

**Western blot analysis of the examined samples**

FVII-$CTP_{3, 5}$ samples were loaded on 4-12% bisTrisgel *(NuPage, invitrogene*) using Precision plus dual color protein marker (Bio-Rad). The SDS-PAGE analysis was performed by western immune-blot using polyclonal anti FVII Ab (R&D systems), anti CTP polyclonal Ab (Adar biotech production) or anti Gla Ab (American diagnostica). In summary, FVII fused to three and five CTP migrated at 80 and 100kDa, respectively (see Figure 31).

**Comparative assessment of FVII *in vitro* potency**

FVII activity assay, which was performed in Sheba medical center, the national coagulation center, is a PT based assay using immunoadsorbed plasma deficient in factor VII (Siemens). The PT reagent is innovin and the assay is performed in the Sysmex CA 1500 instrument. FVII normal range is within 55-145%. Sample activities are summarized in Table 46.

**Table 46:** Sample activity

| Sample | Concentration (mg/ml) according to (NANODROP) | Concentration in tested sample ($\mu$g/ml) | Results (%) | Average-% of plasma |
|---|---|---|---|---|
| FVII-5CTP FVIIS el. Conc. Dial | 2.19 | 2 | 87 | 16% |
| | | 1 | 30 | |
| | | 0.5 | 10 | |
| FVII-5CTP HA 5 100%B conc. Dial | 1 | 2 | 97 | 21% |
| | | 1 | 36 | |
| | | 0.5 | 13 | |
| FVIIS 46 el. Conc. Dial | 3.17 | 2 | 100 | 18% |
| | | 1 | 35 | |
| | | 0.5 | 12 | |
| FVII HA 46 el. Conc. Dial (1) | 1.5 | 2 | 92 | 20% |
| | | 1 | 33 | |
| | | 0.5 | 10 | |

The normal level of circulating FVII in the body is around 0.5$\mu$g/ml. Both, FVII-$CTP_3$ and FVII-$CTP_5$ exhibit about 5-fold reductions in their coagulation activity versus normal human pool plasma.

**Pharmacokinetic study**

Two pharmacokinetic studies were performed in order to determine the FVII-$CTP_3$ and FVII-$CTP_5$ (after FVII select and FVII HA column) pharmacokinetics (PK) profile and parameters. In the first study, FVII-$CTP_3$, and FVII-$CTP_5$ following FVII select/ HA purification were administered in a single intravenous injection to Sprague Dawley rats (six rats per substance) in a dose of 50 $\mu$g/rat.

Blood samples were drawn retro-orbital from 3 rats alternately at 0.083, 0.5 2, 5, 8, 24, 48, 72, 96 and 120 hours post dosing. Citrated plasma (0.38%) was prepared immediately after sampling and stored at -20 until analysis.

In the second study, only samples after HA column were tested. These samples were administered in a single intravenous or subcutaneous injection to Sprague Dawley rats (six rats per substance) using a dose of 100 $\mu$g/rat. Blood samples were collected at the same time points and conditions as at the first study above.

**Table 47.** First study design (FVII select vs. FVII HA).

| Treated Groups | Test Article | No. of animals/ group/ | Dose Route | Dose Level ($\mu$g per animal) | Injected Vol. ($\mu$l) | Conc. ($\mu$g/ml ) | Time-Points (hours post-dose) |
|---|---|---|---|---|---|---|---|
| A | FVII-CTP*3 batch 46 HA | 6 | IV | 50 | 200 | 250 | 0 (Pre-dose) 0.083 0.5, 2, 5, 8, 24, 48, 72, 96, 120 |
| B | FVII-CTP*3 batch 46 FVIIS | 6 | IV | 50 | 200 | 250 | 0 (Pre-dose) 0.083 0.5, 2, 5, 8, 24, 48, 72, 96, 120 |
| C | FVII-CTP*5batch 5 HA | 6 | IV | 50 | 200 | 250 | 0 (Pre-dose) 0.083 0.5, 2, 5, 8, 24, 48, 72, 96, 120 |
| D | FVII -CTP*5 batch 5 FVIIS | 6 | IV | 50 | 200 | 250 | 0 (Pre-dose) 0.083 0.5, 2, 5, 8, 24, 48, 72, 96, 120 |

**Table 18.** Second study design (IV vs. SC)

| Treated Groups | Test Article | No. of animals/ group/ | Dose Route | Dose Level (μg per animal) | Injected Vol. (μl) | Conc. (μg/ml) | Time-Points (hours post-dose) |
|---|---|---|---|---|---|---|---|
| A | FVII-CTP*3 batch 46 HA | 6 | **IV** | 100 | 200 | 500 | 0 (Pre-dose) 0.083 0.5,2,5, 8, 24,48,72, 96,120 |
| B | FVII-CTP*3 batch 46 HA | 6 | **SC** | 100 | 200 | 500 | 0 (Pre-dose) 0.083 0.5,2,5, 8, 24,48,72, 96,120 |
| C | FVII-CTP*5batch 5 HA | 6 | **IV** | 100 | 200 | 500 | 0 (Pre-dose) 0.083 0.5, 2, 5, 8, 24,48,72, 96,120 |
| D | FVII-CTP*5 batch 5 HA | 6 | **SC** | 100 | 200 | 500 | 0 (Pre-dose) 0.083 0.5,2,5, 8, 24,48,72, 96,120 |

The main differences between these two studies are the dosages and the route of administration. In the first study, rats were injected IV with 50 μg\rat, while in the second study, the rats were injected IV or SC with 100 μg\rat (total 500 μg/kg; rats weigh 200 g). The increase in the dosage is due to the change in the type of administration; SC administration requires higher amounts to achieve effects similar to IV administration.

**Analysis of PK study**

FVII concentration in plasma samples were quantified using human FVII Elisa kits (zymutest FVII-Biophen). Pharmacokinetic profiles were calculated and reflect the mean for 3 animals at each time point. Terminal half-live values were calculated using PK solutions 2.0 software. The table below summarizes the calculated FVII concentrations at the different sampling time points. PK profile and a summary of the PK parameters are presented in table below.

**Table 49.** First pharmacokinetic study (FVII select vs. FVII HA) -FVII concentrations (ng\ml).

| Time (hour) | FVII CTP*3 BATCH 46 HA | FVII CTP*3 BATCH 46 FVII S | FVII CTP*5 BATCH 5 HA | FVII CTP*5 BATCH 5 FVII S |
|---|---|---|---|---|
| 0.083 | 1816.3 | 1633.9 | 2064.3 | 1853.5 |
| 0.5 | 1523.7 | 1409.9 | 1351.4 | 1418.0 |
| 2 | 1284.9 | 1041.7 | 1389.7 | 834.4 |
| 5 | 607.9 | 531.6 | 722.7 | 737.2 |
| 8 | 524.2 | 430.0 | 712.2 | 614.6 |
| 24 | 115.5 | 132.9 | 272.5 | 201.8 |
| 48 | 21.1 | 31.6 | 62.3 | 90.4 |
| 72 | 9.5 | 15.8 | 29.1 | 31.8 |
| 96 | BLQ | 5.8 | 7.0 | 16.9 |
| 120 | BLQ | BLQ | 8.5 | 13.4 |

**Table 50.** Second pharmacokinetic study (IV vs. SC) -FVII concentrations (ng\ml).

| Time (hour) | FVII CTP*3 BATCH 46 HA-IV | FVII CTP*5 BATCH 5 HA-IV | FVII CTP*3 BATCH 46 HA-SC | FVII CTP*5 BATCH 5 HA-SC |
|---|---|---|---|---|
| 0.083 | 6452.6 | 6153.3 | 5.0 | BLQ |
| 0.5 | 3930.7 | 3660.6 | 14.5 | 14.6 |
| 2 | 1992.3 | 2176.2 | 113.6 | 96.2 |
| 5 | 1598.9 | 2087.3 | 106.6 | 70.5 |
| 8 | 781.6 | 1075.6 | 188.9 | 129.7 |
| 24 | 268.5 | 627.2 | 155.0 | 239.2 |
| 48 | 51.9 | 143.3 | 43.0 | 88.6 |
| 72 | 8.8 | 39.0 | 7.0 | 36.7 |
| 96 | BLQ | 10.8 | BLQ | 10.4 |
| 120 | BLQ | 8.2 | BLQ | 8.7 |

**Table 51.** PK Analysis- first pharmacokinetic study (FVII S vs. HA).

| | FVII CTP*3 BATCH 46 HA | FVII CTP*3 BATCH 46 FVII S | FVII CTP*5 BATCH 5 HA | FVII CTP*5 BATCH 5 FVII S |
|---|---|---|---|---|
| half-life (0.083-8 hr) (hr) | 4.3 | 4.0 | 5.51 | 5.59 |
| half-life (8-72\96\120hr) (hr) | 11.1 | 12.1 | 16.46 | 20.29 |
| half life (8-72) (hr) | 11.1 | 13.4 | 13.62 | 15.64 |
| AUC(O-t)(obs area) (8-72/96/120hr) | 14566.9 | 13686.4 | 21812.7 | 19307.9 |
| AUC (∞) area(8-72/96/120hr) | 14718.2 | 13788.1 | 22013.9 | 19701 |
| Vd(area)/kg (ml/kg) (8-2/96/120hr) | 271.1 | 316.1 | 269.7 | 371.5 |
| CL(area)/kg(ml/hr/kg) (8-72/96/120hr) | 17.0 | 18.1 | 11.356 | 12.69 |

The addition of five CTP elongated FVII half-life compared to 3 CTPs. Both forms of 5 CTP (i.e FVIIS and FVII HA) were detected at the long time points (96 and 120 hr), while FVII-3 CTP HA and FVIIS -3 CTP were detected until 72 hr and 96 hr, respectively. Based on this fact, the half-life of FVII-5 CTPs is longer than 3CTPs variants (see Fig. 32). Comparing half-life of all examined materials (3 and 5 CTPs) at the same time points (8-72 hr) showed that the half-life are similar, although 5 CTP are quite longer (Fig. 32).

**Table 52: PK analysis - second pharmacokinetic study-(IV vs. SC).**

| | FVII CTP*3 BATCH 46 HA-IV | FVII CTP*5 BATCH 5 HA IV | FVII CTP*3 BATCH 46 HA-SC | FVII CTP*5 BATC H5 HA-SC | Bioviability CTP*3 | Bioviability CTP*5 |
|---|---|---|---|---|---|---|
| half-life (0.083-8 hr) (hr) | 3.0 | 3.9 | -1.8 | -3.18 | | |
| half-life (8-72\96\120hr) (hr) | 9.9 | 14.6 | 13.14 | 22.94 | | |

(continued)

| | FVII CTP*3 BATCH 46 HA-IV | FVII CTP*5 BATCH 5 HA IV | FVII CTP*3 BATCH 46 HA-SC | FVII CTP*5 BATC H5 HA-SC | Bioviability CTP*3 | Bioviability CTP*5 |
|---|---|---|---|---|---|---|
| half-life (8-72) (hr) | 9.9 | 13.0 | 13.14 | 29.47 | | |
| AUC(O-t)(obs area) (8-72/96/120hr) | 28866.8 | 43761.0 | 6600 | 9822.7 | 22.9 | 22.4 |
| AUC (∞) area (8-72/96/120hr) | 28993.0 | 43934.4 | 6733 | 10110.8 | 23.22 | 23.01 |
| Vd(area)/kg (ml/kg) (8-72/96/120hr) | 246.4 | 240.5 | 1407.6 | 1636.8 | | |
| CL(area)/kg ( ml/hr/kg) (8-72/96/120hr) | 17.2 | 11.4 | 74.261 | 49.452 | | |

Again, as observed in the first study, the addition of 5 CTPs elongated FVII half-life as compared to adding 3 CTP, both in the initial and terminal half-life and in both administration ways (IV and SC, see Fig. 33). As expected, following SC administration, FVII was first detected in the blood at a later time point as compared to when it was administered IV.

In the above, two PK studies were summarized. The main purpose of the first study was to check the difference between FVII-3CTP and FVII-5 CTP after 2 different columns: FVII select and FVII HA. In our previous studies, harvest vs. purified proteins were checked and it was found that the difference between 3 and 5 CTP versions of FVII was greater when harvest was injected to the rats.

There was no significant difference between the results of FVII 3\5 CTP after both columns, hence it was decided to inject FVII HA 3\5 CTP in the second study.

***EXAMPLE 8: FVIIa-CTP$_3$ (MOD-5014) SURVIVAL STUDYIN FVIII DEFICIENT MICE FOLLOWING SUBCUTANEOUS INJECTION***

**Study Objective**

To evaluate the efficacy of NovoSeven®, MOD-5014 (FVIIA-CTP$_3$) and MOD-5019 (FVIIA-CTP$_5$) in a tail vein transection study, following subcutaneous administration.

**FVIIa-CTP$_3$(MOD-S014) and FVIIa-CTP$_5$(MOD 5019) analytical properties: Protein determination by A280**

Theoretical extinction coefficient of NovoSeven® was calculated using ProtParam algorithm (http://web.expasy.org/protparam). The calculation is based on amino acid sequence. The calculated extinction coefficient for NovoSeven® is 1.406, and for MOD-5019 is 1.075 (values represent the absorbance of 1 g/L at 280 nm). Extinction coefficient of MOD-5014 was determined by amino acid analysis at Mscan. The extinction coefficients for MOD-5014 is 1.27.

**Clotting assay of FVIIa - STACLOT VIIa-rTF**

FVIIa is derived from intra-chain cleavage of the single-chain FVII. Native tissue factor (TF) is a cofactor of FVIIa, upon binding to TF, FVII mediates the activation of Factor X to Xa, while itself is transformed to FVIIa. The soluble tissue factor is the extra cellular part of native tissue factor. It can no longer activate FVII by auto activation, but the FVIIa bound to tissue factor can activate FX to FXa.

The recombinant soluble tissue factor (rsTF) used in this assay is utilizing the FVIIa specificity to construct a FVIIa clotting test. Recombinant soluble tissue factor (rsTF), in the presence of FVIIa, calcium and phospholipids, produces coagulation of plasma without activating FVII to FVIIa.

The observed clotting time in this system has an inverse relationship with the FVIIa content in the tested sample, with no interference of FVII presence in the sample.

FVIIa activity was evaluated for reconstituted NovoSeven®, and for MOD-5014 and MOD-5019 prior to each study.

FVIIa specific activity (which is calculated as the activity/ ml divided by protein concentration) was calculated based on A280 and is presented in Table 53. When comparing specific activity of the two molecules, which differ in molecular weight, compensation must be made in order to normalize the activity (i.e. because of the molecular weight difference, the number of active sites in 1 mg of NovoSeven® is 1.185-fold higher than in MOD-5014 and 1.307-fold higher than MOD-5019). Hence, calculation of the conversion factor is presented in the following formula:

$$\text{Normalized_SA} = \frac{SA(FVIa - CTP_3)}{MW.(Native_FVII)} \times MW(FVII \quad CTP_3) =$$

$$= \frac{SA(FVIIa \quad CTP_3)}{45079.1Da} \times 53419.5Da = SA(FVIIa - CTP_3) * 1.185$$

**Table 53- MOD-5014 Specific activity compared to NovoSeven®**

| Sample | Protein conc. By A280 (mg/ml) | Specific Activity (U/mg FVIIa) | Fold decrease from ®NovoSeven |
|---|---|---|---|
| **®NovoSeven** | 0.93 | 52,487 | 1.0 |
| **MOD-5014 batch 73** | 1.4 | 25,490 | 2.05 |
| **MOD-5019 batch 9** | 3.0 | 11,698 | 4.48 |

**Study outline**

The most significant measurement is the ability of the protein to induce a clot *in vivo,* after a traumatic event. In order to evaluate the ability of MOD-5014 to stop bleeding, the same FVIII deficient mice model was employed for a bleeding challenge.

FVIII deficient mice were administrated with a single subcutaneous injection of MOD-5014, MOD-5019 or NovoSeven®. Group A and B were dosed with NovoSeven® and MOD-5014 respectively, in equivalent amounts as FVIIa activity. Group C was dosed with MOD-5019 in equivalent amount FVIIa portein as MOD-5014, in order to evaluate the critical factor (activity or amount of protein). The administrated doses were 4.2 mg/kg of NovoSeven®, and 8.6 mg/kg of MOD-5014 and MOD-5019. The tail vein was transected 2.7cm from tail tip 12 hours post administration, and mice survival was recorded for 24 hours.

**Table 54 - Group designation**

| Group | Injection date | Test Article | Administered Dose | | Injected Volume (μl) | No. of mice per group | Bleeding time, hours post dosing |
|---|---|---|---|---|---|---|---|
| | | | mg FVII /Kg | mU/Kg | | | |
| **A** | 13.1.13 | ®NovoSeven | 4.23 | 221,876 | 100 | 10 | 12 |
| **B** | 15.1.13 | MOD-5014, batch 73 | 8.59 | 218,750 | 160 | 10 | 12 |
| **C** | 27.1.13 | MOD-5019, batch 9 | 8.59 | 100,496 | 160 | 10 | 12 |

**RESULTS**

The experiment data is summarized in Table 55 and in Figure 34.

**Table 55.** TVT study results

| Time post TVT (h) | No. of surviving mice | | | % survival | | |
|---|---|---|---|---|---|---|
| | NovoSeven® | MOD-5014 | MOD-5019 | NovoSeven ® | MOD-5014 | MOD-5019 |
| 0 | 9 | 10 | 10 | 100 | 100 | 100 |
| 1 | 9 | 10 | 10 | 100 | 100 | 100 |
| 2 | 9 | 10 | 10 | 100 | 100 | 100 |
| 3 | 8 | 10 | 8 | 89 | 100 | 80 |
| 4 | 6 | 9 | 8 | 67 | 90 | 80 |
| 5 | 5 | 9 | 7 | 56 | 90 | 70 |
| 6 | 4 | 8 | 5 | 44 | 80 | 50 |
| 7 | 3 | 8 | 5 | 33 | 80 | 50 |
| 8 | 2 | 7 | 5 | 22 | 70 | 50 |
| 9 | 1 | 6 | 5 | 11 | 60 | 50 |
| 10 | 1 | 5 | 5 | 11 | 50 | 50 |
| 11 | 1 | 3 | 5 | 11 | 30 | 50 |
| 12 | 1 | 3 | 5 | 11 | 30 | 50 |
| 24 | 1 | 3 | 4 | 11 | 30 | 40 |

24 hours post TVT, only 11% of NovoSeven® injected mice have survived. 30% of MOD-5014 and 40% of MOD-5019 have survived to this time point. Subcutaneously injected MOD-5014 and MOD-5019 shows improved mice survival in comparison to NovoSeven®. Nevertheless, the results are not optimal since more than 50% of the animals died during the experiment.

Factor VIIa, like other coagulation factors, is normally injected intravenously, in order to be directly available in the blood stream. However, the present invention shows that the compositions provided herein are surprisingly more effectively absorbed into the bloodstream after SC administration. To be able to administer FVIIa subcutaneously serves as an advantage as it can be used for prophylactic applications. Subcutaneous injections are also much easier for patients to self-inject, and are advantage when the patients are very young and their veins are small and difficult to find.

Hence, the subcutaneous application can be used for prophylactic treatment,

***EXAMPLE 9: COMPARATIVE PK-PD STUDY OF RECOMBINANT MOD-5014 VS. NOVOSEVEN® FOLLOWING SUBCUTANEOUS ADMINISTRATION IN SD RATS***

**Study Objectives**

To determine the pharmacokinetic and pharmacodynamic parameters of MOD-5014 versus commercial rFVIIa in SD rats following a single SC administration.

To compare two independent experiments (05010 & 05034) containing MOD-5014 products originated from two different clones (clone no. 28 vs. 61) by their pharmacokinetics parameters.

**Experimental Methods**

**Animals**

24 males SD rats arrived from Harlan Laboratories Israel, Ltd, at least 4 days before the injections begin. The animals were healthy young adults, at ~200 gr at study initiation. The body weight variation of animals at the time of treatment initiation should not exceed ± 20% of the mean weight of each sex. The health status of the animals used in this study is examined on arrival. Only animals in good health are acclimatized to laboratory conditions and are used in the study.

## Clotting assay of FVIIa - STACLOT VIIa-Rtf

The recombinant soluble tissue factor (rsTF) used in this assay is utilizing the FVIIa specificity to construct a FVIIa clotting test. rsTF, In the presence of FVIIa, calcium and phospholipids produce coagulation of plasma, without activating FVII to FVIIa.

The observed clotting time in this system has an inverse relationship with the FVIIa content in the tested sample, with no interference of FVII presence in the sample.

FVIIa activity was evaluated for both NovoSeven® following reconstitution and MOD-5014 prior to each study. FVIIa specific activity was calculated based on A280. When comparing specific activity of the two molecules, which differ in MW, compensation must be made in order to normalize the activity (i.e. because of the molecular weight difference, the number of active sites in 1 mg of NovoSeven® is 1.185-fold higher than in MOD-5014).

## PK solver software

The pharmacokinetic parameters were calculated using PK solver software. The IV administration curve analyzed as two compartmental CA bolus, and the SC administration as NCA Extravascular- Log linear trapezoidal analysis. Half-life, AUC, clearance and volume distribution specifications were calculated and the output parameters were studied in comparison between groups of experiments.

## Experimental materials

Experiment no. 05010:

A. NovoSeven® RT: (Lot # AU61553 prepared on 31.7.12*) FVIIa concentration by A280: 0.86 mg/ml. FVIIa Staclot activity assay: 56,867 U/mg. Injected dose: **946μg/kg.** *Pool of NovoSeven® aliquots, all from the same Lot no.
B. Clone 28: MOD-5014 RS12-001: 0.77 mg/ml** based on A280. FVIIa Staclot activity assay: 34,162 U/mg. Injected dose: **850μg** FVIIa/**kg**.

Experiment no. 05034:

A. NovoSeven® RT: (Lot #AU61347 prepared on 1.1.13) FVIIa concentration by A280: 0.82mg/ml, diluted to 0.4 mg/ml with sterile NS buffer. FVIIa Staclot activity assay: 55,688 U/mg. Injected dose: **360μg/kg** and **20,047.7 U/kg.**
B. Clone 61: MOD-5014 Batch 75: 1.9 mg/ml** based on A280, diluted to 0.89 mg/ml with formulation buffer. Injected dose: **20,047.7 U/kg.** FVIIa clotting activity: 25,002* U/mg based on FVIIa Staclot activity assay.
C. Clone 61: MOD-5014 Batch 81A: 2.36 mg/ml based on A280 (filtered on the morning of study day and re-measured at 280nm), diluted to 0.4 mg/ml with formulation buffer. Injected dose: **360μg**FVIIa/**kg**. FVIIa clotting activity: 24943U/mg based on FVIIa Staclot activity assay.
D. Clone 61: MOD-5014 Batch 81A: 2.36 mg/ml based on A280, diluted to 0.89 mg/ml with formulation buffer. Injected dose: **20,047.7 U/kg.** FVIIa clotting activity: 24,943U/mg based on FVIIa Staclot activity assay.

## Study outlines

### Experiment no. 05010

MOD-5014 and NovoSeven® were administered in a single intravenous or subcutaneous injection to SD Rats in a dose of 0.9 mg/kg body weight. Blood samples were drawn from sinus orbital eye from 3 rats alternately at 0.5, 4, 8, 12, 24, 34, 48 and 58 hours post dosing. Citrated plasma (0.32%) was prepared immediately after sampling and stored at - 20°c until analysis. The study was performed at "Science in Action", Nes-Ziona. FVIIa clotting activity level was evaluated and detailed PK analysis was performed at Prolor-Biotech.

**Table 55: Study design 05010**

| Treated Groups | Test Article | No. of animals /group | No. of animals/ group/ Time point | Dose Route | Gender | Dose Level (µg/kg) | Injected Vol. (µl) | Time-Points (hours post-dose) |
|---|---|---|---|---|---|---|---|---|
| A | rFVIIa (Novo Seven®) | 6 | 3 | IV | M | 946 | 220 | 0, 0.5, 4, 8, 12, 24, 34, 48, 58 |
| B | rFVIIa RS12-001 (clone 28) | 6 | 3 | IV | M | 850 | 220 | 0, 0.5, 4, 8, 12, 24, 34, 48, 58 |
| C | rFVIIa (Novo Seven®) | 6 | 3 | SC | M | 946 | 220 | 0, 0.5, 4, 8, 12, 24, 34, 48, 58 |
| D | rFVIIa RS12-001 (clone 28) | 6 | 3 | SC | M | 850 | 220 | 0, 0.5, 4, 8, 12, 24, 34, 48, 58 |

**Experiment no. 05034**

MOD-5014 and NovoSeven® were administered in a single subcutaneous injection to SD Rats in a dose of 0.9 mg/kg body weight. Blood samples were drawn from sinus orbital eye from 3 rats alternately at 0.5, 2, 4, 6, 8, 12, 24, 34, 48 and 72 hours post dosing. Citrated plasma (0.32%) was prepared immediately after sampling and stored at -20°c until analysis. The study was performed at "Science in Action", Nes-Ziona.

FVIIa clotting activity level was evaluated and detailed PK analysis was performed at Prolor-Biotech.

**Table 56: Study design 05034**

| Treated. Groups | Test Article | No. of animals/ group/ Time-point *** | Dose Route | Gender | Dose Level Per Animal (µg/kg) | Dose Level Per Animal (U/kg) | Injected Vol. (µl) | Time-Points (hours post-dose) |
|---|---|---|---|---|---|---|---|---|
| A | FVIIa (NovoSeven®) | 3 | SC | M | **360** | 20047. 7 | 207 | 0, 0.5, 2, 4, 6, 8, 12, 24, 34, 48, 72 |
| B | FVIIa75 (clone 61) | 3 | SC | M | 801.8 4 | **20047.** 7 | 207 | 0, 0.5, 2, 4, 6, 8, 12,24, 34, 48, 72 |

(continued)

| Treated. Groups | Test Article | No. of animals/ group/ Time-point *** | Dose Route | Gender | Dose Level Per Animal (μg/kg) | Dose Level Per Animal (U/kg) | Injected Vol. (μl) | Time-Points (hours post-dose) |
|---|---|---|---|---|---|---|---|---|
| C | FVIIa 81A (clone | 3 | SC | M | **360** | 8979.4 8 | 207 | 0, 0.5, 2, 4, 6, 8, 12, 24, |
| | 61) | | | | | | | 34, 48, 72 |
| D | FVIIa 81A (clone 61) | 3 | SC | M | 803.7 4 | **20047. 7** | 207 | 0, 0.5, 2, 4, 6, 8, 12, 24, 34, 48, 72 |

**RESULTS**

FVIIa activity in blood samples was quantitated using STACLOT VIIa-rTF kit (Stago). Pharmacokinetic profile was calculated for each protein and is the mean of 3 animals at each time point.

**Experiment no. 05010**

After background reduction: 15 mU/ml.

Figure 35 presents the PK profile of FVIIa following IV and SC administration of either NovoSeven® or MOD-5014. Summary of FVIIa activity values for each time point is presented in Table 57. IV and SC administration have different PK pattern (see Fig. 35; after background reduction: 15 mU/ml). The Cmax following IV injection is higher than that obtained following SC injection, due to the presence of the drug immediately after administration in the blood (measured at 0.5hr, Table 57 and Table 58). However, after SC administration drug molecules transfer to intracellular matrix and tissues, thus Cmax can be measured only after 2hr from injection. The total recovery of the drug after SC administration is lower than Cmax value after IV injection.

8hr after injection, NovoSeven® manifested an equal PK pattern when injected by either IV or SC, (After background reduction: 15mU/ml, Fig. 35). Moreover, clotting activity for the NovoSeven® treated mice was undetectable at time points later than 12 hours, while MOD-5014 treated mice continued to retain measurable activity at 58 hours post dosing (Table 57; after background reduction: 15 mU/ml; Fig. 35).

**Table 57.** FVIIa clotting activity of MOD-5014 vs. NovoSeven® following IV or SC administration

| Time (hr) | NovoSeven® IV (A) | | MOD-5014 IV (B) | | NovoSeven® SC (C) | | MOD-5014 SC (D) | |
|---|---|---|---|---|---|---|---|---|
| | mU/ml | %CV | mU/ml | %C V | mU/ml | %C V | mU/ml | %C V |
| 0.5 | 304651.7 | 18.7 | 232818.3 | 5.0 | 11491.7 | 2.4 | 3691.7 | 19.0 |
| 4 | 40068.3 | 7.8 | 62085.0 | 9.5 | 21385.0 | 22.6 | 12018. 3 | 15.8 |
| 8 | 5276.7 | 2.5 | 25931.7 | 6.1 | 5525.0 | 32.5 | 6445.0 | 2.2 |
| 12 | 255.0 | 13.8 | 5633.3 | 9.3 | 297.7 | 41.4 | 924.7 | 24.1 |
| 24 | 1.3 | 7.1 | 251.3 | 11.8 | 1.3 | 89.2 | 249.3 | 60.3 |
| 34 | 0.0 | | 78.3 | 4.5 | 0.0 | | 63.7 | 85.5 |
| 48 | | | 29.0 | 9.9 | 0.0 | | 35.0 | 47.2 |
| 58 | | | 10.3 | 4.6 | 0.0 | | 13.7 | 33.5 |
| After background reduction: 15mU/ml. | | | | | | | | |

**Table 58: PK parameters of MOD-5014 vs. NovoSeven® following IV or SC administration**

| A. IV | | |
|---|---|---|
| **PK Parameters** | **NovoSeven® RT (A)** | **MOD-5014 (RS 12-001) (B)** |
| Half-life-α (0.5-4hr) | 0.24 | 1.04 |
| Half-life-β (4-58hr) | 1.31 | 3.17 |
| AUC o-inf mU/ml*h | 702467.95 | 820778.67 |
| Vss [U/Kg/(mU/ml)] | 0.13 | 0.13 |
| CL [(U/Kg)/(mU/ml)/h] | 0.08 | 0.04 |
| MRT (hr) | 1.74 | 3.62 |
| **B. SC** | | |
| **PK Parameters** | **NovoSeven® RT (B)** | **MOD-5014 (RS 12-001) (C)** |
| Half-Life (hr) | 1.40 | 7.78 |
| Cmax (mU/ml) | 21385.00 | 12018.33 |
| AUC 0-inf (mU/ml*h) | 115099.72 | 84158.87 |
| MRT 0-inf (hr) | 4.32 | 7.04 |
| Vz/F (U/Kg)/(mU/ml) | 0.95 | 3.88 |
| Cl/F (U/Kg)/(mU/ml)/h | 0.47 | 0.35 |

**Experiment no. 05034**

Figure 36 presents the PK profile of FVII a following SC administration of either NovoSeven® or MOD-5017. Two different batches of clone no. 61 (#75 and #81) were examined in the same concentration or the same activity units, compared to NovoSeven®. Summary of FVIIa activity values for each time point is presented in Table 59.

The results indicate a similar PK pattern after SC administration corresponding to previous experiments. Moreover, clotting activity for the NovoSeven® treated mice was undetectable at time points later than 12 hours, while MOD-5014 treated mice continued to retain measurable activity at 24 hours post dosing (Table 59 and Figure 36; and after background reduction: 56 mU/ml (8, 12 hr) or 32 mU/ml (0.5, 2, 6, 14 hr)).

Clone no. 61 batch #81 (D) Cmax (1,301mU/ml) was lower than the Cmax values of clone no. 61 batch #75 (B) and NovoSeven® (A) (3,521mU/ml and 5,908mU/ml respectively), although they were all injected by the same unit activity (Table 6). However, batch #75 (B) and #81 (D) have the same activity units (559 mU/ml and 478 mU/ml respectivaly) measured 8hr after injection (Figure 36 and Table 59; and after background reduction: 56 mU/ml (8, 12 hr) or 32 mU/ml (0.5, 2, 6, 14 hr)).

**Table 59:** FVIIa clotting activity of MOD-5014 (Clone 61 #75, #81) vs. NovoSeven® following single SC administration.

| **Time (hr)** | **NovoSeven® (A)** | | **MOD-5014 Clone 61 Batch 75 (B) - equal U/kg** | | **MOD-5014 Clone 61 Batch 81A (C) - equal conc.FVIIa μg/kg** | | **MOD-5014 Clone 61 Batch 81A (D) - equal U/kg** | |
|---|---|---|---|---|---|---|---|---|
| | **mU/ ml** | **%CV** | **mU/ml** | **%CV** | **mU/ml** | **%CV** | **mU/ml** | **% C V** |
| 0.5 | 3271 .3 | 46.5 | 350.3 | 26.6 | 101.3 | 24.1 | 208.7 | 51.2 |
| 2 | 5908 .0 | 18.1 | 3521.3 | 70.9 | 1294.7 | 7.0 | 1301.3 | 31.6 |
| 6 | 1411 .7 | 23.6 | 1349.7 | 45.6 | 425.3 | 27.6 | 663.0 | 13.4 |
| 8 | 1029 .0 | 12.4 | 559.3 | 52.7 | 152.7 | 19.5 | 478.0 | 25.4 |
| 12 | 121. 3 | 9.9 | 563.0 | 17.4 | 148.7 | 36.3 | 712.7 | 16.2 |
| 24 | 1.0 | 25.0 | 117.0 | 41.9 | 21.3 | 36.4 | 99.0 | 36.7 |

After background reduction: 56mU/ml (8,12hr) or 32mU/ml (0.5, 2, 6, 14hr).

**Table 60:** PK parameters of MOD-5014 (Clone 61 #75, #81) vs. NovoSeven® following single SC administration.

| PK Parameters | NovoSeven® RT (A) | MOD-5014 Clone 61 Batch 75 (B)- equal U/kg | MOD-5014 Clone 61 Batch 81A (C)- equal conc.FVIIa μg/kg | MOD-5014 Clone 61 Batch 81A (D)- equal U/kg |
|---|---|---|---|---|
| Half-Life (hr) | 1.67 | 5.70 | 4.62 | 6.41 |
| Cmax (mU/ml) | 5908.00 | 3521.33 | 1294.67 | 1301.33 |
| AUC 0-inf (mU/ml*h) | 24688.18 | 20456.96 | 6260.23 | 13098.16 |
| MRT 0-inf (hr) | 3.73 | 7.86 | 6.40 | 10.59 |
| Vz/F (U/Kg)/ (mU/m l) | 1.96 | 8.06 | 9.55 | 14.15 |
| Cl/F (U/Kg)/ (mU/m l)/h | 0.81 | 0.98 | 1.43 | 1.53 |

This report summarized two PK studies; 05010 & 05034. We aim to provide specific insight on the impact of CTP fusion to FVII on protein half-life and clearance in subcutaneous administration and address the paradigm of its specific activity following this modification. In these studies, SD rats were administered with a single SC injection of MOD-5014 originated from two clones, and two different batches, compared to recombinant commercial FVIIa (Novo-Seven®). The components were injected at similar FVIIa concentration (μg/Kg) or at the same activity level (U/Kg) and the PK activity based analysis was performed.

The main purpose of the first study was to verify the different PK parameters after IV and SC administration. Based on this study we can conclude that there is a difference between the PK pattern measured after IV or SC administration. A $t^{1/2}$ of 7.78 hr measured after MOD-5014 SC injection, and only 4.2 hr after IV injection. AUC values were the same (Table 58).

The second study however, focuses on the differences between two batches of MOD-5014 clone no. 61, which were injected by the same FVIIa concentration or at an equal activity unit, compare to NovoSeven®. At this study we showed that clone 61 batch #75 manifested better PK parameters than batch #81. Batch #81, which was injected by the same unit activity level, had lower Cmax from an unknown reason. Moreover, the same Cmax was measured when injecting clone 61 batch #81 in two different doses (by FVIIa concentration or by unit activity), instead of 2.5-fold between the two activity values. Following analysis of both studies together, we can conclude that clone 28 manifested prolong $t^{1/2}$ parameter that clone 61 #75 (the better batch) after SC injection (7.78hr and 5.7hr respectively, Table 60). We can also conclude that dissimilar time point samples create different PK pattern, which lead to variation in the PK curves. The patterns of the curves can teach us more about the drug behavior in the blood. Therefore, we decided to determine the time points similar to those detected by Baxter (0, 0.5, 2, 6, 8, 12, 24, 34, 48, 72hr). Moreover, the FVIIa concentration in 05010 experiment was too high, and was revised in the following SC experiment (05034). For future PK studies, we decided to inject the component at 360μg FVIIa/kg for a dose.

Taken all together, we can learn more about our MOD-5014 product after SC administration in order to determine the most quality clone and batch and to decide the best method for MOD-5014 injection amount- by FVIIa concentration or activity units.

While certain features of the invention have been illustrated and described herein, many modifications, substitutions, changes, and equivalents will now occur to those of ordinary skill in the art.

## Claims

**1.** A chorionic gonadotropin carboxy terminal peptide (CTP)-modified coagulation factor consisting of a coagulation factor and three CTPs attached to the carboxy terminus of said coagulation factor, and wherein said CTP-modified coagulation factor is:

a. a CTP-modified Factor IX (FIX) polypeptide consisting of the amino acid sequence of SEQ ID NO: 31 or amino acids 47-545 of SEQ ID NO: 31,
b. a CTP-modified Factor VII (FVII) polypeptide consisting of the amino acid sequence of SEQ ID NO: 25 or amino acids 39-528 of SEQ ID NO: 25, or
c. a CTP-modified activated Factor VII (FVIIa) polypeptide consisting of the amino acid sequence of amino acids 39-528 of SEQ ID NO: 25;

for use in reducing excessive bleeding or bruising in a subject.

**2.** A chorionic gonadotropin carboxy terminal peptide (CTP)-modified coagulation factor consisting of a coagulation factor and three CTPs attached to the carboxy terminus of said coagulation factor, and wherein said CTP-modified coagulation factor is:

a. a CTP-modified Factor IX (FIX) polypeptide consisting of the amino acid sequence of SEQ ID NO: 31 or amino acids 47-545 of SEQ ID NO: 31,
b. a CTP-modified Factor VII (FVII) polypeptide consisting of the amino acid sequence of SEQ ID NO: 25 or amino acids 39-528 of SEQ ID NO: 25, or
c. a CTP-modified activated Factor VII (FVIIa) polypeptide consisting of the amino acid sequence of amino acids 39-528 of SEQ ID NO: 25;

for use in maintaining hemostasis during surgery or during surgical interventions in a subject.

**3.** The CTP-modified coagulation factor for use according to any of claims 1 or 2, wherein at least one of said attached CTPs is glycosylated.

**4.** The CTP-modified coagulation factor for use according to any of claims 1-3, wherein said CTP-modified coagulation factor is a CTP-modified FIX polypeptide consisting of the amino acid sequence of SEQ ID NO: 31 or amino acids 47-545 of SEQ ID NO: 31.

**5.** The CTP-modified coagulation factor for use according to any one of claims 1-3, wherein said CTP-modified coagulation factor is a CTP-modified FVII polypeptide consisting of the amino acid sequence of SEQ ID NO: 25 or amino acids 39-528 of SEQ ID NO: 25.

**6.** The CTP-modified coagulation factor for use according to any one of claims 1-3, wherein said CTP-modified coagulation factor is a CTP-modified FVIIa polypeptide consisting of the amino acid sequence of amino acids 39-528 of SEQ ID NO: 25.

**7.** The CTP-modified coagulation factor for use according to any of claims 1-6, wherein said CTP-modified coagulation factor is administered to the subject in the form of a pharmaceutical composition comprising a pharmaceutically acceptable carrier.

**8.** The CTP-modified coagulation factor for use according to any of claims 1-7, by subcutaneous administration.

**9.** The CTP-modified coagulation factor for use according to any of claims 1-7, by intravenous administration.

**10.** The CTP-modified coagulation factor for use according to any of claims 1-9, wherein said subject is a hemophilic subject, or is a subject suffering from a vitamin K deficiency or a liver disease.

**11.** The CTP-modified coagulation factor for use according to any of claims 1-10, wherein said subject is a child.

## Patentansprüche

**1.** Durch carboxy-terminales Peptid (CTP) von Choriongonadotropin modifizierter Gerinnungsfaktor, bestehend aus einem Gerinnungsfaktor und drei CTPs, die an den Carboxy-Terminus des Gerinnungsfaktors angehängt sind, und wobei der CTP-modifizierte Gerinnungsfaktor folgender ist:

a. ein CTP-modifiziertes Faktor IX (FIX)-Polypeptid, bestehend aus der Aminosäuresequenz SEQ ID NO: 31 oder Aminosäuren 47-545 von SEQ ID NO: 31,
b. ein CTP-modifiziertes Faktor VII (FVII)-Polypeptid, bestehend aus der Aminosäuresequenz SEQ ID NO: 25 oder Aminosäuren 39-528 von SEQ ID NO: 25, oder
c. ein CTP-modifiziertes aktiviertes Faktor VII (FVIIa)-Polypeptid, bestehend aus der Aminosäuresequenz der Aminosäuren 39-528 von SEQ ID NO: 25;

zur Anwendung bei der Reduzierung starker Blutungen oder von Prellungen bei einem Subjekt.

**2.** Durch carboxy-terminales Peptid (CTP) von Choriongonadotropin modifizierter Gerinnungsfaktor, bestehend aus einem Gerinnungsfaktor und drei CTPs, die an den Carboxy-Terminus des Gerinnungsfaktors angehängt sind, und wobei der CTP-modifizierte Gerinnungsfaktor folgender ist:

a. ein CTP-modifiziertes Faktor IX (FIX)-Polypeptid, bestehend aus der Aminosäuresequenz SEQ ID NO: 31 oder Aminosäuren 47-545 von SEQ ID NO: 31,
b. ein CTP-modifiziertes Faktor VII (FVII)-Polypeptid, bestehend aus der Aminosäuresequenz SEQ ID NO: 25 oder Aminosäuren 39-528 von SEQ ID NO: 25, oder

c. ein CTP-modifiziertes aktiviertes Faktor VII (FVIIa)-Polypeptid, bestehend aus der Aminosäuresequenz der Aminosäuren 39-528 von SEQ ID NO: 25;

zur Anwendung beim Aufrechterhalten der Hämostase während einer Operation oder chirurgischen Eingriffen bei einem Subjekt.

3. CTP-modifizierter Gerinnungsfaktor zur Anwendung nach einem der Ansprüche 1 oder 2, wobei mindestens eines der angehängten CTPe glykosyliert ist.

4. CTP-modifizierter Gerinnungsfaktor zur Anwendung nach einem der Ansprüche 1-3, wobei der CTP-modifizierte Gerinnungsfaktor ein CTP-modifiziertes FIX-Polypeptid ist, das aus der Aminosäuresequenz SEQ ID NO: 31 oder Aminosäuren 47-545 von SEQ ID NO: 31 besteht.

5. CTP-modifizierter Gerinnungsfaktor zur Anwendung nach einem der Ansprüche 1-3, wobei der CTP-modifizierte Gerinnungsfaktor ein CTP-modifiziertes FVII-Polypeptid ist, das aus der Aminosäuresequenz SEQ ID NO: 25 oder Aminosäuren 39-528 von SEQ ID NO: 25 besteht.

6. CTP-modifizierter Gerinnungsfaktor zur Anwendung nach einem der Ansprüche 1-3, wobei der CTP-modifizierte Gerinnungsfaktor ein CTP-modifiziertes FVIIa-Polypeptid ist, das aus der Aminosäuresequenz der Aminosäuren 39-528 von SEQ ID NO: 25 besteht.

7. CTP-modifizierter Gerinnungsfaktor zur Anwendung nach einem der Ansprüche 1-6, wobei der CTP-modifizierte Gerinnungsfaktor dem Subjekt in der Form einer pharmazeutischen Zusammensetzung, die einen pharmazeutisch akzeptablen Träger umfasst, verabreicht wird.

8. CTP-modifizierter Gerinnungsfaktor zur Anwendung nach einem der Ansprüche 1-7 zur subkutanen Verabreichung.

9. CTP-modifizierter Gerinnungsfaktor zur Anwendung nach einem der Ansprüche 1-7 zur intravenösen Verabreichung.

10. CTP-modifizierter Gerinnungsfaktor zur Anwendung nach einem der Ansprüche 1-9, wobei das Subjekt ein(e) Hämophiliker(in) ist oder ein Subjekt, das an einem Vitamin K-Mangel oder einer Lebererkrankung leidet.

11. CTP-modifizierter Gerinnungsfaktor zur Anwendung nach einem der Ansprüche 1-10, wobei das Subjekt ein Kind ist.

## Revendications

1. Facteur de coagulation modifié par le peptide carboxy-terminal (CTP) de gonadotrophine chorionique comprenant un facteur de coagulation et trois CTP liés à l'extrémité carboxy dudit facteur de coagulation, et dans lequel ledit facteur de coagulation modifié par CTP est :

a. un polypeptide Facteur IX (FIX) modifié par CTP comprenant la séquence d'acides aminés de SEQ ID NO: 31 ou des acides aminés 47-545 de SEQ ID NO: 31,
b. un polypeptide de Facteur VII (FVII) modifié par CTP comprenant la séquence d'acides aminés de SEQ ID NO: 25 ou des acides aminés 39-528 de SEQ ID NO: 25, ou
c. un polypeptide de Facteur VII (FVIIa) activé modifié par CTP comprenant la séquence d'acides aminés des acides aminés 39-528 de SEQ ID NO: 25 ;

destiné à réduire les saignements excessifs ou les ecchymoses chez un sujet.

2. Facteur de coagulation modifié par le peptide carboxy-terminal de gonadotrophine chorionique (CTP) comprenant un facteur de coagulation et trois CTP liés à l'extrémité carboxy dudit facteur de coagulation, et dans lequel ledit facteur de coagulation modifié par le CTP est :

a. un polypeptide Facteur IX (FIX) modifié par CTP comprenant la séquence d'acides aminés de SEQ ID NO: 31 ou des acides aminés 47-545 de SEQ ID NO: 31,
b. un polypeptide de Facteur VII (FVII) modifié par CTP comprenant la séquence d'acides aminés de SEQ ID NO: 25 ou des acides aminés 39-528 de SEQ ID NO: 25, ou

c. un polypeptide de Facteur VII activé modifié par CTP (FVIIa) comprenant la séquence d'acides aminés des acides aminés 39-528 de SEQ ID NO: 25 ;

destiné à maintenir l'hémostase pendant une intervention chirurgicale sur un sujet.

**3.** Facteur de coagulation modifié par CTP destiné à être utilisé selon l'une quelconque des revendications 1 ou 2, dans lequel au moins l'un desdits CTP liés est glycosylé.

**4.** Facteur de coagulation modifié par CTP destiné à être utilisé selon l'une quelconque des revendications 1 à 3, dans lequel ledit facteur de coagulation modifié par CTP est un polypeptide FIX modifié par CTP comprenant la séquence d'acides aminés de SEQ ID NO: 31 ou des acides aminés 47-545 de SEQ ID NO: 31.

**5.** Facteur de coagulation modifié par CTP destiné à être utilisé selon l'une quelconque des revendications 1 à 3, dans lequel ledit facteur de coagulation modifié par CTP est un polypeptide FVII modifié par CTP comprenant la séquence d'acides aminés de SEQ ID NO: 25 ou des acides aminés 39-528 de SEQ ID NO: 25.

**6.** Facteur de coagulation modifié par CTP destiné à être utilisé selon l'une quelconque des revendications 1 à 3, dans lequel ledit facteur de coagulation modifié par CTP est un polypeptide FVIIa modifié par CTP comprenant la séquence d'acides aminés des acides aminés 39-528 de SEQ ID NO. : 25.

**7.** Facteur de coagulation modifié par CTP destiné à être utilisé selon l'une quelconque des revendications 1 à 6, dans lequel ledit facteur de coagulation modifié par CTP est administré au sujet sous la forme d'une composition pharmaceutique comprenant un support pharmaceutiquement acceptable.

**8.** Facteur de coagulation modifié par CTP destiné à être utilisé selon l'une quelconque des revendications 1 à 7 par administration sous-cutanée.

**9.** Facteur de coagulation modifié par le CTP destiné à être utilisé selon l'une quelconque des revendications 1 à 7 par administration intraveineuse.

**10.** Facteur de coagulation modifié par le CTP destiné à être utilisé selon l'une quelconque des revendications 1 à 9, dans lequel ledit sujet est un sujet hémophile, ou est un sujet souffrant d'une carence en vitamine K ou d'une maladie du foie.

**11.** Facteur de coagulation modifié par CTP destiné à être utilisé selon l'une quelconque des revendications 1 à 10, dans lequel ledit sujet est un enfant.

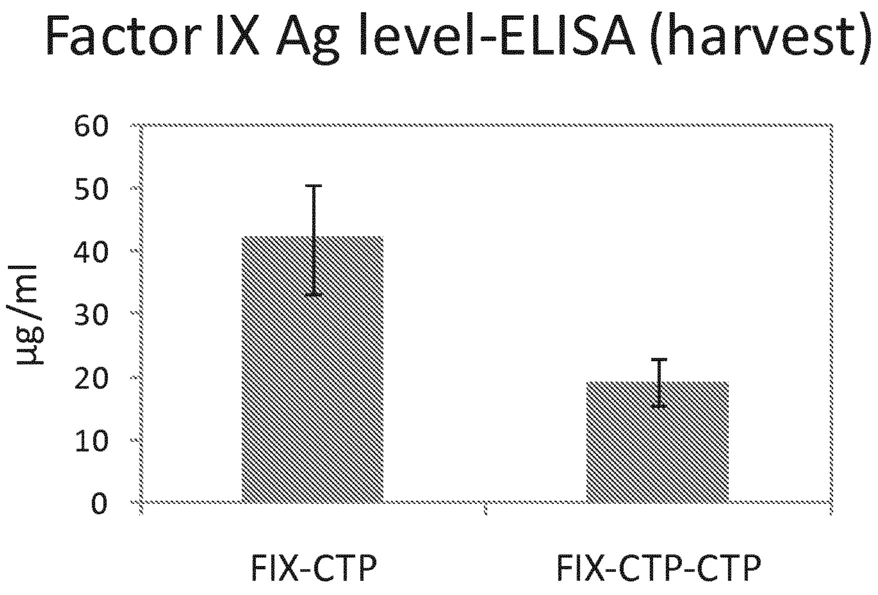


FIGURE 1A

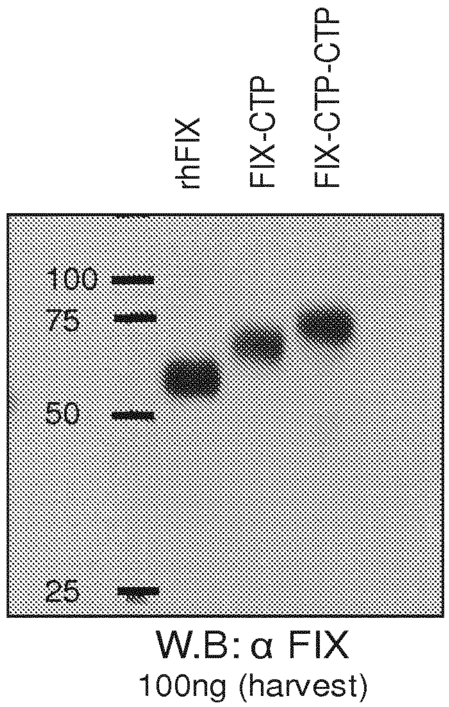


FIGURE 1B

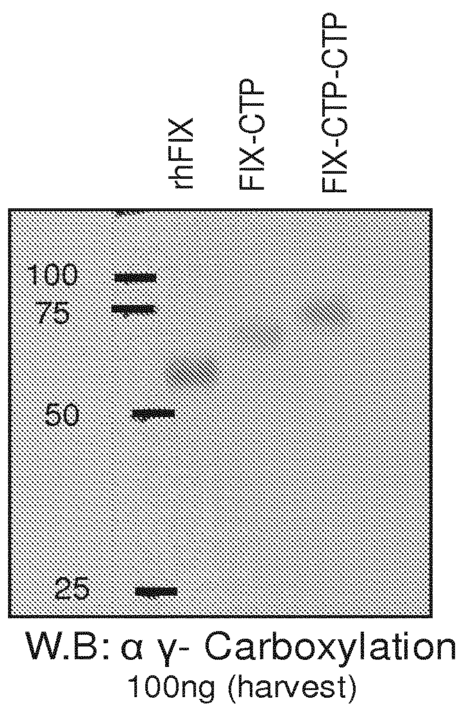


FIGURE 1C

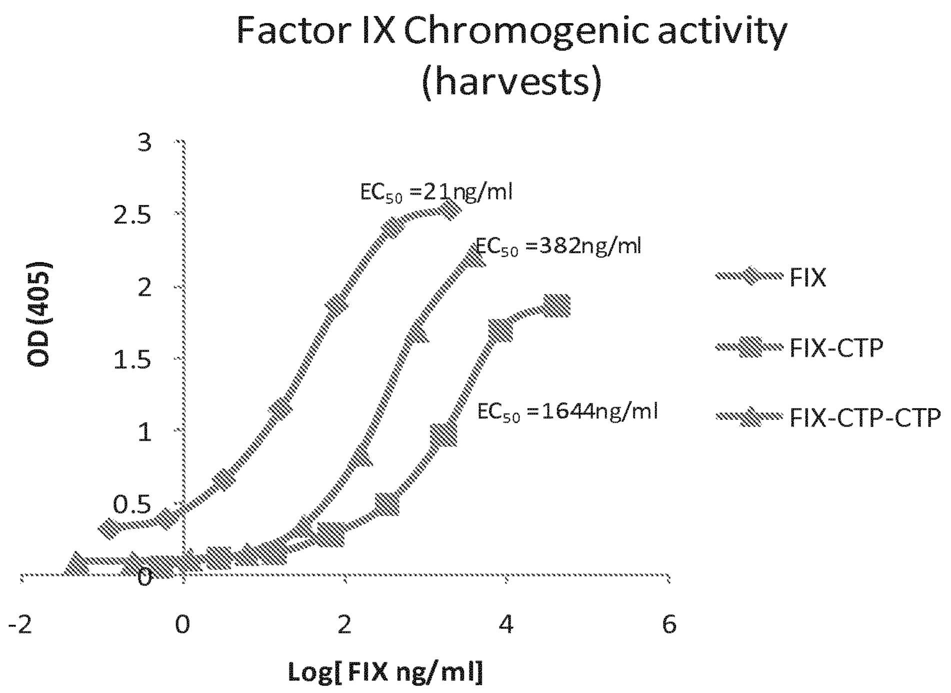


FIGURE 2

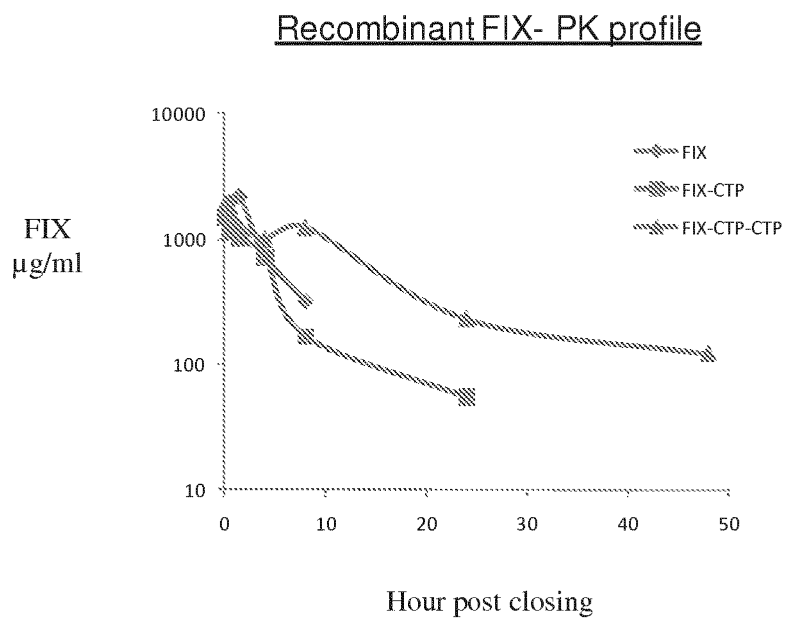


FIGURE 3

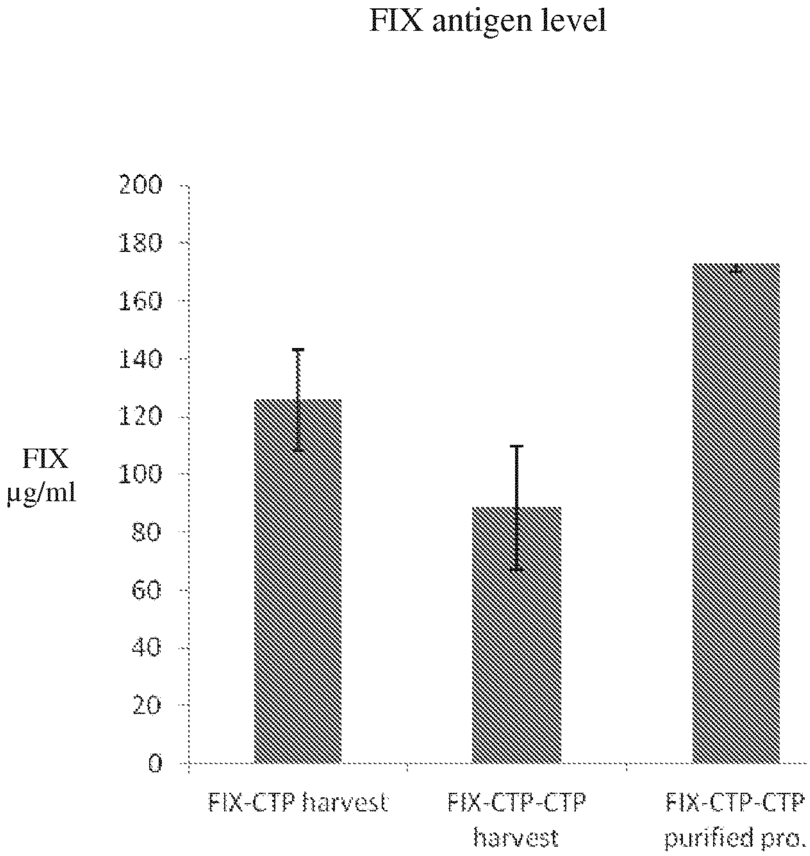


FIGURE 4

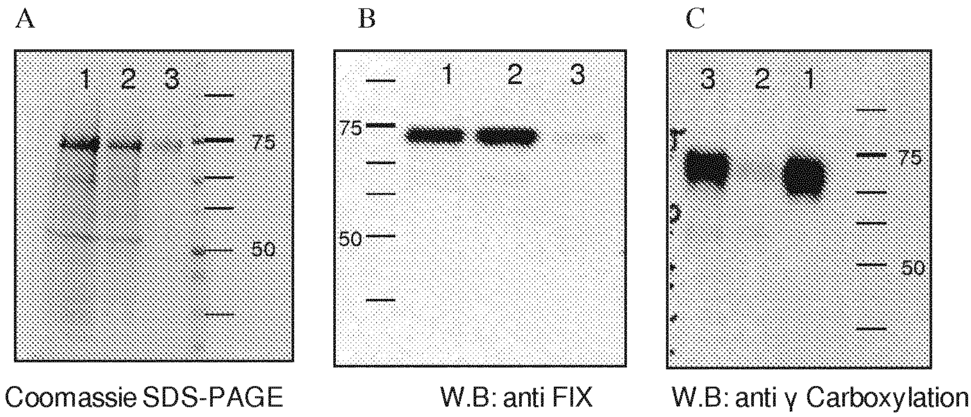


FIGURE 5A

1- FIX-(CTP)$_2$ Harvest
2- Unbound
3- Conc. elution (MOD3012)

FIGURE 5B

FIGURE 5C

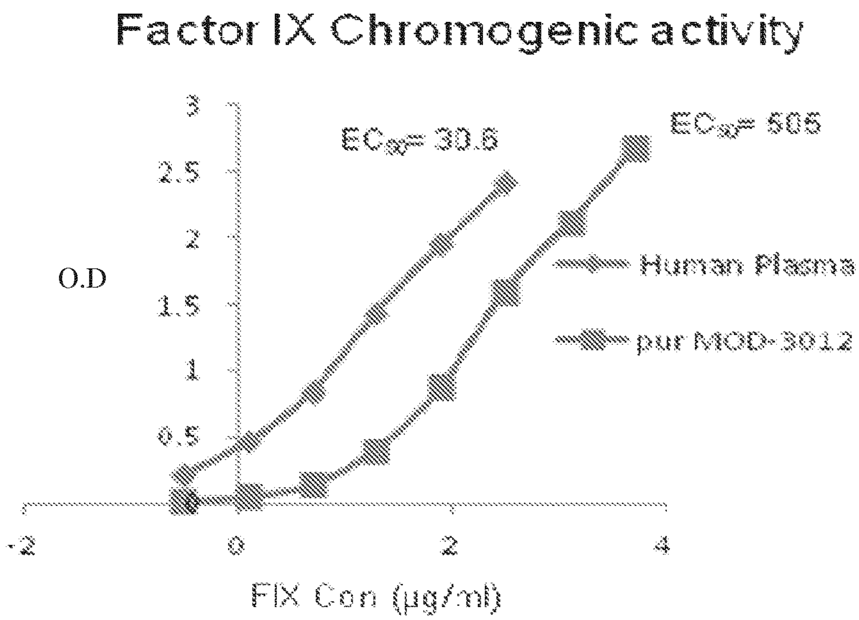


FIGURE 6

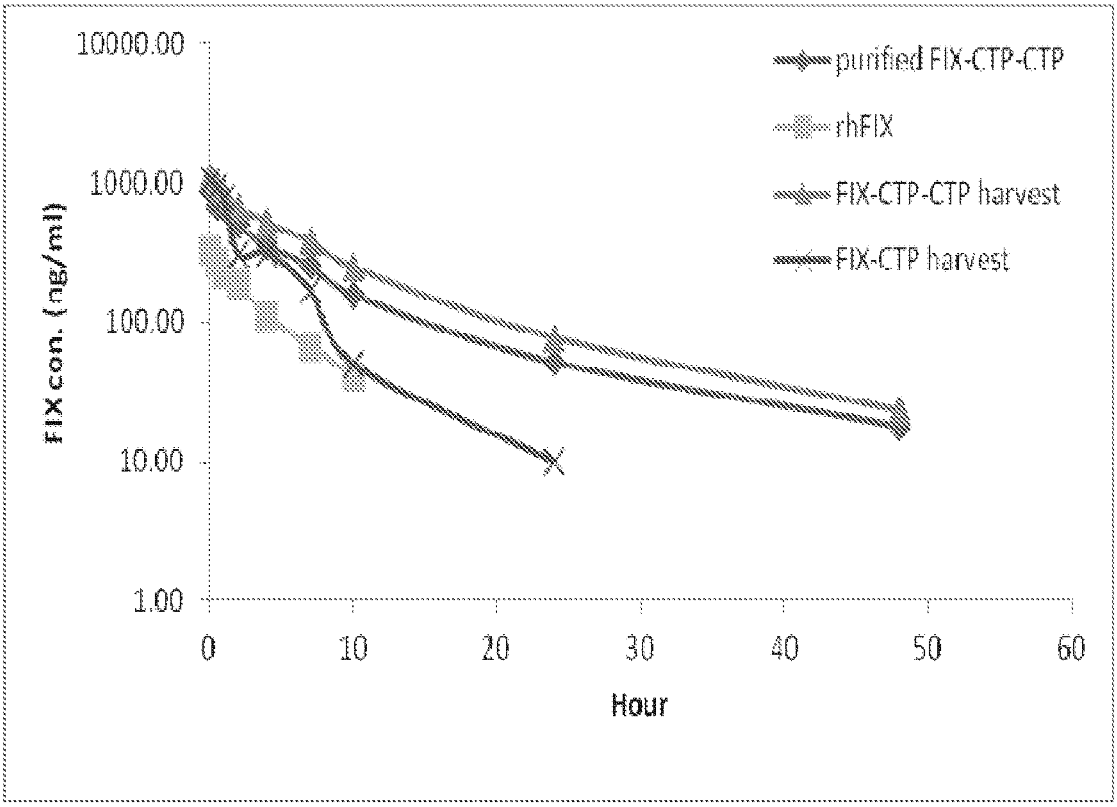


FIGURE 7

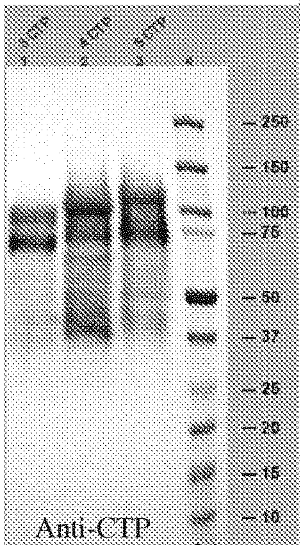


FIGURE 8A

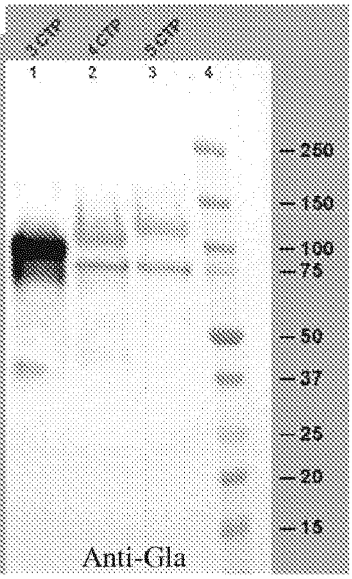


FIGURE 8B

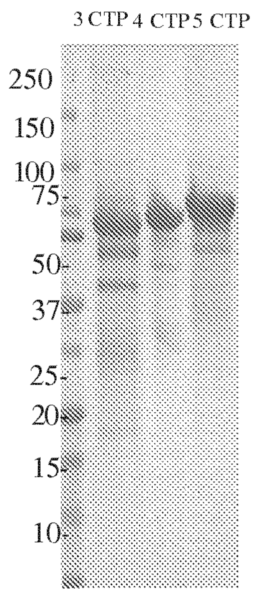


FIGURE 9

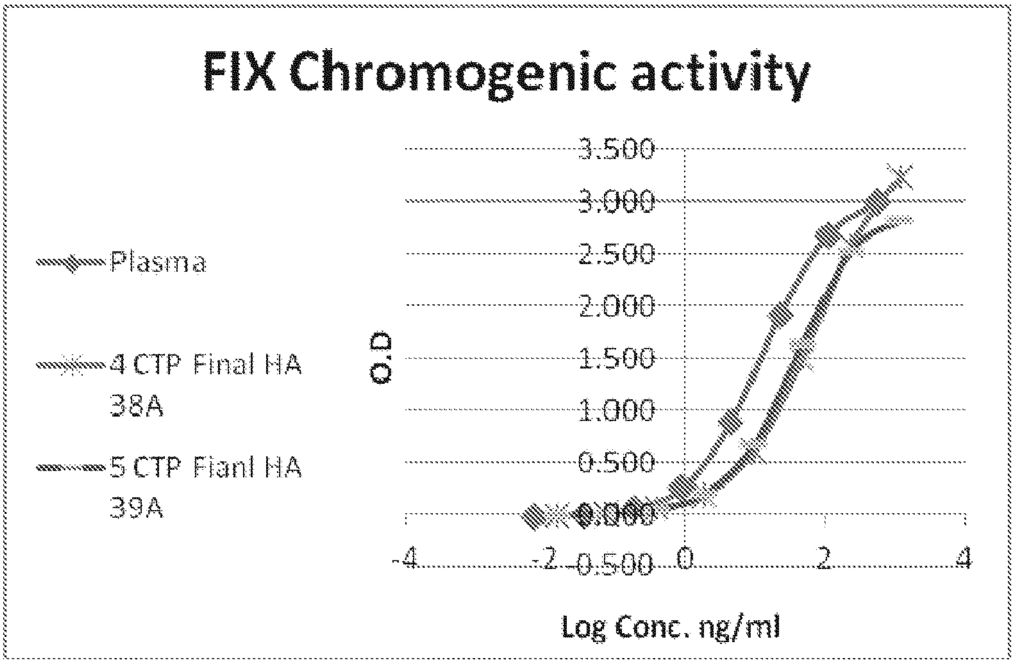


FIGURE 10

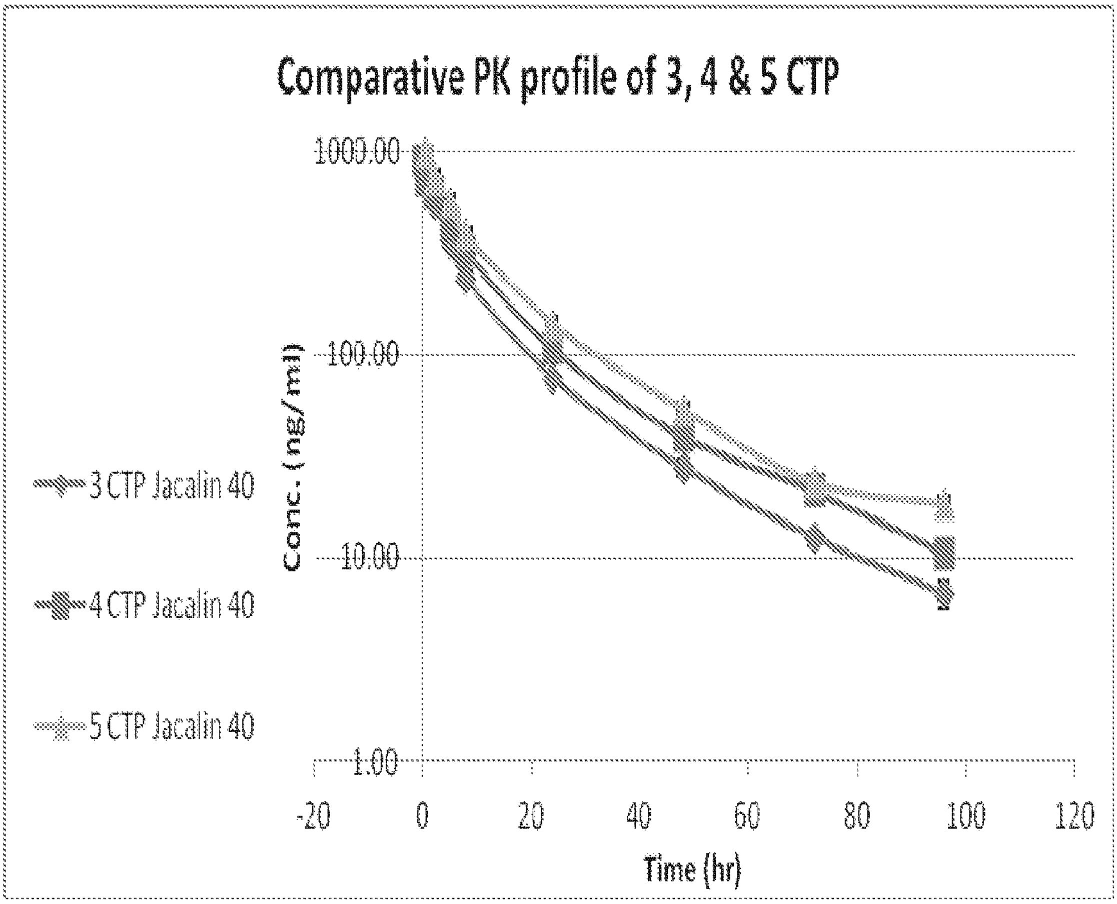


FIGURE 11

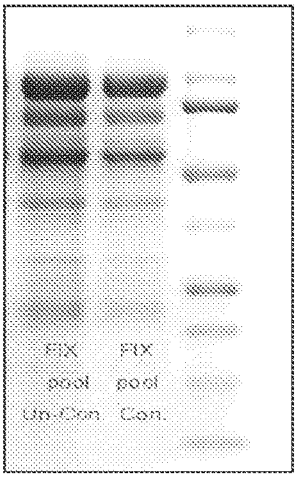


FIGURE 12A

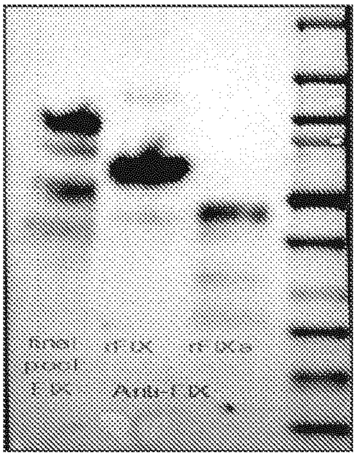


FIGURE 12B

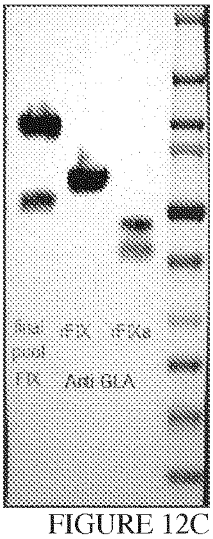


FIGURE 12C

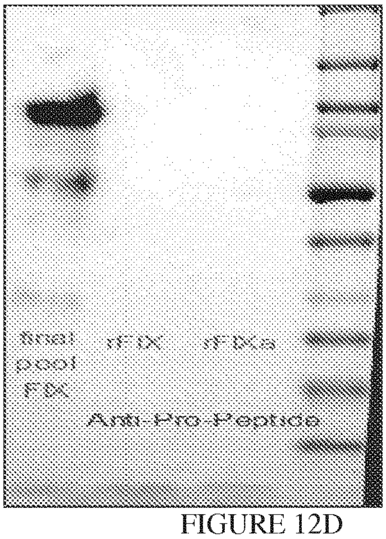


FIGURE 12D

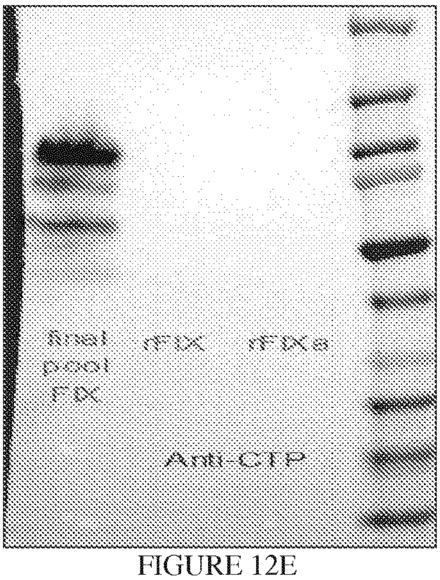


FIGURE 12E

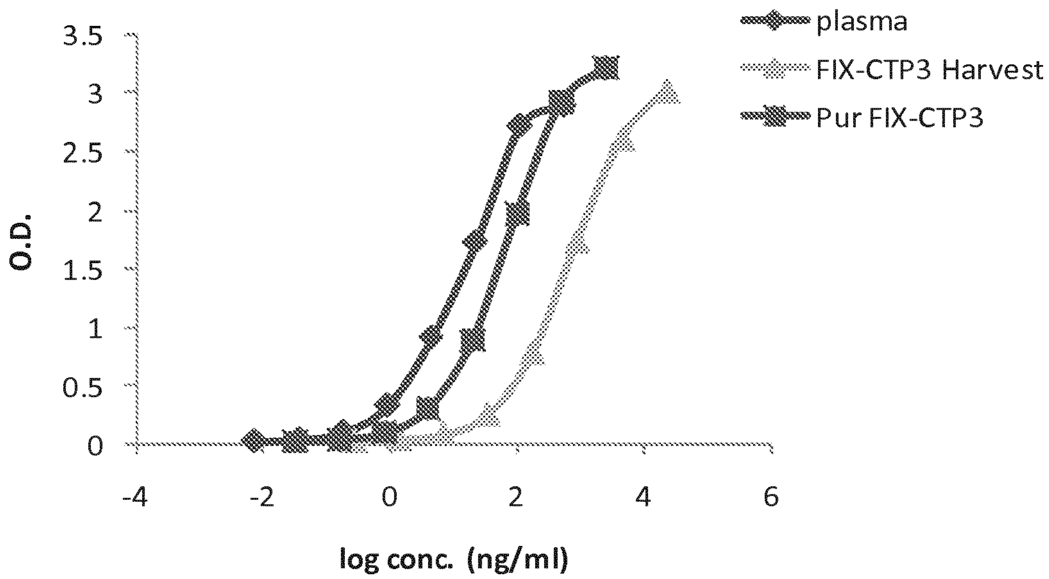


FIGURE 13

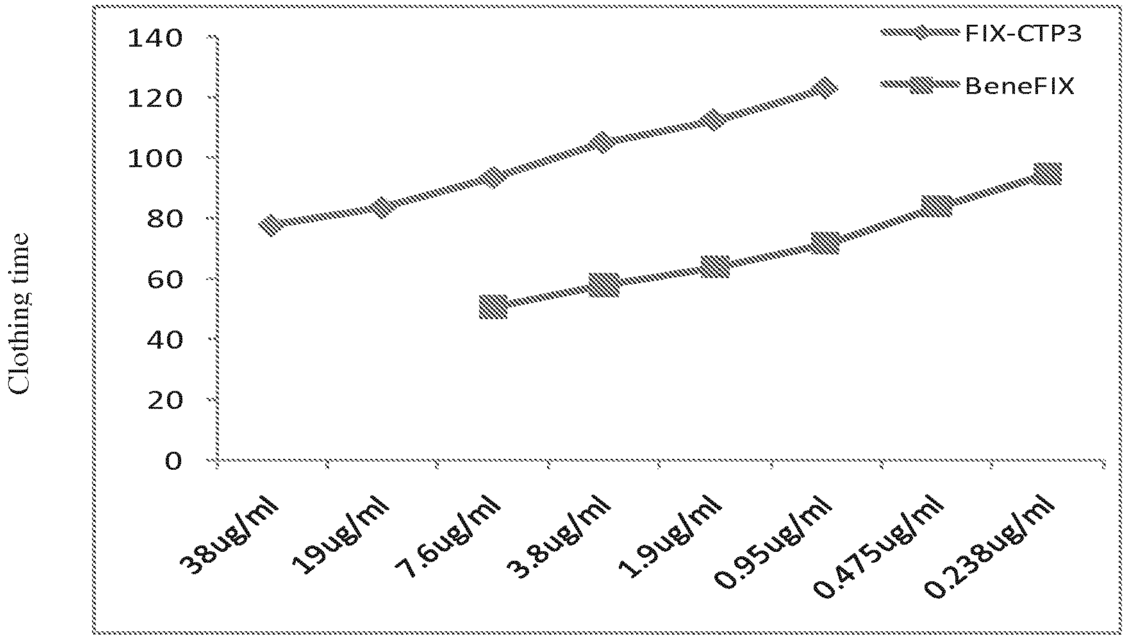


FIGURE 14

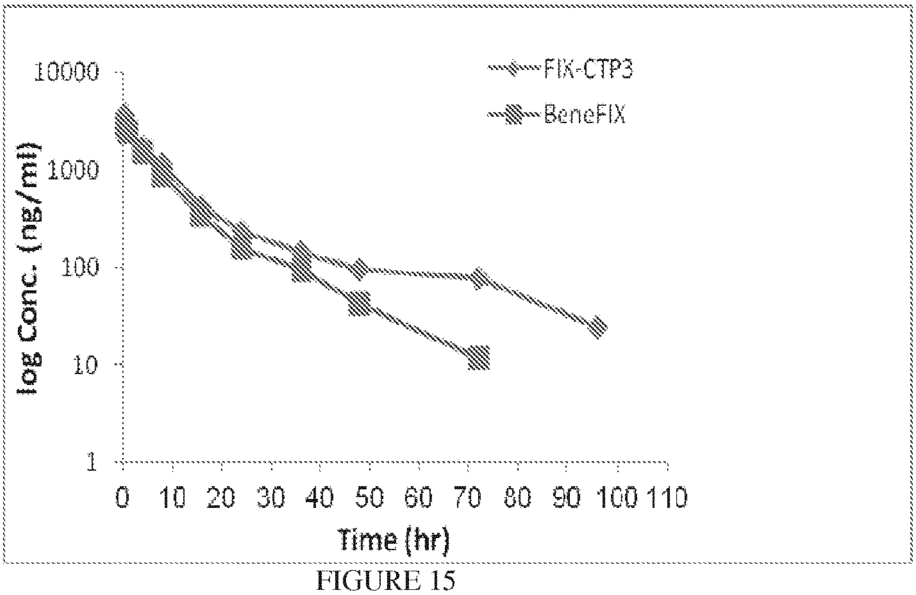


FIGURE 15

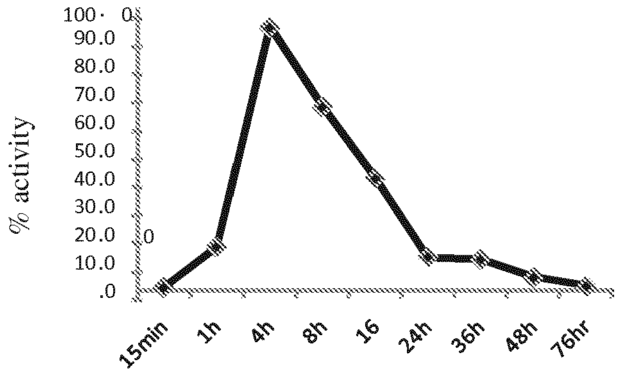


FIGURE 16A

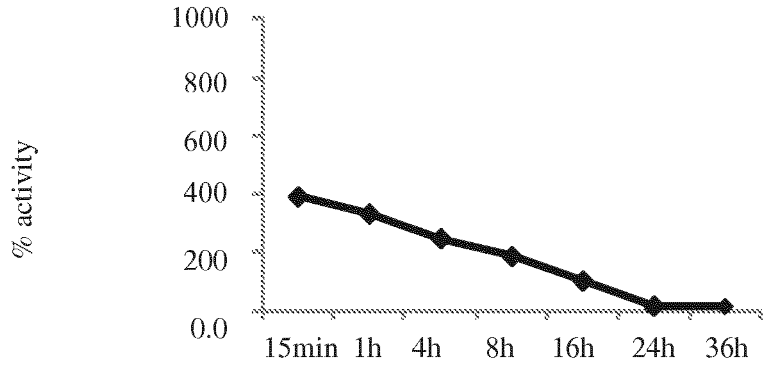


FIGURE 16B

| First bleeding challenge : Hemoglobin OD value | | | |
|---|---|---|---|
| | FIX-$CTP_3$ | BeneFIX | FIXKO |
| #1 | 6.84 | 10.26 | 19.92 |
| #2 | 0.72 | 10.14 | 13.32 |
| #3 | 4.68 | 11.16 | 16.38 |
| #4 | NA | 9.18 | 7.92 |
| #5 | 7.86 | 7.77 | 9.72 |
| #6 | 4.14 | 9.15 | 14.7 |

FIGURE 17A

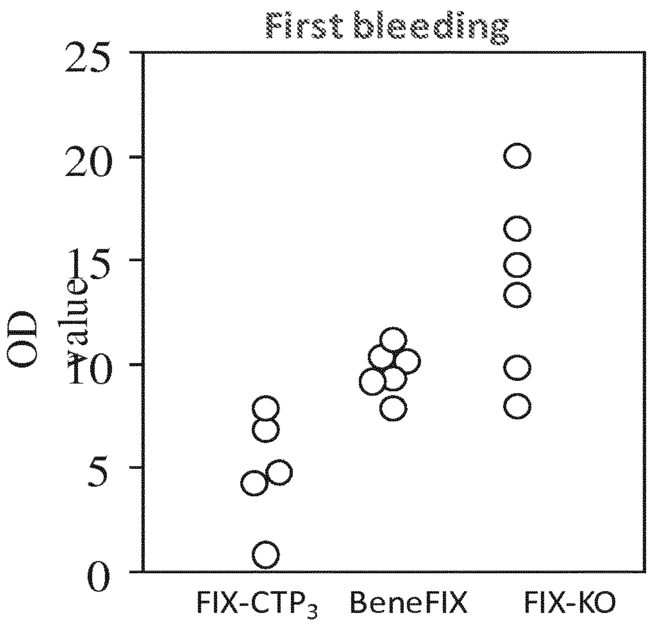


FIGURE 17B

first bleeding time (min)

| | cohort#1 | cohort #3 | FIXKO |
|---|---|---|---|
| #1 | 10 | 10 | 10 |
| #2 | 5.12 | 10 | 8.17 |
| #3 | 10 | 10 | 10 |
| #4 | | 10 | 10 |
| #5 | 10 | 7 | 10 |
| #6 | 10 | 10 | 10 |

FIGURE 17C

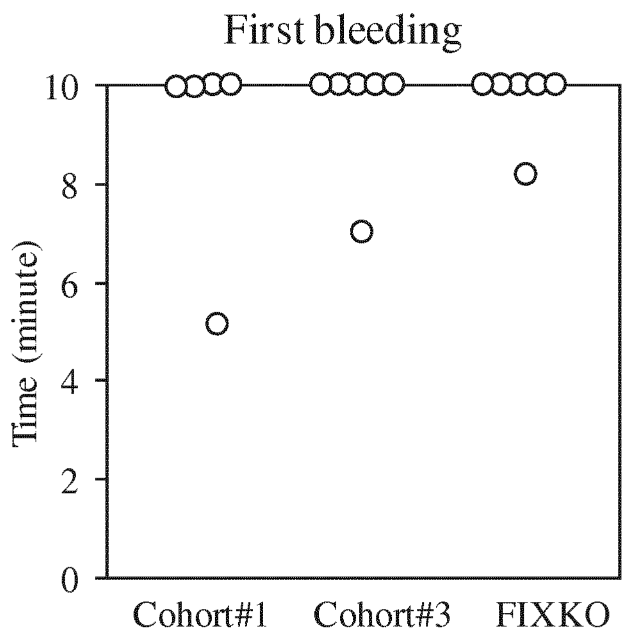


FIGURE 17D

second bleeding: OD value

| | FIX-$CTP_3$ | BeneFIX | FIXKO |
|---|---|---|---|
| #1 | 0.324 | 1.368 | 1.32 |
| #2 | 0.358 | 0.516 | 0.43 |
| #3 | 0.006 | 0.548 | 0.6 |
| #4 | | 0.027 | 1.26 |
| #5 | 0.064 | 0.158 | 0.46 |
| #6 | 0.045 | 0.992 | 0.384 |

FIGURE 18A

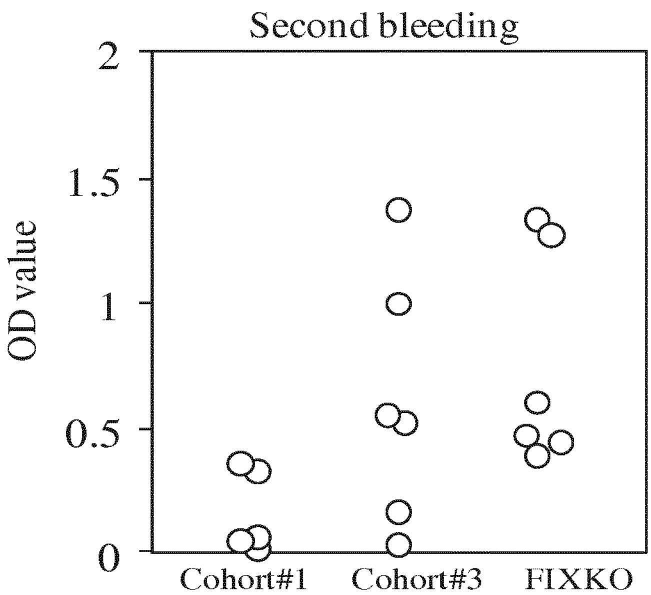


FIGURE 18B

second bleeding time (min)

| | cohort#1 | cohort #3 | FIXKO |
|---|---|---|---|
| #1 | 4.63 | 10 | 10 |
| #2 | 2.5 | 10 | 8.7 |
| #3 | 1.2 | 10 | 7.13 |
| #4 | | 5 | 10 |
| #5 | 3.87 | 7.4 | 10 |
| #6 | 1.83 | 10 | 6.5 |

FIGURE 18C

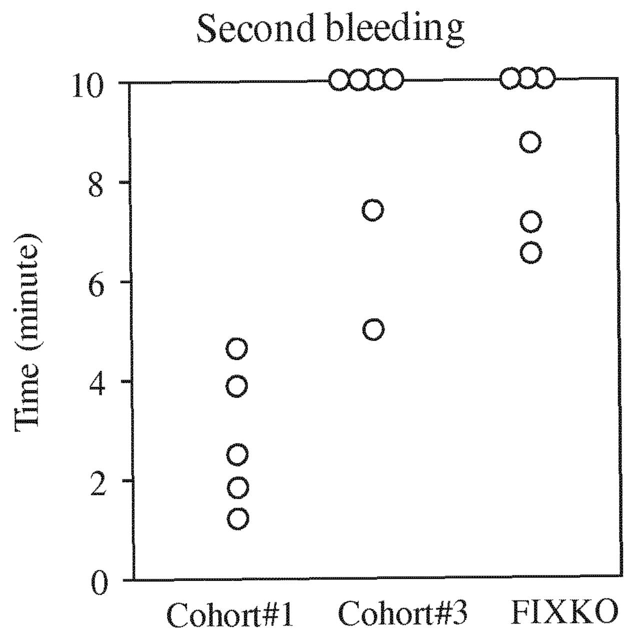


FIGURE 18D

FIGURE 19A

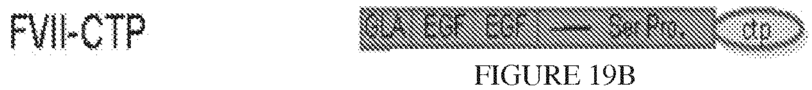


FIGURE 19B

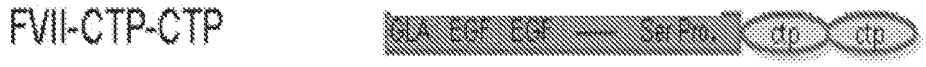


FIGURE 19C

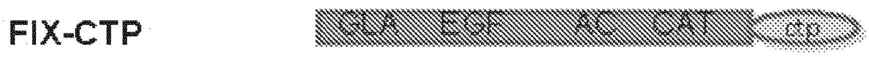


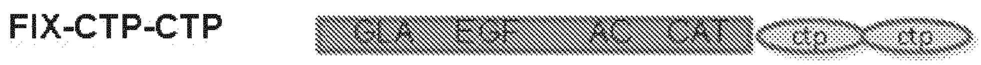


FIGURE 19D

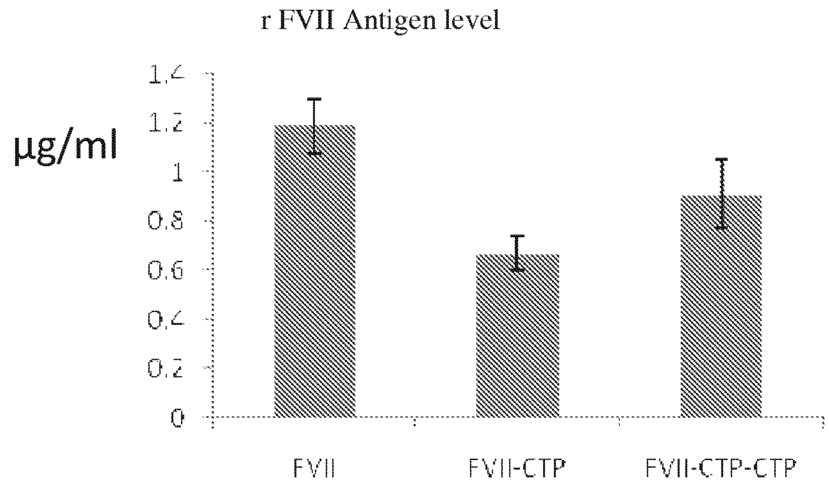


FIGURE 20A

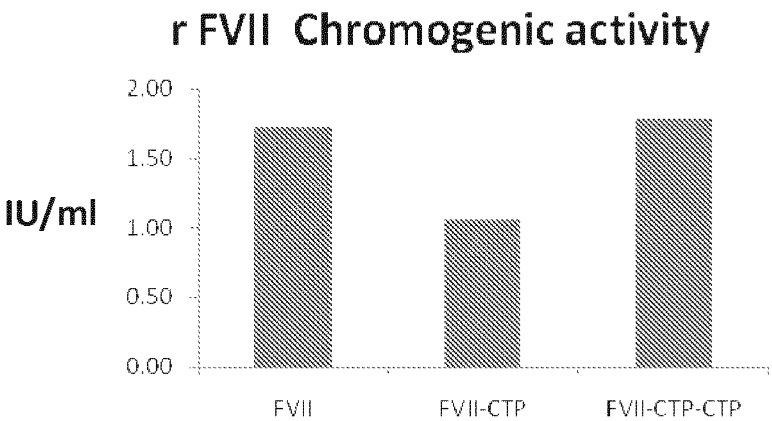


FIGURE 20B

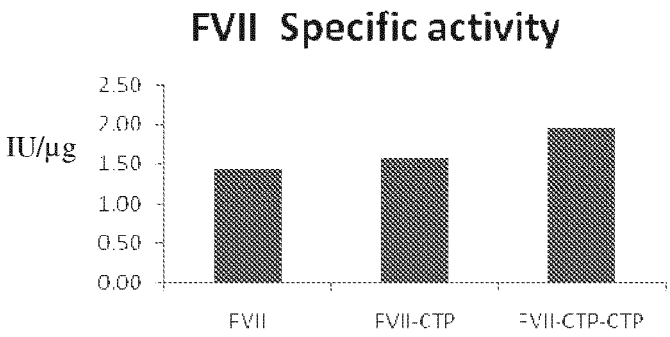


FIGURE 20C

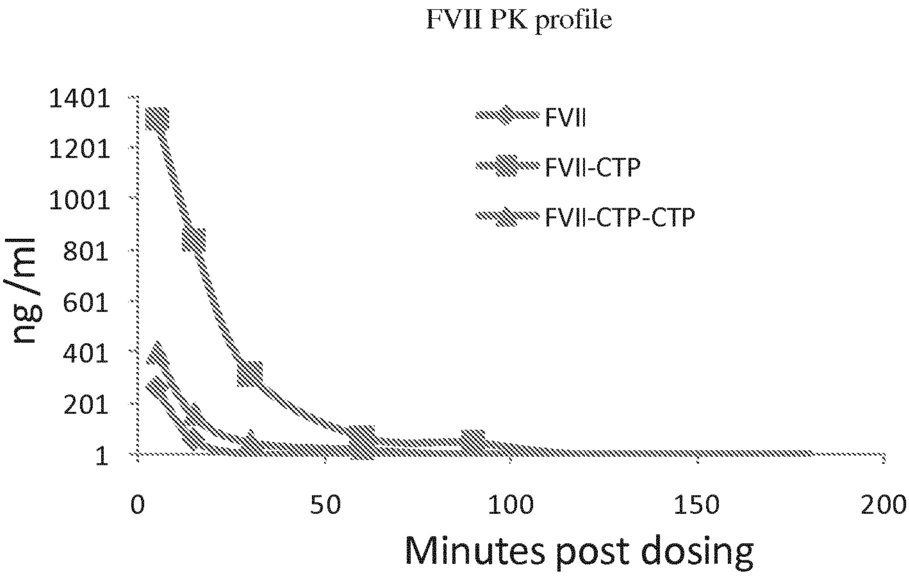


FIGURE 20D

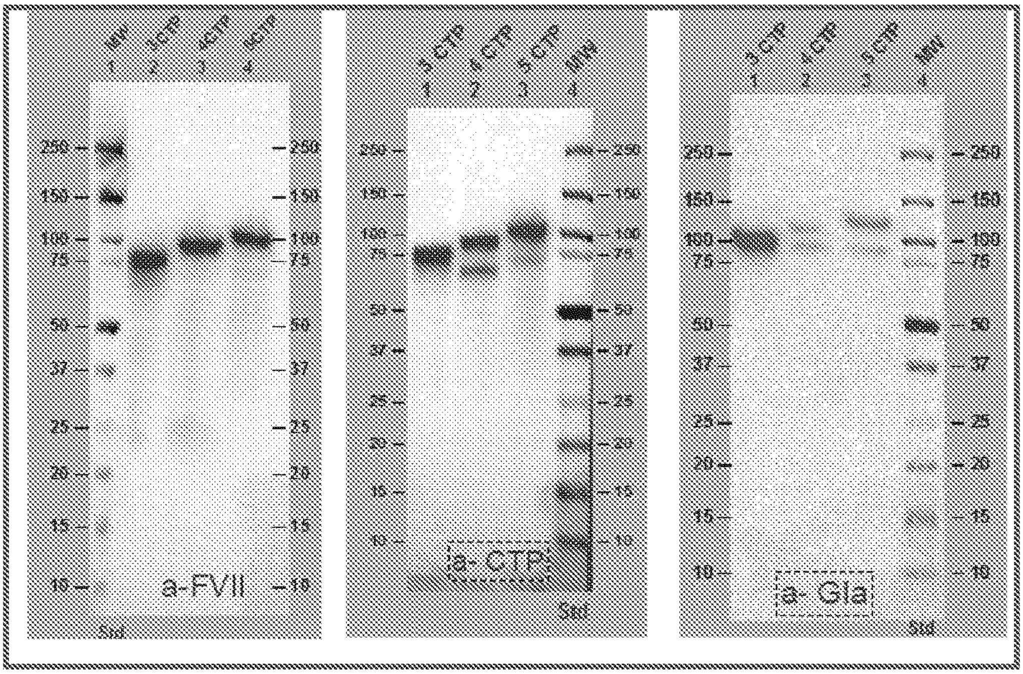


FIGURE 21A FIGURE 21B FIGURE 21C

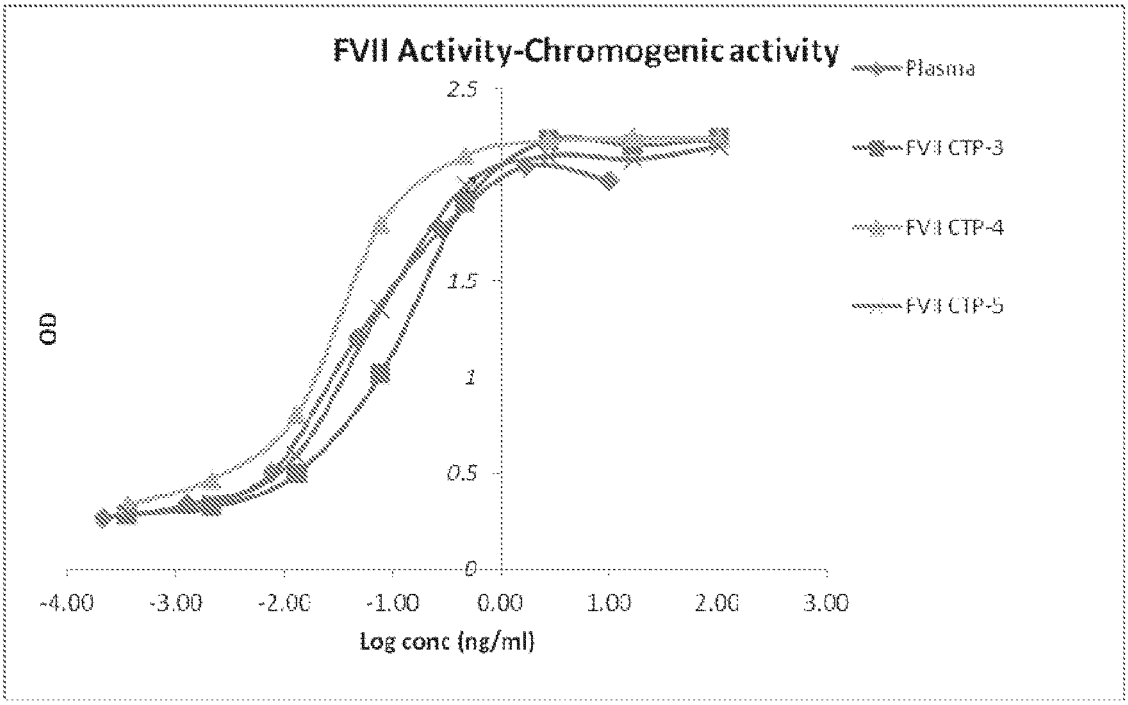


FIGURE 22

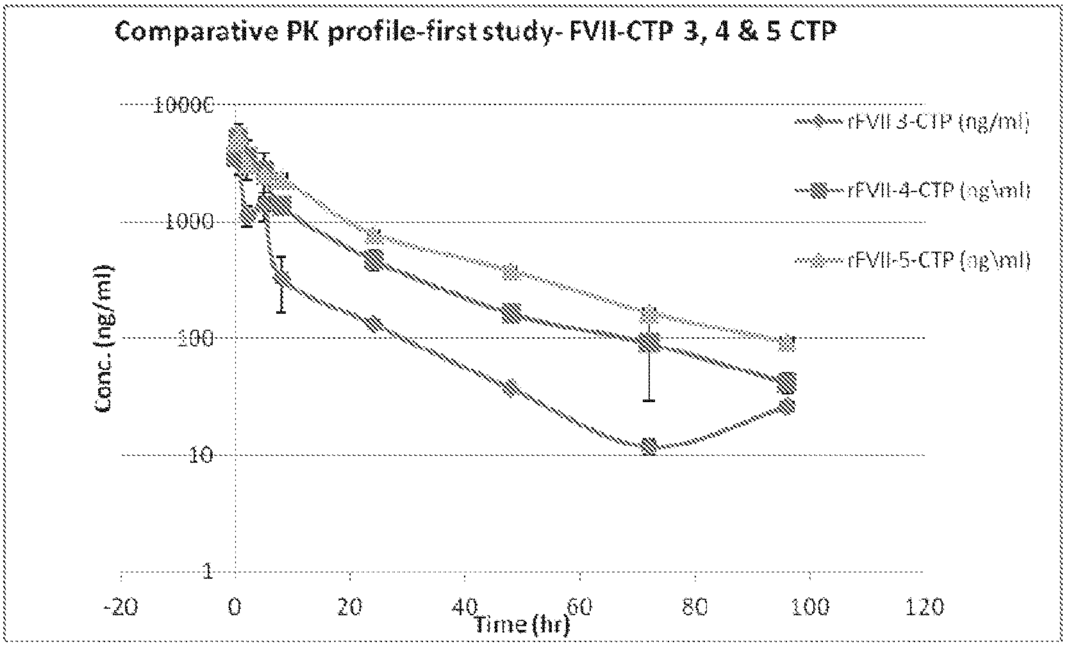


FIGURE 23

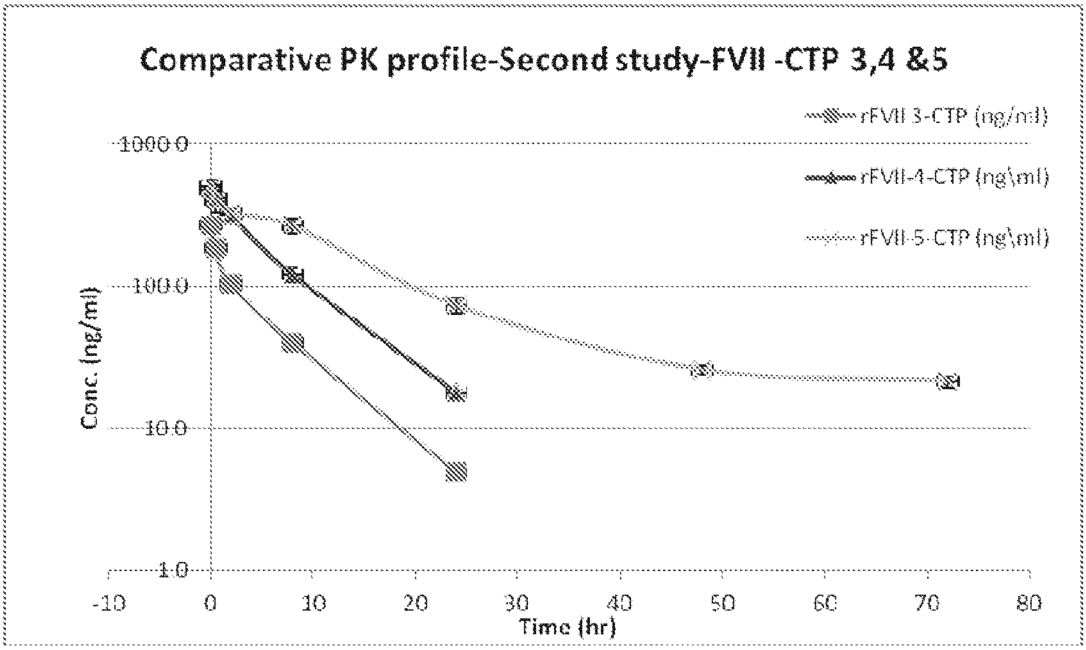


FIGURE 24

FIGURE 25A

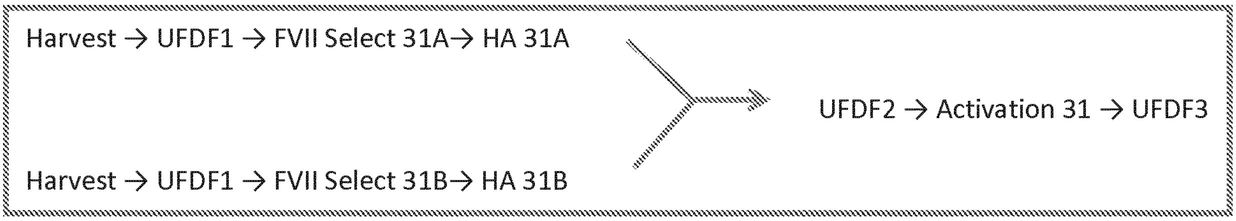


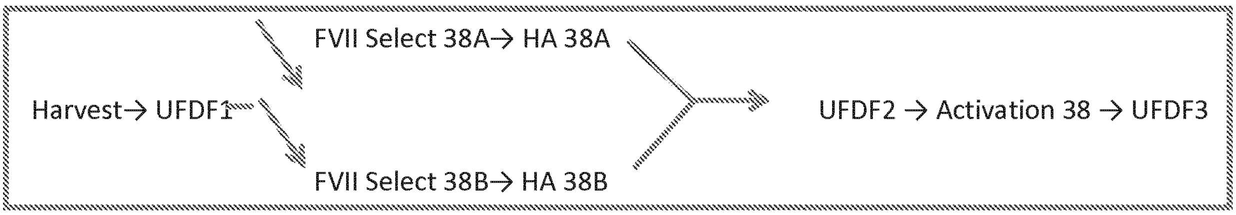


FIGURE 25B

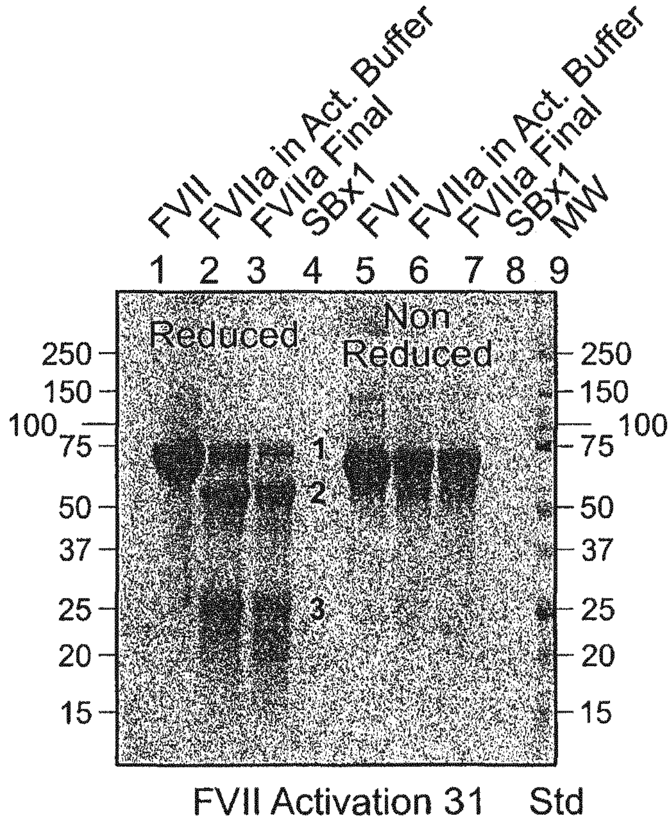


FIGURE 26A

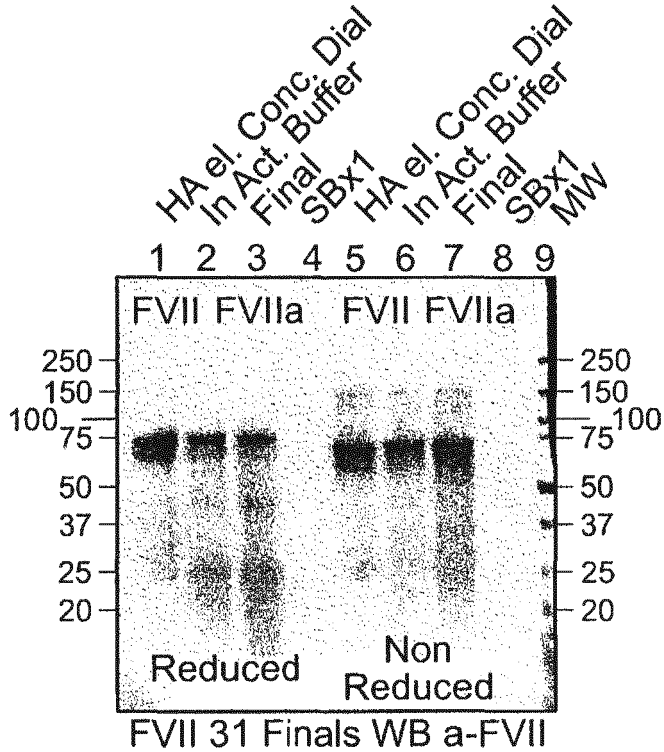


FIGURE 26B

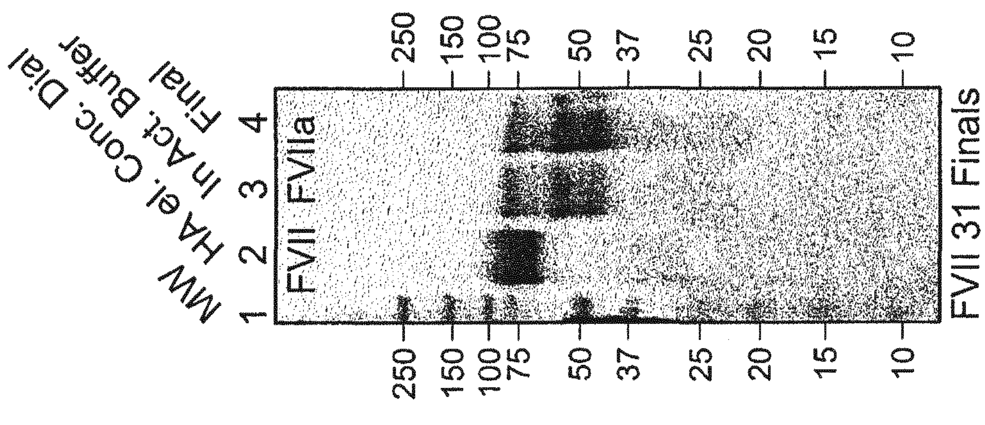


FIGURE 26C

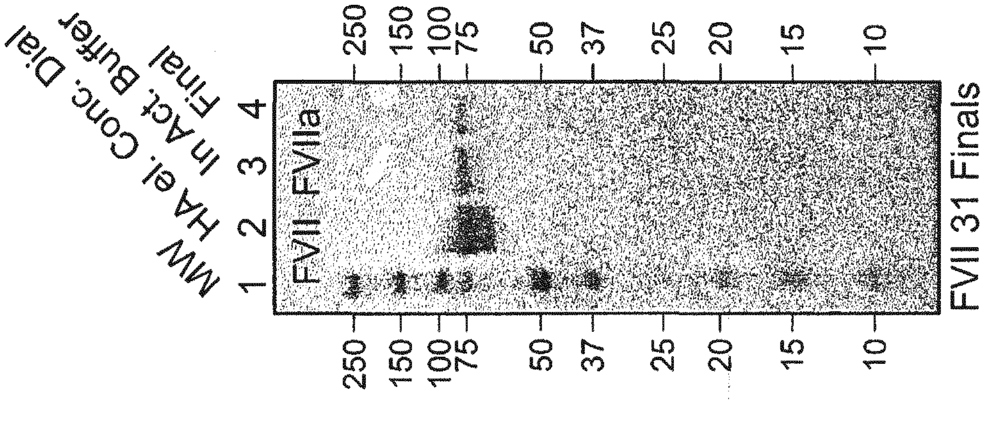


FIGURE 26D

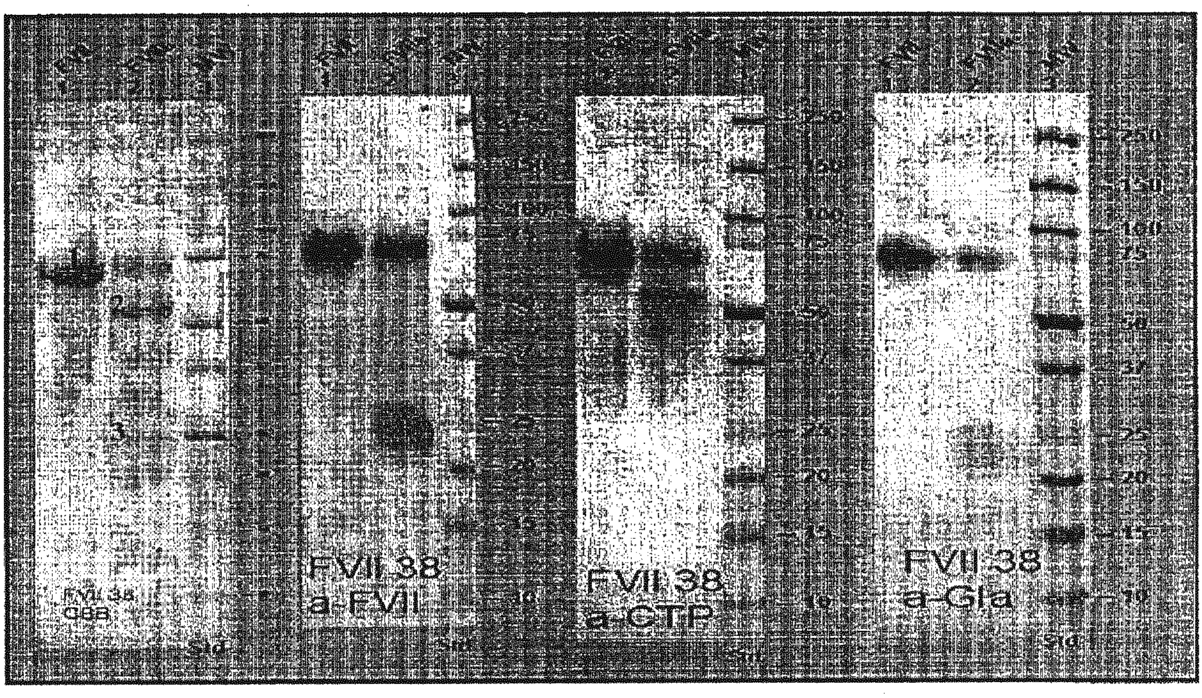


FIGURE 26E FIGURE 26F FIGURE 26G FIGURE 26H

FIGURE 27

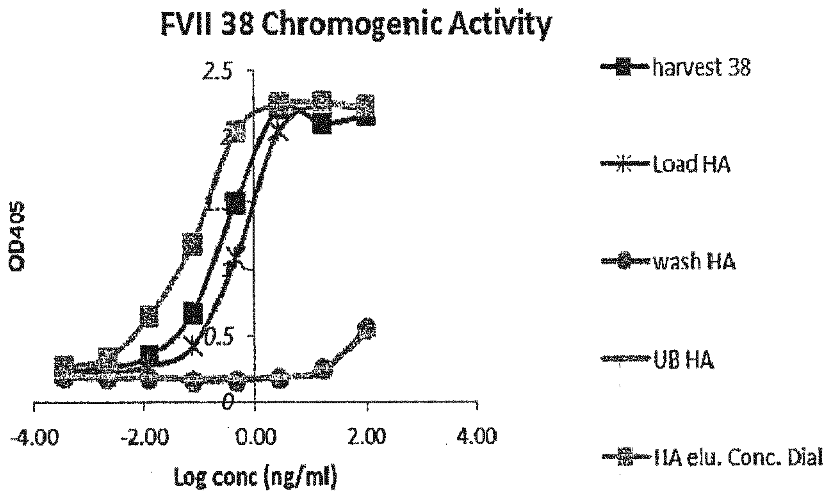

FIGURE 28

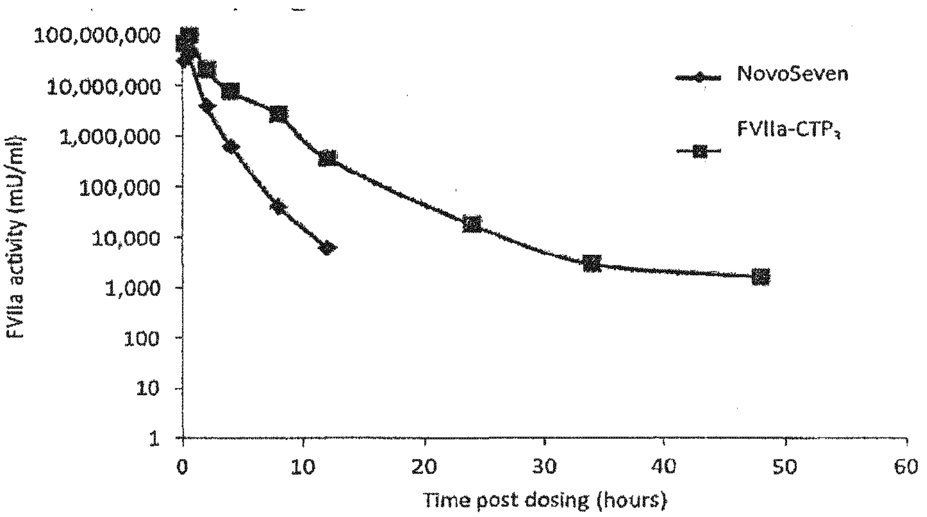


FIGURE 29A

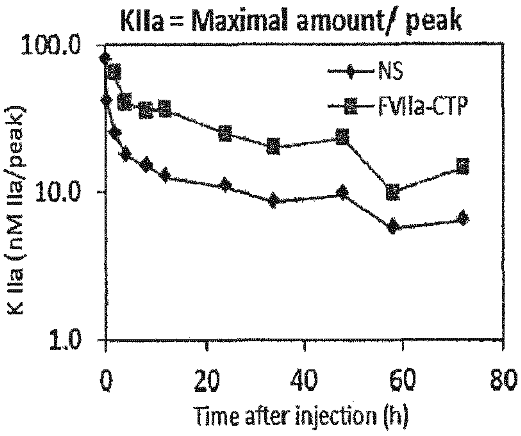


FIGURE 29B

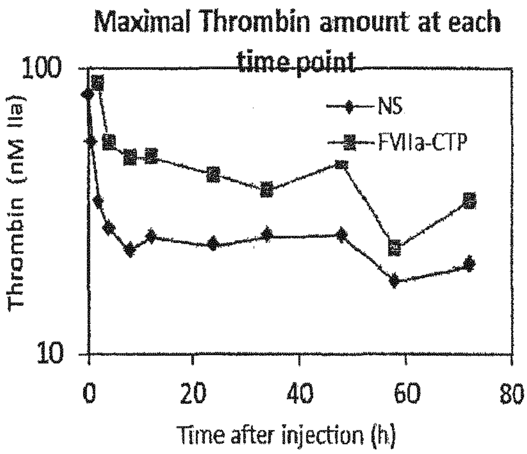


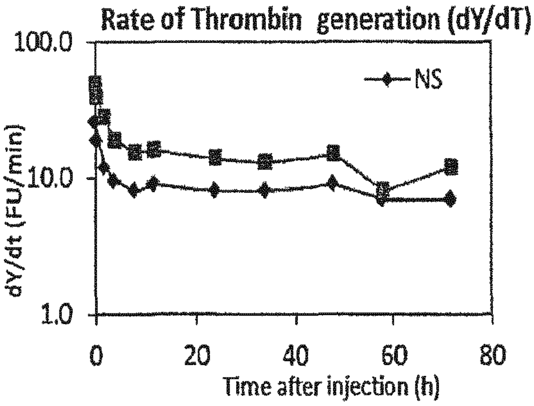


FIGURE 29C

FIGURE 30A

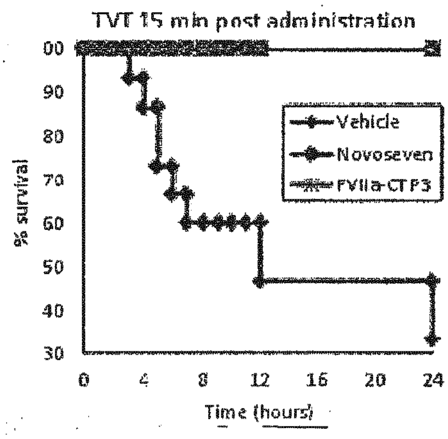


FIGURE 30B

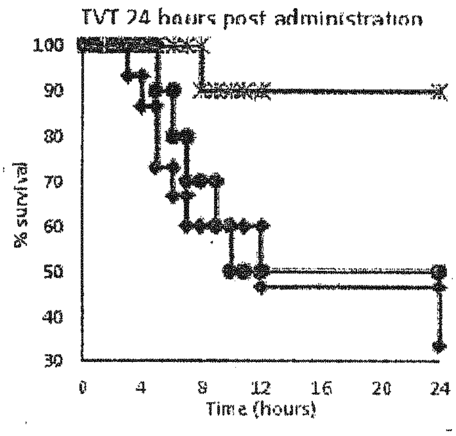


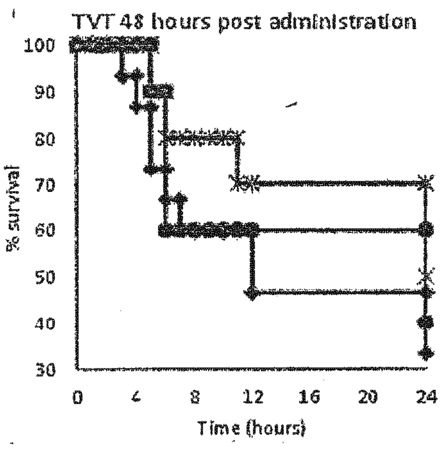


FIGURE 30C

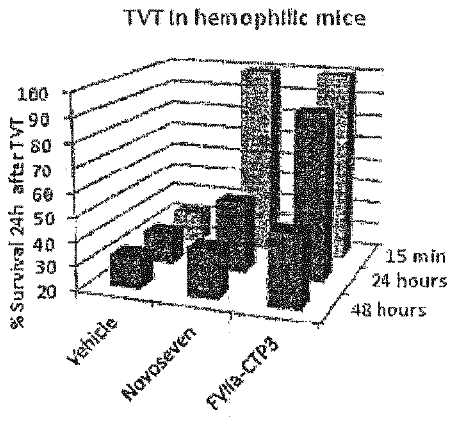


FIGURE 30D

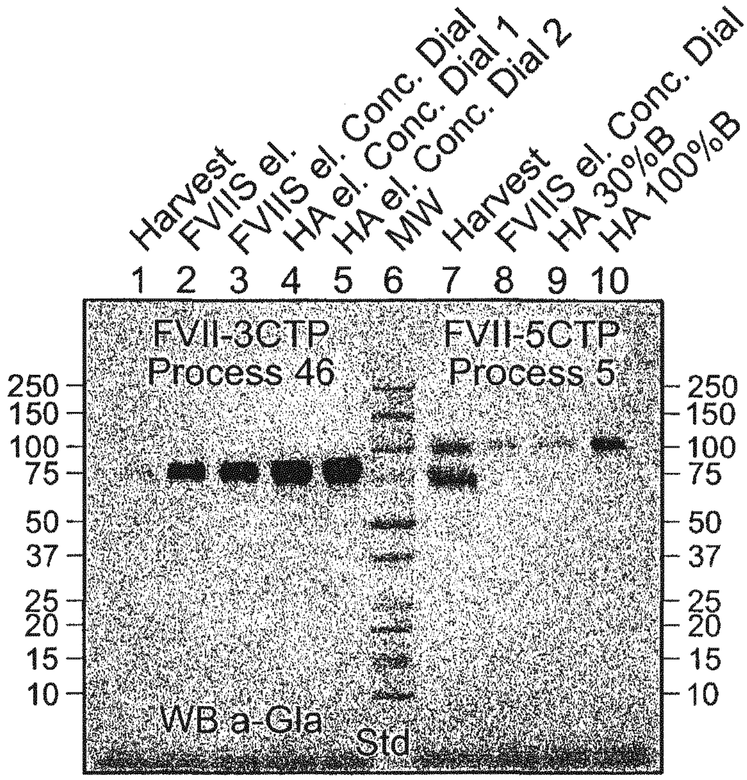


FIGURE 31A

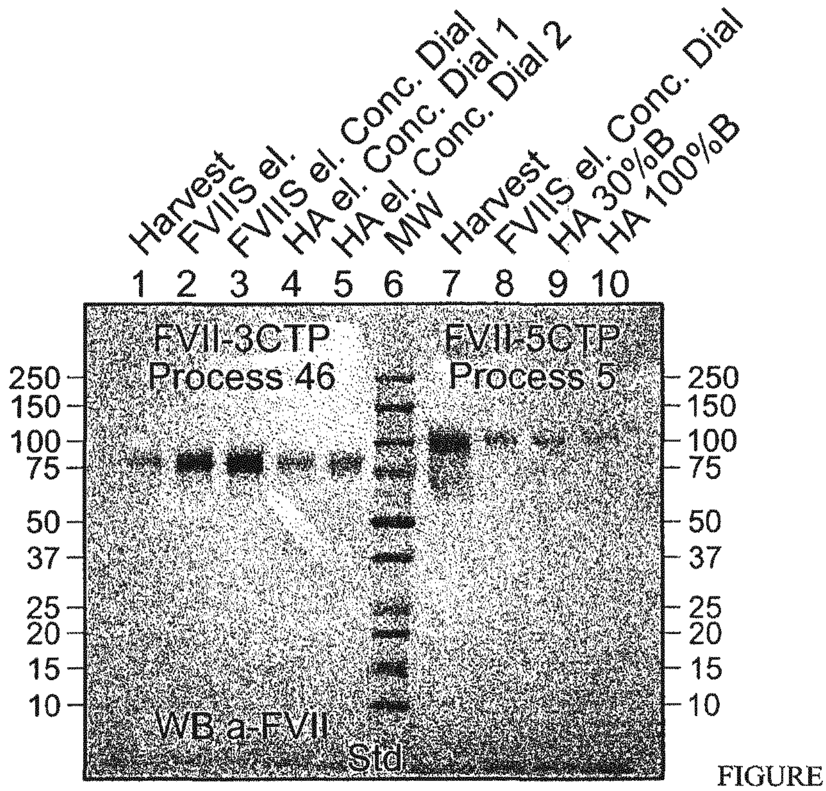


FIGURE 31B

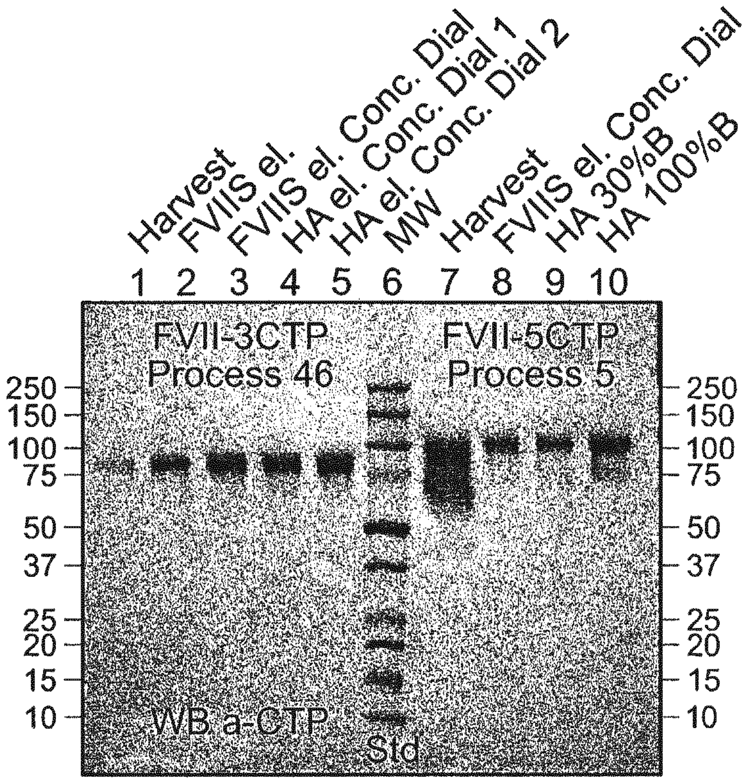


FIGURE 31C

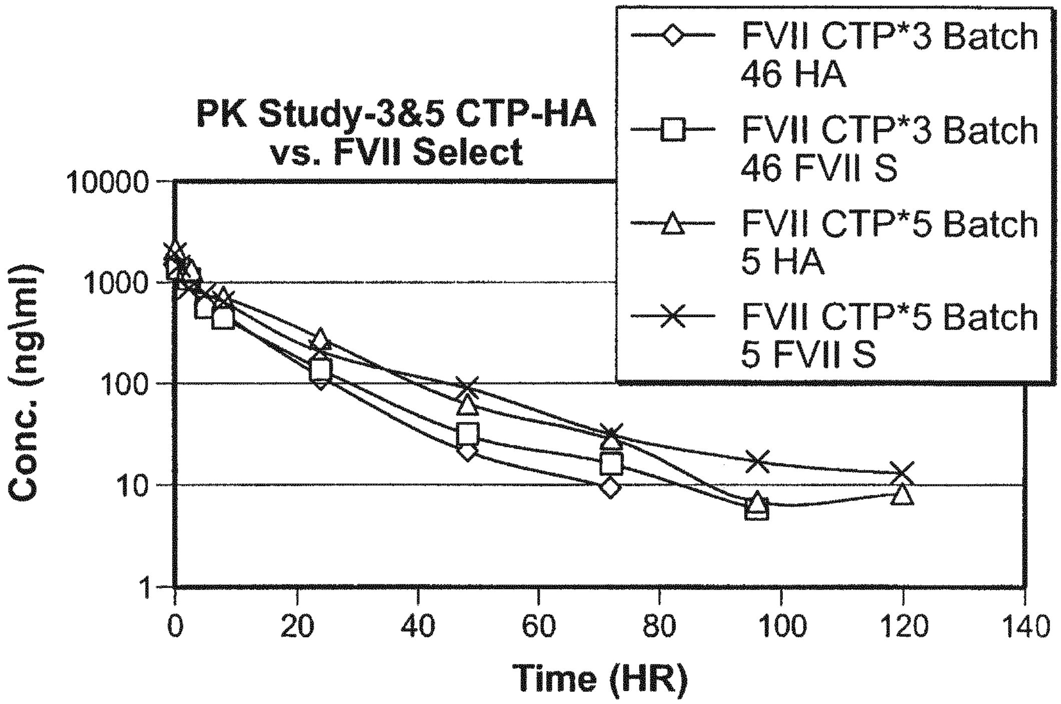


FIGURE 32

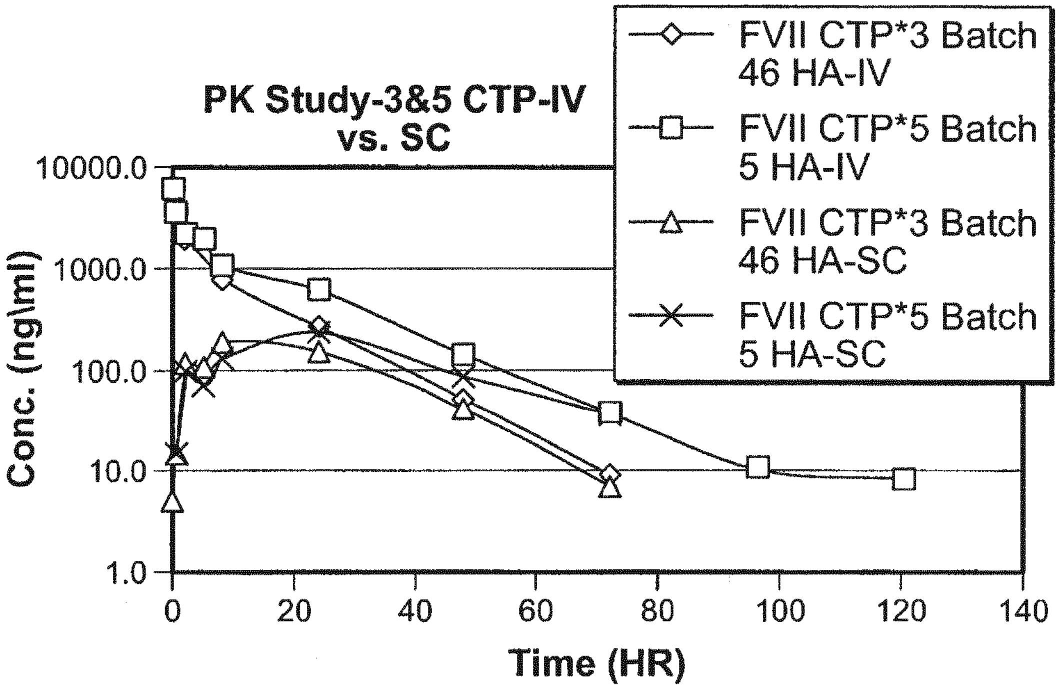


FIGURE 33

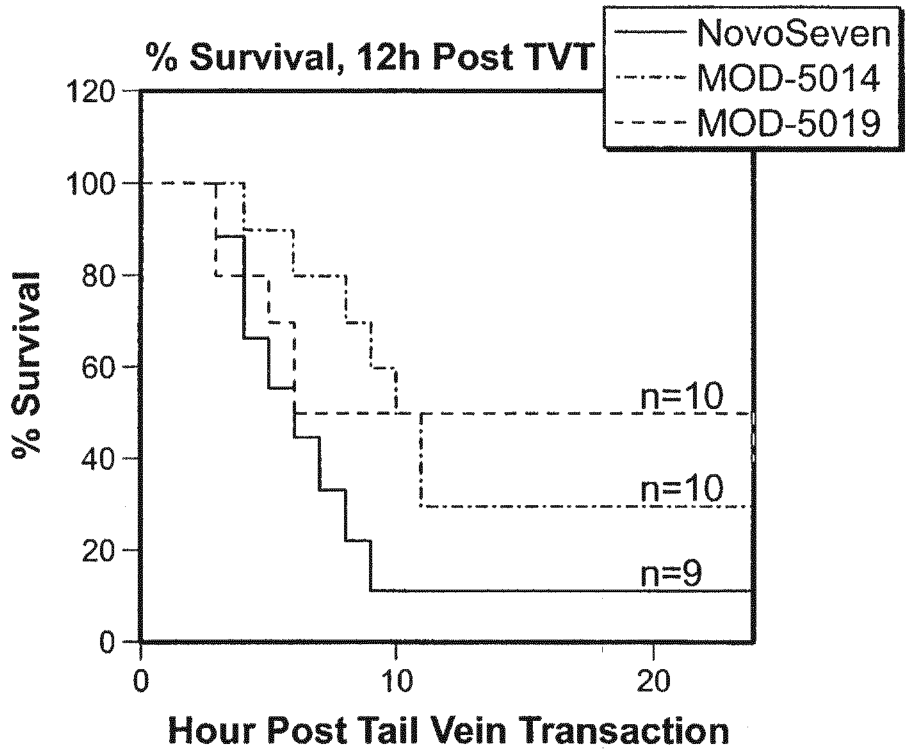


FIGURE 34

FIGURE 35A

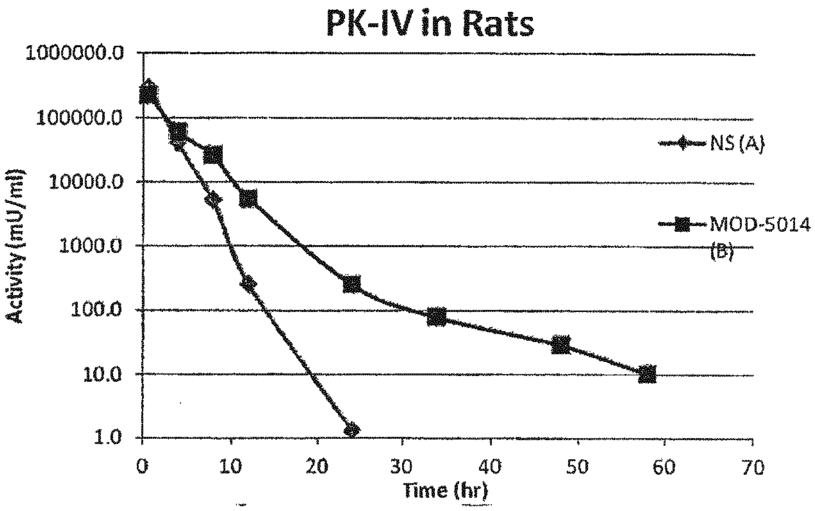


FIGURE 35B

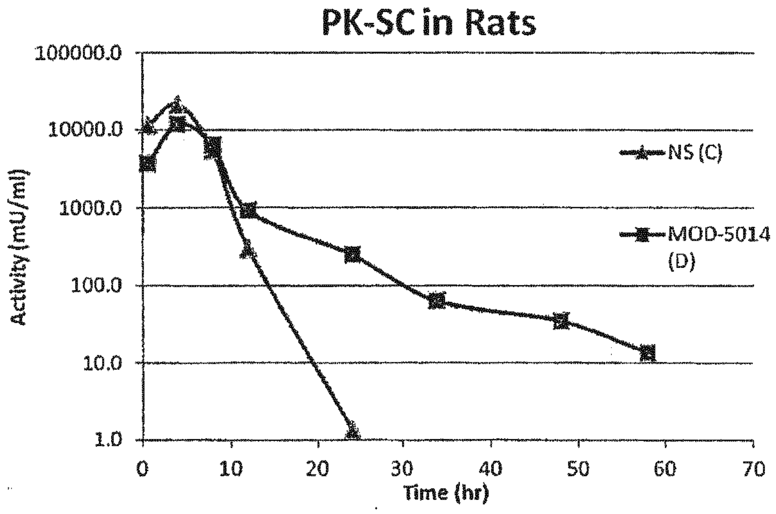

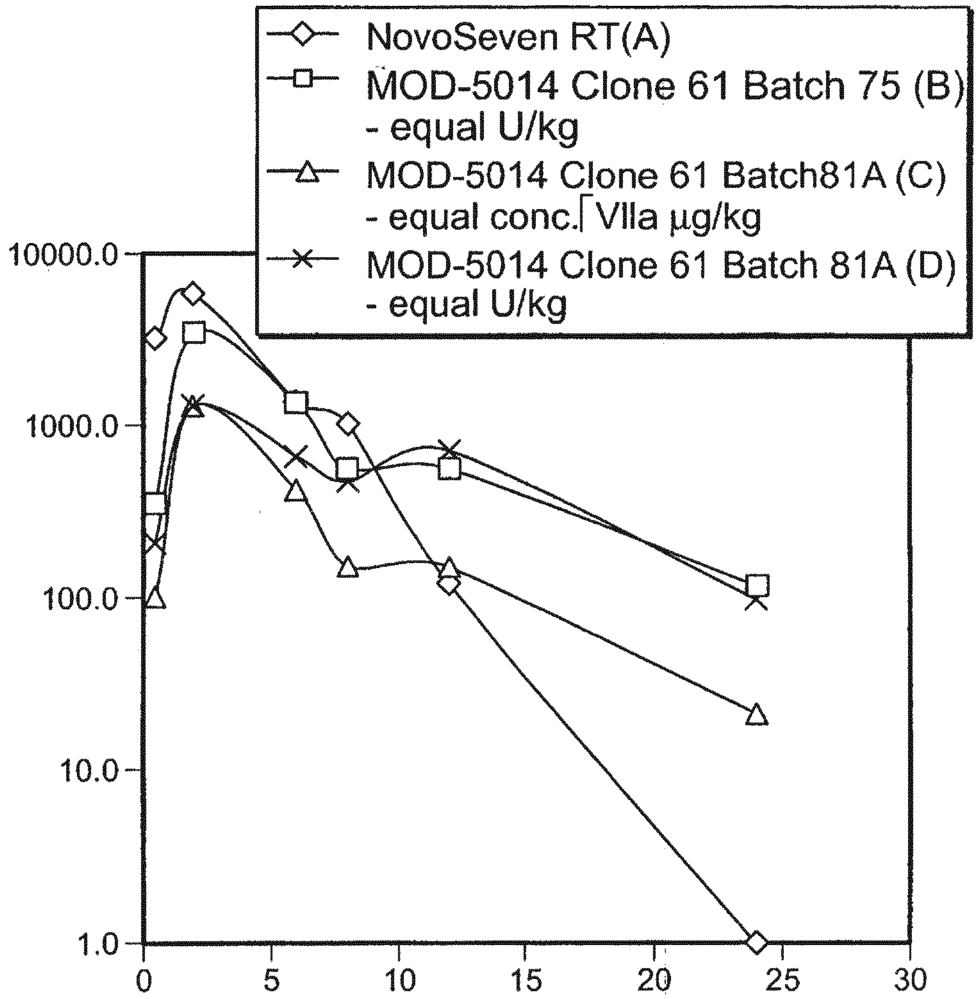
NovoSeven RT(A)
MOD-5014 Clone 61 Batch 75 (B) - equal U/kg
MOD-5014 Clone 61 Batch81A (C) - equal conc.⌈VIIa µg/kg
MOD-5014 Clone 61 Batch 81A (D) - equal U/kg
10000.0
1000.0
100.0
10.0
1.0
0
5
10
15
20
25
30

FIGURE 36

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7553940 B
- US 5712122 A
- US 5932447 A
- US 5464764 A
- US 5487992 A
- EP 0167825 A
- US 4666828 A
- US 4683202 A
- US 4801531 A
- US 5192659 A
- US 5272057 A
- US 3791932 A
- US 3839153 A
- US 3850752 A
- US 3850578 A
- US 3853987 A
- US 3867517 A
- US 3879262 A
- US 3901654 A
- US 3935074 A
- US 3984533 A
- US 3996345 A
- US 4034074 A
- US 4098876 A
- US 4879219 A
- US 5011771 A
- US 5281521 A
- EP 13749077
- US 13372540 B


### Non-patent literature cited in the description


- **BITTER et al.** *Methods in Enzymol.,* 1987, vol. 153, 516-544
- **STUDIER et al.** *Methods in Enzymol.,* 1990, vol. 185, 60-89
- **BRISSON et al.** *Nature,* 1984, vol. 310, 511-514
- **TAKAMATSU et al.** *EMBO J.,* 1987, vol. 6, 307-311
- **CORUZZI et al.** *EMBO J.,* 1984, vol. 3, 1671-1680
- **BROGLI et al.** *Science,* 1984, vol. 224, 838-843
- **GURLEY et al.** *Mol. Cell. Biol.,* 1986, vol. 6, 559-565
- **WEISSBACH ; WEISSBACH.** Methods for Plant Molecular Biology. Academic Press, 1988, 421-463
- Current Protocols in Molecular Biology. John Wiley & Sons, 1992
- **SAMBROOK et al.** Molecular Cloning: A Laboratory Manual. Cold Springs Harbor Laboratory, 1989
- **AUSUBEL et al.** Current Protocols in Molecular Biology. John Wiley and Sons, 1989
- **CHANG et al.** Somatic Gene Therapy. CRC Press, 1995
- **VEGA et al.** Gene Targeting. CRC Press, 1995
- Vectors: A Survey of Molecular Cloning Vectors and Their Uses. Butterworths, 1988
- **GILBOA.** *Biotechniques,* 1986, vol. 4 (6), 504-512
- **BOOTH et al.** *Immunol. Lett.,* 1988, vol. 19, 65-70
- **GARDELLA et al.** *J. Biol. Chem.,* 1990, vol. 265, 15854-15859
- Remington's Pharmaceutical Sciences. Mack Publishing Co,
- **LANGER.** *Science,* 1990, vol. 249, 1527-1533
- **TREAT et al.** Liposomes in the Therapy of Infectious Disease and Cancer. Liss, 1989, 353-365
- **LOPEZ-BERESTEIN.** *LIPOSOMES IN THE THERAPY OF INFECTIOUS DISEASE AND CANCER,* 317-327
- **J. E. DIEDERICHS.** *Pharm./nd.,* 1994, vol. 56, 267-275
- **SEFTON.** *CRC Crit. Ref. Biomed. Eng.,* 1987, vol. 14, 201
- **BUCHWALD et al.** *Surgery,* 1980, vol. 88, 507
- **SAUDEK et al.** *N. Engl. J. Med.,* 1989, vol. 321, 574
- **GOODSON.** *Medical Applications of Controlled Release,* 1984, vol. 2, 115-138
- **FINGL et al.** The Pharmacological Basis of Therapeutics. 1975, 1
- **SPEISER.** *Pharm. Res.,* 1991, vol. 8, 47-54
- **SAMBROOK et al.** Molecular Cloning: A laboratory Manual. 1989

- Current Protocols in Molecular Biology. 1994, vol. I-III
- **PERBAL.** A Practical Guide to Molecular Cloning. John Wiley & Sons, 1988
- **WATSON et al.** Recombinant DNA. Scientific American Books
- Genome Analysis: A Laboratory Manual Series. Cold Spring Harbor Laboratory Press, 1998, vol. 1-4
- Cell Biology: A Laboratory Handbook. 1994, vol. I-III
- **FRESHNEY.** Culture of Animal Cells - A Manual of Basic Technique. Wiley-Liss, 1994
- Current Protocols in Immunology. 1994, vol. I-III
- Basic and Clinical Immunology. Appleton & Lange, 1994
- Selected Methods in Cellular Immunology. W. H. Freeman and Co, 1980
- Oligonucleotide Synthesis. 1984
- Nucleic Acid Hybridization. 1985
- Transcription and Translation. 1984
- Animal Cell Culture. 1986
- Immobilized Cells and Enzymes. IRL Press, 1986
- **PERBAL, B.** A Practical Guide to Molecular Cloning. 1984
- Methods in Enzymology. Academic Press, vol. 1-317
- PCR Protocols: A Guide To Methods And Applications. Academic Press, 1990
- **MARSHAK et al.** Strategies for Protein Purification and Characterization - A Laboratory Course Manual. CSHL Press, 1996

SEQUENCE LISTING

<110> OPKO BIOLOGICS LTD.

<120> LONG-ACTING COAGULATION FACTORS AND USES THEREOF

<130> P-9520-EP16

<150> EP 13749077.7
<151> 2013-02-05

<150> US 13/372,540
<151> 2012-02-14

<160> 45

<170> PatentIn version 3.5

<210> 1
<211> 32
<212> PRT
<213> Homo sapiens

<400> 1

```
Asp Pro Arg Phe Gln Asp Ser Ser Ser Ser Lys Ala Pro Pro Pro Ser
1               5                   10                  15

Leu Pro Ser Pro Ser Arg Leu Pro Gly Pro Ser Asp Thr Pro Ile Leu
            20                  25                  30
```

<210> 2
<211> 28
<212> PRT
<213> Homo sapiens

<400> 2

```
Ser Ser Ser Ser Lys Ala Pro Pro Pro Ser Leu Pro Ser Pro Ser Arg
1               5                   10                  15

Leu Pro Gly Pro Ser Asp Thr Pro Ile Leu Pro Gln
            20                  25
```

<210> 3
<211> 12
<212> PRT
<213> Homo sapiens

<400> 3

```
Ser Ser Ser Ser Lys Ala Pro Pro Pro Ser Leu Pro
1               5                   10
```

<210> 4
<211> 28
<212> PRT
<213> Homo sapiens

<400> 4

```
Ser Ser Ser Ser Lys Ala Pro Pro Pro Ser Leu Pro Ser Pro Ser Arg
1               5                   10                  15

Leu Pro Gly Pro Ser Asp Thr Pro Ile Leu Pro Gln
            20                  25
```

<210> 5
<211> 25
<212> DNA
<213> Artificial Sequence

<220>
<223> PCR primer for Factor VII

<400> 5
ctcgaggaca tggtctccca ggccc 25

<210> 6
<211> 24
<212> DNA
<213> Artificial Sequence

<220>
<223> PCR primer for Factor VII

<400> 6
tctagaatag gtatttttcc acat 24

<210> 7
<211> 22
<212> DNA
<213> Artificial Sequence

<220>
<223> PCR primer for Factor VII

<400> 7
tctagaaaaa agaaatgcca gc 22

<210> 8
<211> 32
<212> DNA
<213> Artificial Sequence

<220>
<223> PCR primer for Factor VII

<400> 8
gcggccgcat cctcagggaa atggggctcg ca 32

<210> 9
<211> 444
<212> PRT
<213> Homo sapiens

<400> 9

```
Met Val Ser Gln Ala Leu Arg Leu Leu Cys Leu Leu Leu Gly Leu Gln
1               5                   10                  15

Gly Cys Leu Ala Ala Val Phe Val Thr Gln Glu Glu Ala His Gly Val
            20                  25                  30

Leu His Arg Arg Arg Arg Ala Asn Ala Phe Leu Glu Glu Leu Arg Pro
        35                  40                  45

Gly Ser Leu Glu Arg Glu Cys Lys Glu Glu Gln Cys Ser Phe Glu Glu
    50                  55                  60

Ala Arg Glu Ile Phe Lys Asp Ala Glu Arg Thr Lys Leu Phe Trp Ile
65                  70                  75                  80

Ser Tyr Ser Asp Gly Asp Gln Cys Ala Ser Ser Pro Cys Gln Asn Gly
                85                  90                  95

Gly Ser Cys Lys Asp Gln Leu Gln Ser Tyr Ile Cys Phe Cys Leu Pro
            100                 105                 110

Ala Phe Glu Gly Arg Asn Cys Glu Thr His Lys Asp Asp Gln Leu Ile
        115                 120                 125

Cys Val Asn Glu Asn Gly Gly Cys Glu Gln Tyr Cys Ser Asp His Thr
    130                 135                 140

Gly Thr Lys Arg Ser Cys Arg Cys His Glu Gly Tyr Ser Leu Leu Ala
145                 150                 155                 160

Asp Gly Val Ser Cys Thr Pro Thr Val Glu Tyr Pro Cys Gly Lys Ile
                165                 170                 175

Pro Ile Leu Glu Lys Arg Asn Ala Ser Lys Pro Gln Gly Arg Ile Val
            180                 185                 190

Gly Gly Lys Val Cys Pro Lys Gly Glu Cys Pro Trp Gln Val Leu Leu
        195                 200                 205

Leu Val Asn Gly Ala Gln Leu Cys Gly Gly Thr Leu Ile Asn Thr Ile
    210                 215                 220

Trp Val Val Ser Ala Ala His Cys Phe Asp Lys Ile Lys Asn Trp Arg
225                 230                 235                 240
```

```
Asn Leu Ile Ala Val Leu Gly Glu His Asp Leu Ser Glu His Asp Gly
                245                 250                 255

Asp Glu Gln Ser Arg Arg Val Ala Gln Val Ile Ile Pro Ser Thr Tyr
            260                 265                 270

Val Pro Gly Thr Thr Asn His Asp Ile Ala Leu Leu Arg Leu His Gln
        275                 280                 285

Pro Val Val Leu Thr Asp His Val Val Pro Leu Cys Leu Pro Glu Arg
    290                 295                 300

Thr Phe Ser Glu Arg Thr Leu Ala Phe Val Arg Phe Ser Leu Val Ser
305                 310                 315                 320

Gly Trp Gly Gln Leu Leu Asp Arg Gly Ala Thr Ala Leu Glu Leu Met
                325                 330                 335

Val Leu Asn Val Pro Arg Leu Met Thr Gln Asp Cys Leu Gln Gln Ser
            340                 345                 350

Arg Lys Val Gly Asp Ser Pro Asn Ile Thr Glu Tyr Met Phe Cys Ala
        355                 360                 365

Gly Tyr Ser Asp Gly Ser Lys Asp Ser Cys Lys Gly Asp Ser Gly Gly
    370                 375                 380

Pro His Ala Thr His Tyr Arg Gly Thr Trp Tyr Leu Thr Gly Ile Val
385                 390                 395                 400

Ser Trp Gly Gln Gly Cys Ala Thr Val Gly His Phe Gly Val Tyr Thr
                405                 410                 415

Arg Val Ser Gln Tyr Ile Glu Trp Leu Gln Lys Leu Met Arg Ser Glu
            420                 425                 430

Pro Arg Pro Gly Val Leu Leu Arg Ala Pro Phe Pro
        435                 440
```

<210> 10
<211> 448
<212> PRT
<213> Homo sapiens

<400> 10

```
Met Val Ser Gln Ala Leu Arg Leu Leu Cys Leu Leu Leu Gly Leu Gln
1               5                   10                  15
```

```
Gly Cys Leu Ala Ala Val Phe Val Thr Gln Glu Glu Ala His Gly Val
            20                  25                  30

Leu His Arg Arg Arg Arg Ala Asn Ala Phe Leu Glu Glu Leu Arg Pro
        35                  40                  45

Gly Ser Leu Glu Arg Glu Cys Lys Glu Glu Gln Cys Ser Phe Glu Glu
    50                  55                  60

Ala Arg Glu Ile Phe Lys Asp Ala Glu Arg Thr Lys Leu Phe Trp Ile
65                  70                  75                  80

Ser Tyr Ser Asp Gly Asp Gln Cys Ala Ser Ser Pro Cys Gln Asn Gly
                85                  90                  95

Gly Ser Cys Lys Asp Gln Leu Gln Ser Tyr Ile Cys Phe Cys Leu Pro
            100                 105                 110

Ala Phe Glu Gly Arg Asn Cys Glu Thr His Lys Asp Asp Gln Leu Ile
        115                 120                 125

Cys Val Asn Glu Asn Gly Gly Cys Glu Gln Tyr Cys Ser Asp His Thr
    130                 135                 140

Gly Thr Lys Arg Ser Cys Arg Cys His Glu Gly Tyr Ser Leu Leu Ala
145                 150                 155                 160

Asp Gly Val Ser Cys Thr Pro Thr Val Glu Tyr Pro Cys Gly Lys Ile
                165                 170                 175

Pro Ile Leu Glu Lys Arg Asn Ala Ser Lys Pro Gln Gly Arg Ile Val
            180                 185                 190

Gly Gly Lys Val Cys Pro Lys Gly Glu Cys Pro Trp Gln Val Leu Leu
        195                 200                 205

Leu Val Asn Gly Ala Gln Leu Cys Gly Gly Thr Leu Ile Asn Thr Ile
    210                 215                 220

Trp Val Val Ser Ala Ala His Cys Phe Asp Lys Ile Lys Asn Trp Arg
225                 230                 235                 240

Asn Leu Ile Ala Val Leu Gly Glu His Asp Leu Ser Glu His Asp Gly
                245                 250                 255

Asp Glu Gln Ser Arg Arg Val Ala Gln Val Ile Ile Pro Ser Thr Tyr
            260                 265                 270
```

```
Val Pro Gly Thr Thr Asn His Asp Ile Ala Leu Leu Arg Leu His Gln
        275                 280                 285

Pro Val Val Leu Thr Asp His Val Val Pro Leu Cys Leu Pro Glu Arg
    290                 295                 300

Thr Phe Ser Glu Arg Thr Leu Ala Phe Val Arg Phe Ser Leu Val Ser
305                 310                 315                 320

Gly Trp Gly Gln Leu Leu Asp Arg Gly Ala Thr Ala Leu Glu Leu Met
                325                 330                 335

Val Leu Asn Val Pro Arg Leu Met Thr Gln Asp Cys Leu Gln Gln Ser
            340                 345                 350

Arg Lys Val Gly Asp Ser Pro Asn Ile Thr Glu Tyr Met Phe Cys Ala
        355                 360                 365

Gly Tyr Ser Asp Gly Ser Lys Asp Ser Cys Lys Gly Asp Ser Gly Gly
    370                 375                 380

Pro His Ala Thr His Tyr Arg Gly Thr Trp Tyr Leu Thr Gly Ile Val
385                 390                 395                 400

Ser Trp Gly Gln Gly Cys Ala Thr Val Gly His Phe Gly Val Tyr Thr
                405                 410                 415

Arg Val Ser Gln Tyr Ile Glu Trp Leu Gln Lys Leu Met Arg Ser Glu
            420                 425                 430

Pro Arg Pro Gly Val Leu Leu Arg Ala Pro Phe Pro Gly Cys Gly Arg
        435                 440                 445
```

<210> 11
<211> 1356
<212> DNA
<213> Homo sapiens

<400> 11

```
ctcgaggaca tggtctccca ggccctcagg ctcctctgcc ttctgcttgg gcttcagggc      60
tgcctggctg cagtcttcgt aacccaggag gaagcccacg gcgtcctgca ccggcgccgg     120
cgcgccaacg cgttcctgga ggagctgcgg ccgggctccc tggagaggga gtgcaaggag     180
gagcagtgct ccttcgagga ggcccgggag atcttcaagg acgcggagag gacgaagctg     240
ttctggattt cttacagtga tggggaccag tgtgcctcaa gtccatgcca gaatgggggc     300
tcctgcaagg accagctcca gtcctatatc tgcttctgcc tccctgcctt cgagggccgg     360

aactgtgaga cgcacaagga tgaccagctg atctgtgtga acgagaacgg cggctgtgag     420
cagtactgca gtgaccacac gggcaccaag cgctcctgtc ggtgccacga ggggtactct     480
ctgctggcag acggggtgtc ctgcacaccc acagttgaat atccatgtgg aaaaatacct     540
attctagaaa aaagaaatgc cagcaaaccc caaggccgaa ttgtgggggg caaggtgtgc     600
cccaaagggg agtgtccatg gcaggtcctg ttgttggtga atggagctca gttgtgtggg     660
gggaccctga tcaacaccat ctgggtggtc tccgcggccc actgtttcga caaaatcaag     720
aactggagga acctgatcgc ggtgctgggc gagcacgacc tcagcgagca cgacggggat     780
gagcagagcc ggcgggtggc gcaggtcatc atccccagca cgtacgtccc gggcaccacc     840
aaccacgaca tcgcgctgct ccgcctgcac cagcccgtgg tcctcactga ccatgtggtg     900
cccctctgcc tgcccgaacg gacgttctct gagaggacgc tggccttcgt gcgcttctca     960
ttggtcagcg gctggggcca gctgctggac cgtggcgcca cggccctgga gctcatggtc    1020
ctcaacgtgc cccggctgat gacccaggac tgcctgcagc agtcacggaa ggtgggagac    1080
tccccaaata tcacggagta catgttctgt gccggctact cggatggcag caaggactcc    1140
tgcaaggggg acagtggagg cccacatgcc acccactacc ggggcacgtg gtacctgacg    1200
ggcatcgtca gctggggcca gggctgcgca accgtgggcc actttggggt gtacaccagg    1260
gtctcccagt acatcgagtg gctgcaaaag ctcatgcgct cagagccacg cccaggagtc    1320
ctcctgcgag ccccatttcc ctgaggatgc ggccgc                              1356
```

<210> 12
<211> 1442
<212> DNA
<213> Artificial Sequence

<220>
<223> CTP-modified Factor VII

<400> 12

```
ctcgaggaca tggtctccca ggccctcagg ctcctctgcc ttctgcttgg gcttcagggc        60
tgcctggctg cagtcttcgt aacccaggag gaagcccacg gcgtcctgca ccggcgccgg       120
cgcgccaacg cgttcctgga ggagctgcgg ccgggctccc tggagaggga gtgcaaggag       180
gagcagtgct ccttcgagga ggcccgggag atcttcaagg acgcggagag gacgaagctg       240
ttctggattt cttacagtga tggggaccag tgtgcctcaa gtccatgcca gaatgggggc       300
tcctgcaagg accagctcca gtcctatatc tgcttctgcc tccctgcctt cgagggccgg       360
aactgtgaga cgcacaagga tgaccagctg atctgtgtga acgagaacgg cggctgtgag       420
cagtactgca gtgaccacac gggcaccaag cgctcctgtc ggtgccacga ggggtactct       480
ctgctggcag acggggtgtc ctgcacaccc acagttgaat atccatgtgg aaaaatacct       540
attctagaaa aaagaaatgc cagcaaaccc caaggccgaa ttgtgggggg caaggtgtgc       600
cccaaagggg agtgtccatg gcaggtcctg ttgttggtga atggagctca gttgtgtggg       660
gggaccctga tcaacaccat ctgggtggtc tccgcggccc actgtttcga caaaatcaag       720
aactggagga acctgatcgc ggtgctgggc gagcacgacc tcagcgagca cgacggggat       780
gagcagagcc ggcgggtggc gcaggtcatc atccccagca cgtacgtccc gggcaccacc       840
aaccacgaca tcgcgctgct ccgcctgcac cagcccgtgg tcctcactga ccatgtggtg       900
cccctctgcc tgcccgaacg gacgttctct gagaggacgc tggccttcgt gcgcttctca       960
ttggtcagcg gctggggcca gctgctggac cgtggcgcca cggccctgga gctcatggtc      1020
ctcaacgtgc cccggctgat gacccaggac tgcctgcagc agtcacggaa ggtgggagac      1080
tccccaaata tcacggagta catgttctgt gccggctact cggatggcag caaggactcc      1140
tgcaaggggg acagtggagg cccacatgcc acccactacc ggggcacgtg gtacctgacc      1200
ggcatcgtga gctggggcca gggctgcgcc accgtgggcc acttcggcgt gtacaccagg      1260
gtgtcccagt acatcgagtg gctgcagaaa ctgatgagaa gcgagcccag acccggcgtg      1320
ctgctgagag ccccccttccc cagcagcagc tccaaggccc ctccccctag cctgcccagc      1380
cctagcagac tgcctgggcc cagcgacacc cccatcctgc cccagtgagg atccgcggcc      1440
gc                                                                     1442
```

<210> 13
<211> 472
<212> PRT
<213> Artificial Sequence

<220>
<223> CTP-modified Factor VII

<400> 13

```
Met Val Ser Gln Ala Leu Arg Leu Leu Cys Leu Leu Leu Gly Leu Gln
1               5                   10                  15

Gly Cys Leu Ala Ala Val Phe Val Thr Gln Glu Glu Ala His Gly Val
            20                  25                  30

Leu His Arg Arg Arg Arg Ala Asn Ala Phe Leu Glu Glu Leu Arg Pro
        35                  40                  45

Gly Ser Leu Glu Arg Glu Cys Lys Glu Glu Gln Cys Ser Phe Glu Glu
    50                  55                  60

Ala Arg Glu Ile Phe Lys Asp Ala Glu Arg Thr Lys Leu Phe Trp Ile
65                  70                  75                  80

Ser Tyr Ser Asp Gly Asp Gln Cys Ala Ser Ser Pro Cys Gln Asn Gly
```

85 90 95

Gly Ser Cys Lys Asp Gln Leu Gln Ser Tyr Ile Cys Phe Cys Leu Pro
100 105 110

Ala Phe Glu Gly Arg Asn Cys Glu Thr His Lys Asp Asp Gln Leu Ile
115 120 125

Cys Val Asn Glu Asn Gly Gly Cys Glu Gln Tyr Cys Ser Asp His Thr
130 135 140

Gly Thr Lys Arg Ser Cys Arg Cys His Glu Gly Tyr Ser Leu Leu Ala
145 150 155 160

Asp Gly Val Ser Cys Thr Pro Thr Val Glu Tyr Pro Cys Gly Lys Ile
165 170 175

Pro Ile Leu Glu Lys Arg Asn Ala Ser Lys Pro Gln Gly Arg Ile Val
180 185 190

Gly Gly Lys Val Cys Pro Lys Gly Glu Cys Pro Trp Gln Val Leu Leu
195 200 205

Leu Val Asn Gly Ala Gln Leu Cys Gly Gly Thr Leu Ile Asn Thr Ile
210 215 220

Trp Val Val Ser Ala Ala His Cys Phe Asp Lys Ile Lys Asn Trp Arg
225 230 235 240

Asn Leu Ile Ala Val Leu Gly Glu His Asp Leu Ser Glu His Asp Gly
245 250 255

Asp Glu Gln Ser Arg Arg Val Ala Gln Val Ile Ile Pro Ser Thr Tyr
260 265 270

Val Pro Gly Thr Thr Asn His Asp Ile Ala Leu Leu Arg Leu His Gln
275 280 285

Pro Val Val Leu Thr Asp His Val Val Pro Leu Cys Leu Pro Glu Arg
290 295 300

Thr Phe Ser Glu Arg Thr Leu Ala Phe Val Arg Phe Ser Leu Val Ser
305 310 315 320

Gly Trp Gly Gln Leu Leu Asp Arg Gly Ala Thr Ala Leu Glu Leu Met
325 330 335

```
Val Leu Asn Val Pro Arg Leu Met Thr Gln Asp Cys Leu Gln Gln Ser
            340                 345                 350

Arg Lys Val Gly Asp Ser Pro Asn Ile Thr Glu Tyr Met Phe Cys Ala
        355                 360                 365

Gly Tyr Ser Asp Gly Ser Lys Asp Ser Cys Lys Gly Asp Ser Gly Gly
    370                 375                 380

Pro His Ala Thr His Tyr Arg Gly Thr Trp Tyr Leu Thr Gly Ile Val
385                 390                 395                 400

Ser Trp Gly Gln Gly Cys Ala Thr Val Gly His Phe Gly Val Tyr Thr
                405                 410                 415

Arg Val Ser Gln Tyr Ile Glu Trp Leu Gln Lys Leu Met Arg Ser Glu
            420                 425                 430

Pro Arg Pro Gly Val Leu Leu Arg Ala Pro Phe Pro Ser Ser Ser Ser
        435                 440                 445

Lys Ala Pro Pro Pro Ser Leu Pro Ser Pro Ser Arg Leu Pro Gly Pro
    450                 455                 460

Ser Asp Thr Pro Ile Leu Pro Gln
465                 470
```

<210> 14
<211> 1535
<212> DNA
<213> Artificial Sequence

<220>
<223> CTP-modified Factor VII

<400> 14

```
ctcgaggaca tggtctccca ggccctcagg ctcctctgcc ttctgcttgg gcttcagggc      60
tgcctggctg cagtcttcgt aacccaggag gaagcccacg gcgtcctgca ccggcgccgg     120
cgcgccaacg cgttcctgga ggagctgcgg ccgggctccc tggagaggga gtgcaaggag     180
gagcagtgct ccttcgagga ggcccgggag atcttcaagg acgcggagag gacgaagctg     240
ttctggattt cttacagtga tggggaccag tgtgcctcaa gtccatgcca gaatgggggc     300
tcctgcaagg accagctcca gtcctatatc tgcttctgcc tccctgcctt cgagggccgg     360
aactgtgaga cgcacaagga tgaccagctg atctgtgtga acgagaacgg cggctgtgag     420
cagtactgca gtgaccacac gggcaccaag cgctcctgtc ggtgccacga ggggtactct     480
ctgctggcag acggggtgtc ctgcacaccc acagttgaat atccatgtgg aaaaatacct     540

attctagaaa aaagaaatgc cagcaaaccc caaggccgaa ttgtgggggg caaggtgtgc     600
cccaaagggg agtgtccatg gcaggtcctg ttgttggtga atggagctca gttgtgtggg     660
gggaccctga tcaacaccat ctgggtggtc tccgcggccc actgtttcga caaaatcaag     720
aactggagga acctgatcgc ggtgctgggc gagcacgacc tcagcgagca cgacggggat     780
gagcagagcc ggcgggtggc gcaggtcatc atccccagca cgtacgtccc gggcaccacc     840
aaccacgaca tcgcgctgct ccgcctgcac cagcccgtgg tcctcactga ccatgtggtg     900
cccctctgcc tgcccgaacg gacgttctct gagaggacgc tggccttcgt gcgcttctca     960
ttggtcagcg gctggggcca gctgctggac cgtggcgcca cggccctgga gctcatggtc    1020
ctcaacgtgc cccggctgat gacccaggac tgcctgcagc agtcacggaa ggtgggagac    1080
tccccaaata tcacggagta catgttctgt gccggctact cggatggcag caaggactcc    1140
tgcaaggggg acagtggagg cccacatgcc acccactacc ggggcacgtg gtacctgacc    1200
ggcatcgtga gctggggcca gggctgcgcc accgtgggcc acttcggcgt gtacaccagg    1260
gtgtcccagt acatcgagtg gctgcagaaa ctgatgagaa gcgagcccag acccggcgtg    1320
ctgctgagag ccccccttccc cagcagcagc tccaaggccc ctccccctag cctgcccagc    1380
cctagcagac tgcctgggcc ctccgacaca ccaatcctgc cacagagcag ctcctctaag    1440
gcccctcctc catccctgcc atccccctcc cggctgccag gcccctctga cacccctatc    1500
ctgcctcagt gatgaaggtc tggatccgcg gccgc                                1535
```

<210> 15
<211> 500
<212> PRT
<213> Artificial Sequence

<220>
<223> CTP-modified Factor VII

<400> 15

```
Met Val Ser Gln Ala Leu Arg Leu Leu Cys Leu Leu Leu Gly Leu Gln
1               5                   10                  15

Gly Cys Leu Ala Ala Val Phe Val Thr Gln Glu Glu Ala His Gly Val
            20                  25                  30

Leu His Arg Arg Arg Arg Ala Asn Ala Phe Leu Glu Glu Leu Arg Pro
        35                  40                  45

Gly Ser Leu Glu Arg Glu Cys Lys Glu Glu Gln Cys Ser Phe Glu Glu
    50                  55                  60

Ala Arg Glu Ile Phe Lys Asp Ala Glu Arg Thr Lys Leu Phe Trp Ile
65                  70                  75                  80
```

Ser Tyr Ser Asp Gly Asp Gln Cys Ala Ser Ser Pro Cys Gln Asn Gly
85 90 95

Gly Ser Cys Lys Asp Gln Leu Gln Ser Tyr Ile Cys Phe Cys Leu Pro
100 105 110

Ala Phe Glu Gly Arg Asn Cys Glu Thr His Lys Asp Asp Gln Leu Ile
115 120 125

Cys Val Asn Glu Asn Gly Gly Cys Glu Gln Tyr Cys Ser Asp His Thr
130 135 140

Gly Thr Lys Arg Ser Cys Arg Cys His Glu Gly Tyr Ser Leu Leu Ala
145 150 155 160

Asp Gly Val Ser Cys Thr Pro Thr Val Glu Tyr Pro Cys Gly Lys Ile
165 170 175

Pro Ile Leu Glu Lys Arg Asn Ala Ser Lys Pro Gln Gly Arg Ile Val
180 185 190

Gly Gly Lys Val Cys Pro Lys Gly Glu Cys Pro Trp Gln Val Leu Leu
195 200 205

Leu Val Asn Gly Ala Gln Leu Cys Gly Gly Thr Leu Ile Asn Thr Ile
210 215 220

Trp Val Val Ser Ala Ala His Cys Phe Asp Lys Ile Lys Asn Trp Arg
225 230 235 240

Asn Leu Ile Ala Val Leu Gly Glu His Asp Leu Ser Glu His Asp Gly
245 250 255

Asp Glu Gln Ser Arg Arg Val Ala Gln Val Ile Ile Pro Ser Thr Tyr
260 265 270

Val Pro Gly Thr Thr Asn His Asp Ile Ala Leu Leu Arg Leu His Gln
275 280 285

Pro Val Val Leu Thr Asp His Val Val Pro Leu Cys Leu Pro Glu Arg
290 295 300

Thr Phe Ser Glu Arg Thr Leu Ala Phe Val Arg Phe Ser Leu Val Ser
305 310 315 320

Gly Trp Gly Gln Leu Leu Asp Arg Gly Ala Thr Ala Leu Glu Leu Met

325 330 335

Val Leu Asn Val Pro Arg Leu Met Thr Gln Asp Cys Leu Gln Gln Ser
340 345 350

Arg Lys Val Gly Asp Ser Pro Asn Ile Thr Glu Tyr Met Phe Cys Ala
355 360 365

Gly Tyr Ser Asp Gly Ser Lys Asp Ser Cys Lys Gly Asp Ser Gly Gly
370 375 380

Pro His Ala Thr His Tyr Arg Gly Thr Trp Tyr Leu Thr Gly Ile Val
385 390 395 400

Ser Trp Gly Gln Gly Cys Ala Thr Val Gly His Phe Gly Val Tyr Thr
405 410 415

Arg Val Ser Gln Tyr Ile Glu Trp Leu Gln Lys Leu Met Arg Ser Glu
420 425 430

Pro Arg Pro Gly Val Leu Leu Arg Ala Pro Phe Pro Ser Ser Ser Ser
435 440 445

Lys Ala Pro Pro Pro Ser Leu Pro Ser Pro Ser Arg Leu Pro Gly Pro
450 455 460

Ser Asp Thr Pro Ile Leu Pro Gln Ser Ser Ser Ser Lys Ala Pro Pro
465 470 475 480

Pro Ser Leu Pro Ser Pro Ser Arg Leu Pro Gly Pro Ser Asp Thr Pro
485 490 495

Ile Leu Pro Gln
500

<210> 16
<211> 1404
<212> DNA
<213> Homo sapiens

<400> 16

```
gcgatcgcca tgcagcgcgt gaacatgatc atggcagaat caccaggcct catcaccatt        60
gccttttagg atatctactc agtgctgaat gtacagtttt tcttgatcat gaaaacgcca       120
acaaaattct gaatcggcca aagaggtata attcaggtaa attggaagag tttgttcaag       180
ggaaccttga gagagaatgt atggaagaaa agtgtagttt tgaagaagca cgagaagttt       240
ttgaaaacac tgaaagaaca actgaatttt ggaagcagta tgttgatgga gatcagtgtg       300
agtccaatcc atgtttaaat ggcggcagtt gcaaggatga cattaattcc tatgaatgtt       360
ggtgtccctt tggatttgaa ggaaagaact gtgaattaga tgtaacatgt aacattaaga       420
atggcagatg cgagcagttt tgtaaaaata gtgctgataa caaggtggtt tgctcctgta       480
ctgagggata tcgacttgca gaaaaccaga agtcctgtga accagcagtg ccatttccat       540
gtggaagagt ttctgtttca caaacttcta agctcacccg tgctgagact gtttttcctg       600
atgtggacta tgtaaattct actgaagctg aaaccatttt ggataacatc actcaaagca       660
cccaatcatt taatgacttc actcgagttg ttggtggaga agatgccaaa ccaggtcaat       720
tcccttggca ggttgttttg aatggtaaag ttgatgcatt ctgtggaggc tctatcgtta       780
atgaaaaatg gattgtaact gctgcccact gtgttgaaac tggtgttaaa attacagttg       840
tcgcaggtga acataatatt gaggagacag aacatacaga gcaaaagcga aatgtgattc       900
gaattattcc tcaccacaac tacaatgcag ctattaataa gtacaaccat gacattgccc       960
ttctggaact ggacgaaccc ttagtgctaa acagctacgt tacacctatt tgcattgctg      1020
acaaggaata cacgaacatc ttcctcaaat ttggatctgg ctatgtaagt ggctggggaa      1080
gagtcttcca caaagggaga tcagctttag ttctccagta ccttagagtt ccacttgttg      1140
accgagccac atgtcttcga tctacaaagt tcaccatcta taacaacatg ttctgtgctg      1200
gcttccatga aggaggtaga gattcatgtc aaggagatag tgggggaccc catgttactg      1260
aagtggaagg gaccagtttc ttaactggaa ttattagctg gggtgaagag tgtgcaatga      1320
aaggcaaata tggaatatat accaaggtat cccggtatgt caactggatt aaggaaaaaa      1380
caaagctcac ttgaacgcgg ccgc                                             1404
```

<210> 17
<211> 461
<212> PRT
<213> Homo sapiens

<400> 17

```
Met Gln Arg Val Asn Met Ile Met Ala Glu Ser Pro Gly Leu Ile Thr
1               5                   10                  15

Ile Cys Leu Leu Gly Tyr Leu Leu Ser Ala Glu Cys Thr Val Phe Leu
            20                  25                  30

Asp His Glu Asn Ala Asn Lys Ile Leu Asn Arg Pro Lys Arg Tyr Asn
        35                  40                  45

Ser Gly Lys Leu Glu Glu Phe Val Gln Gly Asn Leu Glu Arg Glu Cys
    50                  55                  60

Met Glu Glu Lys Cys Ser Phe Glu Glu Ala Arg Glu Val Phe Glu Asn
```

```
65                  70                  75                  80

Thr Glu Arg Thr Thr Glu Phe Trp Lys Gln Tyr Val Asp Gly Asp Gln
                85                  90                  95

Cys Glu Ser Asn Pro Cys Leu Asn Gly Gly Ser Cys Lys Asp Asp Ile
            100                 105                 110

Asn Ser Tyr Glu Cys Trp Cys Pro Phe Gly Phe Glu Gly Lys Asn Cys
        115                 120                 125

Glu Leu Asp Val Thr Cys Asn Ile Lys Asn Gly Arg Cys Glu Gln Phe
    130                 135                 140

Cys Lys Asn Ser Ala Asp Asn Lys Val Val Cys Ser Cys Thr Glu Gly
145                 150                 155                 160

Tyr Arg Leu Ala Glu Asn Gln Lys Ser Cys Glu Pro Ala Val Pro Phe
                165                 170                 175

Pro Cys Gly Arg Val Ser Val Ser Gln Thr Ser Lys Leu Thr Arg Ala
            180                 185                 190

Glu Thr Val Phe Pro Asp Val Asp Tyr Val Asn Ser Thr Glu Ala Glu
        195                 200                 205

Thr Ile Leu Asp Asn Ile Thr Gln Ser Thr Gln Ser Phe Asn Asp Phe
    210                 215                 220

Thr Arg Val Val Gly Gly Glu Asp Ala Lys Pro Gly Gln Phe Pro Trp
225                 230                 235                 240

Gln Val Val Leu Asn Gly Lys Val Asp Ala Phe Cys Gly Gly Ser Ile
                245                 250                 255

Val Asn Glu Lys Trp Ile Val Thr Ala Ala His Cys Val Glu Thr Gly
            260                 265                 270

Val Lys Ile Thr Val Val Ala Gly Glu His Asn Ile Glu Glu Thr Glu
        275                 280                 285

His Thr Glu Gln Lys Arg Asn Val Ile Arg Ile Ile Pro His His Asn
    290                 295                 300

Tyr Asn Ala Ala Ile Asn Lys Tyr Asn His Asp Ile Ala Leu Leu Glu
305                 310                 315                 320
```

Leu Asp Glu Pro Leu Val Leu Asn Ser Tyr Val Thr Pro Ile Cys Ile
325 330 335

Ala Asp Lys Glu Tyr Thr Asn Ile Phe Leu Lys Phe Gly Ser Gly Tyr
340 345 350

Val Ser Gly Trp Gly Arg Val Phe His Lys Gly Arg Ser Ala Leu Val
355 360 365

Leu Gln Tyr Leu Arg Val Pro Leu Val Asp Arg Ala Thr Cys Leu Arg
370 375 380

Ser Thr Lys Phe Thr Ile Tyr Asn Asn Met Phe Cys Ala Gly Phe His
385 390 395 400

Glu Gly Gly Arg Asp Ser Cys Gln Gly Asp Ser Gly Gly Pro His Val
405 410 415

Thr Glu Val Glu Gly Thr Ser Phe Leu Thr Gly Ile Ile Ser Trp Gly
420 425 430

Glu Glu Cys Ala Met Lys Gly Lys Tyr Gly Ile Tyr Thr Lys Val Ser
435 440 445

Arg Tyr Val Asn Trp Ile Lys Glu Lys Thr Lys Leu Thr
450 455 460

<210> 18
<211> 1502
<212> DNA
<213> Artificial Sequence

<220>
<223> CTP-modified Factor IX

<400> 18

gcgatcgcca tgcagcgcgt gaacatgatc atggcagaat caccaggcct catcaccatc 60

tgccttttag gatatctact cagtgctgaa tgtacagttt ttcttgatca tgaaaacgcc 120

aacaaaattc tgaatcggcc aaagaggtat aattcaggta aattggaaga gtttgttcaa 180

gggaaccttg agagagaatg tatggaagaa aagtgtagtt ttgaagaagc acgagaagtt 240

tttgaaaaca ctgaaagaac aactgaattt tggaagcagt atgttgatgg agatcagtgt 300

gagtccaatc catgtttaaa tggcggcagt tgcaaggatg acattaattc ctatgaatgt 360

tggtgtccct ttggatttga aggaaagaac tgtgaattag atgtaacatg taacattaag 420

aatggcagat gcgagcagtt ttgtaaaaat agtgctgata acaaggtggt ttgctcctgt 480

actgagggat atcgacttgc agaaaaccag aagtcctgtg aaccagcagt gccatttcca 540

tgtggaagag tttctgtttc acaaacttct aagctcaccc gtgctgagac tgtttttcct 600

gatgtggact atgtaaattc tactgaagct gaaaccattt tggataacat cactcaaagc 660

acccaatcat ttaatgactt cactcgagtt gttggtggag aagatgccaa accaggtcaa 720

ttcccttggc aggttgtttt gaatggtaaa gttgatgcat tctgtggagg ctctatcgtt 780

aatgaaaaat ggattgtaac tgctgcccac tgtgttgaaa ctggtgttaa aattacagtt 840

gtcgcaggtg aacataatat tgaggagaca gaacatacag agcaaaagcg aaatgtgatt 900

cgaattattc ctcaccacaa ctacaatgca gctattaata agtacaacca tgacattgcc 960

cttctggaac tggacgaacc cttagtgcta aacagctacg ttacacctat ttgcattgct 1020

gacaaggaat acacgaacat cttcctcaaa tttggatctg gctatgtaag tggctgggga 1080

agagtcttcc acaaagggag atcagcttta gttcttcagt accttagagt tccacttgtt 1140

gaccgagcca catgtcttcg atctacaaag ttcaccatct ataacaacat gttctgtgct 1200

ggcttccatg aaggaggtag agattcatgt caaggagata gtgggggacc ccatgttact 1260

gaagtggaag ggaccagttt cttaactgga attattagct ggggtgaaga gtgtgcaatg 1320

aaaggcaaat atggaatata taccaaggta tcccggtatg tcaactggat taaggaaaaa 1380

acaaagctca ctagctccag cagcaaggcc cctcccccga gcctgccctc cccaagcagg 1440

ctgcctgggc cctccgacac accaatcctg ccacagtgat gaaggtctgg atccgcggcc 1500

gc 1502

<210> 19
<211> 489
<212> PRT
<213> Artificial Sequence

<220>
<223> CTP-modified Factor IX

<400> 19

```
Met Gln Arg Val Asn Met Ile Met Ala Glu Ser Pro Gly Leu Ile Thr
1               5                   10                  15

Ile Cys Leu Leu Gly Tyr Leu Leu Ser Ala Glu Cys Thr Val Phe Leu
            20                  25                  30

Asp His Glu Asn Ala Asn Lys Ile Leu Asn Arg Pro Lys Arg Tyr Asn
        35                  40                  45

Ser Gly Lys Leu Glu Glu Phe Val Gln Gly Asn Leu Glu Arg Glu Cys
    50                  55                  60

Met Glu Glu Lys Cys Ser Phe Glu Glu Ala Arg Glu Val Phe Glu Asn
65                  70                  75                  80
```

```
Thr Glu Arg Thr Thr Glu Phe Trp Lys Gln Tyr Val Asp Gly Asp Gln
                85                  90                  95

Cys Glu Ser Asn Pro Cys Leu Asn Gly Gly Ser Cys Lys Asp Asp Ile
            100                 105                 110

Asn Ser Tyr Glu Cys Trp Cys Pro Phe Gly Phe Glu Gly Lys Asn Cys
        115                 120                 125

Glu Leu Asp Val Thr Cys Asn Ile Lys Asn Gly Arg Cys Glu Gln Phe
    130                 135                 140

Cys Lys Asn Ser Ala Asp Asn Lys Val Val Cys Ser Cys Thr Glu Gly
145                 150                 155                 160

Tyr Arg Leu Ala Glu Asn Gln Lys Ser Cys Glu Pro Ala Val Pro Phe
                165                 170                 175

Pro Cys Gly Arg Val Ser Val Ser Gln Thr Ser Lys Leu Thr Arg Ala
            180                 185                 190

Glu Thr Val Phe Pro Asp Val Asp Tyr Val Asn Ser Thr Glu Ala Glu
        195                 200                 205

Thr Ile Leu Asp Asn Ile Thr Gln Ser Thr Gln Ser Phe Asn Asp Phe
    210                 215                 220

Thr Arg Val Val Gly Gly Glu Asp Ala Lys Pro Gly Gln Phe Pro Trp
225                 230                 235                 240

Gln Val Val Leu Asn Gly Lys Val Asp Ala Phe Cys Gly Gly Ser Ile
                245                 250                 255

Val Asn Glu Lys Trp Ile Val Thr Ala Ala His Cys Val Glu Thr Gly
            260                 265                 270

Val Lys Ile Thr Val Val Ala Gly Glu His Asn Ile Glu Glu Thr Glu
        275                 280                 285

His Thr Glu Gln Lys Arg Asn Val Ile Arg Ile Ile Pro His His Asn
    290                 295                 300

Tyr Asn Ala Ala Ile Asn Lys Tyr Asn His Asp Ile Ala Leu Leu Glu
305                 310                 315                 320

Leu Asp Glu Pro Leu Val Leu Asn Ser Tyr Val Thr Pro Ile Cys Ile
```

```
                325                 330                 335

Ala Asp Lys Glu Tyr Thr Asn Ile Phe Leu Lys Phe Gly Ser Gly Tyr
            340                 345                 350

Val Ser Gly Trp Gly Arg Val Phe His Lys Gly Arg Ser Ala Leu Val
        355                 360                 365

Leu Gln Tyr Leu Arg Val Pro Leu Val Asp Arg Ala Thr Cys Leu Arg
    370                 375                 380

Ser Thr Lys Phe Thr Ile Tyr Asn Asn Met Phe Cys Ala Gly Phe His
385                 390                 395                 400

Glu Gly Gly Arg Asp Ser Cys Gln Gly Asp Ser Gly Gly Pro His Val
                405                 410                 415

Thr Glu Val Glu Gly Thr Ser Phe Leu Thr Gly Ile Ile Ser Trp Gly
            420                 425                 430

Glu Glu Cys Ala Met Lys Gly Lys Tyr Gly Ile Tyr Thr Lys Val Ser
        435                 440                 445

Arg Tyr Val Asn Trp Ile Lys Glu Lys Thr Lys Leu Thr Ser Ser Ser
    450                 455                 460

Ser Lys Ala Pro Pro Pro Ser Leu Pro Ser Pro Ser Arg Leu Pro Gly
465                 470                 475                 480

Pro Ser Asp Thr Pro Ile Leu Pro Gln
                485
```

<210> 20
<211> 1585
<212> DNA
<213> Artificial Sequence

<220>
<223> CTP-modified Factor IX

<400> 20

gcgatcgcca tgcagcgcgt gaacatgatc atggcagaat caccaggcct catcaccatc 60

tgccttttag gatatctact cagtgctgaa tgtacagttt ttcttgatca tgaaaacgcc 120

aacaaaattc tgaatcggcc aaagaggtat aattcaggta aattggaaga gtttgttcaa 180

gggaaccttg agagagaatg tatggaagaa aagtgtagtt ttgaagaagc acgagaagtt 240

tttgaaaaca ctgaaagaac aactgaattt tggaagcagt atgttgatgg agatcagtgt 300

gagtccaatc catgtttaaa tggcggcagt tgcaaggatg acattaattc ctatgaatgt 360

tggtgtccct ttggatttga aggaaagaac tgtgaattag atgtaacatg taacattaag 420

aatggcagat gcgagcagtt ttgtaaaaat agtgctgata acaaggtggt ttgctcctgt 480

actgagggat atcgacttgc agaaaaccag aagtcctgtg aaccagcagt gccatttcca 540

tgtggaagag tttctgtttc acaaacttct aagctcaccc gtgctgagac tgtttttcct 600

gatgtggact atgtaaattc tactgaagct gaaaccattt tggataacat cactcaaagc 660

acccaatcat ttaatgactt cactcgagtt gttggtggag aagatgccaa accaggtcaa 720

ttcccttggc aggttgtttt gaatggtaaa gttgatgcat tctgtggagg ctctatcgtt 780

aatgaaaaat ggattgtaac tgctgcccac tgtgttgaaa ctggtgttaa aattacagtt 840

gtcgcaggtg aacataatat tgaggagaca gaacatacag agcaaaagcg aaatgtgatt 900

cgaattattc ctcaccacaa ctacaatgca gctattaata agtacaacca tgacattgcc 960

cttctggaac tggacgaacc cttagtgcta aacagctacg ttacacctat ttgcattgct 1020

acaaggaata cacgaacatc ttcctcaaat ttggatctgg ctatgtaagt ggctggggaa 1080

gagtcttcca caaagggaga tcagctttag ttcttcagta ccttagagtt ccacttgttg 1140

accgagccac atgtcttcga tctacaaagt tcaccatcta taacaacatg ttctgtgctg 1200

gcttccatga aggaggtaga gattcatgtc aaggagatag tgggggaccc catgttactg 1260

aagtggaagg gaccagtttc ttaactggaa ttattagctg ggtgaagag tgtgcaatga 1320

aaggcaaata tggaatatat accaaggtat cccggtatgt caactggatt aaggaaaaaa 1380

caaagctcac tagctccagc agcaaggccc ctcccccgag cctgccctcc ccaagcaggc 1440

tgcctgggcc ctccgacaca ccaatcctgc cacagagcag ctcctctaag gcccctcctc 1500

catccctgcc atccccctcc cggctgcctg gcccctctga cacccctatc ctgcctcagt 1560

gatgaaggtc tggatccgcg gccgc 1585

<210> 21
<211> 517
<212> PRT
<213> Artificial Sequence

<220>

<223> CTP-modified Factor IX

<400> 21

```
Met Gln Arg Val Asn Met Ile Met Ala Glu Ser Pro Gly Leu Ile Thr
1               5                   10                  15

Ile Cys Leu Leu Gly Tyr Leu Leu Ser Ala Glu Cys Thr Val Phe Leu
            20                  25                  30

Asp His Glu Asn Ala Asn Lys Ile Leu Asn Arg Pro Lys Arg Tyr Asn
```

```
            35                  40                  45

Ser Gly Lys Leu Glu Glu Phe Val Gln Gly Asn Leu Glu Arg Glu Cys
    50                  55                  60

Met Glu Glu Lys Cys Ser Phe Glu Glu Ala Arg Glu Val Phe Glu Asn
65                  70                  75                  80

Thr Glu Arg Thr Thr Glu Phe Trp Lys Gln Tyr Val Asp Gly Asp Gln
                85                  90                  95

Cys Glu Ser Asn Pro Cys Leu Asn Gly Gly Ser Cys Lys Asp Asp Ile
            100                 105                 110

Asn Ser Tyr Glu Cys Trp Cys Pro Phe Gly Phe Glu Gly Lys Asn Cys
        115                 120                 125

Glu Leu Asp Val Thr Cys Asn Ile Lys Asn Gly Arg Cys Glu Gln Phe
    130                 135                 140

Cys Lys Asn Ser Ala Asp Asn Lys Val Val Cys Ser Cys Thr Glu Gly
145                 150                 155                 160

Tyr Arg Leu Ala Glu Asn Gln Lys Ser Cys Glu Pro Ala Val Pro Phe
                165                 170                 175

Pro Cys Gly Arg Val Ser Val Ser Gln Thr Ser Lys Leu Thr Arg Ala
            180                 185                 190

Glu Thr Val Phe Pro Asp Val Asp Tyr Val Asn Ser Thr Glu Ala Glu
        195                 200                 205

Thr Ile Leu Asp Asn Ile Thr Gln Ser Thr Gln Ser Phe Asn Asp Phe
    210                 215                 220

Thr Arg Val Val Gly Gly Glu Asp Ala Lys Pro Gly Gln Phe Pro Trp
225                 230                 235                 240

Gln Val Val Leu Asn Gly Lys Val Asp Ala Phe Cys Gly Gly Ser Ile
                245                 250                 255

Val Asn Glu Lys Trp Ile Val Thr Ala Ala His Cys Val Glu Thr Gly
            260                 265                 270

Val Lys Ile Thr Val Val Ala Gly Glu His Asn Ile Glu Glu Thr Glu
        275                 280                 285
```

His Thr Glu Gln Lys Arg Asn Val Ile Arg Ile Ile Pro His His Asn
290 295 300

Tyr Asn Ala Ala Ile Asn Lys Tyr Asn His Asp Ile Ala Leu Leu Glu
305 310 315 320

Leu Asp Glu Pro Leu Val Leu Asn Ser Tyr Val Thr Pro Ile Cys Ile
325 330 335

Ala Asp Lys Glu Tyr Thr Asn Ile Phe Leu Lys Phe Gly Ser Gly Tyr
340 345 350

Val Ser Gly Trp Gly Arg Val Phe His Lys Gly Arg Ser Ala Leu Val
355 360 365

Leu Gln Tyr Leu Arg Val Pro Leu Val Asp Arg Ala Thr Cys Leu Arg
370 375 380

Ser Thr Lys Phe Thr Ile Tyr Asn Asn Met Phe Cys Ala Gly Phe His
385 390 395 400

Glu Gly Gly Arg Asp Ser Cys Gln Gly Asp Ser Gly Gly Pro His Val
405 410 415

Thr Glu Val Glu Gly Thr Ser Phe Leu Thr Gly Ile Ile Ser Trp Gly
420 425 430

Glu Glu Cys Ala Met Lys Gly Lys Tyr Gly Ile Tyr Thr Lys Val Ser
435 440 445

Arg Tyr Val Asn Trp Ile Lys Glu Lys Thr Lys Leu Thr Ser Ser Ser
450 455 460

Ser Lys Ala Pro Pro Pro Ser Leu Pro Ser Pro Ser Arg Leu Pro Gly
465 470 475 480

Pro Ser Asp Thr Pro Ile Leu Pro Gln Ser Ser Ser Ser Lys Ala Pro
485 490 495

Pro Pro Ser Leu Pro Ser Pro Ser Arg Leu Pro Gly Pro Ser Asp Thr
500 505 510

Pro Ile Leu Pro Gln
515

<210> 22
<211> 2413

<212> DNA
<213> Homo sapiens

<400> 22

```
tctagagtcg accccgccat ggagctgagg ccctggttgc tatgggtggt agcagcaaca      60
ggaaccttgg tcctgctagc agctgatgct cagggccaga aggtcttcac caacacgtgg     120
gctgtgcgca tccctggagg cccagcggtg gccaacagtg tggcacggaa gcatgggttc     180
ctcaacctgg gccagatctt cggggactat taccacttct ggcatcgagg agtgacgaag     240
cggtccctgt cgcctcaccg cccgcggcac agccggctgc agagggagcc tcaagtacag     300
tggctggaac agcaggtggc aaagcgacgg actaaacggg acgtgtacca ggagcccaca     360
gaccccaagt ttcctcagca gtggtacctg tctggtgtca ctcagcggga cctgaatgtg     420
aaggcggcct gggcgcaggg ctacacaggg cacggcattg tggtctccat tctggacgat     480
ggcatcgaga agaaccaccc ggacttggca ggcaattatg atcctggggc cagttttgat     540
gtcaatgacc aggaccctga cccccagcct cggtacacac agatgaatga caacaggcac     600
ggcacacggt gtgcggggga agtggctgcg gtggccaaca acggtgtctg tggtgtaggt     660
gtggcctaca acgcccgcat tggaggggtg cgcatgctgg atggcgaggt gacagatgca     720
gtggaggcac gctcgctggg cctgaacccc aaccacatcc acatctacag tgccagctgg     780
ggccccgagg atgacggcaa gacagtggat gggccagccc gcctcgccga ggaggccttc     840
ttccgtgggg ttagccaggg ccgagggggg ctgggctcca tctttgtctg ggcctcgggg     900
aacgggggcc gggaacatga cagctgcaac tgcgacggct acaccaacag tatctacacg     960
ctgtccatca gcagcgccac gcagtttggc aacgtgccgt ggtacagcga ggcctgctcg    1020
tccacactgg ccacgaccta cagcagtggc aaccagaatg agaagcagat cgtgacgact    1080
gacttgcggc agaagtgcac ggagtctcac acgggcacct cagcctctgc cccccttagca    1140
gccggcatca ttgctctcac cctggaggcc aataagaacc tcacatggcg ggacatgcaa    1200
cacctggtgg tacagacctc gaagccagcc cacctcaatg ccaacgactg ggccaccaat    1260
ggtgtgggcc ggaaagtgag ccactcatat ggctacgggc ttttggacgc aggcgccatg    1320
gtggccctgg cccagaattg gaccacagtg gccccccagc ggaagtgcat catcgacatc    1380
ctcaccgagc ccaaagacat cgggaaacgg ctcgaggtgc ggaagaccgt gaccgcgtgc    1440
ctgggcgagc ccaaccacat cactcggctg gagcacgctc aggcgcggct caccctgtcc    1500
tataatcgcc gtggcgacct ggccatccac ctggtcagcc ccatgggcac ccgctccacc    1560
ctgctggcag ccaggccaca tgactactcc gcagatgggt ttaatgactg ggccttcatg    1620
acaactcatt cctgggatga ggatccctct ggcgagtggg tcctagagat tgaaaacacc    1680
agcgaagcca acaactatgg gacgctgacc aagttcaccc tcgtactcta tggcaccgcc    1740
cctgaggggc tgcccgtacc tccagaaagc agtggctgca agaccctcac gtccagtcag    1800
```

gcctgtgtgg tgtgcgagga aggcttctcc ctgcaccaga agagctgtgt ccagcactgc 1860
cctccaggct tcgcccccca agtcctcgat acgcactata gcaccgagaa tgacgtggag 1920
accatccggg ccagcgtctg cgcccctgc cacgcctcat gtgccacatg ccaggggccg 1980
gccctgacag actgcctcag ctgccccagc cacgcctcct tggaccctgt ggagcagact 2040
tgctcccggc aaagccagag cagccgagag tccccgccac agcagcagcc acctcggctg 2100
cccccggagg tggaggcggg gcaacggctg cgggcagggc tgctgccctc acacctgcct 2160
gaggtggtgg ccggcctcag ctgcgccttc atcgtgctgg tcttcgtcac tgtcttcctg 2220
gtcctgcagc tgcgctctgg ctttagtttt cggggggtga aggtgtacac catggaccgt 2280
ggcctcatct cctacaaggg gctgccccct gaagcctggc aggaggagtg cccgtctgac 2340
tcagaagagg acgagggccg gggcgagagg accgccttta tcaaagacca gagcgccctc 2400
tgaacgcggc cgc 2413

<210> 23
<211> 794
<212> PRT
<213> Homo sapiens

<400> 23

Met Glu Leu Arg Pro Trp Leu Leu Trp Val Val Ala Ala Thr Gly Thr
1 5 10 15

Leu Val Leu Leu Ala Ala Asp Ala Gln Gly Gln Lys Val Phe Thr Asn
20 25 30

Thr Trp Ala Val Arg Ile Pro Gly Gly Pro Ala Val Ala Asn Ser Val
35 40 45

Ala Arg Lys His Gly Phe Leu Asn Leu Gly Gln Ile Phe Gly Asp Tyr
50 55 60

Tyr His Phe Trp His Arg Gly Val Thr Lys Arg Ser Leu Ser Pro His
65 70 75 80

Arg Pro Arg His Ser Arg Leu Gln Arg Glu Pro Gln Val Gln Trp Leu
85 90 95

Glu Gln Gln Val Ala Lys Arg Arg Thr Lys Arg Asp Val Tyr Gln Glu
100 105 110

Pro Thr Asp Pro Lys Phe Pro Gln Gln Trp Tyr Leu Ser Gly Val Thr
115 120 125

Gln Arg Asp Leu Asn Val Lys Ala Ala Trp Ala Gln Gly Tyr Thr Gly

```
        130                 135                 140

His Gly Ile Val Val Ser Ile Leu Asp Asp Gly Ile Glu Lys Asn His
145                 150                 155                 160

Pro Asp Leu Ala Gly Asn Tyr Asp Pro Gly Ala Ser Phe Asp Val Asn
                165                 170                 175

Asp Gln Asp Pro Asp Pro Gln Pro Arg Tyr Thr Gln Met Asn Asp Asn
            180                 185                 190

Arg His Gly Thr Arg Cys Ala Gly Glu Val Ala Ala Val Ala Asn Asn
        195                 200                 205

Gly Val Cys Gly Val Gly Val Ala Tyr Asn Ala Arg Ile Gly Gly Val
    210                 215                 220

Arg Met Leu Asp Gly Glu Val Thr Asp Ala Val Glu Ala Arg Ser Leu
225                 230                 235                 240

Gly Leu Asn Pro Asn His Ile His Ile Tyr Ser Ala Ser Trp Gly Pro
                245                 250                 255

Glu Asp Asp Gly Lys Thr Val Asp Gly Pro Ala Arg Leu Ala Glu Glu
            260                 265                 270

Ala Phe Phe Arg Gly Val Ser Gln Gly Arg Gly Gly Leu Gly Ser Ile
        275                 280                 285

Phe Val Trp Ala Ser Gly Asn Gly Gly Arg Glu His Asp Ser Cys Asn
    290                 295                 300

Cys Asp Gly Tyr Thr Asn Ser Ile Tyr Thr Leu Ser Ile Ser Ser Ala
305                 310                 315                 320

Thr Gln Phe Gly Asn Val Pro Trp Tyr Ser Glu Ala Cys Ser Ser Thr
                325                 330                 335

Leu Ala Thr Thr Tyr Ser Ser Gly Asn Gln Asn Glu Lys Gln Ile Val
            340                 345                 350

Thr Thr Asp Leu Arg Gln Lys Cys Thr Glu Ser His Thr Gly Thr Ser
        355                 360                 365

Ala Ser Ala Pro Leu Ala Ala Gly Ile Ile Ala Leu Thr Leu Glu Ala
    370                 375                 380
```

```
Asn Lys Asn Leu Thr Trp Arg Asp Met Gln His Leu Val Val Gln Thr
385                 390                 395                 400

Ser Lys Pro Ala His Leu Asn Ala Asn Asp Trp Ala Thr Asn Gly Val
                405                 410                 415

Gly Arg Lys Val Ser His Ser Tyr Gly Tyr Gly Leu Leu Asp Ala Gly
            420                 425                 430

Ala Met Val Ala Leu Ala Gln Asn Trp Thr Thr Val Ala Pro Gln Arg
        435                 440                 445

Lys Cys Ile Ile Asp Ile Leu Thr Glu Pro Lys Asp Ile Gly Lys Arg
    450                 455                 460

Leu Glu Val Arg Lys Thr Val Thr Ala Cys Leu Gly Glu Pro Asn His
465                 470                 475                 480

Ile Thr Arg Leu Glu His Ala Gln Ala Arg Leu Thr Leu Ser Tyr Asn
                485                 490                 495

Arg Arg Gly Asp Leu Ala Ile His Leu Val Ser Pro Met Gly Thr Arg
            500                 505                 510

Ser Thr Leu Leu Ala Ala Arg Pro His Asp Tyr Ser Ala Asp Gly Phe
        515                 520                 525

Asn Asp Trp Ala Phe Met Thr Thr His Ser Trp Asp Glu Asp Pro Ser
    530                 535                 540

Gly Glu Trp Val Leu Glu Ile Glu Asn Thr Ser Glu Ala Asn Asn Tyr
545                 550                 555                 560

Gly Thr Leu Thr Lys Phe Thr Leu Val Leu Tyr Gly Thr Ala Pro Glu
                565                 570                 575

Gly Leu Pro Val Pro Pro Glu Ser Ser Gly Cys Lys Thr Leu Thr Ser
            580                 585                 590

Ser Gln Ala Cys Val Val Cys Glu Glu Gly Phe Ser Leu His Gln Lys
        595                 600                 605

Ser Cys Val Gln His Cys Pro Pro Gly Phe Ala Pro Gln Val Leu Asp
    610                 615                 620

Thr His Tyr Ser Thr Glu Asn Asp Val Glu Thr Ile Arg Ala Ser Val
625                 630                 635                 640
```

```
Cys Ala Pro Cys His Ala Ser Cys Ala Thr Cys Gln Gly Pro Ala Leu
                645                 650                 655

Thr Asp Cys Leu Ser Cys Pro Ser His Ala Ser Leu Asp Pro Val Glu
            660                 665                 670

Gln Thr Cys Ser Arg Gln Ser Gln Ser Ser Arg Glu Ser Pro Pro Gln
        675                 680                 685

Gln Gln Pro Pro Arg Leu Pro Pro Glu Val Glu Ala Gly Gln Arg Leu
    690                 695                 700

Arg Ala Gly Leu Leu Pro Ser His Leu Pro Glu Val Val Ala Gly Leu
705                 710                 715                 720

Ser Cys Ala Phe Ile Val Leu Val Phe Val Thr Val Phe Leu Val Leu
                725                 730                 735

Gln Leu Arg Ser Gly Phe Ser Phe Arg Gly Val Lys Val Tyr Thr Met
            740                 745                 750

Asp Arg Gly Leu Ile Ser Tyr Lys Gly Leu Pro Pro Glu Ala Trp Gln
        755                 760                 765

Glu Glu Cys Pro Ser Asp Ser Glu Glu Asp Glu Gly Arg Gly Glu Arg
    770                 775                 780

Thr Ala Phe Ile Lys Asp Gln Ser Ala Leu
785                 790
```

<210> 24
<211> 1621
<212> DNA
<213> Artificial Sequence

<220>
<223> CTP-modified Factor VII

<400> 24

ctcgaggaca tggtctccca ggccctcagg ctcctctgcc ttctgcttgg gcttcagggc 60
tgcctggctg cagtcttcgt aacccaggag gaagcccacg gcgtcctgca ccggcgccgg 120
cgcgccaacg cgttcctgga ggagctgcgg ccgggctccc tggagaggga gtgcaaggag 180
gagcagtgct ccttcgagga ggcccgggag atcttcaagg acgcggagag gacgaagctg 240
ttctggattt cttacagtga tggggaccag tgtgcctcaa gtccatgcca gaatgggggc 300
tcctgcaagg accagctcca gtcctatatc tgcttctgcc tccctgcctt cgagggccgg 360
aactgtgaga cgcacaagga tgaccagctg atctgtgtga acgagaacgg cggctgtgag 420
cagtactgca gtgaccacac gggcaccaag cgctcctgtc ggtgccacga ggggtactct 480
ctgctggcag acggggtgtc ctgcacaccc acagttgaat atccatgtgg aaaaatacct 540
attctagaaa aaagaaatgc cagcaaaccc caaggccgaa ttgtgggggg caaggtgtgc 600
cccaaagggg agtgtccatg gcaggtcctg ttgttggtga atggagctca gttgtgtggg 660
gggaccctga tcaacaccat ctgggtggtc tccgcggccc actgtttcga caaaatcaag 720
aactggagga acctgatcgc ggtgctgggc gagcacgacc tcagcgagca cgacggggat 780
gagcagagcc ggcgggtggc gcaggtcatc atccccagca cgtacgtccc gggcaccacc 840
aaccacgaca tcgcgctgct ccgcctgcac cagcccgtgg tcctcactga ccatgtggtg 900
cccctctgcc tgcccgaacg gacgttctct gagaggacgc tggccttcgt gcgcttctca 960
ttggtcagcg gctggggcca gctgctggac cgtggcgcca cggccctgga gctcatggtc 1020
ctcaacgtgc cccggctgat gacccaggac tgcctgcagc agtcacggaa ggtgggagac 1080
tccccaaata tcacggagta catgttctgt gccggctact cggatggcag caaggactcc 1140
tgcaaggggg acagtggagg cccacatgcc acccactacc ggggcacgtg gtacctgacc 1200
ggcatcgtga gctggggcca gggctgcgcc accgtgggcc acttcggcgt gtacaccagg 1260
gtgtcccagt acatcgagtg gctgcagaaa ctgatgagaa gcgagcccag acccggcgtg 1320
ctgctgagag ccccccttccc cagcagcagc tccaaggccc ctccccctag cctgcccagc 1380
cctagcagac tgcctgggcc cagtgacacc cctatcctgc ctcagtccag ctccagcaag 1440
gccccacccc ctagcctgcc ttctccttct cggctgcctg gccccagcga tactccaatt 1500
ctgccccagt cctccagcag taaggctccc cctccatctc tgccatcccc cagcagactg 1560
ccaggccctt ctgatacacc catcctccca cagtgatgag gatccgcggc cgcttaatta 1620
a 1621

<210> 25
<211> 528
<212> PRT
<213> Artificial Sequence

<220>
<223> CTP-modified Factor VII

<400> 25

```
Met Val Ser Gln Ala Leu Arg Leu Leu Cys Leu Leu Leu Gly Leu Gln
1               5                   10                  15

Gly Cys Leu Ala Ala Val Phe Val Thr Gln Glu Glu Ala His Gly Val
            20                  25                  30

Leu His Arg Arg Arg Arg Ala Asn Ala Phe Leu Glu Glu Leu Arg Pro
```

35 40 45

Gly Ser Leu Glu Arg Glu Cys Lys Glu Glu Gln Cys Ser Phe Glu Glu
50 55 60

Ala Arg Glu Ile Phe Lys Asp Ala Glu Arg Thr Lys Leu Phe Trp Ile
65 70 75 80

Ser Tyr Ser Asp Gly Asp Gln Cys Ala Ser Ser Pro Cys Gln Asn Gly
85 90 95

Gly Ser Cys Lys Asp Gln Leu Gln Ser Tyr Ile Cys Phe Cys Leu Pro
100 105 110

Ala Phe Glu Gly Arg Asn Cys Glu Thr His Lys Asp Asp Gln Leu Ile
115 120 125

Cys Val Asn Glu Asn Gly Gly Cys Glu Gln Tyr Cys Ser Asp His Thr
130 135 140

Gly Thr Lys Arg Ser Cys Arg Cys His Glu Gly Tyr Ser Leu Leu Ala
145 150 155 160

Asp Gly Val Ser Cys Thr Pro Thr Val Glu Tyr Pro Cys Gly Lys Ile
165 170 175

Pro Ile Leu Glu Lys Arg Asn Ala Ser Lys Pro Gln Gly Arg Ile Val
180 185 190

Gly Gly Lys Val Cys Pro Lys Gly Glu Cys Pro Trp Gln Val Leu Leu
195 200 205

Leu Val Asn Gly Ala Gln Leu Cys Gly Gly Thr Leu Ile Asn Thr Ile
210 215 220

Trp Val Val Ser Ala Ala His Cys Phe Asp Lys Ile Lys Asn Trp Arg
225 230 235 240

Asn Leu Ile Ala Val Leu Gly Glu His Asp Leu Ser Glu His Asp Gly
245 250 255

Asp Glu Gln Ser Arg Arg Val Ala Gln Val Ile Ile Pro Ser Thr Tyr
260 265 270

Val Pro Gly Thr Thr Asn His Asp Ile Ala Leu Leu Arg Leu His Gln
275 280 285

```
Pro Val Val Leu Thr Asp His Val Val Pro Leu Cys Leu Pro Glu Arg
    290                 295                 300

Thr Phe Ser Glu Arg Thr Leu Ala Phe Val Arg Phe Ser Leu Val Ser
305                 310                 315                 320

Gly Trp Gly Gln Leu Leu Asp Arg Gly Ala Thr Ala Leu Glu Leu Met
                325                 330                 335

Val Leu Asn Val Pro Arg Leu Met Thr Gln Asp Cys Leu Gln Gln Ser
            340                 345                 350

Arg Lys Val Gly Asp Ser Pro Asn Ile Thr Glu Tyr Met Phe Cys Ala
        355                 360                 365

Gly Tyr Ser Asp Gly Ser Lys Asp Ser Cys Lys Gly Asp Ser Gly Gly
    370                 375                 380

Pro His Ala Thr His Tyr Arg Gly Thr Trp Tyr Leu Thr Gly Ile Val
385                 390                 395                 400

Ser Trp Gly Gln Gly Cys Ala Thr Val Gly His Phe Gly Val Tyr Thr
                405                 410                 415

Arg Val Ser Gln Tyr Ile Glu Trp Leu Gln Lys Leu Met Arg Ser Glu
            420                 425                 430

Pro Arg Pro Gly Val Leu Leu Arg Ala Pro Phe Pro Ser Ser Ser Ser
        435                 440                 445

Lys Ala Pro Pro Pro Ser Leu Pro Ser Pro Ser Arg Leu Pro Gly Pro
    450                 455                 460

Ser Asp Thr Pro Ile Leu Pro Gln Ser Ser Ser Ser Lys Ala Pro Pro
465                 470                 475                 480

Pro Ser Leu Pro Ser Pro Ser Arg Leu Pro Gly Pro Ser Asp Thr Pro
                485                 490                 495

Ile Leu Pro Gln Ser Ser Ser Ser Lys Ala Pro Pro Pro Ser Leu Pro
            500                 505                 510

Ser Pro Ser Arg Leu Pro Gly Pro Ser Asp Thr Pro Ile Leu Pro Gln
        515                 520                 525
```

<210> 26
<211> 1607

<212> DNA
<213> Artificial Sequence

<220>
<223> CTP-modified Factor VII

<400> 26

```
ctcgaggaca tggtctccca ggccctcagg ctcctctgcc ttctgcttgg gcttcagggc      60
tgcctggctg cagtcttcgt aacccaggag gaagcccacg gcgtcctgca ccggcgccgg     120
cgcgccaacg cgttcctgga ggagctgcgg ccgggctccc tggagaggga gtgcaaggag     180
gagcagtgct ccttcgagga ggcccgggag atcttcaagg acgcggagag gacgaagctg     240
ttctggattt cttacagtga tggggaccag tgtgcctcaa gtccatgcca gaatgggggc     300
tcctgcaagg accagctcca gtcctatatc tgcttctgcc tccctgcctt cgagggccgg     360
aactgtgaga cgcacaagga tgaccagctg atctgtgtga acgagaacgg cggctgtgag     420
cagtactgca gtgaccacac gggcaccaag cgctcctgtc ggtgccacga ggggtactct     480
ctgctggcag acggggtgtc ctgcacaccc acagttgaat atccatgtgg aaaaatacct     540
attctagaaa aaagaaatgc cagcaaaccc caaggccgaa ttgtgggggg caaggtgtgc     600
cccaaagggg agtgtccatg gcaggtcctg ttgttggtga atggagctca gttgtgtggg     660
gggaccctga tcaacaccat ctgggtggtc tccgcggccc actgtttcga caaaatcaag     720
aactggagga acctgatcgc ggtgctgggc gagcacgacc tcagcgagca cgacggggat     780
gagcagagcc ggcgggtggc gcaggtcatc atccccagca cgtacgtccc gggcaccacc     840
aaccacgaca tcgcgctgct ccgcctgcac cagcccgtgg tcctcactga ccatgtggtg     900
cccctctgcc tgcccgaacg gacgttctct gagaggacgc tggccttcgt gcgcttctca     960
ttggtcagcg gctggggcca gctgctggac cgtggcgcca cggccctgga gctcatggtc    1020
ctcaacgtgc cccggctgat gacccaggac tgcctgcagc agtcacggaa ggtgggagac    1080
tccccaaata tcacggagta catgttctgt gccggctact cggatggcag caaggactcc    1140
tgcaaggggg acagtggagg cccacatgcc acccactacc ggggcacgtg gtacctgacc    1200
ggcatcgtga gctggggcca gggctgcgcc accgtgggcc acttcggcgt gtacaccagg    1260
gtgtcccagt acatcgagtg gctgcagaaa ctgatgagaa gcgagcccag acccggcgtg    1320
ctgctgagag ccccccttccc cagcagcagc tccaaggccc ctccccctag cctgcccagc    1380
cctagcagac tgcctgggcc cagtgacacc cctatcctgc ctcagtccag ctccagcaag    1440
gccccacccc ctagcctgcc ttctccttct cggctgcctg gccccagcga tactccaatt    1500
ctgccccagt cctccagcag taaggctccc cctccatctc tgccatcccc cagcagactg    1560
ccaggccctt ctgatacacc catcctccca cagtgatgag gatccgc                  1607
```

<210> 27
<211> 532
<212> PRT
<213> Artificial Sequence

<220>
<223> CTP-modified Factor VII

<400> 27

```
Leu Glu Asp Met Val Ser Gln Ala Leu Arg Leu Leu Cys Leu Leu Leu
1               5                   10                  15

Gly Leu Gln Gly Cys Leu Ala Ala Val Phe Val Thr Gln Glu Glu Ala
            20                  25                  30

His Gly Val Leu His Arg Arg Arg Arg Ala Asn Ala Phe Leu Glu Glu
        35                  40                  45

Leu Arg Pro Gly Ser Leu Glu Arg Glu Cys Lys Glu Glu Gln Cys Ser
    50                  55                  60

Phe Glu Glu Ala Arg Glu Ile Phe Lys Asp Ala Glu Arg Thr Lys Leu
65                  70                  75                  80

Phe Trp Ile Ser Tyr Ser Asp Gly Asp Gln Cys Ala Ser Ser Pro Cys
                85                  90                  95

Gln Asn Gly Gly Ser Cys Lys Asp Gln Leu Gln Ser Tyr Ile Cys Phe
            100                 105                 110

Cys Leu Pro Ala Phe Glu Gly Arg Asn Cys Glu Thr His Lys Asp Asp
        115                 120                 125

Gln Leu Ile Cys Val Asn Glu Asn Gly Gly Cys Glu Gln Tyr Cys Ser
    130                 135                 140

Asp His Thr Gly Thr Lys Arg Ser Cys Arg Cys His Glu Gly Tyr Ser
145                 150                 155                 160

Leu Leu Ala Asp Gly Val Ser Cys Thr Pro Thr Val Glu Tyr Pro Cys
                165                 170                 175

Gly Lys Ile Pro Ile Leu Glu Lys Arg Asn Ala Ser Lys Pro Gln Gly
            180                 185                 190

Arg Ile Val Gly Gly Lys Val Cys Pro Lys Gly Glu Cys Pro Trp Gln
        195                 200                 205

Val Leu Leu Leu Val Asn Gly Ala Gln Leu Cys Gly Gly Thr Leu Ile
    210                 215                 220
```

Asn Thr Ile Trp Val Val Ser Ala Ala His Cys Phe Asp Lys Ile Lys
225 230 235 240

Asn Trp Arg Asn Leu Ile Ala Val Leu Gly Glu His Asp Leu Ser Glu
245 250 255

His Asp Gly Asp Glu Gln Ser Arg Arg Val Ala Gln Val Ile Ile Pro
260 265 270

Ser Thr Tyr Val Pro Gly Thr Thr Asn His Asp Ile Ala Leu Leu Arg
275 280 285

Leu His Gln Pro Val Val Leu Thr Asp His Val Val Pro Leu Cys Leu
290 295 300

Pro Glu Arg Thr Phe Ser Glu Arg Thr Leu Ala Phe Val Arg Phe Ser
305 310 315 320

Leu Val Ser Gly Trp Gly Gln Leu Leu Asp Arg Gly Ala Thr Ala Leu
325 330 335

Glu Leu Met Val Leu Asn Val Pro Arg Leu Met Thr Gln Asp Cys Leu
340 345 350

Gln Gln Ser Arg Lys Val Gly Asp Ser Pro Asn Ile Thr Glu Tyr Met
355 360 365

Phe Cys Ala Gly Tyr Ser Asp Gly Ser Lys Asp Ser Cys Lys Gly Asp
370 375 380

Ser Gly Gly Pro His Ala Thr His Tyr Arg Gly Thr Trp Tyr Leu Thr
385 390 395 400

Gly Ile Val Ser Trp Gly Gln Gly Cys Ala Thr Val Gly His Phe Gly
405 410 415

Val Tyr Thr Arg Val Ser Gln Tyr Ile Glu Trp Leu Gln Lys Leu Met
420 425 430

Arg Ser Glu Pro Arg Pro Gly Val Leu Leu Arg Ala Pro Phe Pro Ser
435 440 445

Ser Ser Ser Lys Ala Pro Pro Pro Ser Leu Pro Ser Pro Ser Arg Leu
450 455 460

Pro Gly Pro Ser Asp Thr Pro Ile Leu Pro Gln Ser Ser Ser Ser Lys
465 470 475 480

Ala Pro Pro Pro Ser Leu Pro Ser Pro Ser Arg Leu Pro Gly Pro Ser
485 490 495

Asp Thr Pro Ile Leu Pro Gln Ser Ser Ser Ser Lys Ala Pro Pro Pro
500 505 510

Ser Leu Pro Ser Pro Ser Arg Leu Pro Gly Pro Ser Asp Thr Pro Ile
515 520 525

Leu Pro Gln Gly
530

<210> 28
<211> 1775
<212> DNA
<213> Artificial Sequence

<220>
<223> CTP-modified Factor VII

<400> 28

ctcgaggaca tggtctccca ggccctcagg ctcctctgcc ttctgcttgg gcttcagggc 60

tgcctggctg cagtcttcgt aacccaggag gaagcccacg gcgtcctgca ccggcgccgg 120

cgcgccaacg cgttcctgga ggagctgcgg ccgggctccc tggagaggga gtgcaaggag 180

gagcagtgct ccttcgagga ggcccgggag atcttcaagg acgcggagag gacgaagctg 240

ttctggattt cttacagtga tggggaccag tgtgcctcaa gtccatgcca gaatgggggc 300

tcctgcaagg accagctcca gtcctatatc tgcttctgcc tccctgcctt cgagggccgg 360

aactgtgaga cgcacaagga tgaccagctg atctgtgtga acgagaacgg cggctgtgag 420

cagtactgca gtgaccacac gggcaccaag cgctcctgtc ggtgccacga ggggtactct 480

ctgctggcag acggggtgtc ctgcacaccc acagttgaat atccatgtgg aaaaatacct 540

attctagaaa aaagaaatgc cagcaaaccc caaggccgaa ttgtgggggg caaggtgtgc 600

cccaaagggg agtgtccatg gcaggtcctg ttgttggtga atggagctca gttgtgtggg 660

gggaccctga tcaacaccat ctgggtggtc tccgcggccc actgtttcga caaaatcaag 720

aactggagga acctgatcgc ggtgctgggc gagcacgacc tcagcgagca cgacggggat 780

gagcagagcc ggcgggtggc gcaggtcatc atccccagca cgtacgtccc gggcaccacc 840

aaccacgaca tcgcgctgct ccgcctgcac cagcccgtgg tcctcactga ccatgtggtg 900

cccctctgcc tgcccgaacg gacgttctct gagaggacgc tggccttcgt gcgcttctca 960

ttggtcagcg gctggggcca gctgctggac cgtggcgcca cggccctgga gctcatggtc 1020

ctcaacgtgc cccggctgat gacccaggac tgcctgcagc agtcacggaa ggtgggagac 1080

```
tccccaaata tcacggagta catgttctgt gccggctact cggatggcag caaggactcc     1140
tgcaaggggg acagtggagg cccacatgcc acccactacc ggggcacgtg gtacctgacc     1200
ggcatcgtga gctggggcca gggctgcgcc accgtgggcc acttcggcgt gtacaccagg     1260
gtgtcccagt acatcgagtg gctgcagaaa ctgatgagaa gcgagcccag acccggcgtg     1320
ctgctgagag ccccccttccc cagcagcagc tccaaggccc ctcccccctag cctgcccagc   1380
cctagcagac tgcctgggcc ctctgacacc cctatcctgc ctcagtccag ctcctctaag     1440
gctccaccac cttccctgcc tagcccttca agactgccag gccctagcga tacaccaatt     1500
ctgccccagt cctccagcag caaggctccc ccacctagcc tgccttctcc atcaaggctg     1560
cctggcccat ccgatacccc aattttgcct cagagcagct ctagcaaggc acctcccccc     1620
agtctgccct ctccaagcag actccctggc ccttcagaca ctccaatcct cccacagtcc     1680
tctagctcta aagctccacc tcccagcctg cccagcccta gtagactccc cggaccttct     1740
gataccccca tcttgcccca gtgatgagga tccgc                                1775
```

<210> 29
<211> 589
<212> PRT
<213> Artificial Sequence

<220>
<223> CTP-modified Factor VII

<400> 29

```
Leu Glu Asp Met Val Ser Gln Ala Leu Arg Leu Leu Cys Leu Leu Leu
1               5                   10                  15

Gly Leu Gln Gly Cys Leu Ala Ala Val Phe Val Thr Gln Glu Glu Ala
            20                  25                  30

His Gly Val Leu His Arg Arg Arg Arg Ala Asn Ala Phe Leu Glu Glu
        35                  40                  45

Leu Arg Pro Gly Ser Leu Glu Arg Glu Cys Lys Glu Glu Gln Cys Ser
    50                  55                  60

Phe Glu Glu Ala Arg Glu Ile Phe Lys Asp Ala Glu Arg Thr Lys Leu
65                  70                  75                  80

Phe Trp Ile Ser Tyr Ser Asp Gly Asp Gln Cys Ala Ser Ser Pro Cys
                85                  90                  95

Gln Asn Gly Gly Ser Cys Lys Asp Gln Leu Gln Ser Tyr Ile Cys Phe
            100                 105                 110
```

```
Cys Leu Pro Ala Phe Glu Gly Arg Asn Cys Glu Thr His Lys Asp Asp
        115                 120                 125

Gln Leu Ile Cys Val Asn Glu Asn Gly Gly Cys Glu Gln Tyr Cys Ser
    130                 135                 140

Asp His Thr Gly Thr Lys Arg Ser Cys Arg Cys His Glu Gly Tyr Ser
145                 150                 155                 160

Leu Leu Ala Asp Gly Val Ser Cys Thr Pro Thr Val Glu Tyr Pro Cys
                165                 170                 175

Gly Lys Ile Pro Ile Leu Glu Lys Arg Asn Ala Ser Lys Pro Gln Gly
            180                 185                 190

Arg Ile Val Gly Gly Lys Val Cys Pro Lys Gly Glu Cys Pro Trp Gln
        195                 200                 205

Val Leu Leu Leu Val Asn Gly Ala Gln Leu Cys Gly Gly Thr Leu Ile
    210                 215                 220

Asn Thr Ile Trp Val Val Ser Ala Ala His Cys Phe Asp Lys Ile Lys
225                 230                 235                 240

Asn Trp Arg Asn Leu Ile Ala Val Leu Gly Glu His Asp Leu Ser Glu
                245                 250                 255

His Asp Gly Asp Glu Gln Ser Arg Arg Val Ala Gln Val Ile Ile Pro
            260                 265                 270

Ser Thr Tyr Val Pro Gly Thr Thr Asn His Asp Ile Ala Leu Leu Arg
        275                 280                 285

Leu His Gln Pro Val Val Leu Thr Asp His Val Val Pro Leu Cys Leu
    290                 295                 300

Pro Glu Arg Thr Phe Ser Glu Arg Thr Leu Ala Phe Val Arg Phe Ser
305                 310                 315                 320

Leu Val Ser Gly Trp Gly Gln Leu Leu Asp Arg Gly Ala Thr Ala Leu
                325                 330                 335

Glu Leu Met Val Leu Asn Val Pro Arg Leu Met Thr Gln Asp Cys Leu
            340                 345                 350

Gln Gln Ser Arg Lys Val Gly Asp Ser Pro Asn Ile Thr Glu Tyr Met
        355                 360                 365
```

```
Phe Cys Ala Gly Tyr Ser Asp Gly Ser Lys Asp Ser Cys Lys Gly Asp
    370                 375                 380

Ser Gly Gly Pro His Ala Thr His Tyr Arg Gly Thr Trp Tyr Leu Thr
385                 390                 395                 400

Gly Ile Val Ser Trp Gly Gln Gly Cys Ala Thr Val Gly His Phe Gly
                405                 410                 415

Val Tyr Thr Arg Val Ser Gln Tyr Ile Glu Trp Leu Gln Lys Leu Met
            420                 425                 430

Arg Ser Glu Pro Arg Pro Gly Val Leu Leu Arg Ala Pro Phe Pro Ser
        435                 440                 445

Ser Ser Ser Lys Ala Pro Pro Pro Ser Leu Pro Ser Pro Ser Arg Leu
    450                 455                 460

Pro Gly Pro Ser Asp Thr Pro Ile Leu Pro Gln Ser Ser Ser Ser Lys
465                 470                 475                 480

Ala Pro Pro Pro Ser Leu Pro Ser Pro Ser Arg Leu Pro Gly Pro Ser
                485                 490                 495

Asp Thr Pro Ile Leu Pro Gln Ser Ser Ser Ser Lys Ala Pro Pro Pro
            500                 505                 510

Ser Leu Pro Ser Pro Ser Arg Leu Pro Gly Pro Ser Asp Thr Pro Ile
        515                 520                 525

Leu Pro Gln Ser Ser Ser Ser Lys Ala Pro Pro Pro Ser Leu Pro Ser
    530                 535                 540

Pro Ser Arg Leu Pro Gly Pro Ser Asp Thr Pro Ile Leu Pro Gln Ser
545                 550                 555                 560

Ser Ser Ser Lys Ala Pro Pro Pro Ser Leu Pro Ser Pro Ser Arg Leu
                565                 570                 575

Pro Gly Pro Ser Asp Thr Pro Ile Leu Pro Gln Gly Ser
            580                 585
```

<210> 30
<211> 1673
<212> DNA
<213> Artificial Sequence

<220>

<223> CTP-modified Factor IX

<400> 30

```
tctagagtcg accccgccat gcagcgcgtg aacatgatca tggcagaatc accaggcctc      60
atcaccatct gccttttagg atatctactc agtgctgaat gtacagtttt tcttgatcat     120
gaaaacgcca acaaaattct gaatcggcca aagaggtata attcaggtaa attggaagag     180
tttgttcaag ggaaccttga gagagaatgt atggaagaaa agtgtagttt tgaagaagca     240
cgagaagttt ttgaaaacac tgaaagaaca actgaatttt ggaagcagta tgttgatgga     300
gatcagtgtg agtccaatcc atgtttaaat ggcggcagtt gcaaggatga cattaattcc     360
tatgaatgtt ggtgtccctt tggatttgaa ggaaagaact gtgaattaga tgtaacatgt     420
aacattaaga atggcagatg cgagcagttt tgtaaaaata gtgctgataa caaggtggtt     480
tgctcctgta ctgagggata tcgacttgca gaaaaccaga agtcctgtga accagcagtg     540
ccatttccat gtggaagagt ttctgtttca caaacttcta agctcacccg tgctgaggca     600
gtttttcctg atgtggacta tgtaaattct actgaagctg aaaccatttt ggataacatc     660
actcaaagca cccaatcatt taatgacttc actcgagttg ttggtggaga agatgccaaa     720
ccaggtcaat tcccttggca ggttgttttg aatggtaaag ttgatgcatt ctgtggaggc     780
tctatcgtta atgaaaaatg gattgtaact gctgcccact gtgttgaaac tggtgttaaa     840
attacagttg tcgcaggtga acataatatt gaggagacag aacatacaga gcaaaagcga     900
aatgtgattc gaattattcc tcaccacaac tacaatgcag ctattaataa gtacaaccat     960
gacattgccc ttctggaact ggacgaaccc ttagtgctaa acagctacgt tacacctatt    1020
tgcattgctg acaaggaata cacgaacatc ttcctcaaat ttggatctgg ctatgtaagt    1080
ggctggggaa gagtcttcca caaagggaga tcagctttag ttcttcagta ccttagagtt    1140
ccacttgttg accgagccac atgtcttcga tctacaaagt tcaccatcta taacaacatg    1200
ttctgtgctg gcttccatga aggaggtaga gattcatgtc aaggagatag tgggggaccc    1260
catgttactg aagtggaagg gaccagtttc ttaactggaa ttattagctg gggtgaagag    1320
tgtgcaatga aaggcaaata tggaatatat accaaggtat cccggtatgt caactggatt    1380
aaggaaaaaa caaagctcac tagctccagc agcaaggccc ctcccccgag cctgccctcc    1440
ccaagcaggc tgcctgggcc cagtgacacc cctatcctgc ctcagtccag ctccagcaag    1500
gccccacccc ctagcctgcc ttctccttct cggctgcctg gccccagcga tactccaatt    1560
ctgccccagt cctccagcag taaggctccc cctccatctc tgccatcccc cagcagactg    1620
ccaggccctt ctgatacacc catcctccca cagtgatgag gatccgcggc cgc           1673
```

<210> 31

<211> 545
<212> PRT
<213> Artificial Sequence

<220>
<223> CTP-modified Factor IX

<400> 31

```
Met Gln Arg Val Asn Met Ile Met Ala Glu Ser Pro Gly Leu Ile Thr
1               5                   10                  15

Ile Cys Leu Leu Gly Tyr Leu Leu Ser Ala Glu Cys Thr Val Phe Leu
            20                  25                  30

Asp His Glu Asn Ala Asn Lys Ile Leu Asn Arg Pro Lys Arg Tyr Asn
        35                  40                  45

Ser Gly Lys Leu Glu Glu Phe Val Gln Gly Asn Leu Glu Arg Glu Cys
    50                  55                  60

Met Glu Glu Lys Cys Ser Phe Glu Glu Ala Arg Glu Val Phe Glu Asn
65                  70                  75                  80

Thr Glu Arg Thr Thr Glu Phe Trp Lys Gln Tyr Val Asp Gly Asp Gln
                85                  90                  95

Cys Glu Ser Asn Pro Cys Leu Asn Gly Gly Ser Cys Lys Asp Asp Ile
            100                 105                 110

Asn Ser Tyr Glu Cys Trp Cys Pro Phe Gly Phe Glu Gly Lys Asn Cys
        115                 120                 125

Glu Leu Asp Val Thr Cys Asn Ile Lys Asn Gly Arg Cys Glu Gln Phe
    130                 135                 140

Cys Lys Asn Ser Ala Asp Asn Lys Val Val Cys Ser Cys Thr Glu Gly
145                 150                 155                 160

Tyr Arg Leu Ala Glu Asn Gln Lys Ser Cys Glu Pro Ala Val Pro Phe
                165                 170                 175

Pro Cys Gly Arg Val Ser Val Ser Gln Thr Ser Lys Leu Thr Arg Ala
            180                 185                 190

Glu Ala Val Phe Pro Asp Val Asp Tyr Val Asn Ser Thr Glu Ala Glu
        195                 200                 205

Thr Ile Leu Asp Asn Ile Thr Gln Ser Thr Gln Ser Phe Asn Asp Phe
    210                 215                 220
```

```
Thr Arg Val Val Gly Gly Glu Asp Ala Lys Pro Gly Gln Phe Pro Trp
225                 230                 235                 240

Gln Val Val Leu Asn Gly Lys Val Asp Ala Phe Cys Gly Gly Ser Ile
                245                 250                 255

Val Asn Glu Lys Trp Ile Val Thr Ala Ala His Cys Val Glu Thr Gly
            260                 265                 270

Val Lys Ile Thr Val Val Ala Gly Glu His Asn Ile Glu Glu Thr Glu
        275                 280                 285

His Thr Glu Gln Lys Arg Asn Val Ile Arg Ile Ile Pro His His Asn
    290                 295                 300

Tyr Asn Ala Ala Ile Asn Lys Tyr Asn His Asp Ile Ala Leu Leu Glu
305                 310                 315                 320

Leu Asp Glu Pro Leu Val Leu Asn Ser Tyr Val Thr Pro Ile Cys Ile
                325                 330                 335

Ala Asp Lys Glu Tyr Thr Asn Ile Phe Leu Lys Phe Gly Ser Gly Tyr
            340                 345                 350

Val Ser Gly Trp Gly Arg Val Phe His Lys Gly Arg Ser Ala Leu Val
        355                 360                 365

Leu Gln Tyr Leu Arg Val Pro Leu Val Asp Arg Ala Thr Cys Leu Arg
    370                 375                 380

Ser Thr Lys Phe Thr Ile Tyr Asn Asn Met Phe Cys Ala Gly Phe His
385                 390                 395                 400

Glu Gly Gly Arg Asp Ser Cys Gln Gly Asp Ser Gly Gly Pro His Val
                405                 410                 415

Thr Glu Val Glu Gly Thr Ser Phe Leu Thr Gly Ile Ile Ser Trp Gly
            420                 425                 430

Glu Glu Cys Ala Met Lys Gly Lys Tyr Gly Ile Tyr Thr Lys Val Ser
        435                 440                 445

Arg Tyr Val Asn Trp Ile Lys Glu Lys Thr Lys Leu Thr Ser Ser Ser
    450                 455                 460

Ser Lys Ala Pro Pro Pro Ser Leu Pro Ser Pro Ser Arg Leu Pro Gly
465                 470                 475                 480
```

```
Pro Ser Asp Thr Pro Ile Leu Pro Gln Ser Ser Ser Ser Lys Ala Pro
                485                 490                 495

Pro Pro Ser Leu Pro Ser Pro Ser Arg Leu Pro Gly Pro Ser Asp Thr
            500                 505                 510

Pro Ile Leu Pro Gln Ser Ser Ser Ser Lys Ala Pro Pro Pro Ser Leu
        515                 520                 525

Pro Ser Pro Ser Arg Leu Pro Gly Pro Ser Asp Thr Pro Ile Leu Pro
    530                 535                 540

Gln
545
```

<210> 32
<211> 1757
<212> DNA
<213> Artificial Sequence

<220>
<223> CTP-modified Factor IX

<400> 32

tctagagtcg accccgccat gcagcgcgtg aacatgatca tggcagaatc accaggcctc 60
atcaccatct gccttttagg atatctactc agtgctgaat gtacagtttt tcttgatcat 120
gaaaacgcca acaaaattct gaatcggcca aagaggtata attcaggtaa attggaagag 180
tttgttcaag ggaaccttga gagagaatgt atggaagaaa agtgtagttt tgaagaagca 240
cgagaagttt ttgaaaacac tgaaagaaca actgaatttt ggaagcagta tgttgatgga 300
gatcagtgtg agtccaatcc atgtttaaat ggcggcagtt gcaaggatga cattaattcc 360
tatgaatgtt ggtgtccctt tggatttgaa ggaaagaact gtgaattaga tgtaacatgt 420
aacattaaga atggcagatg cgagcagttt tgtaaaaata gtgctgataa caaggtggtt 480
tgctcctgta ctgagggata tcgacttgca gaaaaccaga agtcctgtga accagcagtg 540
ccatttccat gtggaagagt ttctgtttca caaacttcta agctcacccg tgctgaggca 600
gtttttcctg atgtggacta tgtaaattct actgaagctg aaaccatttt ggataacatc 660
actcaaagca cccaatcatt taatgacttc actcgagttg ttggtggaga agatgccaaa 720
ccaggtcaat tcccttggca ggttgttttg aatggtaaag ttgatgcatt ctgtggaggc 780
tctatcgtta atgaaaaatg gattgtaact gctgcccact gtgttgaaac tggtgttaaa 840
attacagttg tcgcaggtga acataatatt gaggagacag aacatacaga gcaaaagcga 900
aatgtgattc gaattattcc tcaccacaac tacaatgcag ctattaataa gtacaaccat 960
gacattgccc ttctggaact ggacgaaccc ttagtgctaa acagctacgt tacacctatt 1020
tgcattgctg acaaggaata cacgaacatc ttcctcaaat ttggatctgg ctatgtaagt 1080
ggctggggaa gagtcttcca caaagggaga tcagctttag ttcttcagta ccttagagtt 1140
ccacttgttg accgagccac atgtcttcga tctacaaagt tcaccatcta taacaacatg 1200
ttctgtgctg gcttccatga aggaggtaga gattcatgtc aaggagatag tggggggaccc 1260
catgttactg aagtggaagg gaccagtttc ttaactggaa ttattagctg gggtgaagag 1320
tgtgcaatga aaggcaaata tggaatatat accaaggtat cccggtatgt caactggatt 1380
aaggaaaaaa caaagctcac tagctccagc agcaaggccc ctcccccgag cctgccctcc 1440
ccaagcaggc tgcctgggcc ctctgacacc cctatcctgc ctcagtccag ctcctctaag 1500
gccccaccac cttccctgcc tagcccttca agactgccag gccctagcga tacaccaatt 1560
ctgccccagt cctccagcag caaggctccc ccacctagcc tgccttctcc atcaaggctg 1620
cctggcccat ccgatacccc aattttgcct cagagcagct ctagcaaggc acctcccccc 1680
agtctgccct ctccaagcag actccctggc ccttcagaca ctcccattct gccacagtga 1740
tgaggatccg cggccgc 1757

<210> 33
<211> 583
<212> PRT
<213> Artificial Sequence

<220>
<223> CTP-modified Factor IX

<400> 33

```
Ser Arg Val Asp Pro Ala Met Gln Arg Val Asn Met Ile Met Ala Glu
1               5                   10                  15

Ser Pro Gly Leu Ile Thr Ile Cys Leu Leu Gly Tyr Leu Leu Ser Ala
            20                  25                  30

Glu Cys Thr Val Phe Leu Asp His Glu Asn Ala Asn Lys Ile Leu Asn
        35                  40                  45

Arg Pro Lys Arg Tyr Asn Ser Gly Lys Leu Glu Glu Phe Val Gln Gly
    50                  55                  60

Asn Leu Glu Arg Glu Cys Met Glu Glu Lys Cys Ser Phe Glu Glu Ala
65                  70                  75                  80

Arg Glu Val Phe Glu Asn Thr Glu Arg Thr Thr Glu Phe Trp Lys Gln
                85                  90                  95

Tyr Val Asp Gly Asp Gln Cys Glu Ser Asn Pro Cys Leu Asn Gly Gly
```

```
            100                 105                 110

Ser Cys Lys Asp Asp Ile Asn Ser Tyr Glu Cys Trp Cys Pro Phe Gly
        115                 120                 125

Phe Glu Gly Lys Asn Cys Glu Leu Asp Val Thr Cys Asn Ile Lys Asn
    130                 135                 140

Gly Arg Cys Glu Gln Phe Cys Lys Asn Ser Ala Asp Asn Lys Val Val
145                 150                 155                 160

Cys Ser Cys Thr Glu Gly Tyr Arg Leu Ala Glu Asn Gln Lys Ser Cys
                165                 170                 175

Glu Pro Ala Val Pro Phe Pro Cys Gly Arg Val Ser Val Ser Gln Thr
            180                 185                 190

Ser Lys Leu Thr Arg Ala Glu Ala Val Phe Pro Asp Val Asp Tyr Val
        195                 200                 205

Asn Ser Thr Glu Ala Glu Thr Ile Leu Asp Asn Ile Thr Gln Ser Thr
    210                 215                 220

Gln Ser Phe Asn Asp Phe Thr Arg Val Val Gly Gly Glu Asp Ala Lys
225                 230                 235                 240

Pro Gly Gln Phe Pro Trp Gln Val Val Leu Asn Gly Lys Val Asp Ala
                245                 250                 255

Phe Cys Gly Gly Ser Ile Val Asn Glu Lys Trp Ile Val Thr Ala Ala
            260                 265                 270

His Cys Val Glu Thr Gly Val Lys Ile Thr Val Val Ala Gly Glu His
        275                 280                 285

Asn Ile Glu Glu Thr Glu His Thr Glu Gln Lys Arg Asn Val Ile Arg
    290                 295                 300

Ile Ile Pro His His Asn Tyr Asn Ala Ala Ile Asn Lys Tyr Asn His
305                 310                 315                 320

Asp Ile Ala Leu Leu Glu Leu Asp Glu Pro Leu Val Leu Asn Ser Tyr
                325                 330                 335

Val Thr Pro Ile Cys Ile Ala Asp Lys Glu Tyr Thr Asn Ile Phe Leu
            340                 345                 350
```

```
Lys Phe Gly Ser Gly Tyr Val Ser Gly Trp Gly Arg Val Phe His Lys
        355                 360                 365

Gly Arg Ser Ala Leu Val Leu Gln Tyr Leu Arg Val Pro Leu Val Asp
    370                 375                 380

Arg Ala Thr Cys Leu Arg Ser Thr Lys Phe Thr Ile Tyr Asn Asn Met
385                 390                 395                 400

Phe Cys Ala Gly Phe His Glu Gly Gly Arg Asp Ser Cys Gln Gly Asp
                405                 410                 415

Ser Gly Gly Pro His Val Thr Glu Val Glu Gly Thr Ser Phe Leu Thr
            420                 425                 430

Gly Ile Ile Ser Trp Gly Glu Glu Cys Ala Met Lys Gly Lys Tyr Gly
        435                 440                 445

Ile Tyr Thr Lys Val Ser Arg Tyr Val Asn Trp Ile Lys Glu Lys Thr
    450                 455                 460

Lys Leu Thr Ser Ser Ser Ser Lys Ala Pro Pro Pro Ser Leu Pro Ser
465                 470                 475                 480

Pro Ser Arg Leu Pro Gly Pro Ser Asp Thr Pro Ile Leu Pro Gln Ser
                485                 490                 495

Ser Ser Ser Lys Ala Pro Pro Pro Ser Leu Pro Ser Pro Ser Arg Leu
            500                 505                 510

Pro Gly Pro Ser Asp Thr Pro Ile Leu Pro Gln Ser Ser Ser Ser Lys
        515                 520                 525

Ala Pro Pro Pro Ser Leu Pro Ser Pro Ser Arg Leu Pro Gly Pro Ser
    530                 535                 540

Asp Thr Pro Ile Leu Pro Gln Ser Ser Ser Ser Lys Ala Pro Pro Pro
545                 550                 555                 560

Ser Leu Pro Ser Pro Ser Arg Leu Pro Gly Pro Ser Asp Thr Pro Ile
                565                 570                 575

Leu Pro Gln Gly Ser Ala Ala
            580
```

<210> 34
<211> 1840

<212> DNA
<213> Artificial Sequence

<220>
<223> CTP-modified Factor IX

<400> 34

```
ctagagtcga ccccgccatg cagcgcgtga acatgatcat ggcagaatca ccaggcctca      60
tcaccatctg ccttttagga tatctactca gtgctgaatg tacagttttt cttgatcatg     120
aaaacgccaa caaaattctg aatcggccaa agaggtataa ttcaggtaaa ttggaagagt     180
ttgttcaagg gaaccttgag agagaatgta tggaagaaaa gtgtagtttt gaagaagcac     240
gagaagtttt tgaaaacact gaaagaacaa ctgaattttg gaagcagtat gttgatggag     300
atcagtgtga gtccaatcca tgtttaaatg gcggcagttg caaggatgac attaattcct     360
atgaatgttg gtgtcccttt ggatttgaag gaaagaactg tgaattagat gtaacatgta     420
acattaagaa tggcagatgc gagcagtttt gtaaaaatag tgctgataac aaggtggttt     480
gctcctgtac tgagggatat cgacttgcag aaaaccagaa gtcctgtgaa ccagcagtgc     540
catttccatg tggaagagtt tctgtttcac aaacttctaa gctcacccgt gctgaggcag     600
tttttcctga tgtggactat gtaaattcta ctgaagctga aaccattttg gataacatca     660
ctcaaagcac ccaatcattt aatgacttca ctcgagttgt tggtggagaa gatgccaaac     720
caggtcaatt cccttggcag gttgttttga atggtaaagt tgatgcattc tgtggaggct     780
ctatcgttaa tgaaaaatgg attgtaactg ctgcccactg tgttgaaact ggtgttaaaa     840
ttacagttgt cgcaggtgaa cataatattg aggagacaga acatacagag caaaagcgaa     900
atgtgattcg aattattcct caccacaact acaatgcagc tattaataag tacaaccatg     960
acattgccct tctggaactg gacgaaccct tagtgctaaa cagctacgtt acacctattt    1020
gcattgctga caaggaatac acgaacatct tcctcaaatt tggatctggc tatgtaagtg    1080
gctggggaag agtcttccac aaagggagat cagctttagt tcttcagtac cttagagttc    1140
cacttgttga ccgagccaca tgtcttcgat ctacaaagtt caccatctat aacaacatgt    1200
tctgtgctgg cttccatgaa ggaggtagag attcatgtca aggagatagt gggggacccc    1260
atgttactga agtggaaggg accagtttct taactggaat tattagctgg ggtgaagagt    1320
gtgcaatgaa aggcaaatat ggaatatata ccaaggtatc ccggtatgtc aactggatta    1380
aggaaaaaac aaagctcact agctccagca gcaaggcccc tcccccgagc ctgccctccc    1440
caagcaggct gcctgggccc tctgacaccc ctatcctgcc tcagtccagc tcctctaagg    1500
ctccaccacc ttccctgcct agcccttcaa gactgccagg ccctagcgat acaccaattc    1560
tgccccagtc ctccagcagc aaggctcccc cacctagcct gccttctcca tcaaggctgc    1620
ctggcccatc cgatacccca attttgcctc agagcagctc tagcaaggca cctcccccca    1680
gtctgccctc tccaagcaga ctccctggcc cttcagacac tccaatcctc ccacagtcct    1740
```

ctagctctaa agctccacct cccagcctgc ccagccctag tagactcccc ggaccttctg 1800

ataccccccat cttgccccag tgatgaggat ccgcggccgc 1840

<210> 35
<211> 610
<212> PRT
<213> Artificial Sequence

<220>
<223> CTP-modified Factor IX

<400> 35

Arg Val Asp Pro Ala Met Gln Arg Val Asn Met Ile Met Ala Glu Ser
1 5 10 15

Pro Gly Leu Ile Thr Ile Cys Leu Leu Gly Tyr Leu Leu Ser Ala Glu
20 25 30

Cys Thr Val Phe Leu Asp His Glu Asn Ala Asn Lys Ile Leu Asn Arg
35 40 45

Pro Lys Arg Tyr Asn Ser Gly Lys Leu Glu Glu Phe Val Gln Gly Asn
50 55 60

Leu Glu Arg Glu Cys Met Glu Glu Lys Cys Ser Phe Glu Glu Ala Arg
65 70 75 80

Glu Val Phe Glu Asn Thr Glu Arg Thr Thr Glu Phe Trp Lys Gln Tyr
85 90 95

Val Asp Gly Asp Gln Cys Glu Ser Asn Pro Cys Leu Asn Gly Gly Ser
100 105 110

Cys Lys Asp Asp Ile Asn Ser Tyr Glu Cys Trp Cys Pro Phe Gly Phe
115 120 125

Glu Gly Lys Asn Cys Glu Leu Asp Val Thr Cys Asn Ile Lys Asn Gly
130 135 140

Arg Cys Glu Gln Phe Cys Lys Asn Ser Ala Asp Asn Lys Val Val Cys
145 150 155 160

Ser Cys Thr Glu Gly Tyr Arg Leu Ala Glu Asn Gln Lys Ser Cys Glu
165 170 175

Pro Ala Val Pro Phe Pro Cys Gly Arg Val Ser Val Ser Gln Thr Ser
180 185 190

```
Lys Leu Thr Arg Ala Glu Ala Val Phe Pro Asp Val Asp Tyr Val Asn
        195                 200                 205

Ser Thr Glu Ala Glu Thr Ile Leu Asp Asn Ile Thr Gln Ser Thr Gln
    210                 215                 220

Ser Phe Asn Asp Phe Thr Arg Val Val Gly Gly Glu Asp Ala Lys Pro
225                 230                 235                 240

Gly Gln Phe Pro Trp Gln Val Val Leu Asn Gly Lys Val Asp Ala Phe
                245                 250                 255

Cys Gly Gly Ser Ile Val Asn Glu Lys Trp Ile Val Thr Ala Ala His
            260                 265                 270

Cys Val Glu Thr Gly Val Lys Ile Thr Val Val Ala Gly Glu His Asn
        275                 280                 285

Ile Glu Glu Thr Glu His Thr Glu Gln Lys Arg Asn Val Ile Arg Ile
    290                 295                 300

Ile Pro His His Asn Tyr Asn Ala Ala Ile Asn Lys Tyr Asn His Asp
305                 310                 315                 320

Ile Ala Leu Leu Glu Leu Asp Glu Pro Leu Val Leu Asn Ser Tyr Val
                325                 330                 335

Thr Pro Ile Cys Ile Ala Asp Lys Glu Tyr Thr Asn Ile Phe Leu Lys
            340                 345                 350

Phe Gly Ser Gly Tyr Val Ser Gly Trp Gly Arg Val Phe His Lys Gly
        355                 360                 365

Arg Ser Ala Leu Val Leu Gln Tyr Leu Arg Val Pro Leu Val Asp Arg
    370                 375                 380

Ala Thr Cys Leu Arg Ser Thr Lys Phe Thr Ile Tyr Asn Asn Met Phe
385                 390                 395                 400

Cys Ala Gly Phe His Glu Gly Gly Arg Asp Ser Cys Gln Gly Asp Ser
                405                 410                 415

Gly Gly Pro His Val Thr Glu Val Glu Gly Thr Ser Phe Leu Thr Gly
            420                 425                 430

Ile Ile Ser Trp Gly Glu Glu Cys Ala Met Lys Gly Lys Tyr Gly Ile
        435                 440                 445
```

Tyr Thr Lys Val Ser Arg Tyr Val Asn Trp Ile Lys Glu Lys Thr Lys
450 455 460

Leu Thr Ser Ser Ser Ser Lys Ala Pro Pro Pro Ser Leu Pro Ser Pro
465 470 475 480

Ser Arg Leu Pro Gly Pro Ser Asp Thr Pro Ile Leu Pro Gln Ser Ser
485 490 495

Ser Ser Lys Ala Pro Pro Pro Ser Leu Pro Ser Pro Ser Arg Leu Pro
500 505 510

Gly Pro Ser Asp Thr Pro Ile Leu Pro Gln Ser Ser Ser Ser Lys Ala
515 520 525

Pro Pro Pro Ser Leu Pro Ser Pro Ser Arg Leu Pro Gly Pro Ser Asp
530 535 540

Thr Pro Ile Leu Pro Gln Ser Ser Ser Ser Lys Ala Pro Pro Pro Ser
545 550 555 560

Leu Pro Ser Pro Ser Arg Leu Pro Gly Pro Ser Asp Thr Pro Ile Leu
565 570 575

Pro Gln Ser Ser Ser Ser Lys Ala Pro Pro Pro Ser Leu Pro Ser Pro
580 585 590

Ser Arg Leu Pro Gly Pro Ser Asp Thr Pro Ile Leu Pro Gln Gly Ser
595 600 605

Ala Ala
610

<210> 36
<211> 20
<212> DNA
<213> Artificial Sequence

<220>
<223> Primer 101 for FIX-(CTP)2

<400> 36
gtttagtgaa ccgtcagaat 20

<210> 37
<211> 20
<212> DNA
<213> Artificial Sequence

<220>

<223> Primer 103-R for FIX-(CTP)2

<400> 37
ttgaggaaga tgttcgtgta 20

<210> 38
<211> 20
<212> DNA
<213> Artificial Sequence

<220>
<223> Primer 98 for FIX-(CTP)2

<400> 38
attacagttg tcgcaggtga 20

<210> 39
<211> 30
<212> DNA
<213> Artificial Sequence

<220>
<223> Primer 99-Rfor FIX-(CTP)2

<400> 39
gctggagcta gtgagctttg ttttttcctt 30

<210> 40
<211> 25
<212> DNA
<213> Artificial Sequence

<220>
<223> Primer 100 for FIX-(CTP)2

<400> 40
gctcactagc tccagcagca aggcc 25

<210> 41
<211> 23
<212> DNA
<213> Artificial Sequence

<220>
<223> Primer 27-R for FIX-(CTP)2

<400> 41
ttttcactgc attctagttg tgg 23

<210> 42
<211> 20
<212> DNA
<213> Artificial Sequence

<220>
<223> Primer 75

<400> 42

ctcccagttc aattacagct 20

<210> 43
<211> 27
<212> DNA
<213> Artificial Sequence

<220>
<223> Primer 122r

<400> 43
ggaaaaactg cctcagcacg ggtgagc 27

<210> 44
<211> 32
<212> DNA
<213> Artificial Sequence

<220>
<223> Primer 123

<400> 44
gtgctgaggc agtttttcct gatgtggact at 32

<210> 45
<211> 18
<212> DNA
<213> Artificial Sequence

<220>
<223> Primer 124r

<400> 45
caacacagtg ggcagcag 18